# EUROPEAN PATENT APPLICATION

(11) **EP 0 160 876 A1**
(43) Date of publication of application: **13.11.1985**
(21) Application number: 85104843.9
(22) Date of filing: 22.04.1985
(51) Int. Cl.: C07D 487/04, A61K 31/40, C07F 7/18, C07D 205/08, C07D 211/46, C07D 211/54, C07D 279/12, C07D 207/12

(54) **1-Hetero-6-/1-Hydroxyethyl/-2-SR8-1-carbadethiapen-2-EM-3-carboxylic acids**

(30) Priority: 23.04.1984 US 603005
(71) Applicant: Merck & Co., Inc., Rahway New Jersey 07065-0900 (US)
(72) Inventor: Cama, Lovji D., Cresskill New Jersey 07626 (US); Christensen, Burton G., Cliffside New Jersey 07010 (US); Shih, David H., Manalapan New Jersey 07726 (US)
(74) Representative: Abitz, Walter, Dr.-Ing.

(57) **Abstract**

Disclosed are 1-hetero-6-[1-hydroxyethyl)-2-SR⁸-1-carbadethiapen-2-em-3-COOR⁴ compounds having the structure: 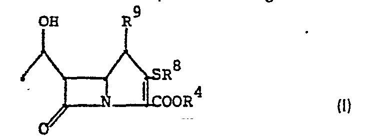 wherein R⁴ is, e.g., hydrogen, R⁸ is, e.g., alkyl and R⁹ is, e.g., halogen; the pharmaceutically acceptable salt and ester derivatives thereof and use as antibiotics. Also disclosed are processes for the preparation of such compounds and pharmaceutical compositions comprising such compounds.

## Description

### BACKGROUND OF THE INVENTION

This invention relates to 1-hetero-6-[1-hydroxyethyl]-2-SR⁸-1-carbadethiapen-2-em-3-carboxylic acids (I) and the pharmaceutically acceptable salt, ester and amide derivatives thereof which are useful as antibiotics: 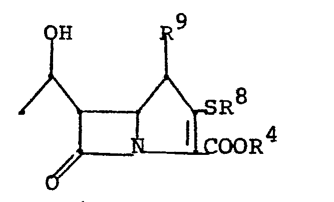 wherein: R is H; a pharmaceutically acceptable cation, e.g. of an alkali metal, an alkaline earth metal, silver, aluminum and the like, or NH⁺₄, quaternary ammonium or a pharmaceutically acceptable ester moiety such as C₁₋₁₂ linear, branched or cyclic alkyl; or a group having the formula: 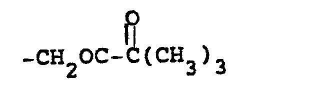 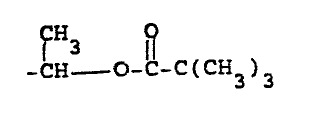 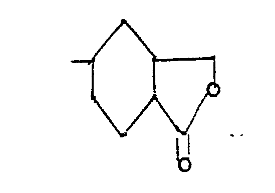 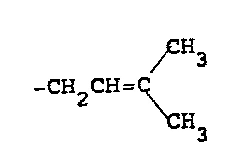 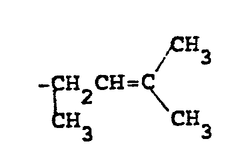 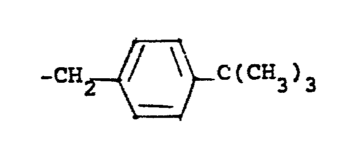 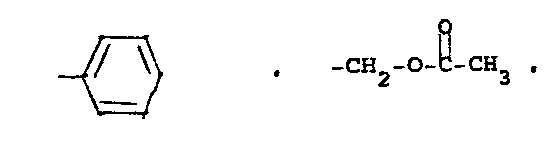 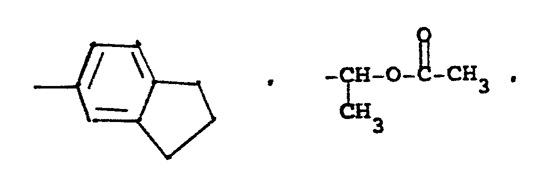 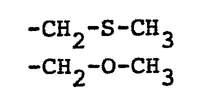 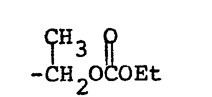 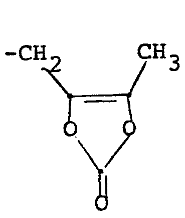 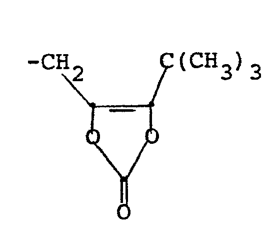 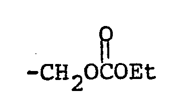 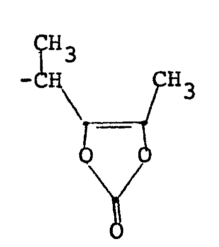 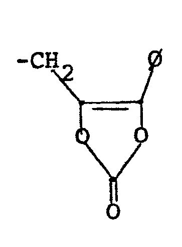 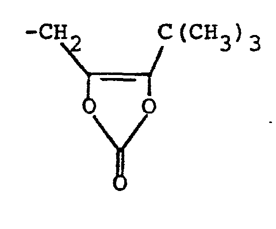 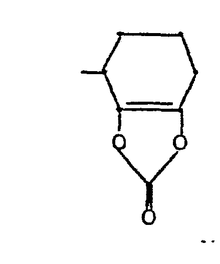 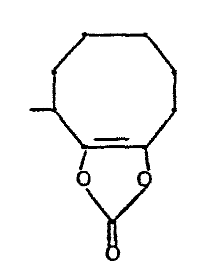 or 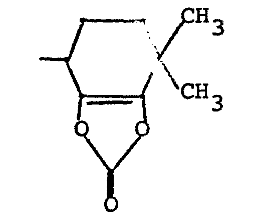 _{R}⁹ is halogen, OR, OSO₂R, 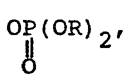 SH, SR, 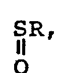 SO₂R, NH₂, NHR, N(R₂), N⊕(R₃), 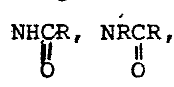 wherein R is H, C₁-C₆ alkyl, C₂-C₆ alkenyl, C₂-C₆ alkynyl; phenyl; phenylalkyl having 7-12 carbon atoms; cycloalkyl having 3-6 carbon atoms; cycloalkylalkyl having 3-6 carbon atoms in the ring and 1-6 carbon atoms in the alkyl moiety; heterocyclic groups including heterocyclyl, heterocyclyalkyl, heteroaryl, and heteroarylalkyl; wherein, relative to said heterocyclic group rings, the rings comprise 3-6 atoms, 1-4 of which are selected from O, N, or S, and the alkyl moiety comprises 1-6 carbon atoms; representative examples of such heterocyclic values for R include:

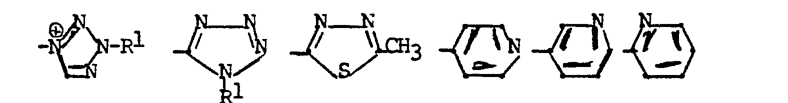

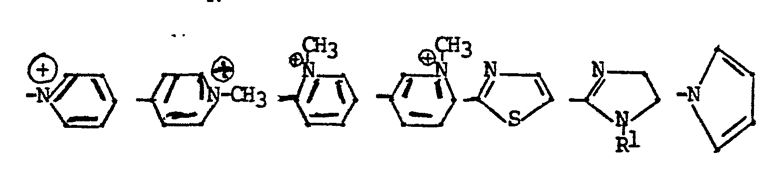

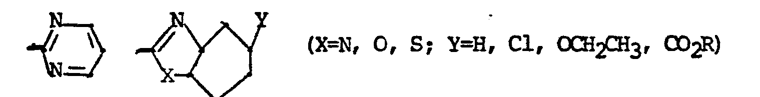

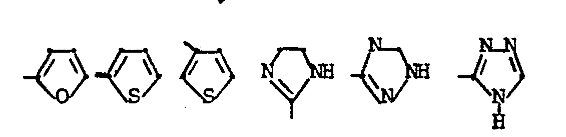

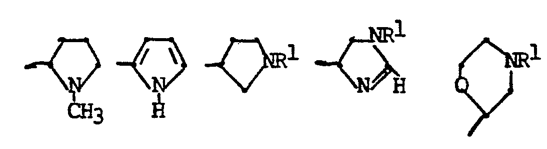

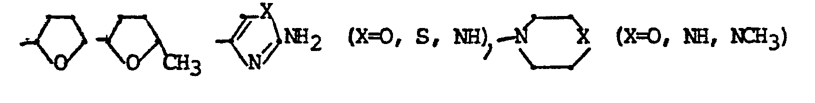

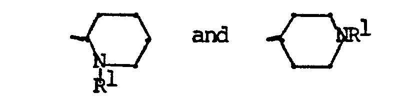

The foregoing acyclic and ring structure values for R may be substituted by one or more members selected from: Cl, Br, F, O_{H}, _{OR}¹, 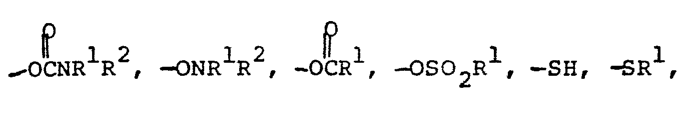 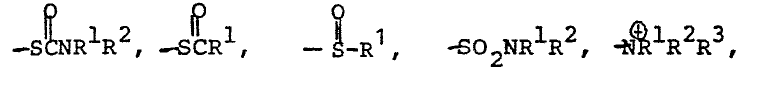 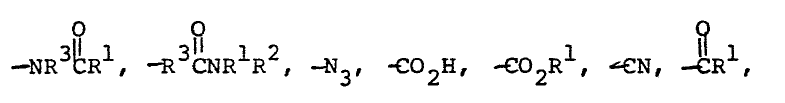 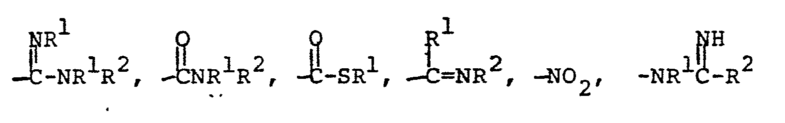 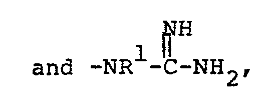 wherein relative to the above-listed substituents on _{R}, the _{R}¹, R and R³ are independently selected from: hydrogen, C₁₋₁₀ alkyl, C₂₋₁₀ alkenyl, and C₂₋₁₀ alkynyl, having cycloalky, cycloalkylalkyl, and alkylcycloalkyl, having 3-6 carbon atoms in the cycloalkyl ring and 1-6 carbon atoms in the alkyl moieties; aryl, such as phenyl; aralkyl, aralkenyl, and aralkynyl wherein the aryl moiety is phenyl and the aliphatic portion has 1-6 carbon atoms; heterocyclic groups including heteroalkyl, heteroaralkyl, heterocyclyl and heterocyclylalkyl and wherein the hetero atom or atoms in the above-named heterocyclic moieties are selected from the group consisting of 1-4 oxygen, nitrogen or sulphur atoms and wherein the alkyl moieties associated with said heterocyclic moieties have 1-6 carbon atoms.

Certain of the previously recited values for _{R}⁹ are additionally useful as leaving groups in the synthesis of I bearing 1-substituent _{R}⁹, generically defined above; such R⁹ leaving groups include: OSO₂Ø. 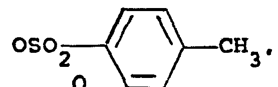 OSO₂Me, OSO₂CF₃, 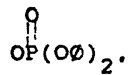 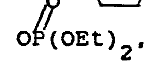 Cl, I, Br, OCCH₃ (Ø=phenyl; Me=CH₃, Et=ethyl). O

Prepared _{R}⁹ groups are halogen especially chloro and fluoro.

R⁸ is independently selected from the group consisting of hydrogen, substituted and unsubstituted: alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; aryl, such as phenyl; aralkyl, aralkenyl, and aralkynyl wherein the aryl moiety is phenyl and the aliphatic portion has 1-6 carbon atoms; heterocyclic groups as hereinabove defined including heteroaryl, heteroaralkyl, heterocyclyl and heterocyclylalkyl, wherein the heteroatom or atoms are selected from O, S, N such as 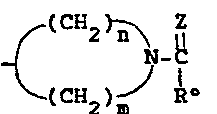 wherein n is 1-4, preferably 1-2 and m is 1-4, preferably 1-2: Z is NH, NR¹, O or S, preferably NR¹ or NH; and R° is substituted or unsubstituted C₁₋₆ alkyl, C₆₋₁₀ non-heteroaryl, _{C3-6} cycloalkyl, C₂₋₆ alkenyl or C₂₋₆ alkynyl: wherein the substitutent or substituents relative to the above-named radical values for R⁸ are selected from the group consisting of: halo (chloro, bromo, fluoro) -OH hydroxy 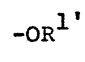 alkoxy, aryloxy 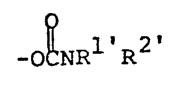 carbamoyloxy 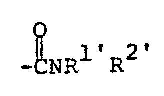 carbamoyl 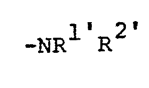 amino 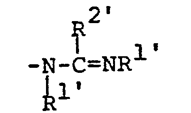 amidino 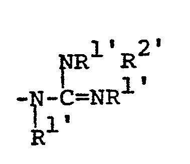 guanidino 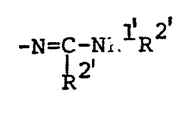 amidino 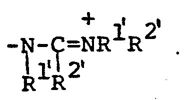 amidinium 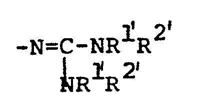 guanidino and 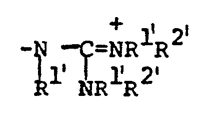 guanidinium 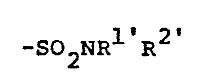 sulfamoyl 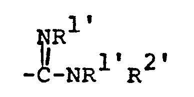 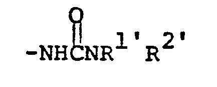 ureido 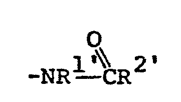 amido 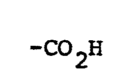 carboxy 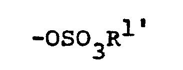 sulphate 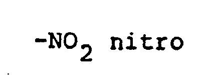 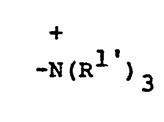 ammonium (R^{1'} groups independently chosen) 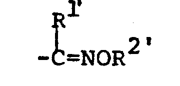 oximino 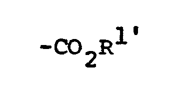 carboxylate 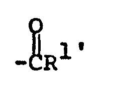 acyl 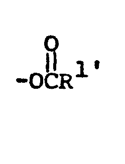 acyloxy 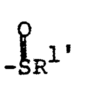 alkyl and aryl sulfinyl 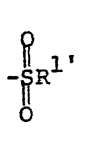 alkyl and aryl sulfonyl 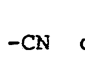 :yano 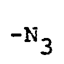 azido 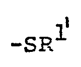 alkyl- and arylthio 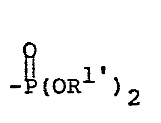 phosphono 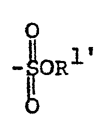 sulfo 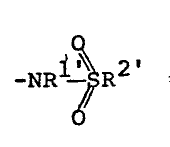 sulfonamido wherein, the groups R^{1'} and R^{2'} are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; aryl, such as phenyl; aralkyl, aralkenyl, and aralkynyl wherein the aryl moiety is phenyl and the aliphatic portion has 1-6 carbon atoms; heterocyclic groups as defined above, including heteroalkyl, heteroaralkyl, heterocyclyl and heterocyclylalkyl and wherein the hetero atom or atoms in the above-named heterocyclic moieties are selected from the group consisting of 1-4 oxygen, nitrogen or sulphur atoms and wherein the alkyl moieties associated with said heterocyclic moieties have 1-6 carbon atoms.

Relative to Structure I, above and the generic definition of its 2-substituents R⁸, specific recitation of preferred values for R⁸ is given below. However, for purposes of definition, European Patent Application 80102076.9 (Publication Number 0017992) is incorporated herein by reference to the extent that it defines R⁸ relative to the 1-carbadethiapenem nucleus Ia: 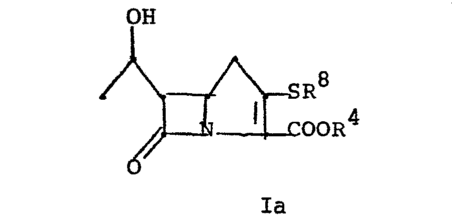 1-carbadethiapenem The incorporated European Patent Application is directed to the substituted 2-SR⁸⁻1-carbadethiapen-2-em-3-carboxylic acids of Structure Ia, and its 6-(1-hydroxyethyl), 2-(-SR⁸), and 3-(_{COOR}⁴) substituent definitions are included and incorporated to the 1-substituted carbapenem Structure I of the present application.
_{R}⁸ is additionally defined to be "carbamimidoyl", which is defined by the following structures: 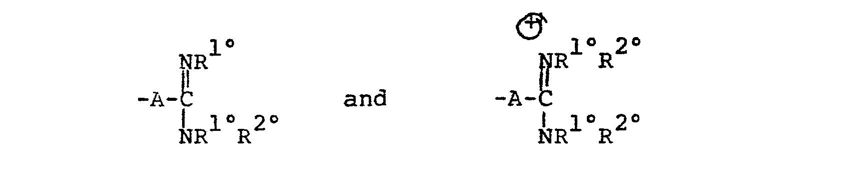 wherein A, the cyclic or acylic connecting group, and _{R}¹⁰ and R^{2°} are defined below. The definition of _{R}⁸ also embraces cyclic structures, which may be generically represented, for example, thusly: 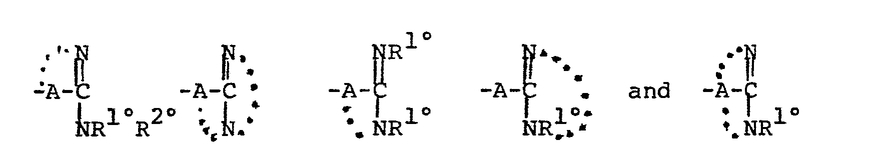 wherein the dotted lines indicate that the nitrogen atoms of the so-called carbamimidoyl function may participate in the formation of the cyclic structures indicated above. Representative specific embodiments for R⁸ fallow, but, in the generic sense, the components: R^{1°}, R^{2°} and A are defined, thusly:
A, the cyclic or acyclic connector, is selected from the group consisting of alkyl, alkenyl, and alkynyl having 1-10 carbon atoms, preferably 1-6 carbon atoms, which may be interrupted by a hetero atom selected from O, S or N, or by a ring such as phc yl, cycloalkyl, cycloalkenyl, heterocyclyl or heteroaryl wherein such cyclic interrruptions comprise 3-6 ring atoms selected from C, O, S and N; cycloalkyl, cycloalkenyl having 3-6 carbon atoms; heterocyclyl; heteroaryl; and phenyl; A also represents a direct, single bond connecting the indicated S and C atoms.
R^{1°} and _{R}²⁰ are independently selected from hydrogen and the previously defined values for the group A, such as: alkyl, aryl, cycloalkyl, heteroarlkyl, alkylaryl, alkylarylalkyl, and heterocyclyl and heteroaryl.
R⁴ is hydrogen, a removable protecting group, a synthetically useful salt moiety, or a pharmaceutically acceptable salt or ester moiety. _{R}⁴ is additionally defined below.

It should be noted that the final products of this invention (I) can exist in either neutral or zwitterionic (internal salt) forms. In the zwitterionic form, the basic function is protonated and positively charged and the carboxyl group is deprotonated and negatively charged. The zwitterionic form is the predominant species under most conditions and is in equilibrium with a minor amount of the uncharged, neutral species. The equilibrium process is conveniently visualized as an internal acid-base neutralization. The neutral and zwitterionic forms are shown below. 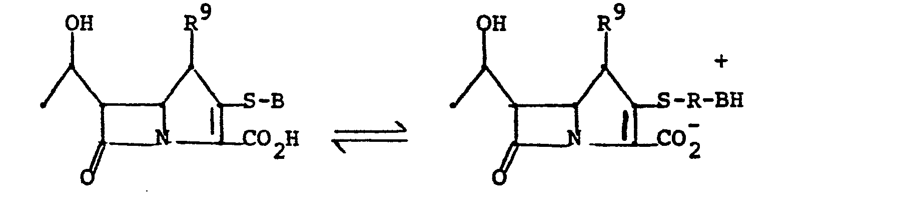 wherein B is the carbamimidoyl group.

Further, the final products of this invention I wherein R⁸ contains a positively charged quaternary nitrogen function such as the "carbamimidinium" can exist as zwitterionic (internal salt) forms or as external salt forms. The preferred form of this product group is the zwitterionic or internal salt form. These forms are shown below: 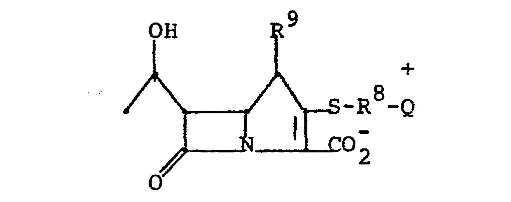 Zwitterionic (internal salt) Form 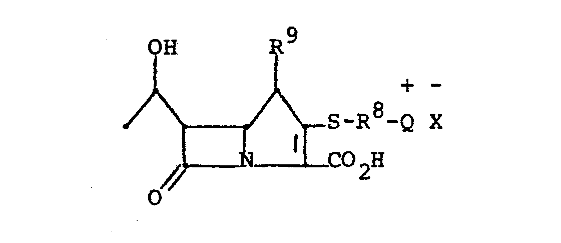 Extern 1 Salt Form wherein Q represents the quaternized nitrogen group, and wherein X is a pharmaceutically acceptable anion such as those listed in U.S. Patent 4,194,047, issued 3/18/80, which is incorporated herein by reference.

This invention also relates to the carboxyl derivatives of I which are antibiotics and which may be represented by the following generic structure (I): 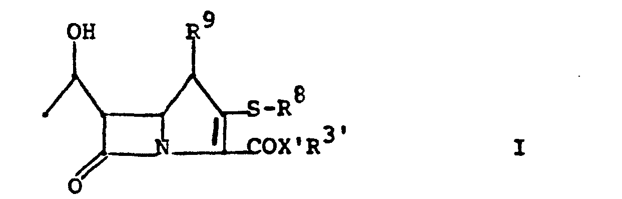 wherein X' is oxygen, sulphur or NR' (R' = H or lower alkyl having 1-6 carbon atoms); and R^{3'} is, hydrogen, or inter alia is representatively selected to provide the pharmaceutically acceptable salt, ester, anhydride (R^{3'} is acyl), and amide moieties known in bicyclic β-lactam antibiotic art; R^{3'} may also be readily removable blocking group. The definition of R^{3'} is given in greater detail below.

Preferred R⁸ groups are selected from C^{H}3 CH₂^{CH}₃ CH(CH₃)₂ 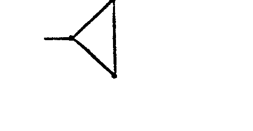 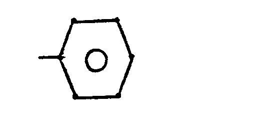 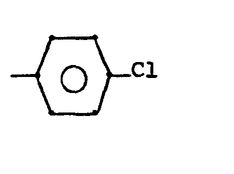 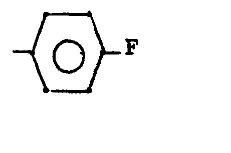 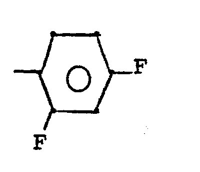 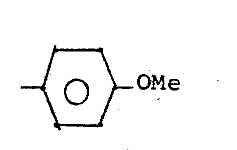 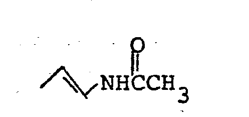 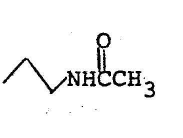 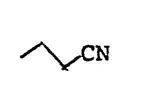 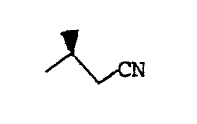 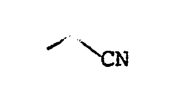 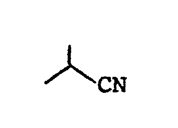 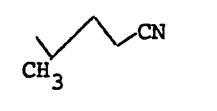 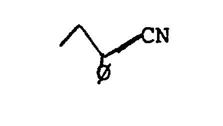 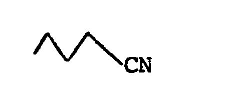 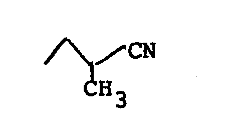 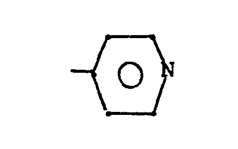 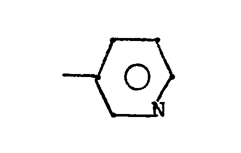 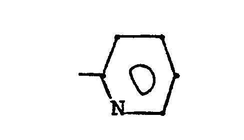 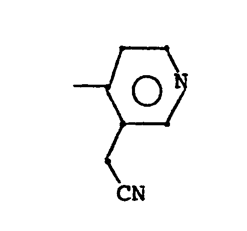 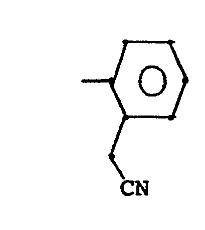 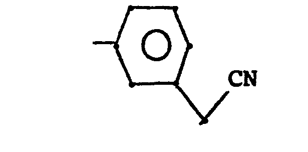 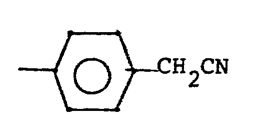 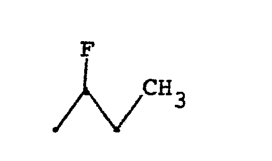 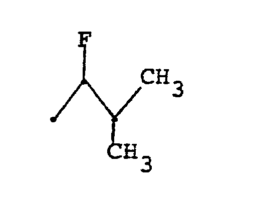 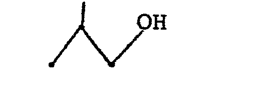 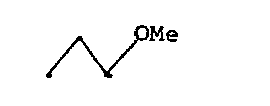 Me ^{= CH}₃ 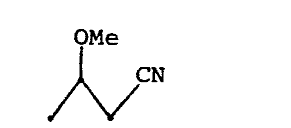 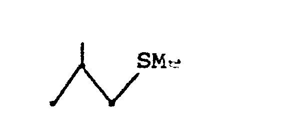 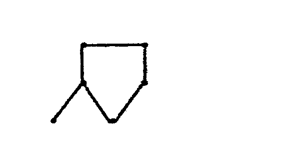 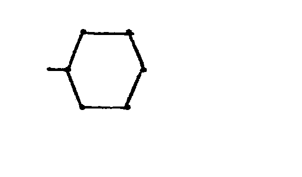 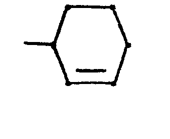 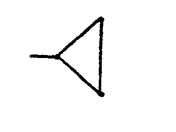 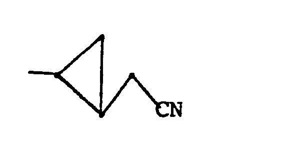 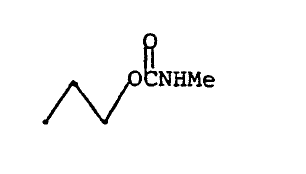 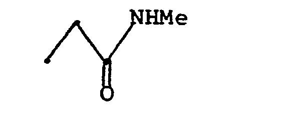 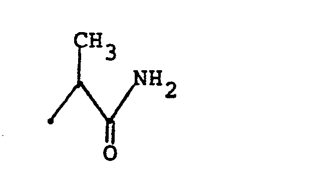 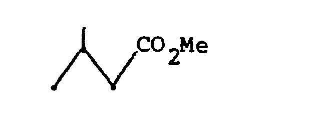 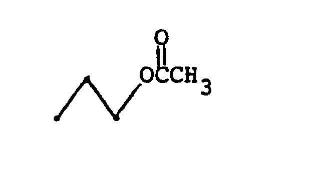 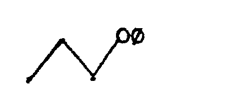 Ø = phenyl 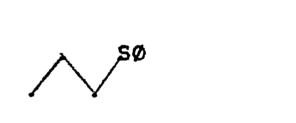 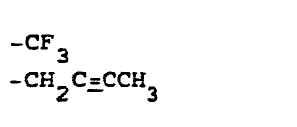 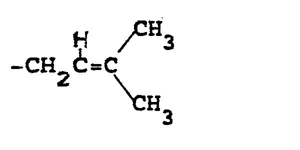 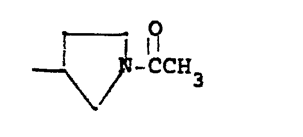 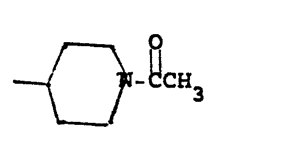 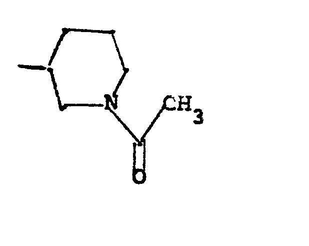 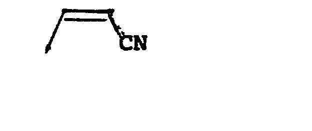 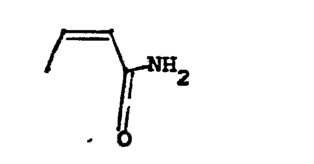 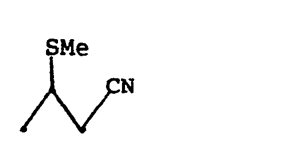 and 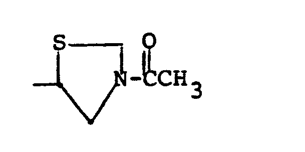 Most preferred R⁸ groups are: 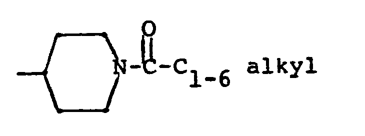 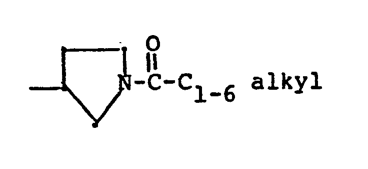 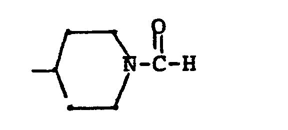 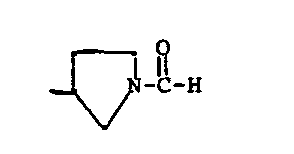 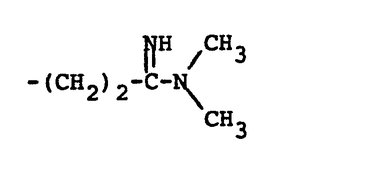 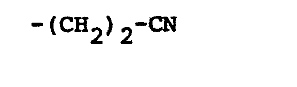 -CH₂(CH₃)-CH₂-CN and

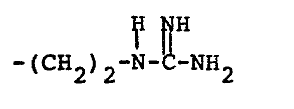

This invention also relates to processes for the preparation of such compounds I; pharmaceutical compositions comprising such compounds; and to methods of treatment comprising administering such compounds and compositions when an antibiotic effect is indicated.

There is a continuing need for new antibiotics. For example, effectiveness of a given antibiotic may diminish because continued wide scale usage selectively may give rise to resistant strains of pathogens. In addition, many known antibiotics suffer from the disadvantage of being effective only against certain types of microorganisms. Accordingly, the search for new antibiotics continues.

Thus, it is an object of the present invention to provide a novel class of antibiotics which are useful in animal and human therapy and in inanimate systems. These antibiotics are active against a broad range of pathogens which representatively include both Gram positive bacteria such as S. aureau, Strep. pyogenes, and B. subtilis, and Gram negative bacteria such as E. coli, Pseudomonas, Proteus morganii, Serratia, and Klebsiella. Further objects of this invention are to provide chemical processes for the preparation of such antibiotics and their nontoxic, pharmaceutically acceptable salts; pharmaceutical compositions comprising such antibiotics; and to provide methods of treatment comprising administering such antibiotics and compositions when an antibiotic effect is indicated.

### DETAILED DESCRIPTION OF THE INVENTION

The compounds of the present invention (I, above) are conveniently prepared by the following scheme: DIAGRAM I

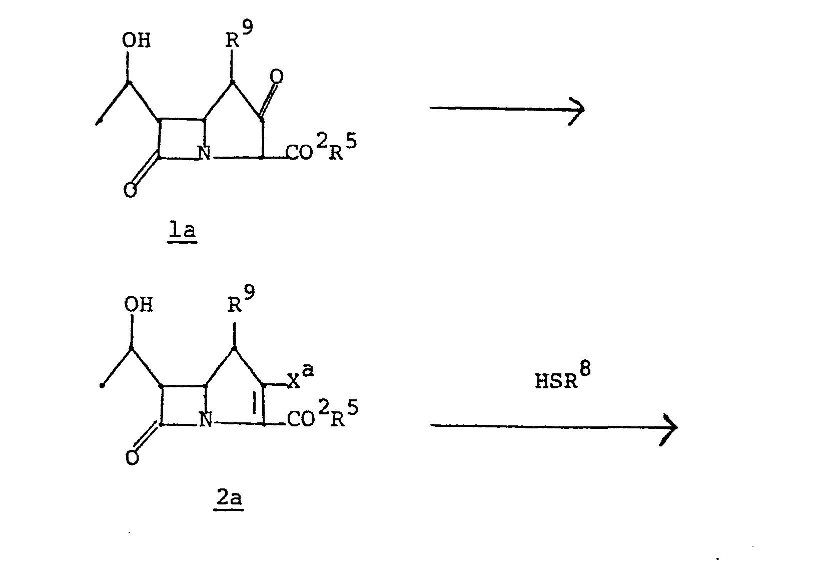

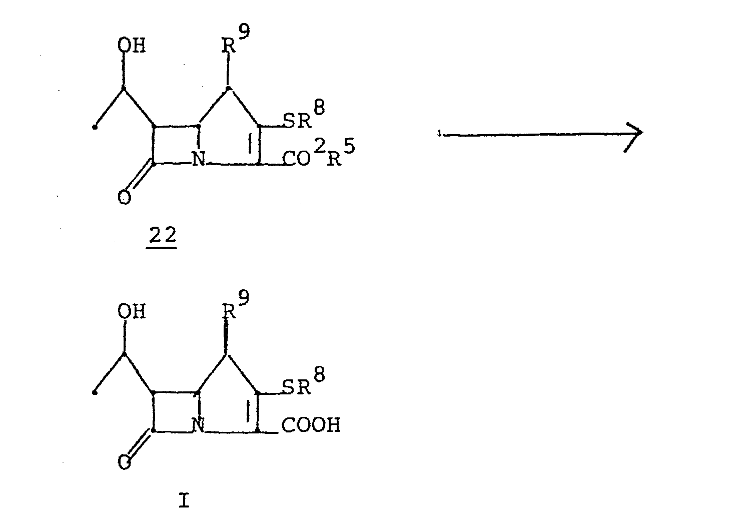

In words relative to the above reaction scheme, Diagram I, the step la to 2a to establish leaving group X is accomplished by acylating the bicyclic keto ester la with an acylating agent RX^{a} such as p-toluenesulfonic acid anhydride, p-nitro- phenylsulfonic acid anhydride, 2,4,6-triisopropyl- phenylsulfonic acid anhydride, methanesulfonic acid anhydride, trifluoromethane sulfonic acid anhydride, diphenyl chlorophosphate, toluenesulfonyl chloride, p-bronophenylsulfonyl chloride, or the like; wherein X^{a} is the corresponding leaving group such as toluene sulfonyloxy, p-nitrophenylsulfonyloxy, benzenesulfonyloxy, diphenylphosphoryl, and other leaving groups which are established by conventional procedures and are well known in the art. Typically, the above acylation to establish leaving group _{X}^{a} is conducted in a solvent such as methylene chloride, acetonitrile or dimethylformamide, in the presence of a base such as diisopropylethylamine, triethylamine, 4-dimethylaminopyridine, or the like, at a temperature of from -20 to 40° for from 0.1 to 5 hours. The leaving group X^{a} of intermediate 2a can also be halogen. The halogen leaving group is established by treating la with a halogenating agent such as φ₃PCl₂, φ₃PBr₂, (φO)₃PBr₂, oxalyl chloride or the like in a solvent such as CH₂Cl₂, CH₃CN, THF, or the like in the presence of a base such as diisopropylethylamine, triethylamine, or 4-dimethylaminopyridine or the like. [φ = phenyl.]

The reaction 2a to 22 is accomplished by treating 2a in a solvent such as dioxane, dimethylformamide, dimethylsulfoxide, acetonitrile, hexamethylphosphoramide, or the like, in the presence of an approximately equivalent to excess of the mercaptan reagent HSR⁸, wherein R is defined above, in the presence of a base such as sodium hydrogen carbonate, potassium carbonate, triethylamine, diisopropylethylamine, or the like at a temperature of from -40 to 25°C for from 30 sec. to 1 hour.

The final deblocking step 22 to I is accomplished by conventional procedures such as solvolysis or hydrogenation. The conditions of deblocking 22 to I are thus: typically 22 in a solvent such as tetrahydrofuran-water, tetrahydrofuran-ethanol-water, dioxane-water, dioxane-ethanol-water, n-butanol-water, or the like containing pH 7 morpholinopropanesulfonic acid-sodium hydroxide ,uffer, pH 7 phosphate buffer, dipotassium hydrogen .osphate, sodium bicarbonate, or the like, is treater under a hydrogen pressure of from 1 to 4 atmospheres in the presence of a catalyst such as platinum oxide, palladium on charcoal, or palladium hydroxide on charcoal, or the like, at a temperature of from 0 to 50°C for from 0.25 to 4 hours to provide _{I}. Photolysis, when R⁵ is a group such as o-nitrobenzyl, for example, may also be used for deblocking.

Relative to Diagram I, the bicyclic keto ester la may be obtained by the following scheme, Diagram II.

### DIAGRAM II

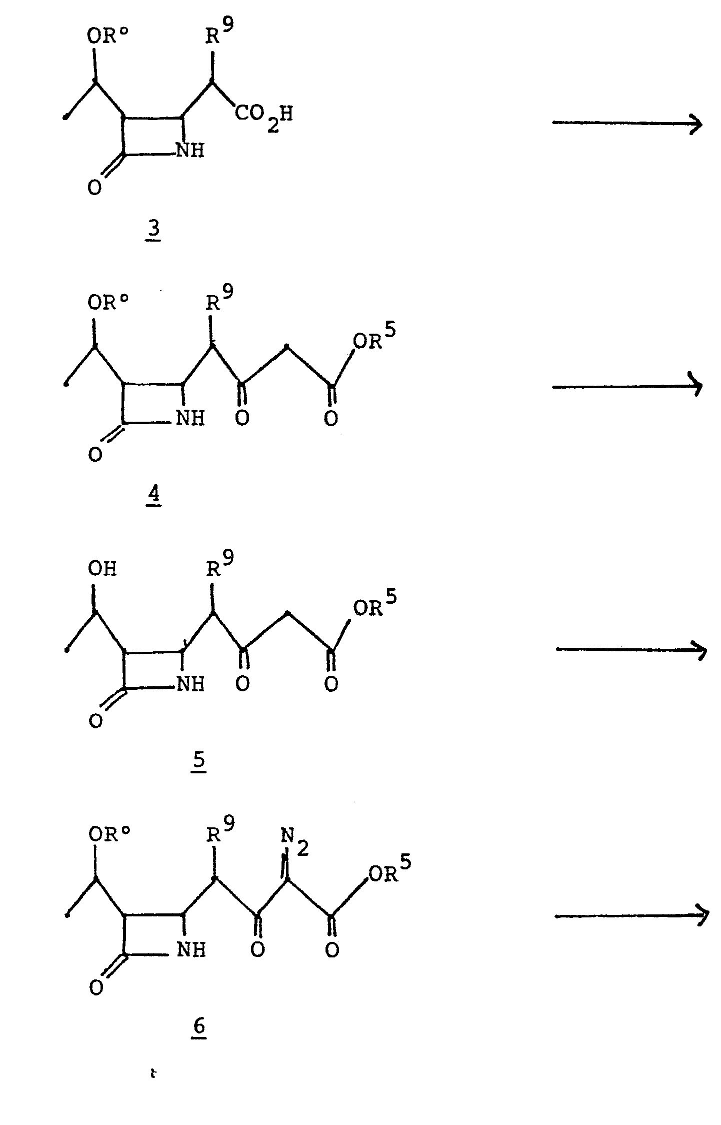

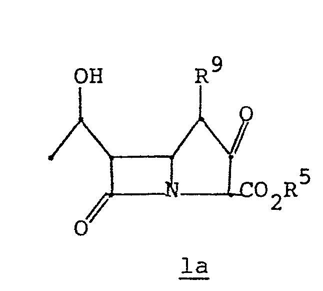

The conversion 3 to 4 is accomplished by treating 3 with l,l'-carbonyldiimidazole, or the like, in a solvent such as tetrahydrofuran (THF), dimethoxyethane, acetonitrile, or the like, at a temperature of from 0 to 70°C, followed by the addition of 1.1 to 3.0 equivalent of (R⁵O₂CCH₂CO₂)₂Mg, at a temperature of from 0 to 70°C for from 1 to 48 hours. (R°=triorganosilyl or p-nitrobenzoxyl carbonyl). The group R⁵ is a pharmaceutically acceptable ester moiety or a readily removable carboxyl protecting group such as p-nitrobenzyl, benzyl, or the like.

Removal of protecting group wherein R° is triorganosilyl such as t-butyldimethylsilyl is accomplished by acidic aqueous hydrolysis of 4 in a solvent such as methanol, ethanol, tetrahydrofuran, dioxane, or the like, in the presence of an acid such as hydrochloric, sulfuric, acetic or the like at a temperature of from 0 to 100°C for from 0.5 to 18 hours. [The term "triorganosilyl" preferable embraces those wherein the organo moiety is independently chosen from alkyl having 1-6 carbon atoms, phenyl, and phenylalkyl.]

The diazo species 6 is prepared from 5 by treating 5 in a solvent such as CH₃CN, CH₂Cl₂, THF, or the like with an azide such as p-carboxy- benzenesulfonylazide, toluenesulfonylazide, methane- sulfonylazide, or the like in the presence of a base such as triethylamine, pyridine, diethylamine or the like for from 1 to 50 hours at 0-50°C.

Cyclization (6 to la) is accomplished by treating 6 in a solvent such as benzene, toluene, THF, cyclohexane, ethylacetate or the like at a temperature of from 25 to 110°C for from 1-5 hours in the presence of a catalyst such as bis (acetyl- acetonato)Cu(II)[Cu(acac)₂], CuSO₄, Cu powder, Rh₂(OAc)₄ or Pd(OAc)₂. Alternatively, the cyclization may be accomplished by irradiating 6 through a pyrex filter (a wave length greater than 300nm) in a solvent such as benzene, CC1₄, diethylether, or the like, at a temperature of from 0-25°C for from 0.5 to 2 hours.

Relative to Diagram II, the following scheme, Diagram III, furnishes starting material 3.

### DIAGRAM III

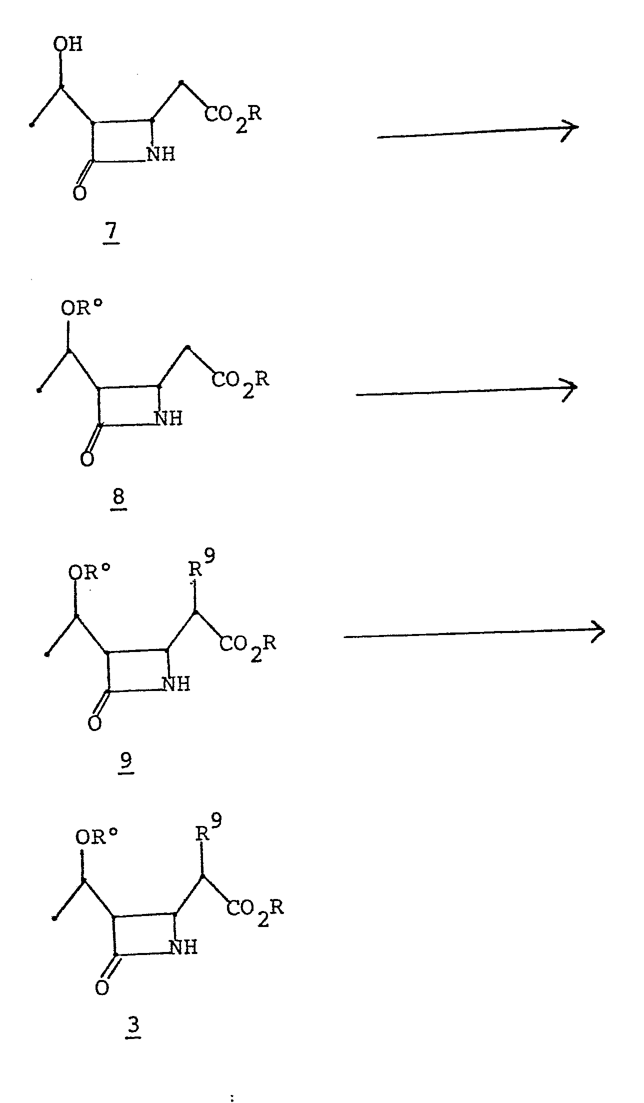

In words relative to Diagram III, the free hydroxy function of the azetidinone carboxylate 7 (R is a conventional protecting group, such as, alkyl or aralkyl, for example, methyl, ethyl, benzyl, 2,2,2-trichloroethyl, methoxymethyl, p-methoxybenzyl, or the like) is selectively protected by treating 7 with 1-2 eq of a triorganosilylating agent such as t-butyldimethylchlorosilane in a solvent such as DMF, CH₂C1₂, or the like, in the presence of 2-5 eq. of imidazole at room temperature for 1 to 8 hrs. to give 8 (R=CH₃, R°=t-butyldimethylsilyl, for example). The preparation of chiral precursor 7 (above) is known; see: EP Patent Application No. 80102338.3 (filed April 30, 1980), publication No. 0038869, publication date November 4, 1981; European Patent Application No. 81102270.6 "Process for The Preparation of 1-Carbapenems; and Intermediates via silyl-substituted Dithioacetals"; and European Patent Publication No. 37081 "Process for The Preparation of 1-Cabapenems, and Intermediates via Trithioorthoacetates"; which are incorporated herein by reference. Treatment of 8 at -78°C under a nitrogen atmosphere with 2-2.5 eq. of a base such as lithium diisopropylamide, or the like , and 2.0 to 2.5 equivalents of hexamethylphosphoric triamide (HMPTA) in a solvent such as THF, ether, or the like, for from 10 min. to 30 min. yields a dianion intermediate, from which the halo- or oxygenated intermediates may be obtained directly. For the preparation of halo azetidinone 9 (wherein R⁹ is halogen), the dianion so obtained is then treated with 1 to 100 eq. of a halogenating reagent such as N-bromo succinimide, N-chloro succinimide, N-iodo succinimide, N-bromo acetamide, N-chloro acetamide, N-iodo acetamide, or an aryl sulfonyl halide, such as, p-toluene sulfonyl chloride or bromide at -78° to -40° from 10 minutes to 1 hour. The reaction is then quenched with saturated ammonium chloride solution to give species 9. Hydrolysis of 9 in the presence of 1.0 eq of NaOH in methanol, followed by acidic work up gives intermediate 3 (wherein R⁹=ₕₐₗₒ).

For the preparation of hydroxy substituted azetidinone 9 (wherein R⁹=OH), the dianion obtained as described above may be treated with molecular oxygen in the presence of 1.0 eg of trimethylphosphite at -78° to -40° from 10 minutes to 1 hour, followed by the work up described above to give 9 (_{R}⁹ =_{'OH}). Activiating, for subsequent derivatization, of such hydroxyl species may be achieved by conventional means; for example, treatment of 9 (R⁹ = OH) with 1.1 eg of methanesulfonyl chloride or p-toluene sulfonyl O chloride or (C₆ H₅-O)₂ PC1 converts the hydroxy group to the corresponding methane sulfonate p-tolue.esulfonate or diphenoxy phosphate leaving group.

Displacement of the halogen or oxygen leaving group (R^{9 =} halogen, or OSO₂CH₃, for example) may be carried out by treatment of 9, (Diagram III) or any of the intermediates of Diagram II with another leaving group or the finally desired heterosubstituent R⁹. This displacement can be achieved by treatment of the mentioned intermediates in solvents such as DMSO, DMF, THF, HMPA, DME with 1 to 100 equivalents of a suitable nucleophile R⁹⁻ in the presence of 1.0 to 100 equivalents of a suitable base such as Et₃N, diisopropylethylamine, NaH, KOtBu, or the like, at 0° to 100° from 1 to 24 hours. If desired the R⁹ substituent so introduced can be protected by methods known in the art for processing through the remaining steps. Preferred protecting groups for exposed oxygen and nitrogen groups are THP ethers, triorganosily, and para- and ortho-nitrobenzyloxycarbonyl, and the like.

The following section representatively demonstrates the conversion of intermediate R⁹ bearing species to other intermediate and final species bearing R⁹ substituents of pharmacological (antibacterial) interest, which substituents are most conveniently established via the derivatization schemes herein disclosed.

### Chemical Derivatization of I-Heteroatom Substituted Carbapenem I:

### (A) Substitution Reactions at C-1 position:

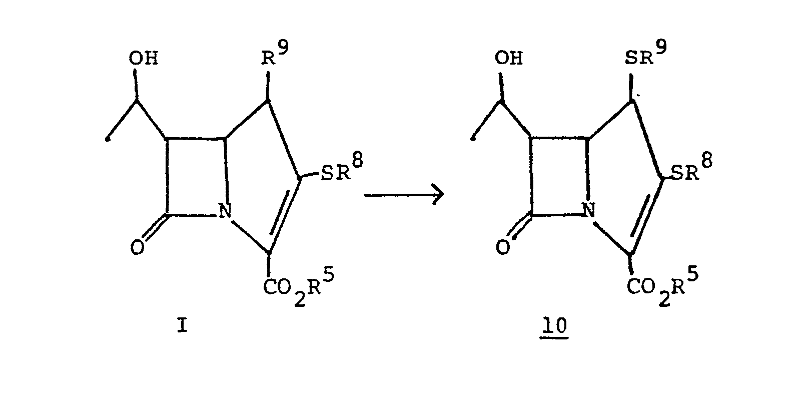

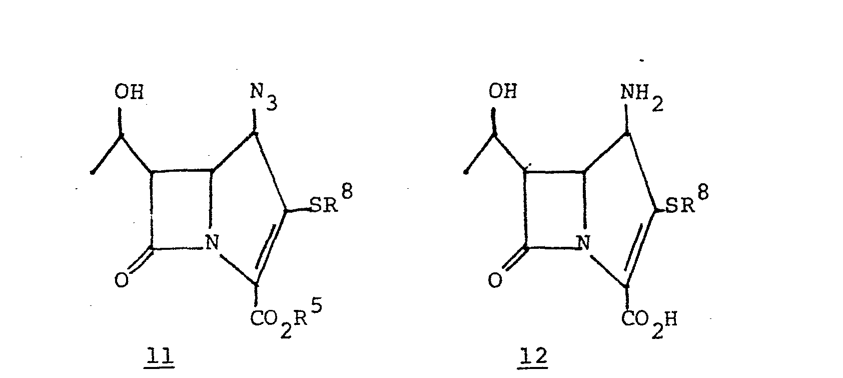

The 1-heteroatom substituted carbapenem I (wherein R⁹ is a leaving group, such as halogen or OMs) is treated with 1-2 eq. of mercaptan of choice (_{R}⁹ is as originally, generically defined) in the presence of 1 to 2 eq. of base such as diisopropylethylamine, triethylamine, dicyclohexylethylamine, and the like in a solvent such as DMF, DMSO, CH₂Cl₂, acetonitrile, THF, or the like at a temperature of from -20° to 60° for from 10 minutes to 6 hours to give 1-mercapto analogue 10. Representative mercaptans includes alkyl mercaptans, arylme.captans, and heteroarylmercaptans such as CH₃^{SH,} ₂H₅SH, 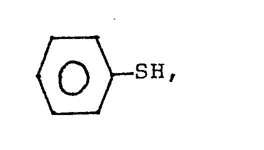 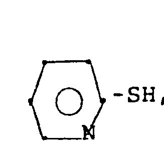 , and the like.

Preparation of 1-amino analogue 12 can also be derived from I. Thus, Compound I (R⁹=halogen or OMs, for example) is treated with lithium azide in DMF or DME at a temperature of from 0°C to 60°C for from 10 minutes to 6 hours to generated azido intermediate 11 which is then hydrogenated in THF-buffer solution in the presence of 10% Pd/C catalysts for from 30 minutes to 3 hours at a temperature of from 0°C to 25°C. Such 1-amino species can be derivatized to various 1-NHR, 1-NR, emobodiments of the thienamycin derivatization invention.

### (B) Derivatization of 1-Amino Carbapenem

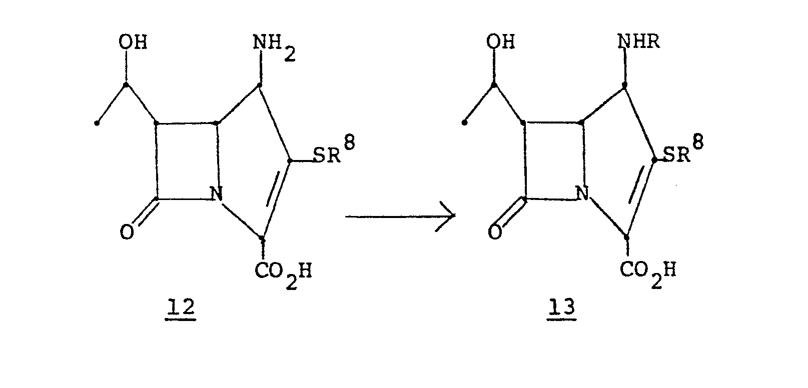

(1) N-Acylation: The 1-amino carbapenem 12 dissolved in 50% dioxane 0.1 M phosphate buffer is adjusted and maintained between pH 8.5-9.5 with 10% NaOH while treated with an acylating agent such as acyl halide or anhydride at a temperature of from 0 to 25°C for from 10 minutes to 1 hour. In this regard U.S. Patent 4,194,047 e.g.is incorporated herein by reference to the extent that it teaches such N-derivatization.
(2) N-Amidination: The aminocarbapenem 12 dissolved in 50% dioxane 0.1 M phosphate buffer at a temperature of from 0°C to 25°C is treated with an imidate between pH 8.5-9.5 for from 10 minutes to 1 hour. In this regard U.S. Patent 4,194,047 e.g.is incorporated herein by reference to the extent that it teaches such N-derivatization.
(3) N-Alkylation: The dioxane-phosphate buffer solution of 12 is treated with excess alkylating agent such as alkyl halide at a temperature of from 0° to 25°C, between pH 8.5-9.5 for from 10 minutes to 1 hours. In this regard U.S. Pate.t 4,194,047 e.gis incorporated herein by reference to the extent that it teaches such N-derivatizations.

In addition to the synthesis via the bicylic keto-ester, the compounds I of the present invention can also be prepared by the following exchange reaction:

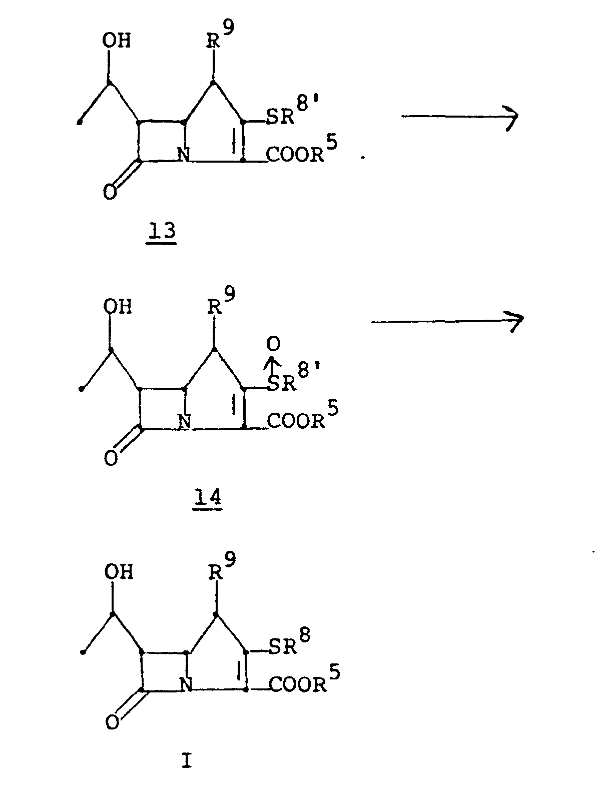

Thus, in specific terms, carbapenem 13 is converted to sulfoxide 14 by treating 13 with 1.0 eq of m-perbenzonic acid in a solvent such as methylene chloride, chloroform or the like at a temperature of from 0°C to 25°C for from 10 minutes to 60 minutes to give an epimeric mixture of sulfoxides 14 which is then treated with 1 to 2 eq of the desired mercaptan (_{R}⁸SH) in a solvent such as DMF, DME, THF, CH₃CN, methylene chloride, or the like, in the presence of 1.0 to 2.0 eg of a tertiary amine such as diisopropylethylamine, triethylamine, or the like, at a temperature of from 0° to 25°C for from 10 minutes to 2 hours to produce the desired carbapenem I. Relative to the foregoing scheme, R⁵ is a readily removable protecting group such as p-nitrobenzyl, benzyl, or the like.

### PREFERRED VALUES FOR _{R}⁹

In the generic structure (I):

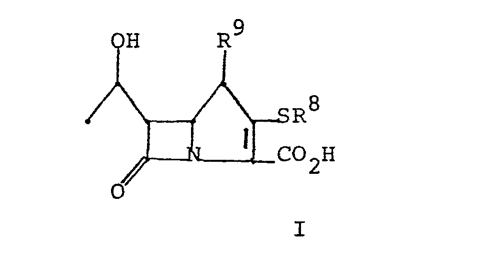

The following is representative and a preferred definition for R9: Preferred R⁹ groups:

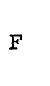

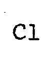

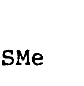

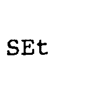

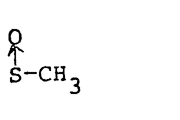

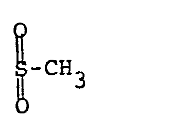

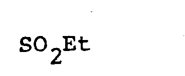

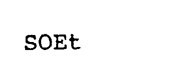

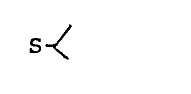

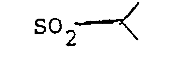

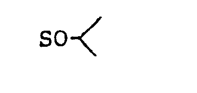

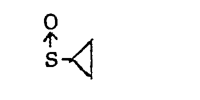

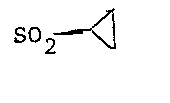

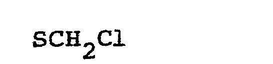

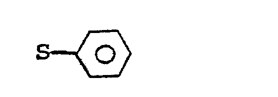

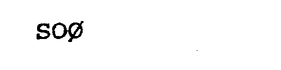

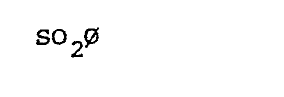

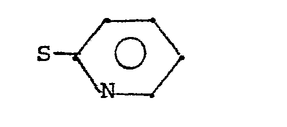

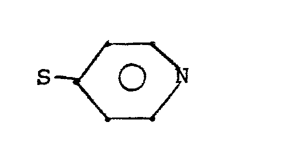

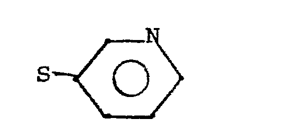

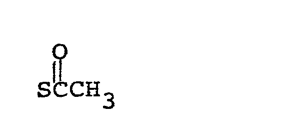

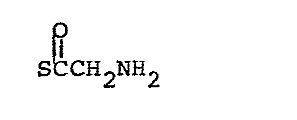

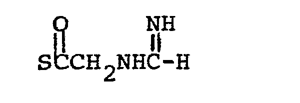

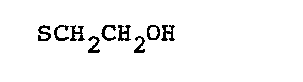

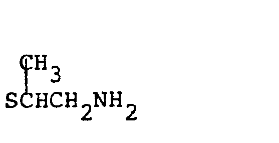

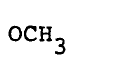

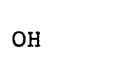

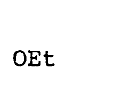

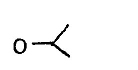

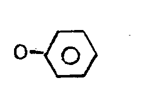

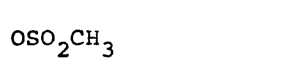

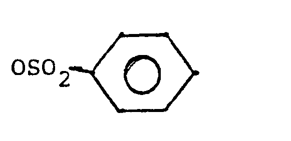

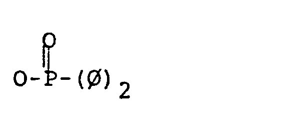

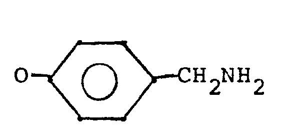

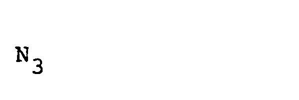

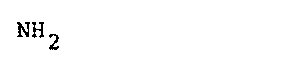

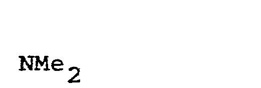

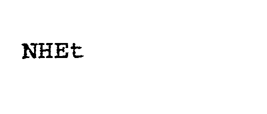

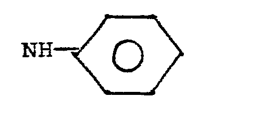

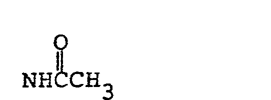

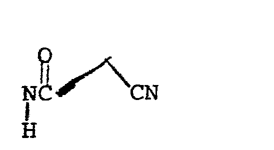

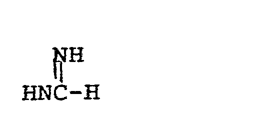

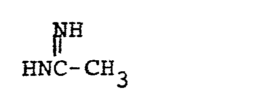

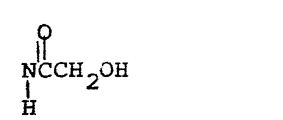

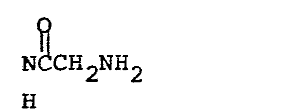

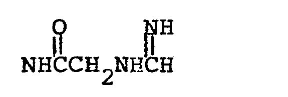

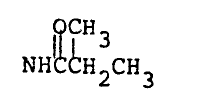

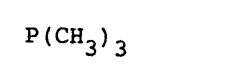

### Most Preferred R⁹ groups:

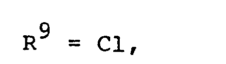

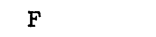

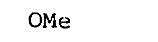

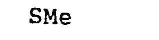

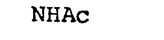

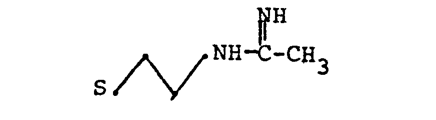

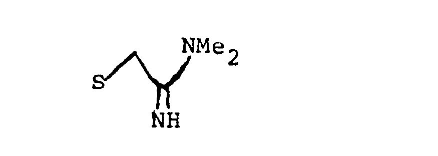

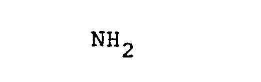

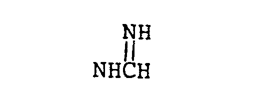

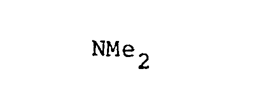

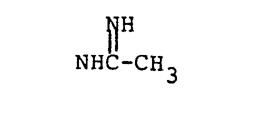

### _{HSR}⁸ REAGENTS

Relative to the foregoing description of the invention, suitable reagents, HSR⁸, which are utilized in the transformation 2a or 14 to I, are listed below. The list is arranged according to structural and functional characteristics of this side chain -SR⁸; annotation is provided where necessary. It should be noted that only HSR⁸ reagents are expressly shown. The thia side chain of choice -SR⁸ is derived from the corresponding mercaptan reagent HSR⁸, and thus the following list serves to further specifically disclose -SR⁸ side chains of I which are of special interest. When the mercaptan contains a functional group which might interfere with the intended course of reaction, the offending group is covered. For example, when a basic nitrogen group is encountered (-NHR or -NH₂, for example) it is usually protected by acylation (e.g., -C0₂PNB) and when a carboxyl group (-C0₂H) is present, it is usually protected by esterification (e.g., PNB ester). Such protection also facilitates in the purification of products by chromatographic means. (PNB is p-nitrobenzyl.) Such protection is, however, not a necessary requirement for introduction of the -SR⁸ side chain.

It is recognized that SR8 side chains in which the R⁸ group contains one or more chiral centers can be added as racemic or diastereomeric mixtures to provide mixtures of diastereomeric products or can be added as resolved, somerically pure reagents to provide diastereomerically pure products. Since antibacterial activity and other pharmacological properties vary among isomers, it is frequently advantageous to prepare isomerically pure products by the introducton of resolved -SR⁸ side chains.

1.) Aliphatic Mercaptans: HSR⁸ is 1-10 carbon alkyl, cycloalkyl, alkenyl, cycloalkenyl, or alkynyl; _{R}⁸ may be branched or unbranched, Examples 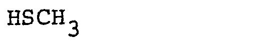 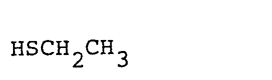 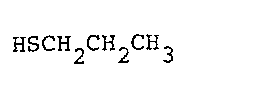 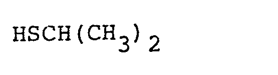 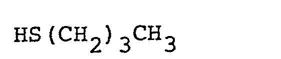 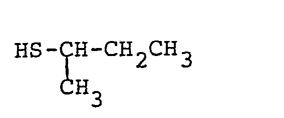 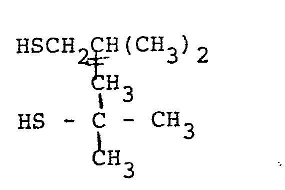 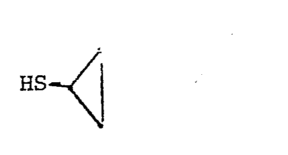 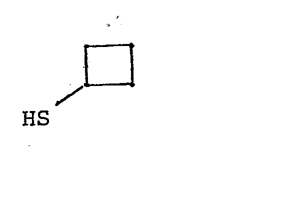 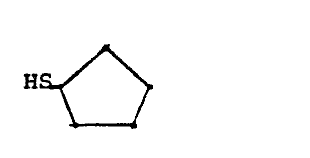 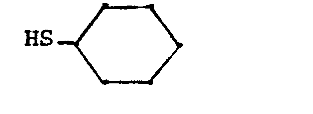 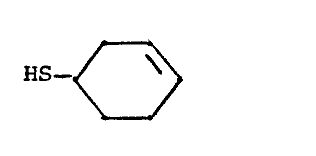 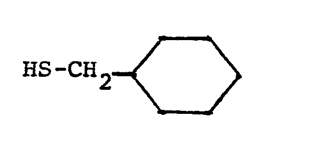 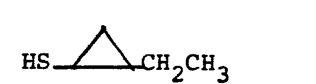 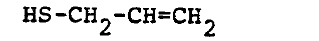 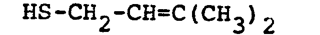 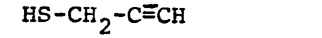 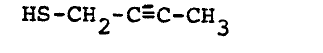
2.) Substituted Aliphatic Mercaptans: HSR⁸ wherein R⁸ is a 1-10 carbon branched or unbranched alkyl, cycloalkyl, alkenyl, cycloalkenyl, or alkynyl group substituted by one or more halo, OH, OR¹, 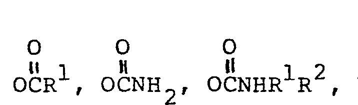 NH₂, NHR¹, NR¹R², 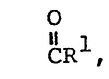 CO₂H, CO₂R¹, CONH₂, CON_{HR}¹, CONHR¹R², _{CN}, SR¹, 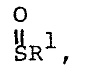 SO₂R¹, SO₂NH₂, SO₂NHR¹, SO₂NR¹R², NHCR¹ 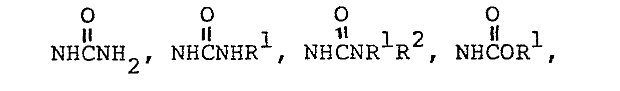 wherein _{R}¹ and R² are as previously defined relative to substituents on R⁸. Preferred substituents are basic nitrogen-containing groups.

### EXAMPLES

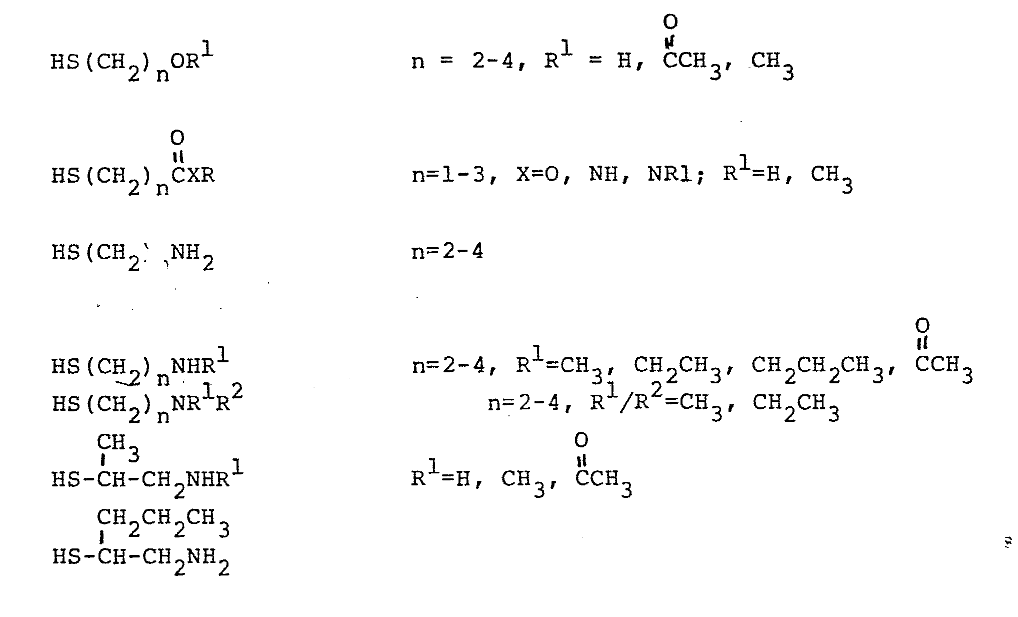

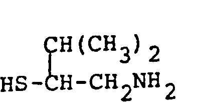

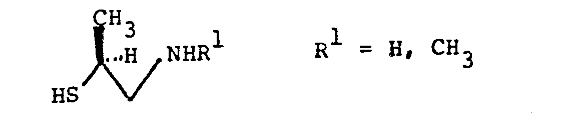

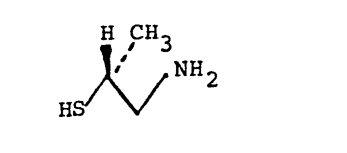

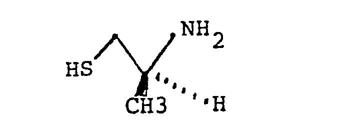

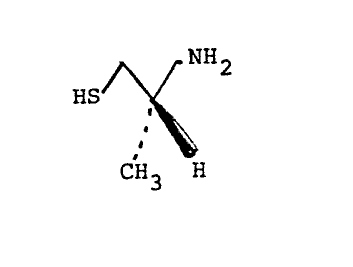

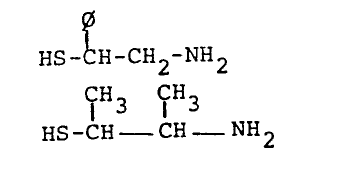

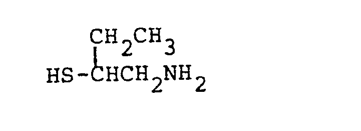

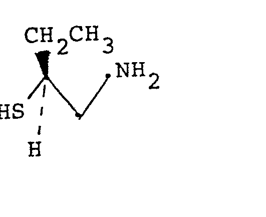

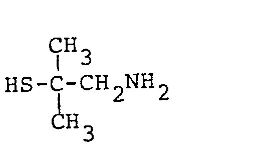

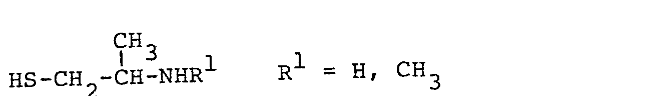

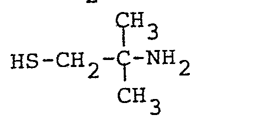

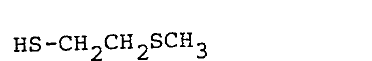

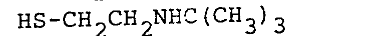

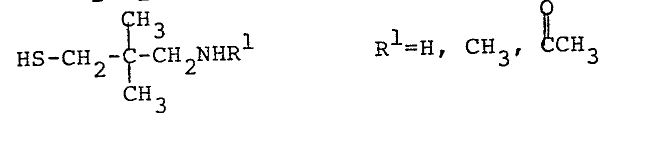

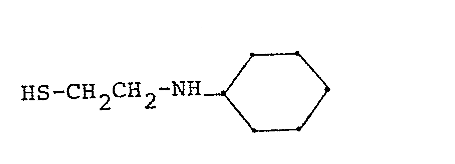

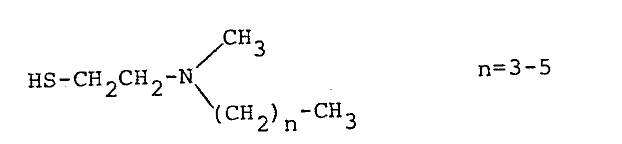

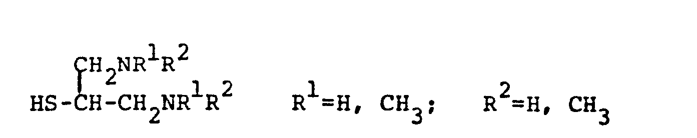

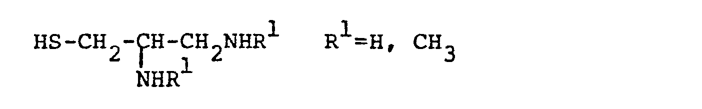

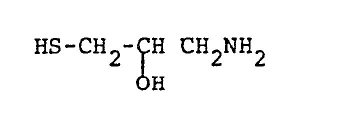

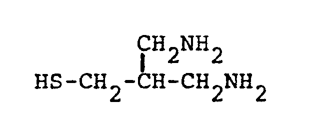

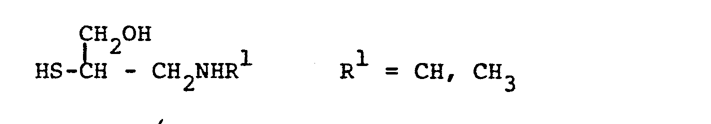

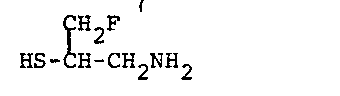

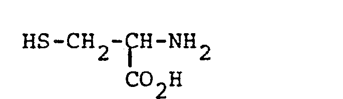

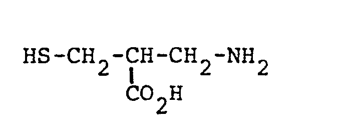

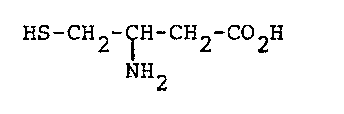

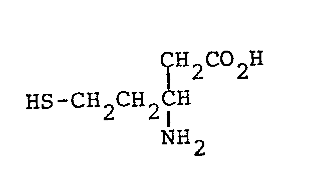

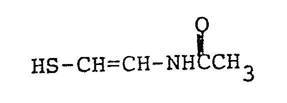

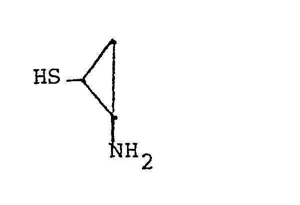

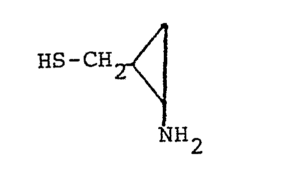

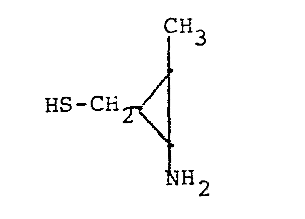

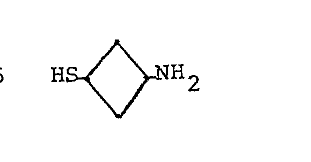

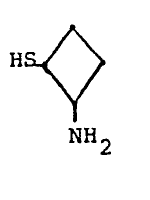

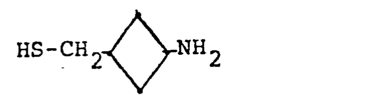

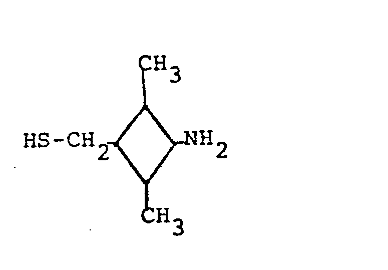

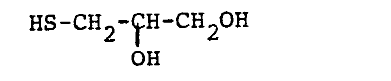

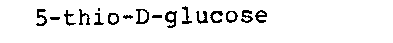

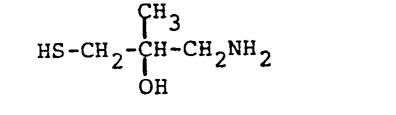

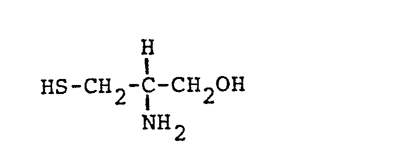

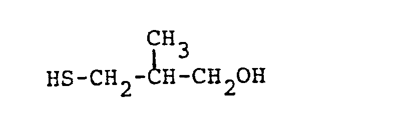

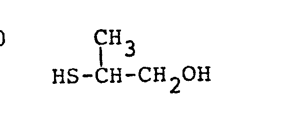

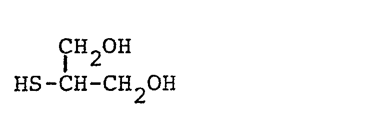

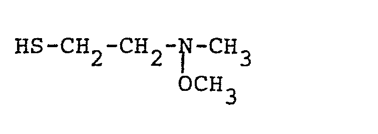

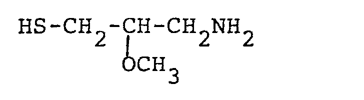

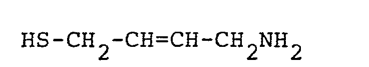

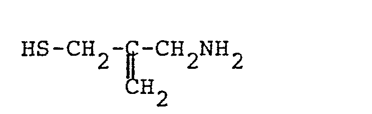

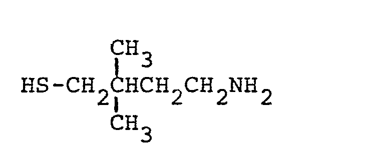

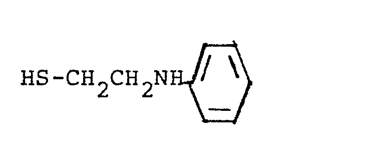

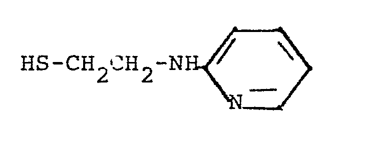

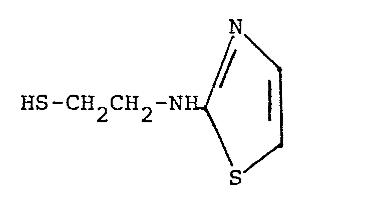

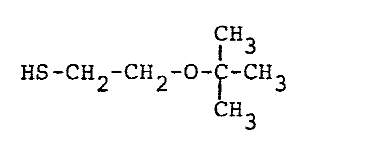

3.) Aryl Mercaptans: HSR⁸ wherein R⁸ is phenyl or substituted phenyl. The substituents are independently selected from those previously defined for R⁸. Especially preferred substituents include alkyl, halo, cyano, hydroxy, alkoxy, acyloxy, acyl, carboxy, mercapto, sulfinyl, sulfonyl, amino, substituted amino, aminoalkyl, substituted aminoalkyl, amido, and ureido. 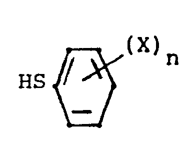 n= 1, 2 or 3 X= F, Cl, Br, CN, OH, OR, 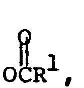 NH₂, NHR¹, NR¹R², CH₂NH₂, CH₂NR¹R², CO₂H, CO₂R¹, COR¹, CONH₂, CONR¹R², R¹CONH, R¹_{NHCONH}, SR¹, 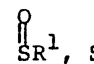 SO₂R¹, CH₃, CF₃; R¹ and R² are as previously defined under R⁸.

### Examples

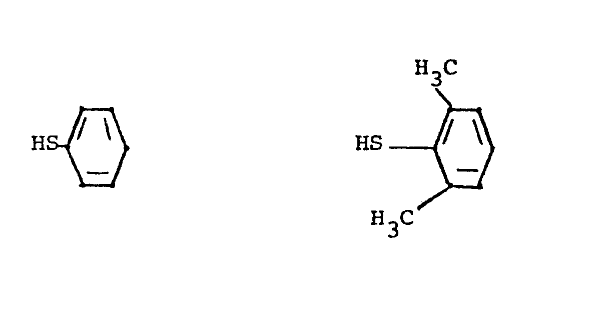

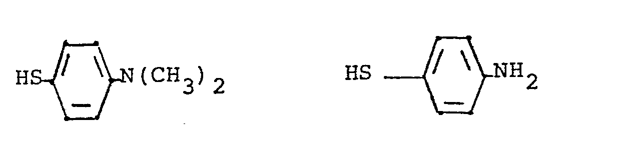

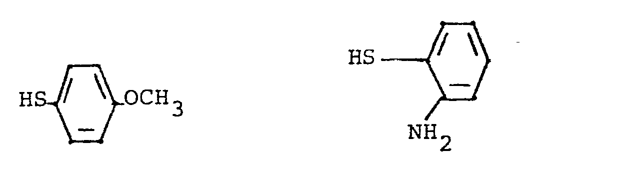

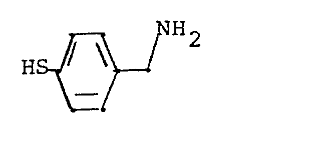

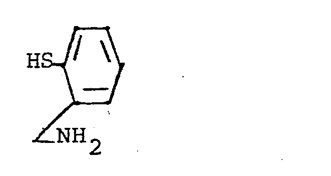

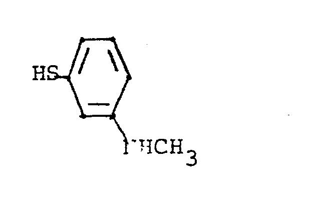

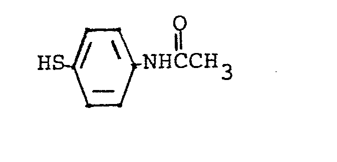

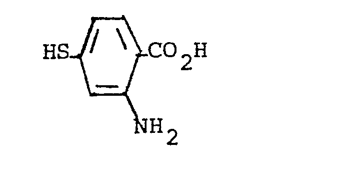

4.) Heteroaryl Mercaptans: HSR⁸ wherein R⁸ is a substituted or unsubstituted heteroaryl group concontaining 1-4 0, N or S atoms. Typical substituents include those mentioned above under "Aryl Mercaptans".

### Examples

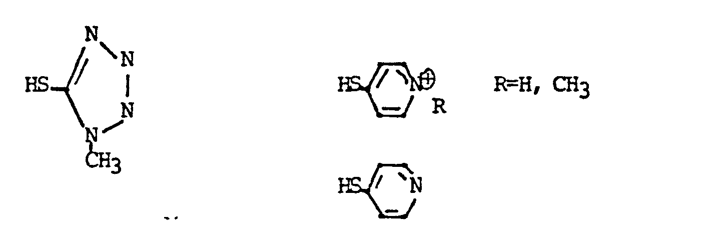

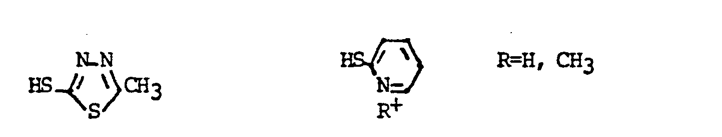

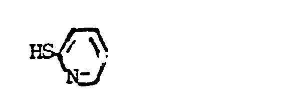

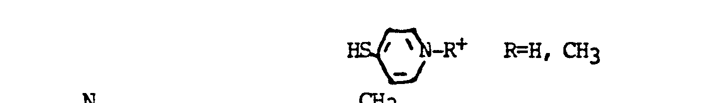

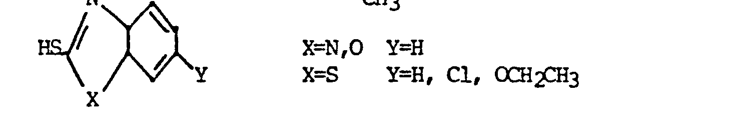

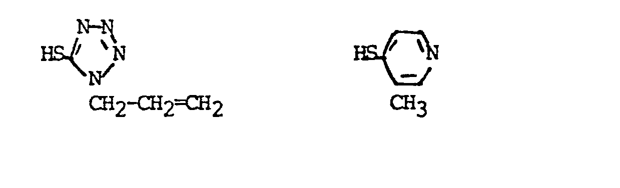

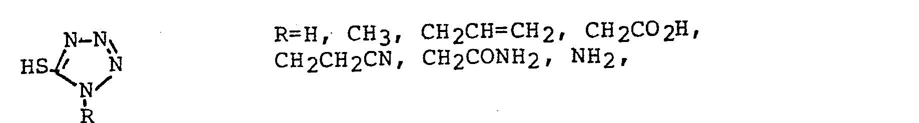

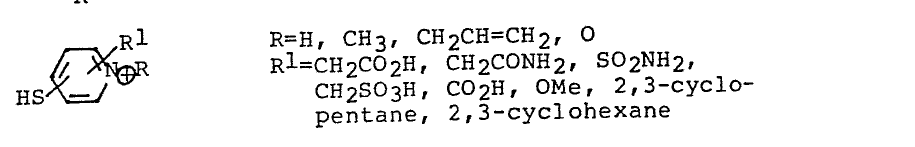

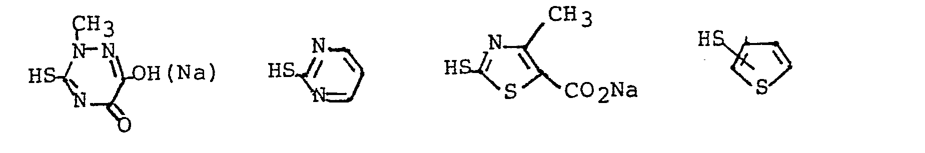

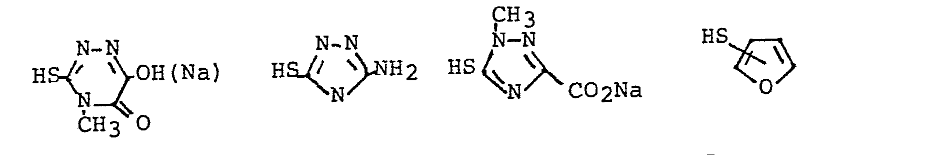

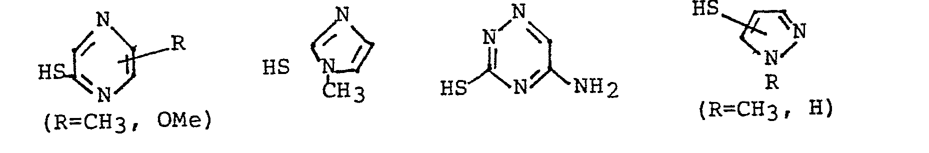

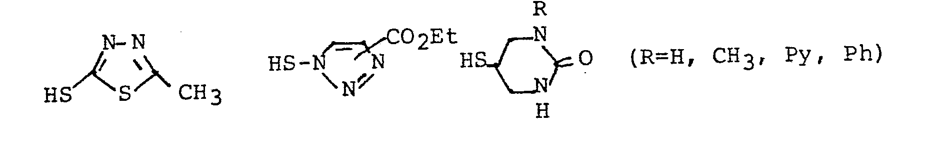

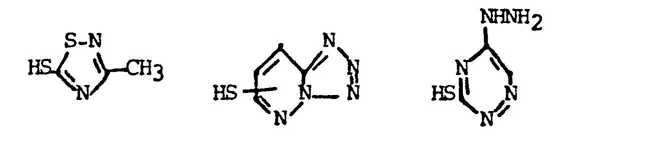

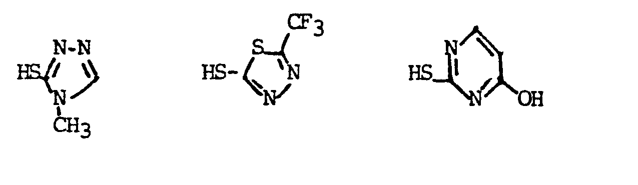

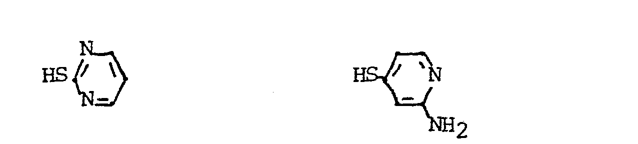

5.) Arylaliphatic Mercaptans: HSR8 where IR⁸ is a 1 -6 carbon branched or unbranched alkyl, cycloalkyl, alkenyl, or alkynyl group substituted by a phenyl or substituted phenyl kgroup. Typical phenyl substituents include those mentioned under "Aryl Mercaptans".

### Examples

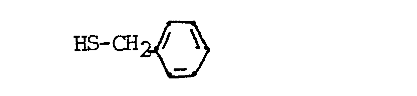

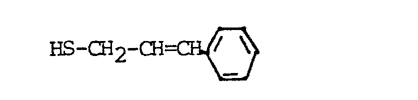

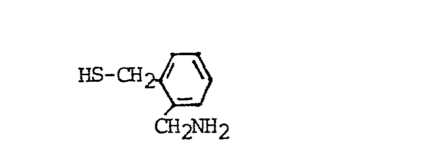

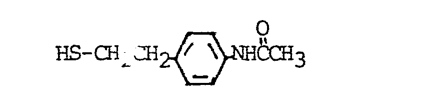

6.) Heteroarylaliphatic and Heterocyclylaliphatic, and heterocyclic Mercaptans HSR⁸ wherein R⁸ is a 1-6 carbon branched or unbranched alkyl, cycloalkyl, alkenyl, or alkynyl group substituted by a heteroaryl or heterocyclyl group containing 1-4, 0, N, or S atoms. The heteroaryl or heterocyclic group is unsubstituted or substituted by those substituents mentioned under "Aryl Mercaptans", (No. 3 above).

### Examples

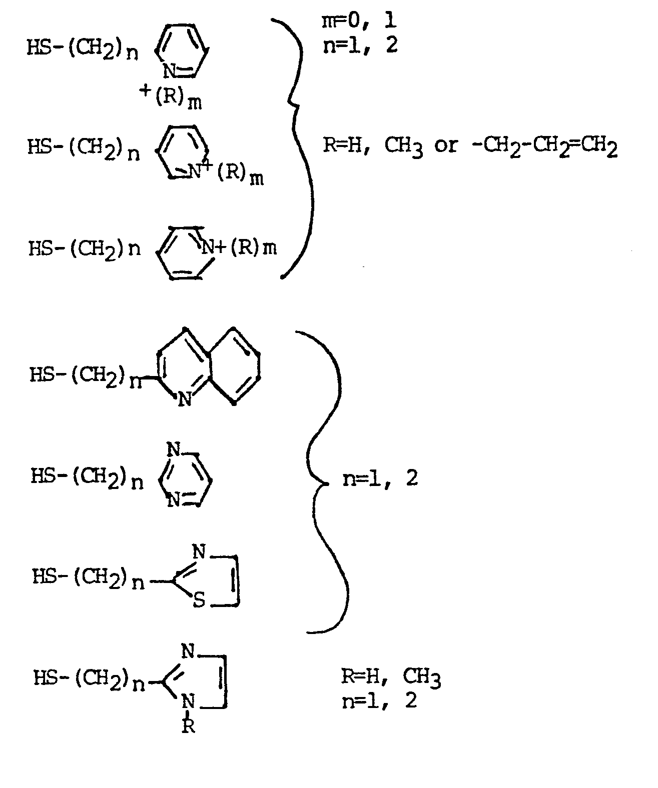

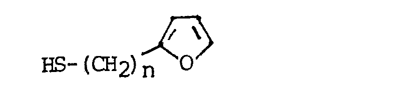

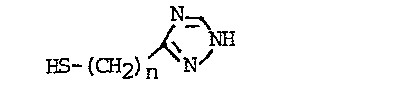

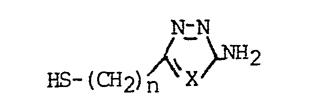

X=NH, S 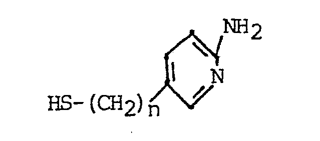 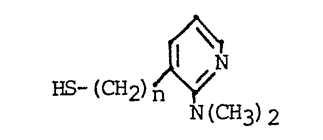 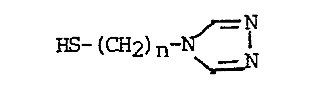 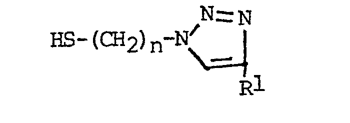 R¹=OCH₂CH₃ 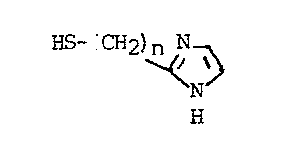 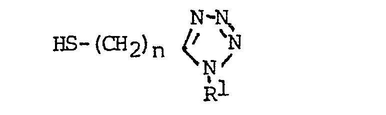 R¹=H, CH₃, -CH₂-CH=CH₂ n=1, 2 in above formula 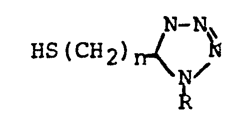 R=H, CH₃, CH₂CH=CH₂, CH₂CO₂H, CH₂CH₂CN, CH₂CONH₂, NH₂, 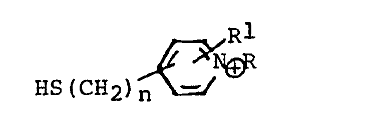 R=H, CH₃, CH₂CH=CH₂, 0 R^{l}=CH₂C0₂H, CH₂CONH₂, S0₂NH₂, CH₂SO₃H, C0₂H, OMe, 2,3-cyclopentane, 2,3-cyclohexane 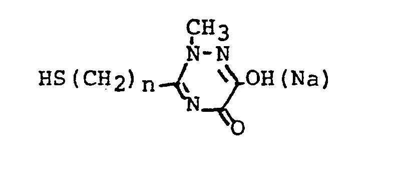 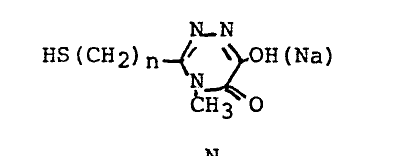 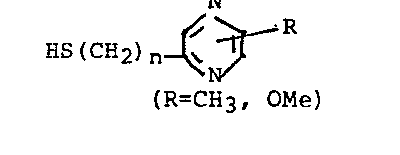 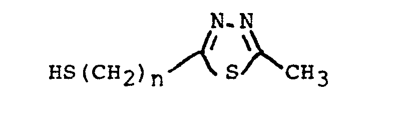 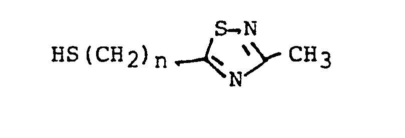 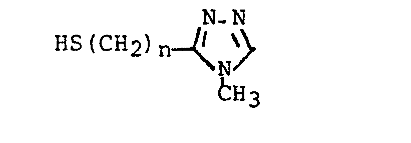 n = 1, 2, 3 in all the above formulae 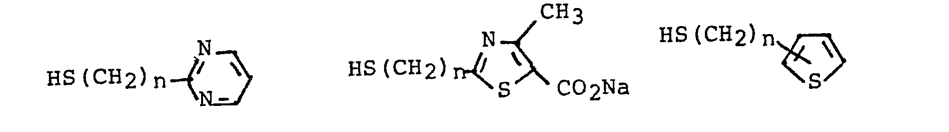 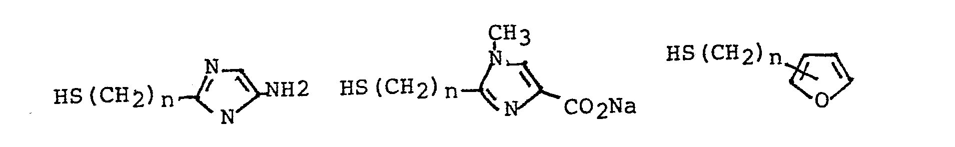 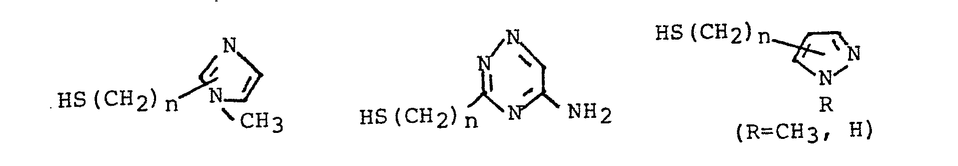 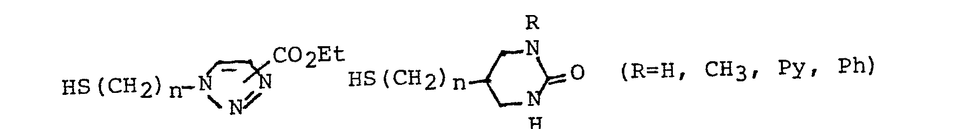 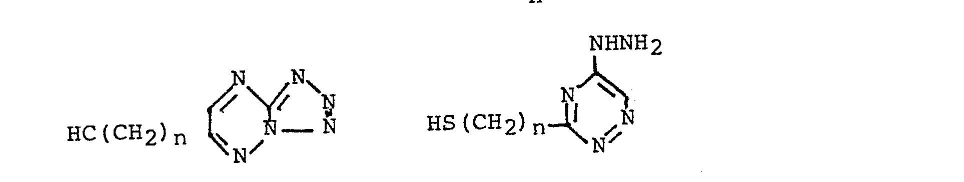 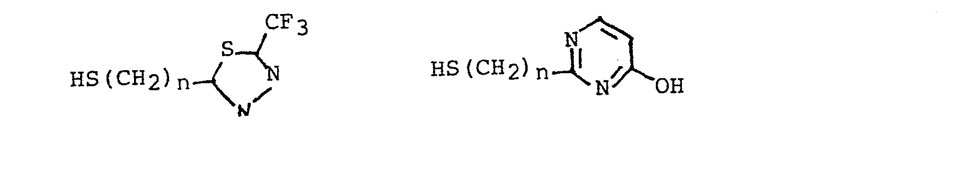 n = 1, 2, 3 in the above formulae 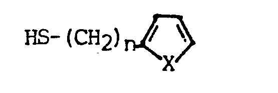 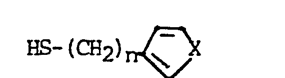 X=O, S, NH 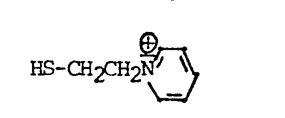 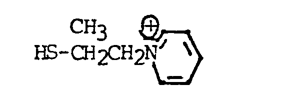 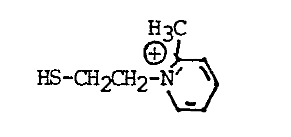 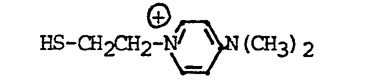 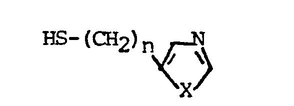 X=O, S, NH 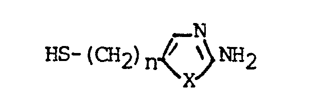 X=O, S, NH 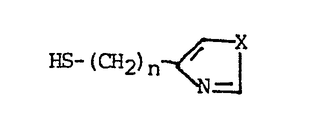 X=O, S, NH 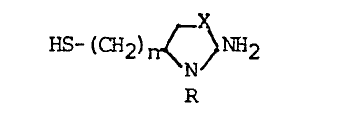 X=O, S, NH R=H, CH₃ 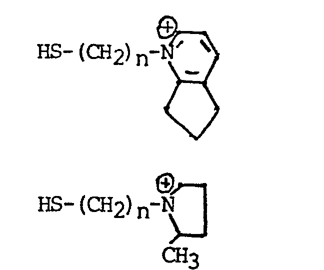 n=1,2 or 3 in the above formulae 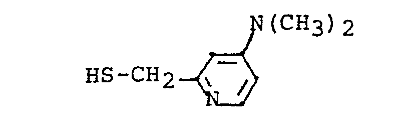 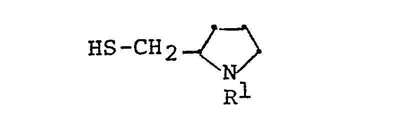 _{Rl = H}, C^{H}3 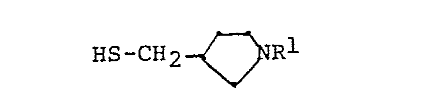 _{R}l = H, CH₃ 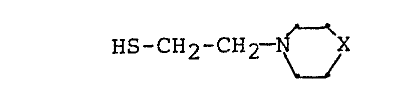 X = O, NH, NCH₃ 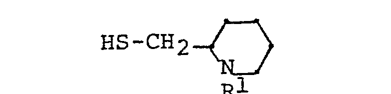 _{R}l = H, CH3 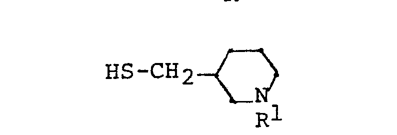 _{R}l = H, CH₃ 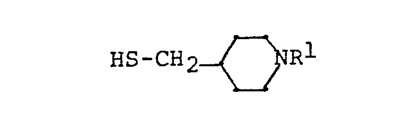 _{R}l = H, CH3 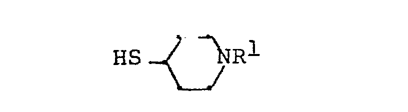 _{R}l = H, CH3 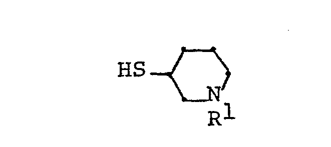 _{R}l = H, CH3 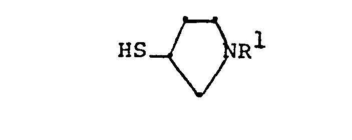 R¹ = H, CH₃ 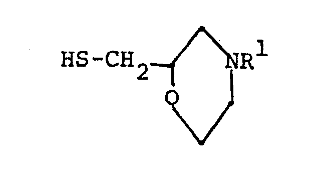 R¹ = H, ^{CH}₃ 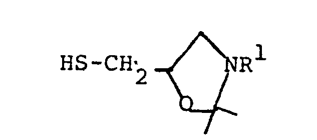 R¹ = H, ^{CH}₃

7.) Alkyl-Heteroatom-Alkyl Mercaptans, HSR⁸ Wherein R is -(CH₂)ₙX(CH₂)ₘR¹⁰ wherin n = 2 to 4, m = 2 to 4; x is NR, O or S; and wherein R° is H, CH₃, CH₂CH₃, CH₂CH₂OH, or CH₂NH₂ and R¹⁰ is OH, NH₂, NHCH₃, N(CH₃)₂, 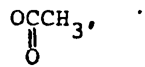 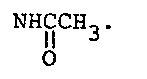 Note, in the above representation, the methylene carbons may be branched; for example: 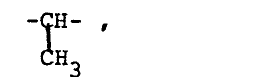 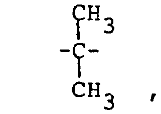 and the like. The following HSR⁸ are representative of this class:

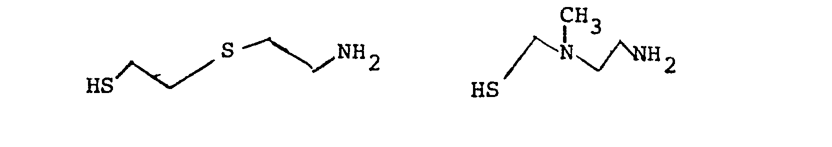

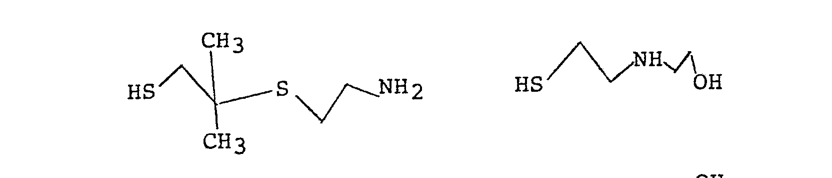

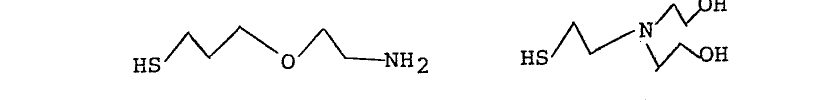

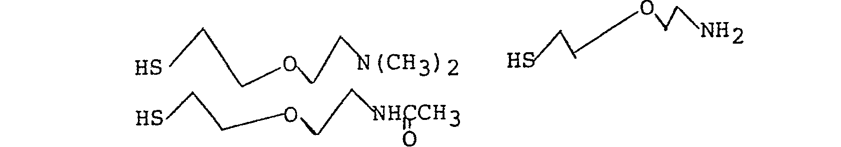

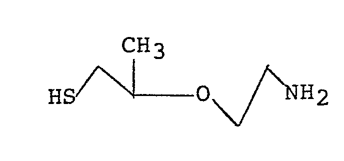

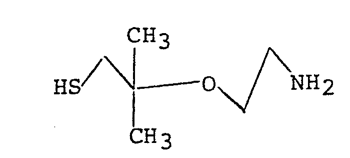

8.) Amidino and Amidinium Mercaptans HSR⁸ Wherein _{R}⁸ is: 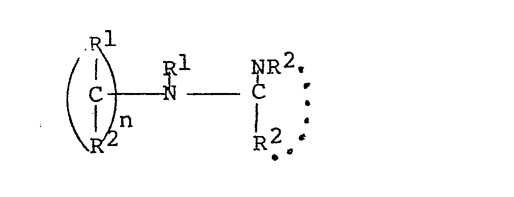 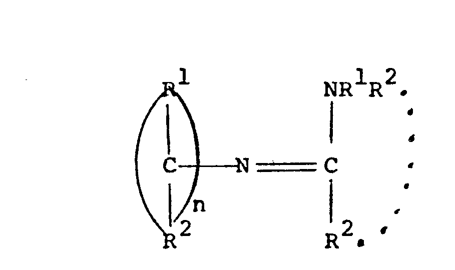 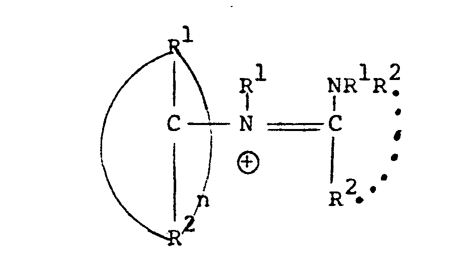 and wherein n = 2-6; R1 and R² are as initially defined under R⁸; and the dotted line indicates provision for the ring formed by the joinder of substituents carried by the imino carbon atoms. Such amidino and amidinium embodiments of final products I are also conveniently obtained by N-derivatization of the corresponding amino embodiment I according to the procedure disclosed in U.S. Patent 4,194,047 which patent is incorporated herein by reference since the N-derivatization of thienamycin disclosed in the incorporated by reference patent is strictly analogous to the N-derivatization contemplated to achieve the amidino embodiments characterized herein. The following reaction summarizes such N-derivatization: 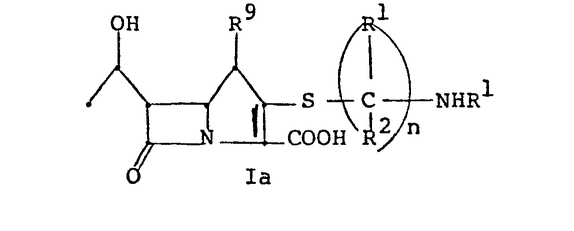 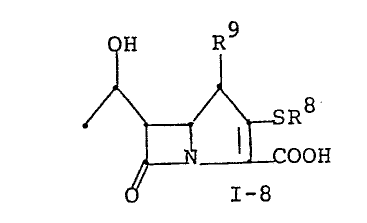 wherein: relative to 1-8, R⁸ is defined above in this category No. 8.

Relative to the amidino embodiments characterized under this heading, representatively preferred values for R¹ and R² attached to the carbon atom include:
H, CH₃, CH₂CH₃, CH₂OH, OCH₃, CH₂NH₂, F, phenyl, CF₃, CH(CH₃)₂, CH₂CH₂CH₃, CH₂^{F} benzyl, SCH₃, N(CH₃)₂, N⁺(CH₃)₃X (X defined above) CN

Representatively preferred values for R¹ and R² attached to the nitrogen atoms include:
H, phenyl, CH(CH₃)₂, C(CH₃)₃, ^{NH}₂, CH₃, NHCH₃, N(CH₃)₂, CH₂CH₃, CH₂CH₂⁰H, - (CH₂)₄ - , -CH₂CH₂-O-CH₂CH₂, O^{CH}3

Representatively preferred values for R² attached to the imino carbon atom include:
H, CH₃, CH₂CH₃, phenyl

The following values for HSR⁸ are also classified under the amidino mercaptans, giving rise to amidino embodiments of I:
_{R}⁸ is: 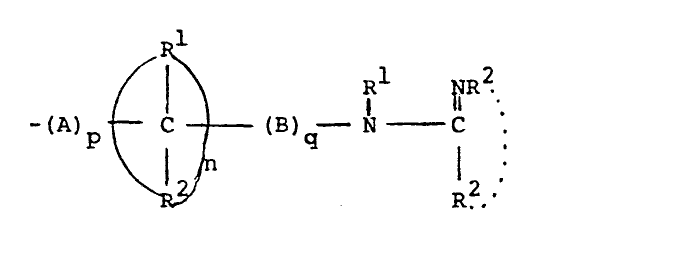 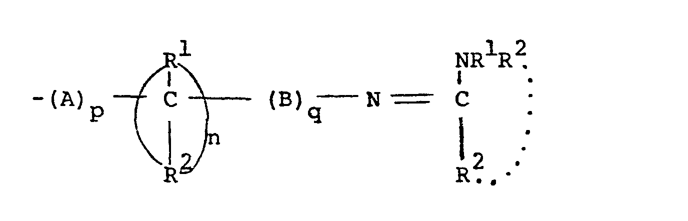 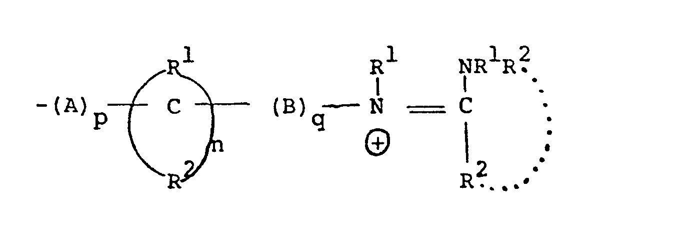 wherein _{R}¹, R² and n are as defined immediately above; p and q are 0 or 1; A and B are selected from: the aforementioned values for R⁸ expressed in bivalent form (-R⁸-) from categories No.'s 1-7; thus, A and B (or - R⁸-) are selected from: cycloalkyl, alkenyl, cycloalkenyl, alkynyl (see Class No. 1, above); substituted: cycloalkyl, alkenyl, cycloalkenyl, alkynyl (see Class No. 2, above); phenyl and substituted phenyl (see Class No. 3, above); substituted and unsubstituted heteraryl (see Class No. 4, above); aryl aliphatic (see Class No. 5, above); heteroarylaliphatic, heterocyclylaliphatic, and heterocyclic (see Class No. 6, above); and alkylheteroatom-alkyl (see Class No. 7, above); and B can also be selected from -O- and -NR¹-.

### EXAMPLES

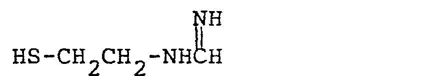

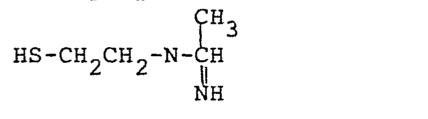

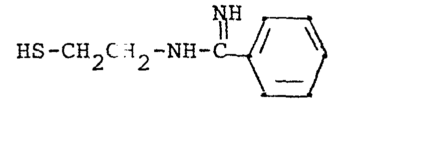

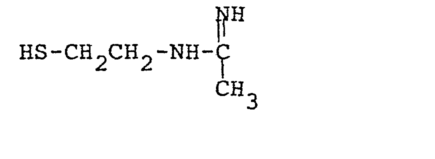

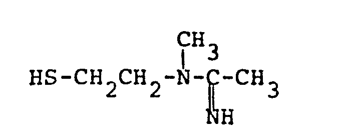

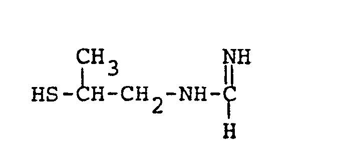

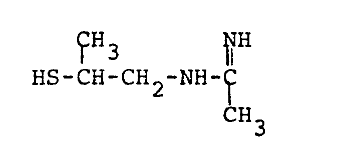

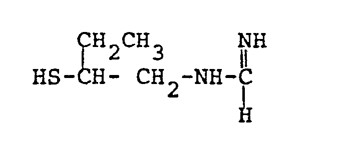

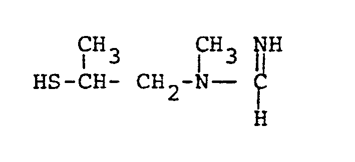

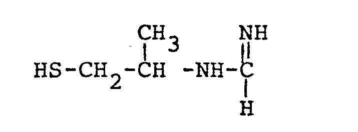

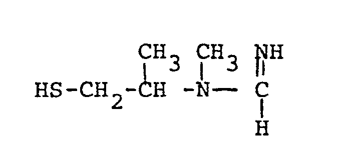

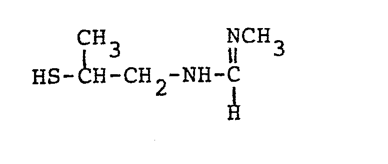

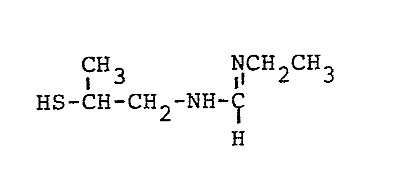

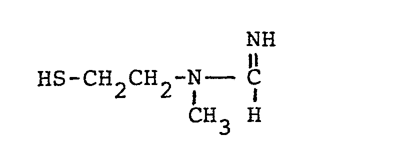

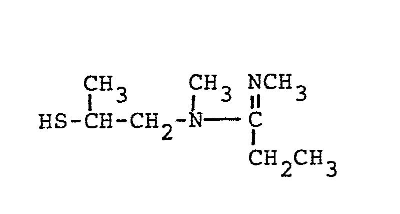

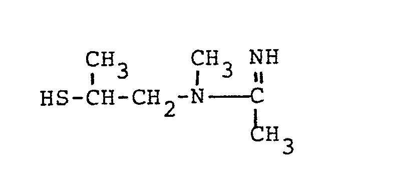

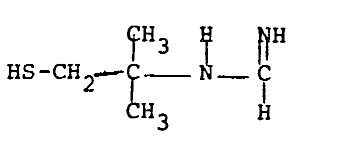

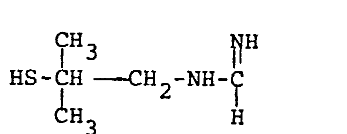

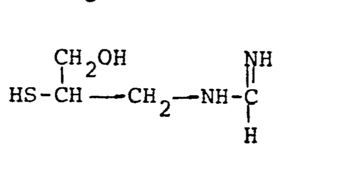

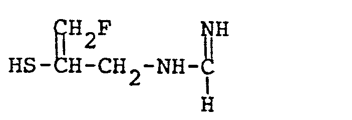

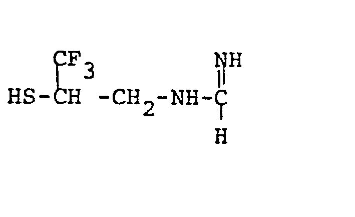

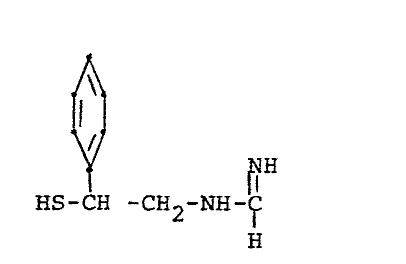

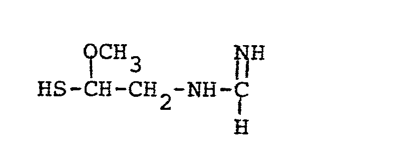

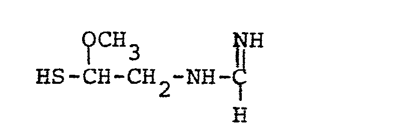

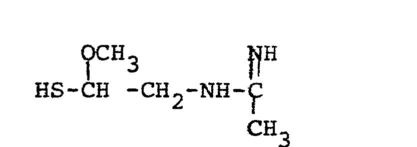

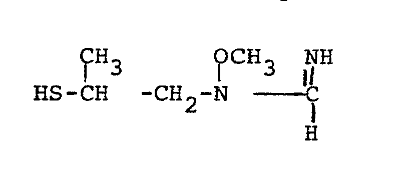

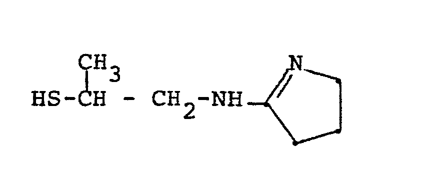

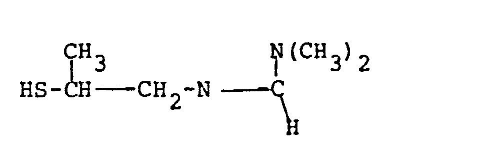

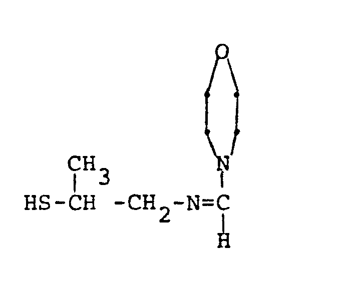

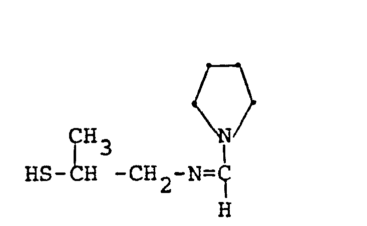

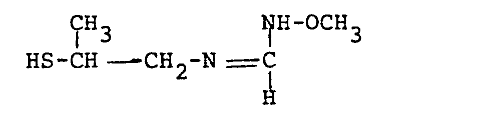

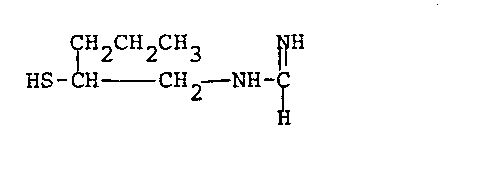

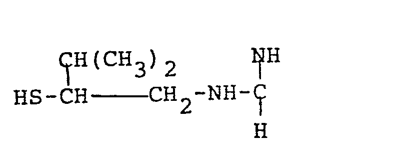

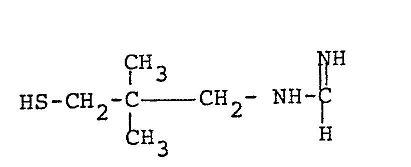

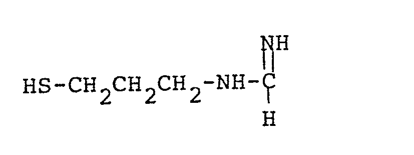

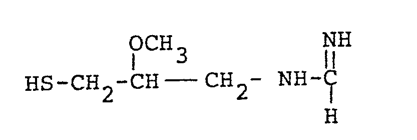

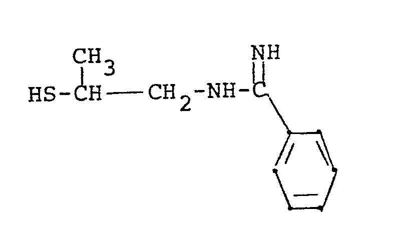

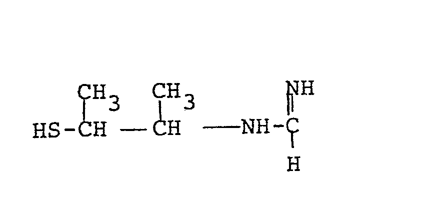

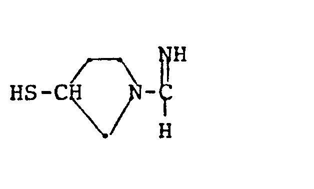

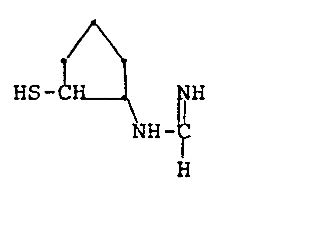

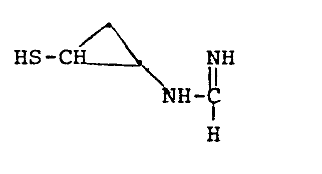

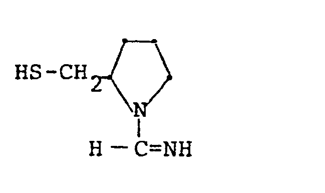

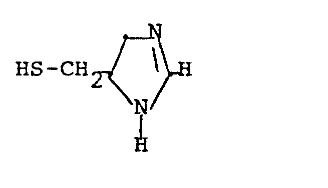

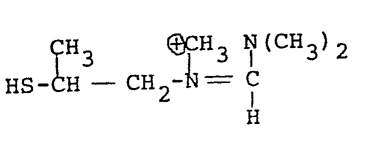

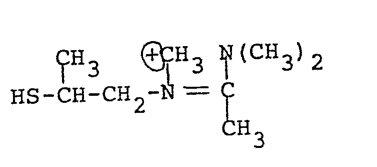

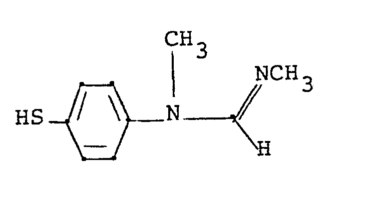

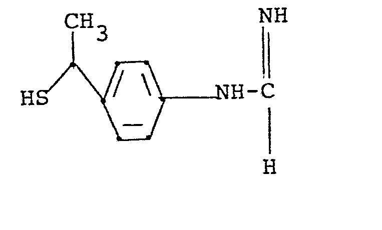

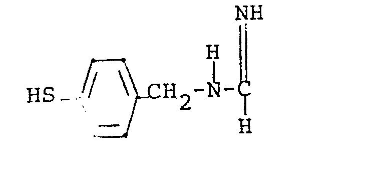

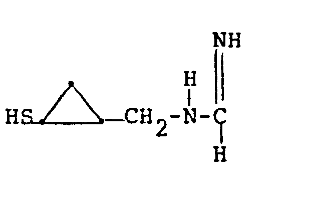

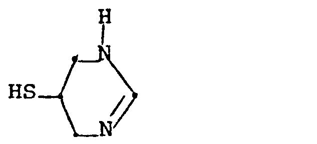

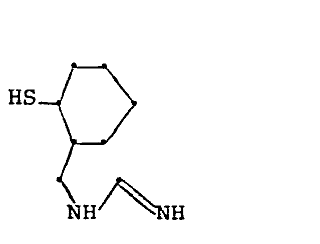

9.) Guanidino and Guanidinium Mercaptans HSR⁸ Wherein R⁸ is: 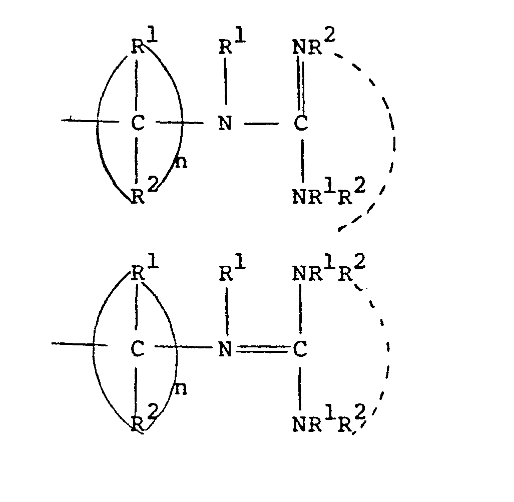 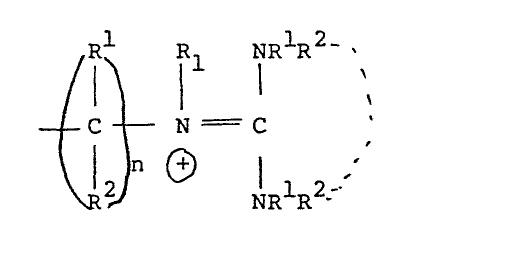 and wherein n = 2-6; Rl and R2 are as initially defined under R8; and the dotted line indicates provision for the ring formed by the joinder of substituents carried by the mino carbon atom. Such guanidino and guanidinium embodiments of the final products I are conveniently obtained by N-derivatization of the corresponding amino embodiments according to procedures disclosed in U.S. Patent 4,194,047 as was explained under 8 above. Such guanidino embodiments are also conveniently prepared directly following the procedure described in European Patent Publication No. 50334, which publication is incorporated herein by reference. It should be noted that the cited application is directed to 1-carbadethiapenems, but thai the disclosed process is useful by analogy. Representatively preferred values for R and R² attached to the carbon atom include:

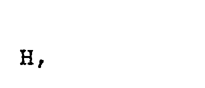

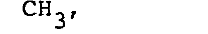

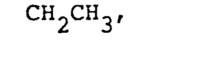

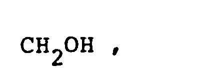

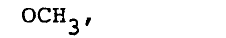

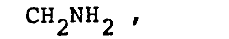

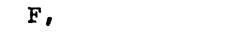

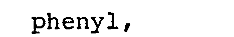

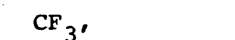

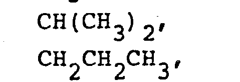

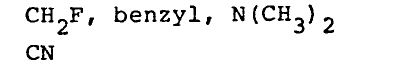

Representatively preferred values for R¹ and _{R}² attached to nitrogen atoms include:

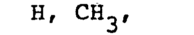

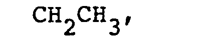

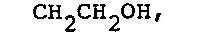

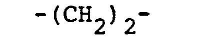

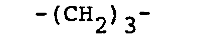

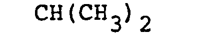

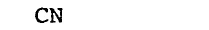

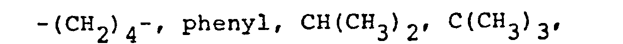

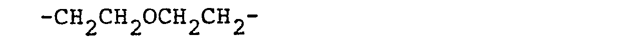

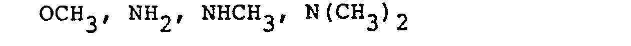

The following values for HSR⁸ are also classified under the guanidino mercaptans, giving rise to the guanidino embodiments of I:
_{R}⁸ is: 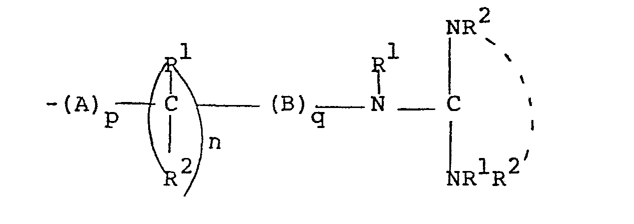 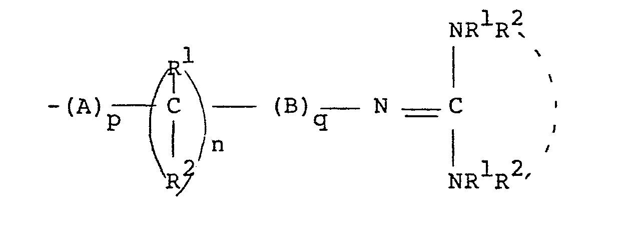 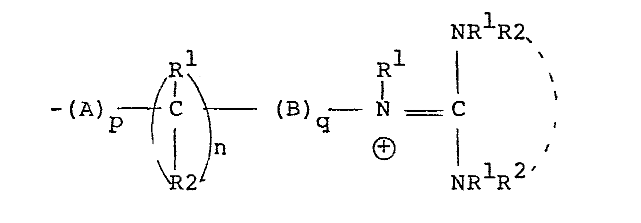 wherein R¹ and R² and n are as defined immediately above; p and q are 0 or 1; A and B are selected from: the aforementioned values for R⁸ expressed in bivalent form from categories No.'s 1-7. Thus, A and B (or -R ) are selected from: cycloalkyl, alkenyl, cycloalkenyl, alkynyl (see Class No. 1, above); substituted: cycloalkyl, alkenyl, cycloalkenyl, alkynyl (see Class No. 2, above); phenyl and substit ted phenyl (see Class No. 3, above); substituted and unsubstituted Heteroaryl (see Class No. 4, above; aryl aliphatic (see Class No. 5, above); heteroaryl-aliphatic, heterocyclylaliphatic, and heterocyclic (see Class No. 6 above); and alkyl-heteroatom-alkyl (see Class No. 7, above); B is also selected from -0- and -NR'-.

### EXAMPLES

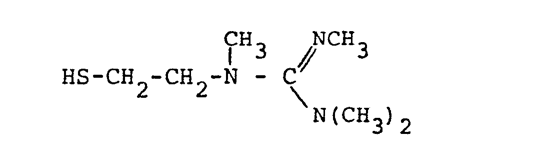

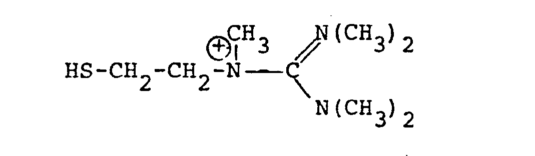

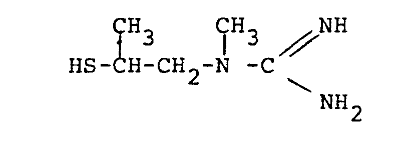

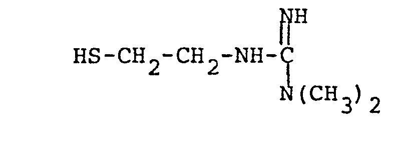

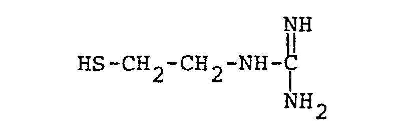

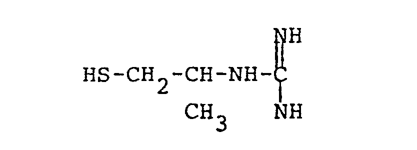

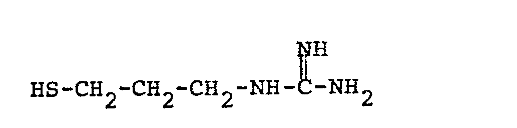

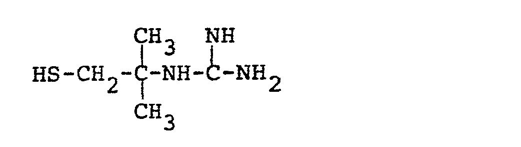

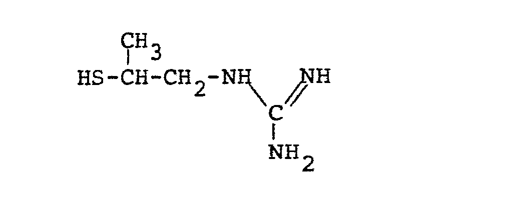

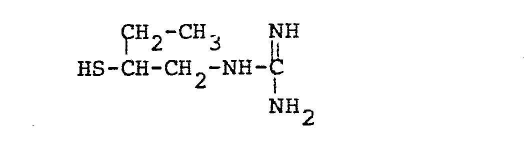

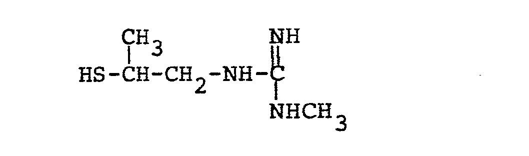

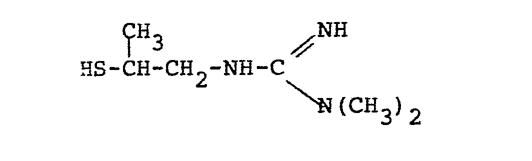

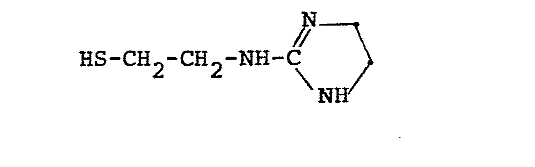

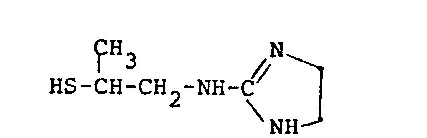

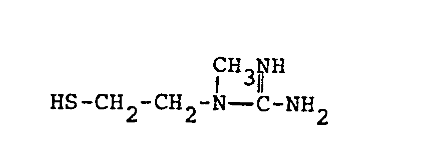

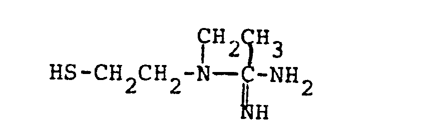

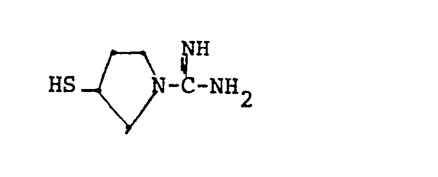

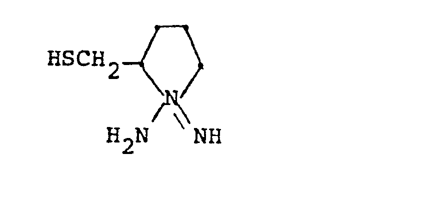

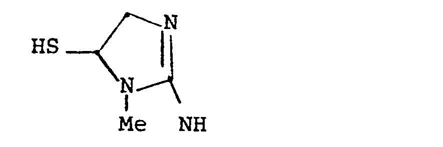

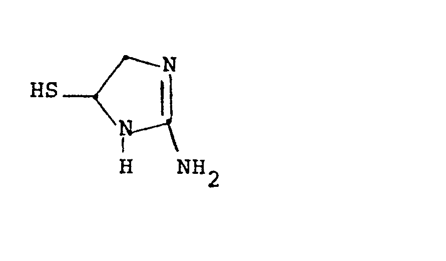

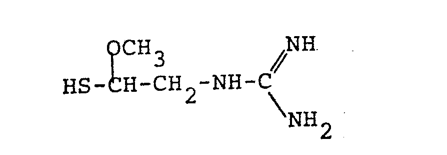

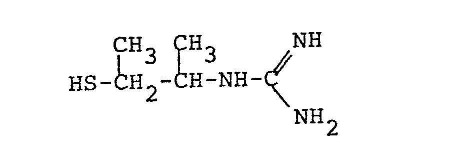

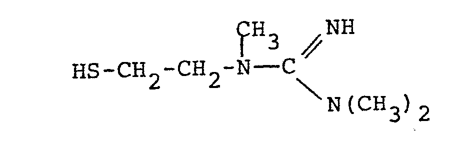

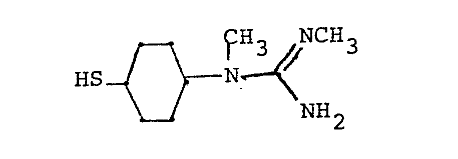

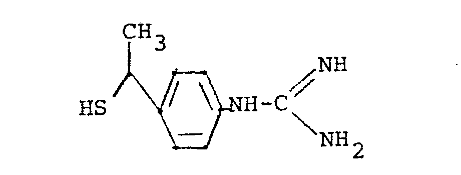

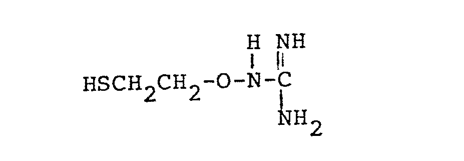

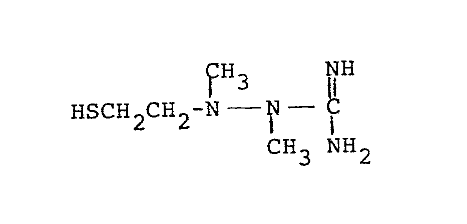

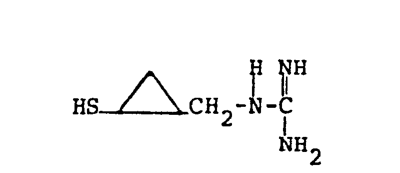

### HSR⁸ REAGENTS, CONTINUED

Relative to the foregoing description of the invention (I), suitable carbamimidoyl and carbamimidinium mercaptans HSR⁸ which are utilized in the transformation II to I are listed below. Wherein _{R}⁸ is: 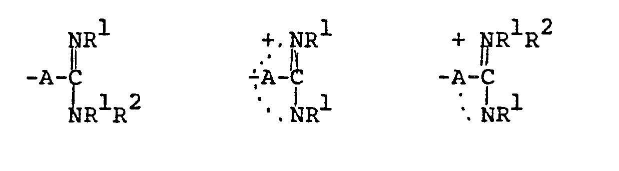 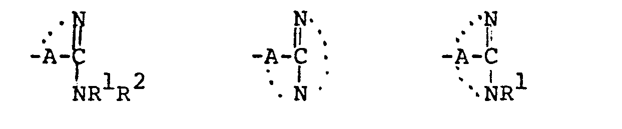 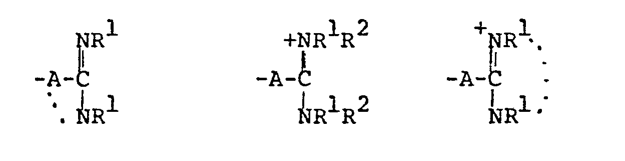 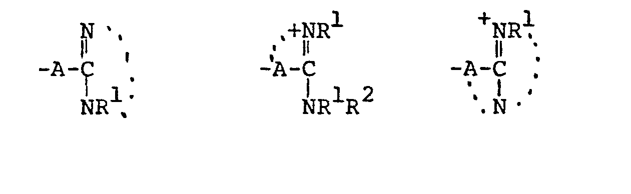 and wherein R¹ and R² are as initially defined under R⁸_{;} the two nitrogen atoms demonstrated in the above structure may participate in cyclic structures which are indicated by the dotted lines; A is a connecting group between the sulfur atom and carbamimidoyl function. It should be noted that while not all conical forms of R⁸ are reproduced herein, the foregoing list is representative and constitutes together with the associated text a definition of the "carbamimidoyl" group of the present invention.

_{R}¹ and _{R}² are independently selected from the group consisting of hydrogen; substituted and unsubstituted: straight and branched alkyl having from 1 to 6 carbon atoms; cycloalkyl having 3 to 6 carbon atoms; cycloalkylalkyl wherein the cycloalkyl moiety comprises 3 to 6 carbon atoms and the alkyl moiety comprises 1 to 6 carbon atoms; alkylcycloalkyl wherein the alkyl moiety comprises 1 to 6 carbon atoms and the cycloalkyl moiety comprises 3 to 6 carbon atoms; aryl such as phenyl; arylalkyl such at benzyl; heterocyclyl (saturated and unsaturated) comprising mono- and bicyclic structures having from 5 to 10 ring atoms, wherein one or more of the heteroatoms is selected from oxygen, nitrogen, or sulfur, such as thiophene, imidazole, tetrazolyl, furyl, pyridine; heterocyclylalkyl groups which comprise the immediately preceding heterocyclyl moieties and the alkyl moiety comprises 1 to 6 carbon atoms. The substituent or subs Ltuents relative to the above-named radicals comprising _{R}¹ and _{R}² are selected from the group consisting of amino, hydroxy, cyano, carboxyl, nitro, chloro, bromo, fluoro, alkoxy, and alkylthio having from 1 to 6 carbon atoms, mercapto, perhaloalkyl having 1 to 3 carbon atoms, guanidino, amidino, sulfamoyl. When located on the same nitrogen atom, the substituents R¹ and _{R}² can be joined to form a cyclic group comprising 3-8 atoms. The resulting ring can contain additional O, S or N atoms. For example, -NR¹R² can be taken as morpholino, pyrrolidino, piperidino, azetidinyl or the like.

Particularly preferred groups under the definition of R^{l}/R² are: hydrogen; substituted and unsubstituted: straight and branched loweralkyl having from 1 to 6 carbon atoms; cycloalkyl having from 3 to 6 carbon atoms, cycloalkylalkyl wherein the cycloalkyl moiety comprises 3 to 6 carbon atoms and the alkyl moiety comprises 1 to 6 carbon atoms; aryl such as phenyl, arylalkyl such as benzyl; the substituents on the above-named radicals are selected from fluoro, hydroxy, mercaptocyano, alkoxy and alkylthio having from 1 to 3 carbon atoms.

In defining the bivalent, cyclic or acyclic connector group "A", it is to be noted that the recited radicals of definition are to be read both left to right and right to left. Thus, the preferred connecting groups "A" are selected from: substituted and unsubstituted: loweralkyl having from 1-6 carbon atoms; cycloalkyl having from 3-10 atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; loweralkenyl having from 2-10 carbon atoms, cycloalkenyl having from 3 to 10 carbon atoms, cycloalkenylalkyl wherein the cycloalkenyl moiety comprises 3 to 10 carbon atoms; and the alkyl moiety comprises 1 to 6 carbon atoms; alkynyl having from 2 to 10 carbon atoms; aryl such as phenyl and naphthyl; arylalkyl and alkylaryl such as benzyl, phenethyl and the like; heteroalkyl, alkylheteroalkyl, arylheteroarlky and alkylheteroaryl wherein the hetero atoms are selected from the group of sulfur, oxygen and nitrogen, and the alkyl moiety has 1 to 6 carbon atoms, and the aryl moiety is phenyl; heterocyclyl (saturated and unsaturated) comprising mono- and bicyclic structures having 5 to 10 ring atoms wherein one or more of the hetero atoms is selected from oxygen, nitrogen, or sulphur such as thiophene, imidazole, pyridine, furyl and the like; heterc-yclyalkyl wherein the heterocyclyl moiety comprises from 3 to 10 atoms and the alkyl moiety comprises from 1 to 6 atoms; the substituent (or substituents) relative to the above-named radicals are selected from the group consisting of amino, hydroxyl, cyano, carboxyl, nitro, chloro, bromo, fluoro, alkoxy having from 1 to 6 carbon atoms, mercapto, perhaloloweralkyl such as trifluoromethyl and alkylthio having from 1-6 carbon atoms.

A particularly preferred class of connecting groups "A" are selected from: a single bond connecting the sulfur and carbamimidoyl function; substituted and unsubstituted: straight and branched loweralkyl having from 1 to 6 carbon atoms, cycloalkyl having from 3 to 6 carbon atoms; phenyl; heterocyclyl such as thiophene, imidazole, pyridine, and furane; alkylheteroalkyl wherein alkyl moiety comprises 1 to 3 carbon atoms and the hetero atoms are sulfur, oxygen and nitrogen; the substituents relative to the above-named radicals are: amino, hydroxyl, chloro, bromo, fluoro, cyano, carboxyl alkoxy having from 1 to 3 carbon atoms, mercapto, trifluoromethyl, and alkylthio having from 1 to 3 carbon atoms.

Representative examples of such preferred -SR⁸ groups (represented as HSR⁸) are:

### EXAMPLES

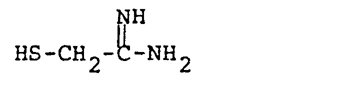

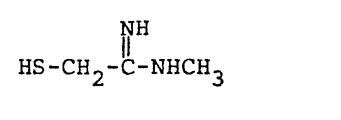

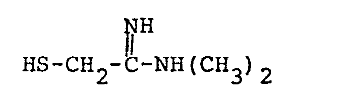

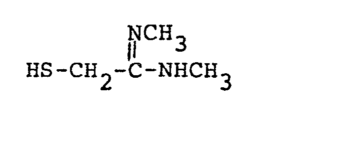

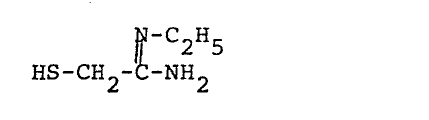

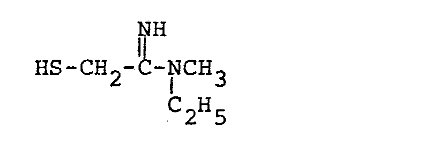

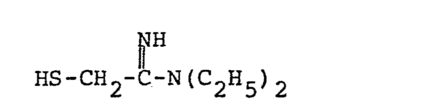

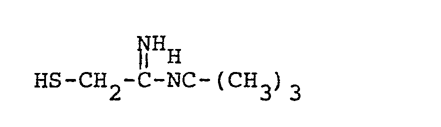

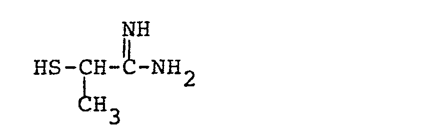

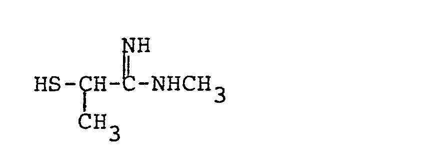

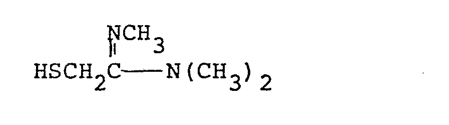

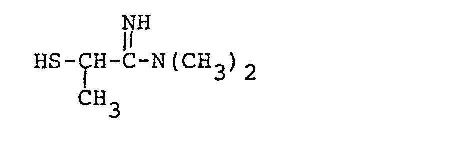

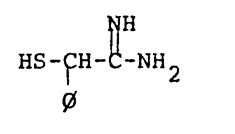

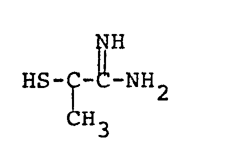

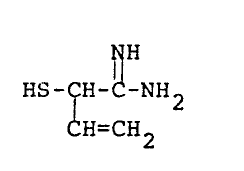

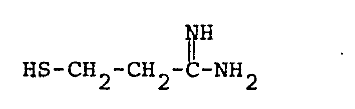

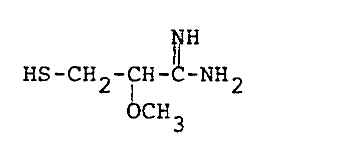

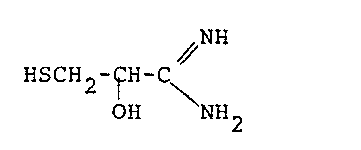

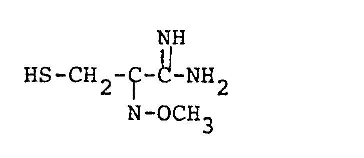

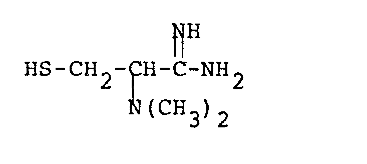

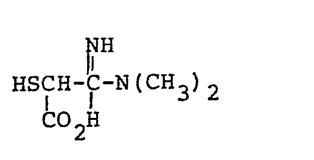

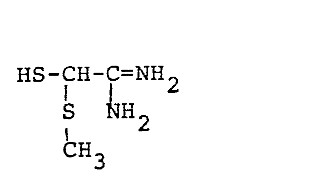

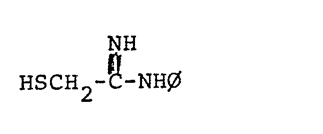

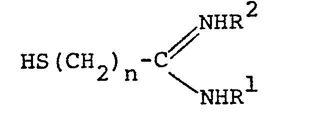

ₙ = 2-5, R² = H, CH₃ R¹ = H, ^{CH}₃ 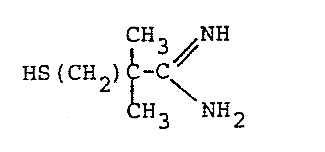 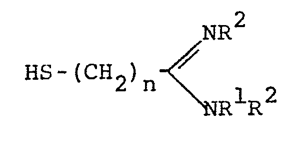 n = 2-5, R¹, R² = H, CH₃ 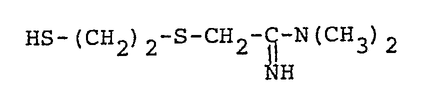 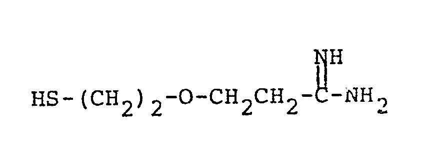 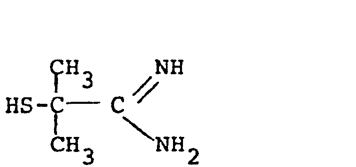 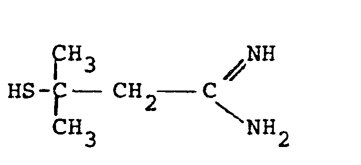 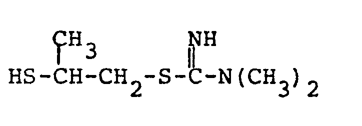 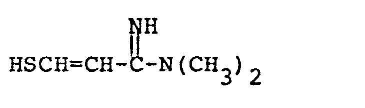 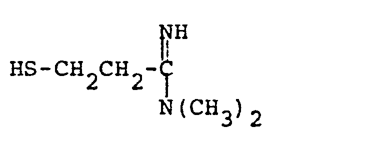 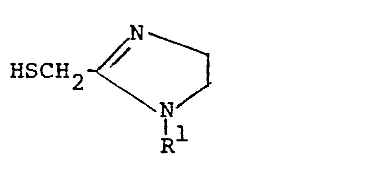 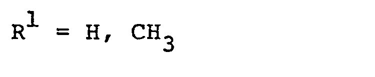 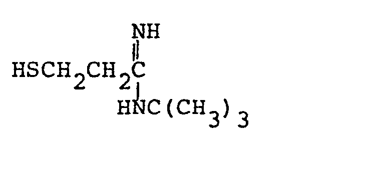 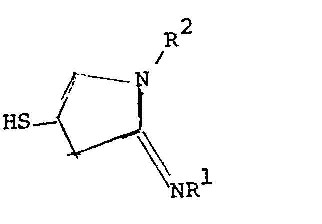 R¹ = H, ^{CH}₃ R² _{= H}, ^{CH}₃ 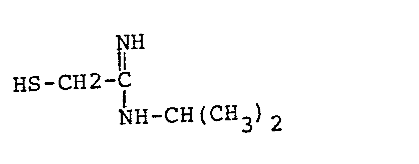 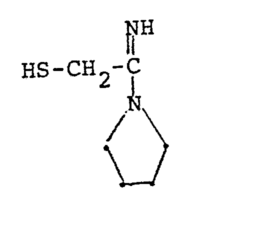 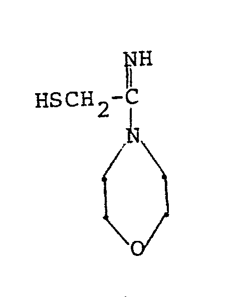 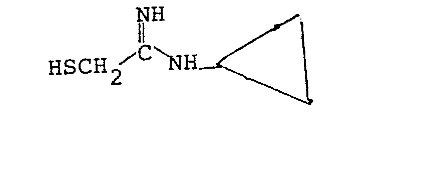 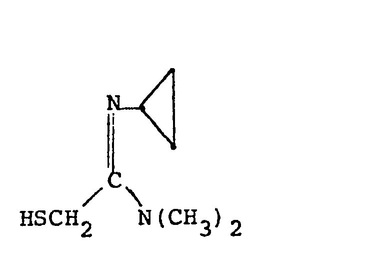 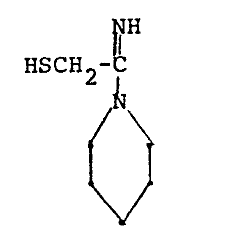 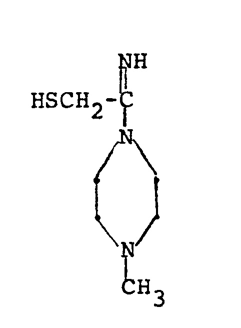 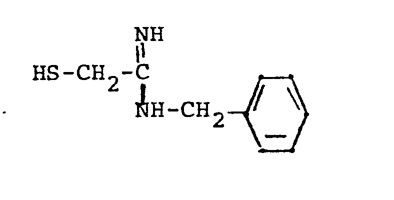 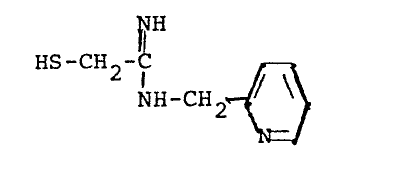 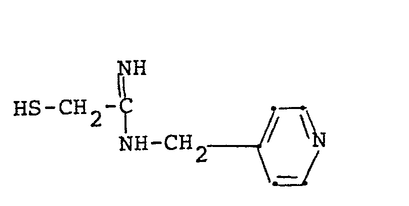 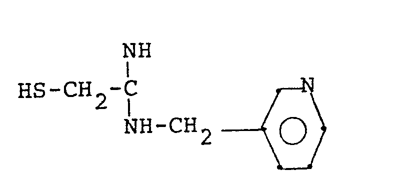 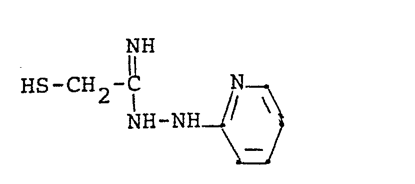 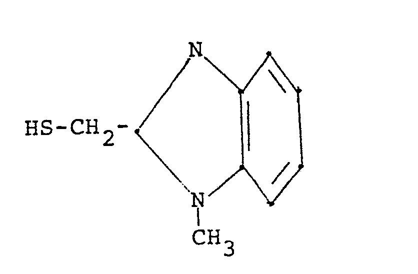 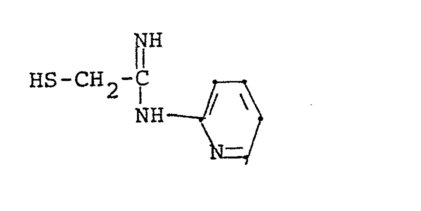 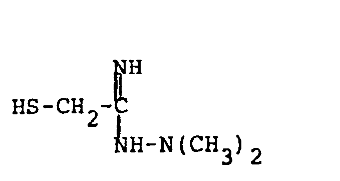 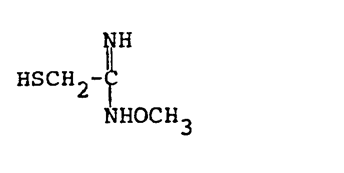 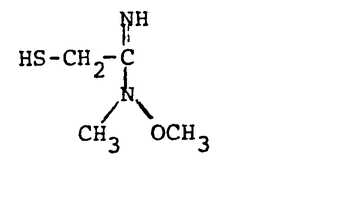 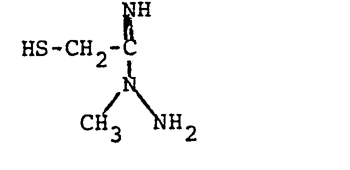 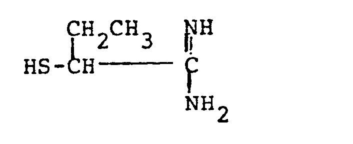 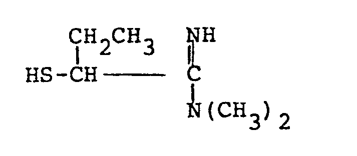 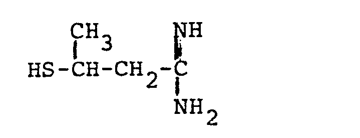 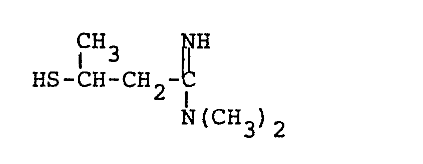 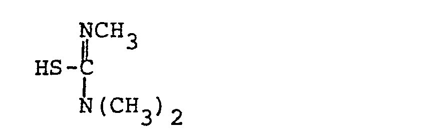 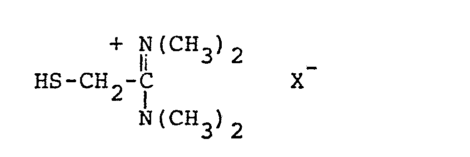 X = any compatible anion 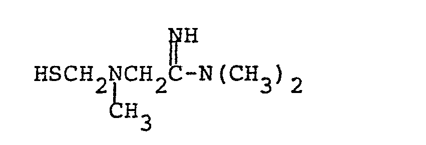 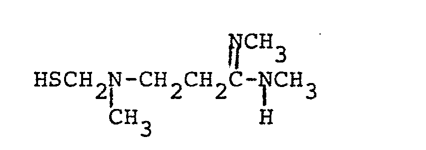 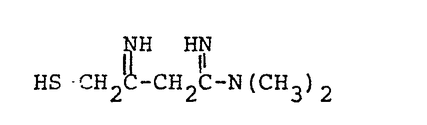 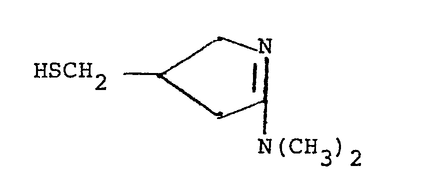 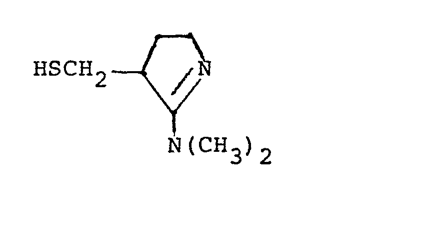 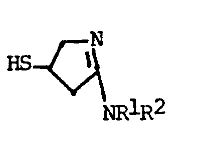 _{R}l = H, CH₃ _{R}2 = H, CH₃ 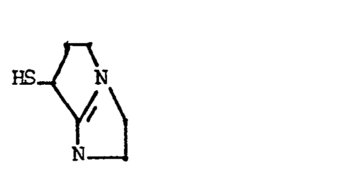 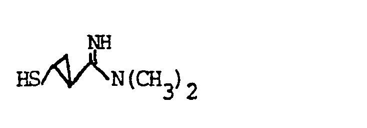 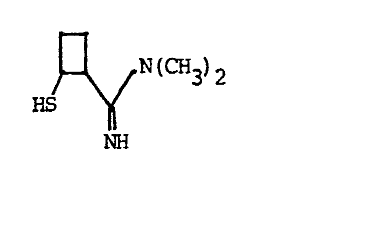 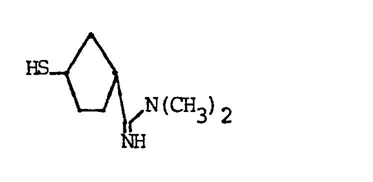 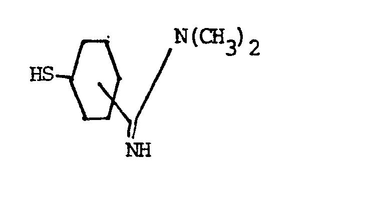 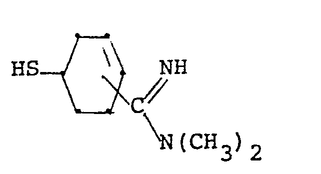 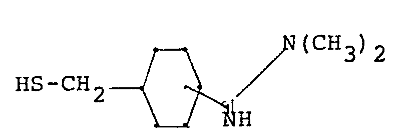 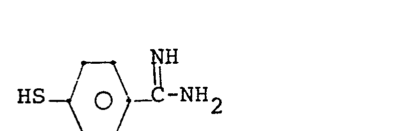 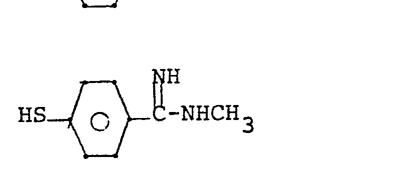 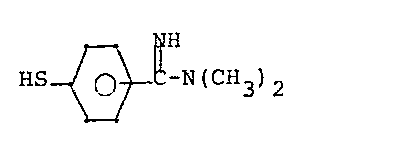 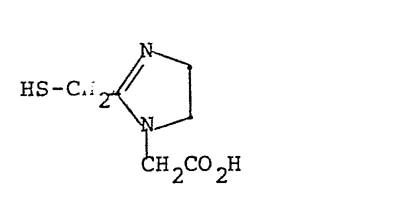 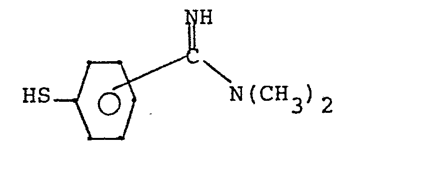 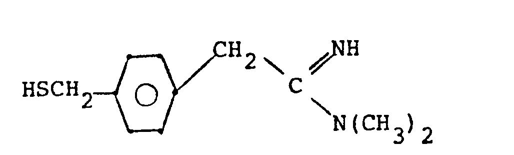 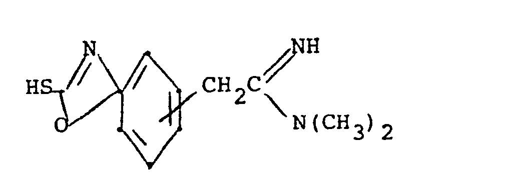 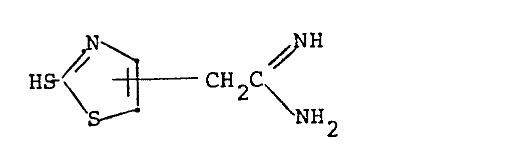 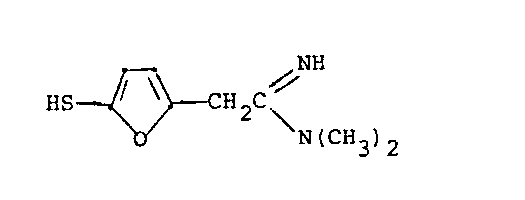 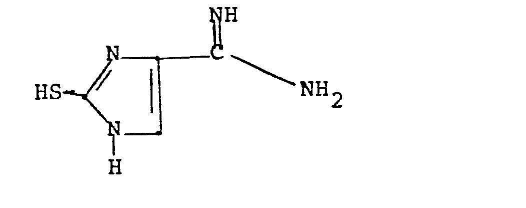 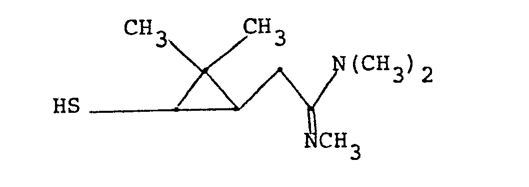 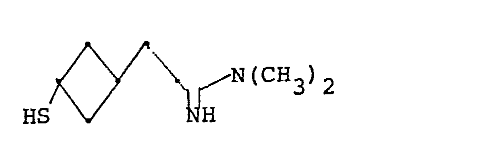 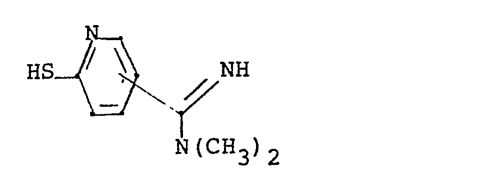 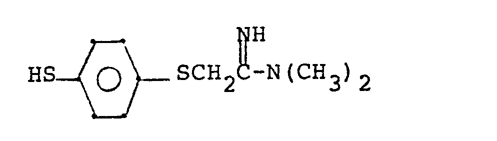 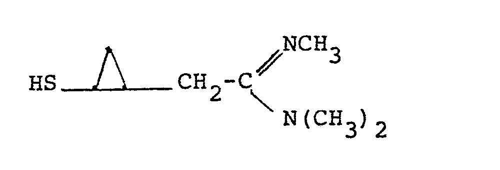 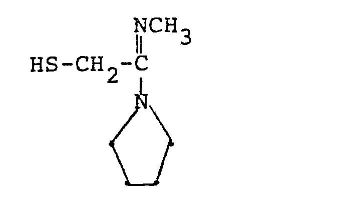 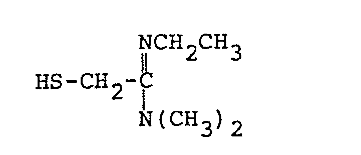 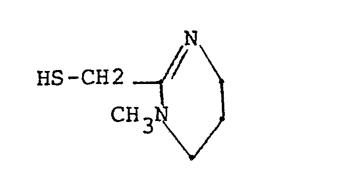 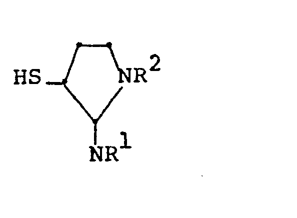 R¹ = H, CH₃ R² = H, CH₃ 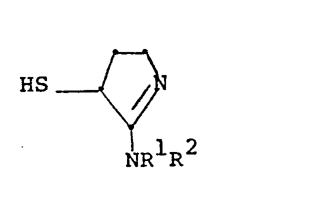 R¹ = H, CH₃ R² = H, CH₃ 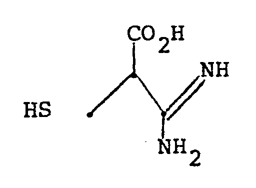 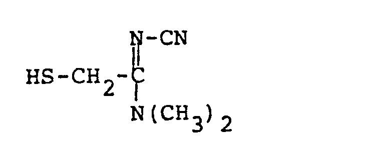 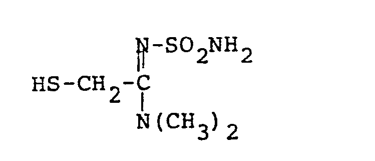 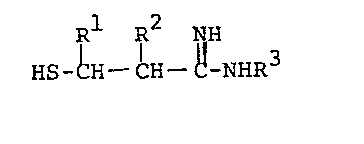 R¹ = C^{H}3 _{R}² = CH₃, N(CH₃)2, ^{OC}H₃ R³ = H, C^{H}₃ 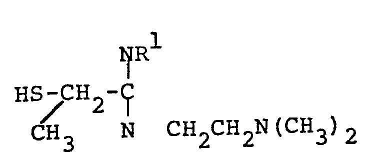 R¹ = H, C^{H}₃ 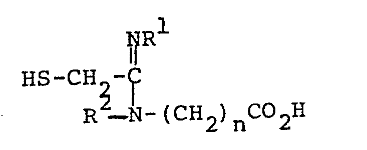 R¹ = H, ^{CH}₃ R² = H, ^{CH}₃ n = 1 or 2 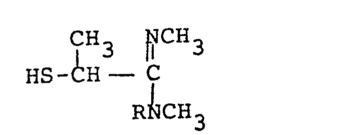 _{R} = H, ^{CH}₃ 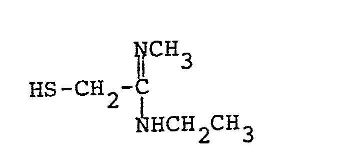 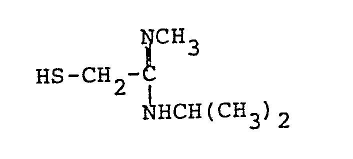 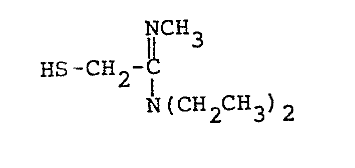 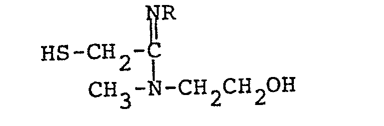 R ₌ CH₂CH₃, ^{CH}₃, ^{H} 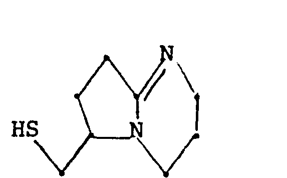 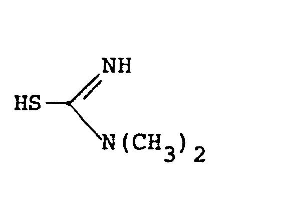 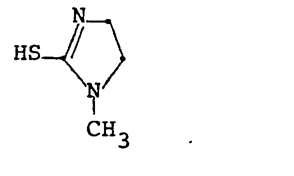 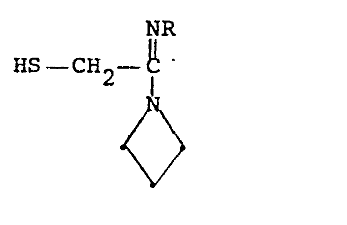 _{R} = H, ^{CH}₃

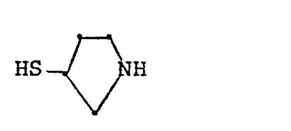

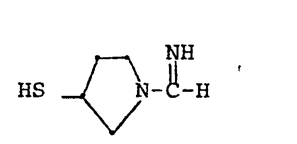

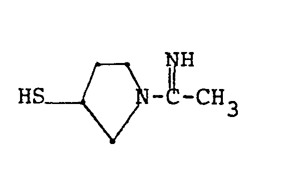

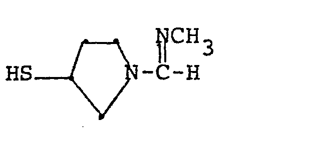

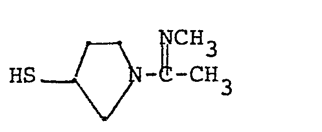

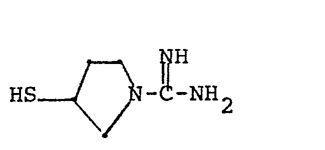

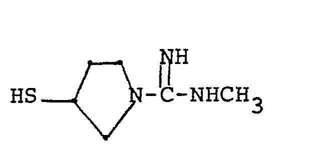

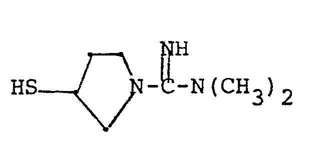

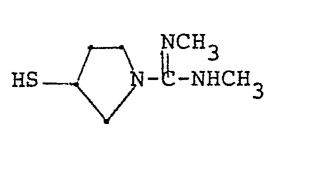

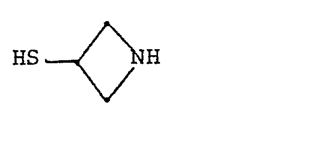

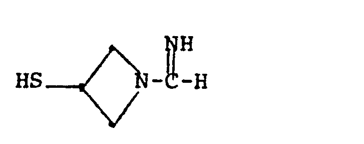

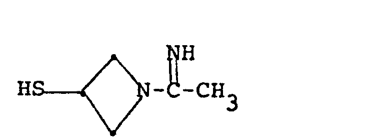

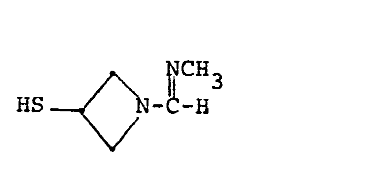

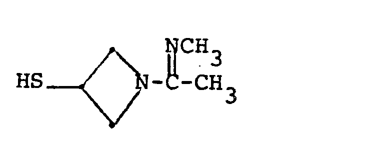

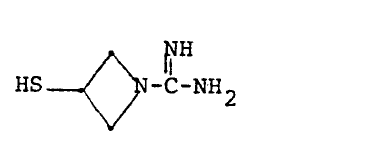

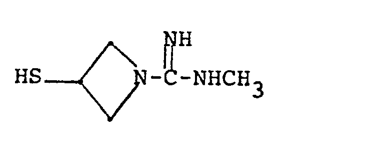

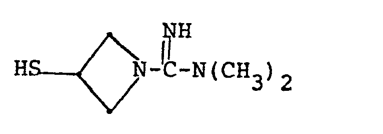

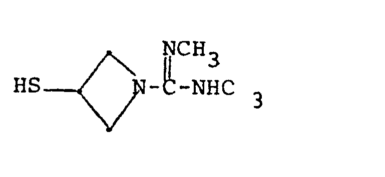

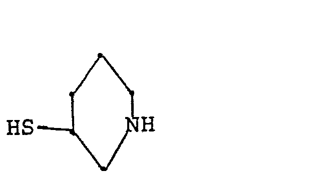

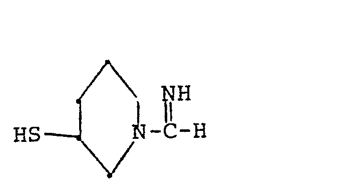

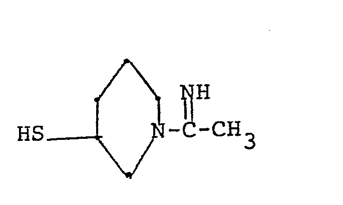

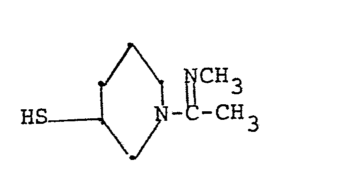

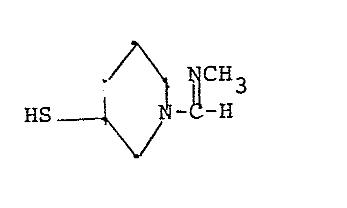

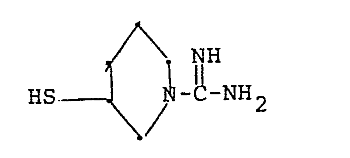

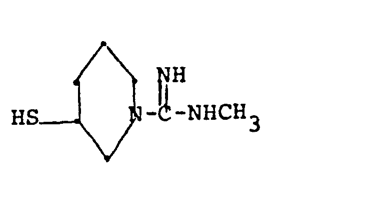

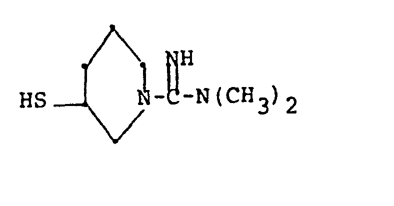

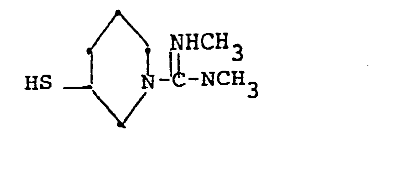

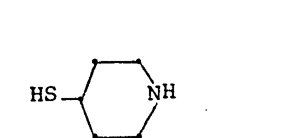

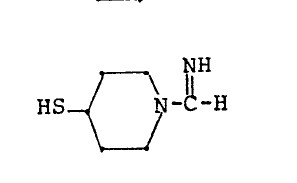

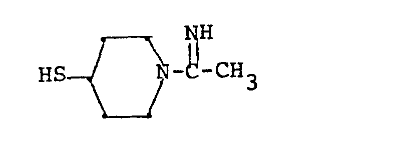

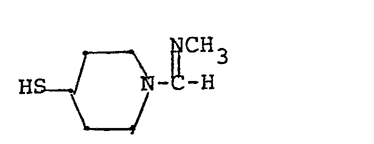

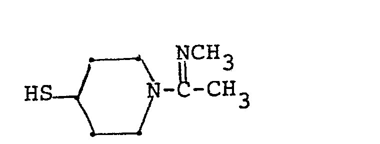

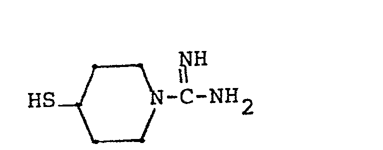

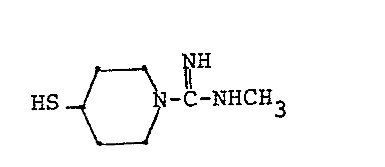

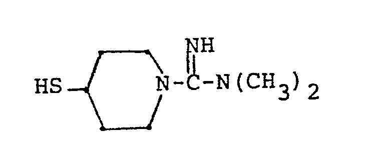

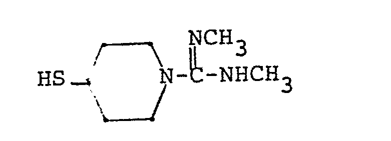

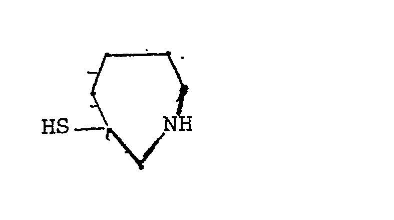

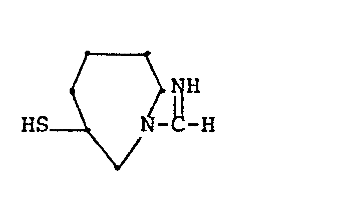

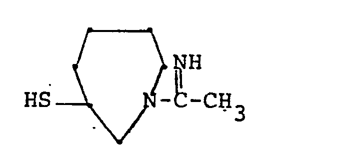

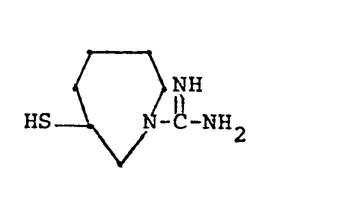

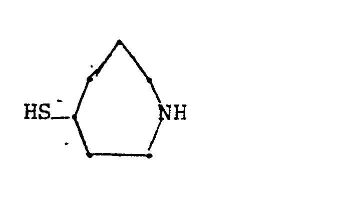

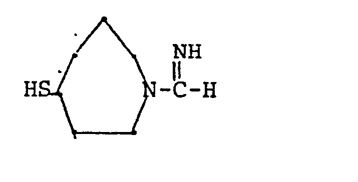

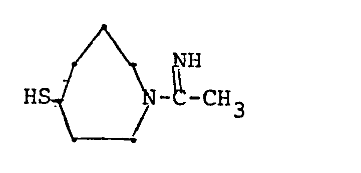

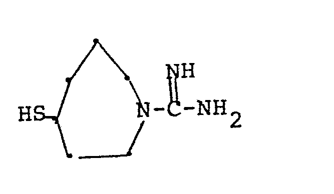

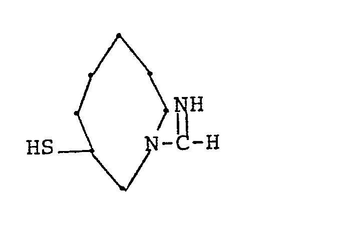

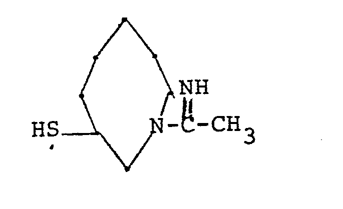

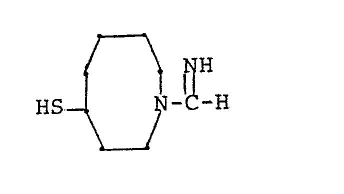

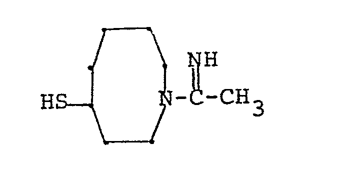

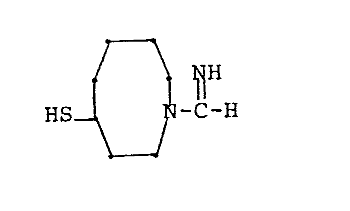

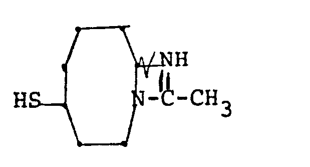

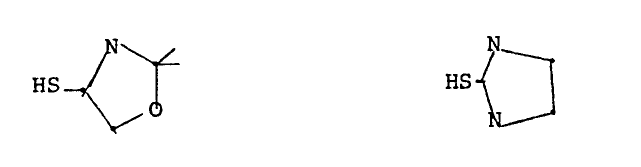

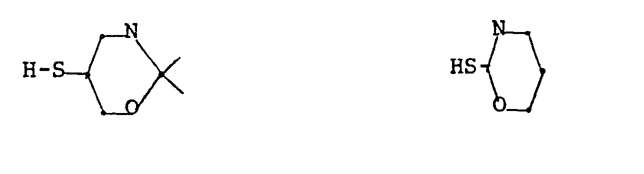

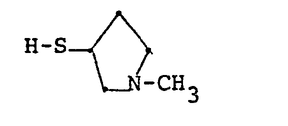

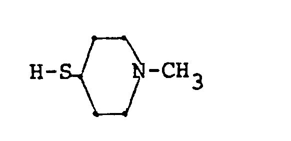

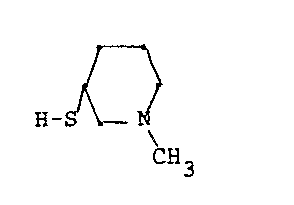

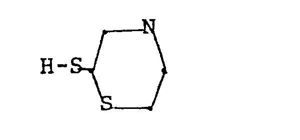

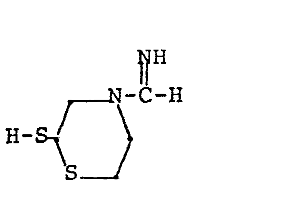

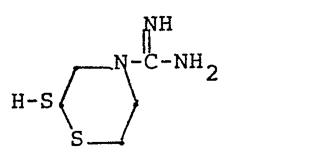

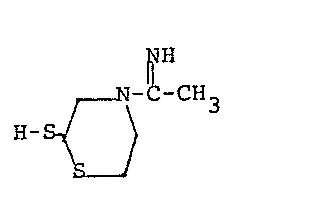

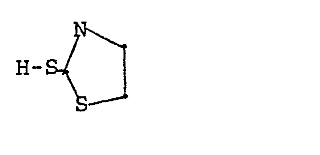

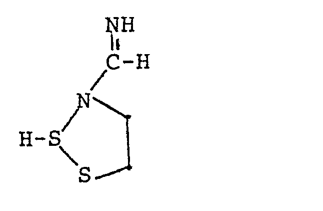

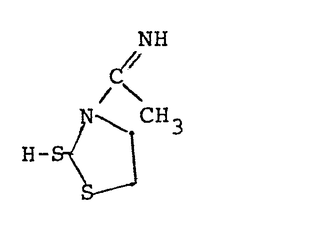

### ADDITIONALLY PREFERRED -SR⁸ VALUES

The following HSR⁸ reagents establish -SR⁸ substituents which are characterized by their lack of strong polarity, and are additionally preferred.

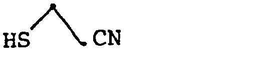

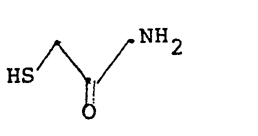

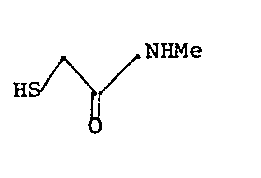

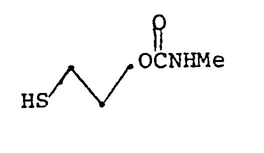

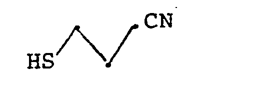

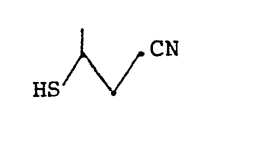

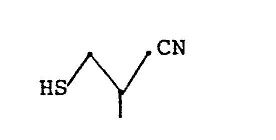

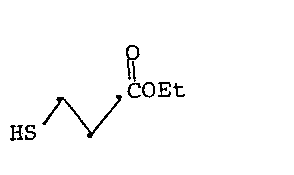

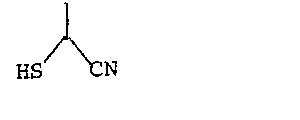

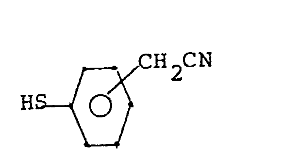

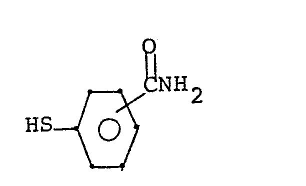

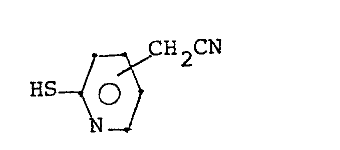

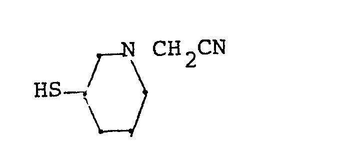

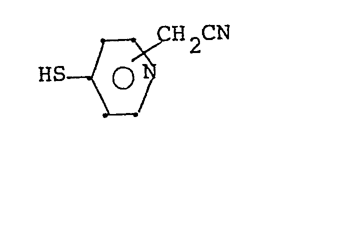

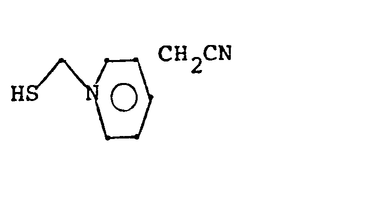

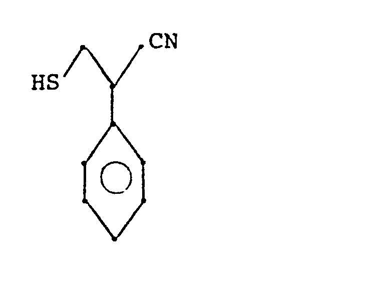

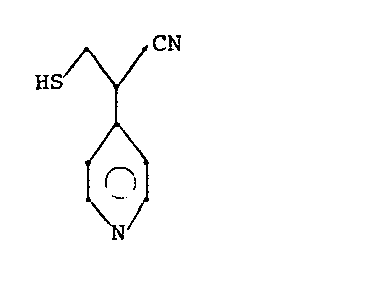

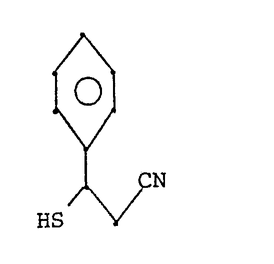

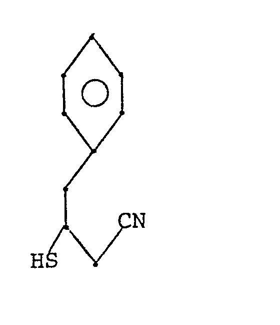

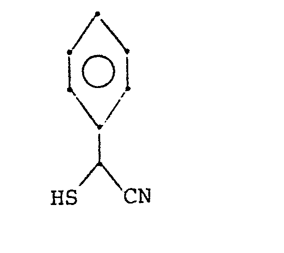

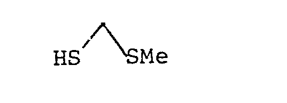

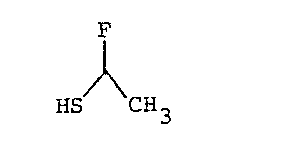

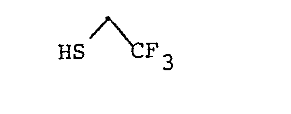

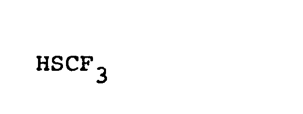

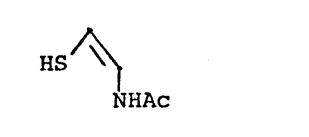

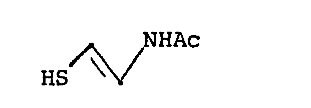

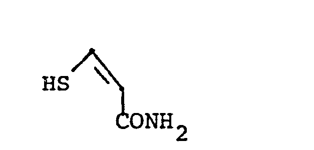

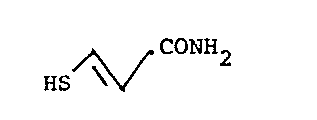

Again it is to be recognized that SR side chains in which the R⁸ group contains one or more chiral centers can be added as racemic or diastereomeric mixtures to provide mixtures of diastereomeric products or can be added as resolved, isomerically pure reagents to provide diastereomerically pure products. Since antibacterial activity and other pharmacological properties vary among isomers, it is frequently advantageous to prepare isomerically pure products by the introduction of resolved -SR⁸ side chains.

As noted above, the compounds of the present invention may also generally be represented by the following structural formula: 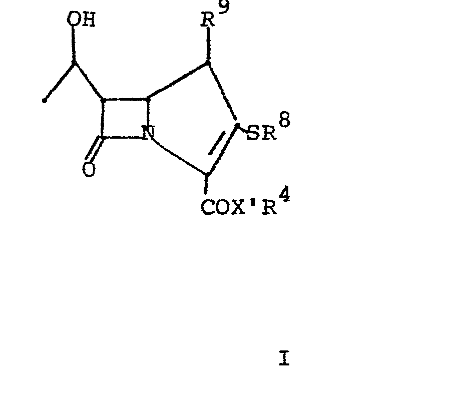 wherein X' is oxygen, sulfur or NR' (R' is hydrogen or loweralkyl having from 1 to 6 carbon atoms); and R⁴ is hydrogen, or, inter alia, is representatively selected to provide the pharmaceutically acceptable salt, ester, anhydride (R⁴ is acyl), and amide moieties known in the bicyclic 8-lactam antibiotic art; R⁴ may also be a readily removable blocking group or a synthetic useful salt moiety. A synthetically useful salt moiety consists of a highly lipophilic carboxylate cation which imparts organic solubility to the molecule. This type of carboxylate derivative allows reactions, and particularly the displacement reaction of II to I, to be conducted in an organic solvent. Representative examples of such highly lipophilic carboxylate cations R⁴ are ammonium salts R^{a}₄N⁺ wherein R^{a} are independently selected from 1-16 carbon alkyl groups or 7 to 10 carbon aralkyl groups. A particularly useful example of this type is the N,N-dimethyl-N-benzyl-N-hexadecyl ammonium salt.

### Identification of the Radical -_{COX}'_{R}⁴

In the generic representation of the compounds of the present invention (I, above), the radical represented by -COX^{I}R⁴ is, inter alia, -COOH (X' is oxygen and R⁴ is hydrogen) and all radicals known to be effective as pharmaceutically acceptable ester, anhydride (R⁴ is acyl) and amide radicals in the bicyclic β-lactam antibiotic art, such as the cephalosporins and penicillins and nuclear analogues thereof.

Suitable, but representative, blocking esters R⁴ (X'= O) include those selected from the following list which is representative:
(ᵢ) _{R}⁴ = CR^{a}R^{b}R^{c} wherein at least one of _{R}^{a}, _{R}^{b}, and R^{c} is an electron-donor, e.g., p-methoxyphenyl. The remaining _{R}^{a}, _{R}^{b} and R^{c} groups may be hydrogen or organic substituting groups. Suitable ester groups of this type include p-methoxybenzyloxycarbonyl.
(ii) _{R}^{4 =} CR^{a}R^{b}R^{c} wherein at least one of _{R}^{a}, R^{b} and R^{c} is an electron-attracting group, e.g., p-nitrophenyl, trichloromethyl, and o-nitrophenyl. Suitable esters of this type include p-nitrobenzyloxycarbonyl, 2,2,2-trichloroethoxy- carbc yl, and acetonyloxycarbonyl.
(iii) _{R} = CR^{a}R^{b}R^{c} wherein at least two of R^{a}, R^{b} and R^{c} are hydrocarbon such as alkyl, e.g., methyl or ethyl, or aryl, e.g., phenyl and the remaining R^{a}, R^{b} and R^{c} group, if there is one, is hydrogen. Suitable esters of this type include t-butyloxycarbonyl, diphenylmethoxycarbonyl, triphenylmethoxycarbonyl, and allyloxycarbonyl.

Silyl esters. This category of blocking groups, may conveniently be prepared from a halosilane of the formula: R^{4°}₃ SiX° wherein X° is a halogen such as chloro or bromo and R^{4'} is independently chosen from: alkyl, having 1-6 carbon atoms, phenyl, or phenylalkyl. Suitable esters of this type include t-butyldiphenylsilylcarbonyl.

Pharmaceutically acceptable carboxyl derivatives of the present invention are those derived by reacting I with alcohols, acylating reagents and the like. For example, esters and amides of interest are the above-listed starting materials and final products having the -COX'R⁴ grout at the 3-position; wherein X' is oxygen, sulfur or N_{R}' (R' is H or R⁴), and R⁴ is alkyl having 1-6 carbon atoms, straight or branched, such as methyl, ethyl, t-butyl, and the like; carbonylmethyl, including phenacyl; aminoalkyl including 2-methylaminoethyl, 2-diethylaminoethyl; alkanoyloxyalkyl wherein the alkanoyloxy portion is straight or branched and has 1-6 carbon atoms and the alkyl portion has 1-6 carbon atoms, such as pivaloyloxymethyl; haloalkyl wherein halo is chloro, and the alkyl portion is straight or branched having 1-6 carbon atoms, e.g., 2,2,2-trichloroethyl; alkenyl having 1-4 carbon atoms, such as 2-propenyl, 3-butenyl, and 4-butenyl; aralkyl and lower alkoxyl-and nitro- substituted aralkyl such as benzyl, benzhydryl, o-nitrobenzyl, p-methoxybenzyl, and p-nitrobenzyl; phthalidyl; benzyloxyalkyl having 8-10 carbon atoms, such as benzyloxymethyl, and (4-nitro) benzyloxymethyl.

In addition to the esters (and thio esters) listed above, amides are also embraced by the present invention, i.e., wherein X' is the -NR'- group. Representative of such amides are those wherein R' is selected from the group consisting of hydrogen and alkyl such as methyl and ethyl.

The most preferred -COX^{I}R⁴ radicals of the present invention are those wherein (relative to Structure I above), X' is oxygen and R⁴ is hydrogen; loweralkyl having 1-4 carbon atoms; lower alkenyl such as 3-methylbutenyl, allyl, 2,2,2-trichloroethyl, 4-butenyl and the like; benzyl and substituted benzyl such as o-nitrobenzyl, p-nitrobenzyl; pivaloyloxymethyl, 3-phthalidyl; phenacyl, acetonyl; and triorganosilyl, such as trimethylsilyl.

Removal of the preferred protecting groups is categorized as follows: a.) for hydroxyl and amino functionalities bearing p-nitrobenzyloxycarbonyl protection or a carboxyl functionality possessing a p-nitrobenzyl moiety deprotection is accomplished by catalytic hydrogenation over a transition metal catalyst such as palladium supported on carbon, palladium hydroxide on carbon, or plantinum oxide, in an inert solvent or solvent mixtures, which maybe buffered in the usual way, such as tetrahydrofuran, dioxane, ethyl acetate, ethanol, and water, at a temperature of from 0°C to ambient temperature, at a pressure of from 1 atmosphere to 5 atomospheres of hydrogen, for a period of from a few minutes to twelve hours; b.) for hydroxyl functions covered by a t-butyldimethylsilyl (TBDMS) group removal is accomplished according to the procedure of G. Just and T. J. Liak, Can. J. Chem., 56, 211 (1978), which comprises treating the TBDMS ether derivative with N-tetrabutyl ammonium fluoride in the presence of acetic acid in an inert solvent such as tetrahydrofuran at a temperature of from -78°C to ambient temperature for from a few minutes to 72 hours; c.) for carboxyl moieties possessing an allyl moiety and for hydroxyl and amino groups bearing an allyloxycarbonyl function deprotection is accomplished by treatment with a combination of triphenyl phosphine, tetrakistriphenylphosphine palladium (0), and 2-ethylhexanoic acid or its sodium or potassium salts in a suitable aprotic solvent such as ethylacetate, methylene chloride, tetrahydrofuran, or diethylether. Use of either sodium or potassium 2-ethylhexanoate provides the corresponding salt; whereas 2-ethylhexanoic acid provides the free carboxylic acid. The process for removing an allylic group from allylic esters, carbonates and carbamates is described in European Patent Application 13,633 (_{S}chering Corp.) and in S.W. McCombie, et al., Tetrahedron Letters, 22, 3489 (1981).

Relative to the generic description of the compounds of the present invention. I: 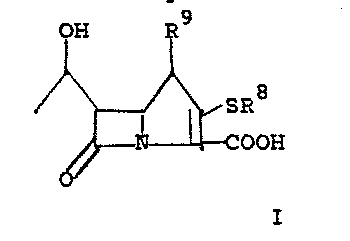 the following representative drawings depict the most preferred configuration about atoms 5, 6 and 8, which is 5S, 65, 8R:

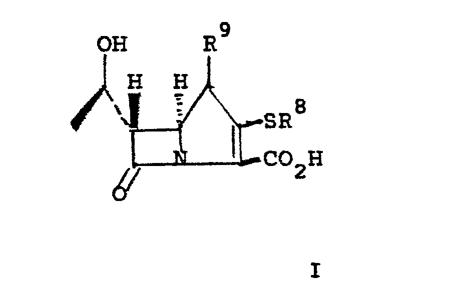

The compounds of the present invention (I) are valuable antibiotics active against various Gram-positive and Gram-negative bacteria and accordingly find utility in human and veterinary medicine. Representative pathogens which are sensitive to antibiotics I include: Staphylococcus aureus, Escherichia coli, Klebsiella pneumoniae, Bacillus subtilis, Salmonella typhosa, Psuedomonas and Bacterium proteus. The antibacterials of the invention are not limited to utility as medicaments; they may be used in all manner of industry, for example: additives to animal feed, preservation of food, disinfectants, and in other industrial systems where control of bacterial growth is desired. For.example, they may be employed in aqueous compositions in concentrations ranging from 0.1 to 100 parts of antibiotic per million parts of solution in order to destroy or inhibit the growth of harmful bacteria on medical and dental equipment and as bactericides in industrial applications, for example in waterbased paints and in the white water of paper mills to inhibit the growth of harmful bacteria.

The products of this invention may be used in any of a variety of pharmaceutical preparations. They may be employed in capsule, powder form, in liquid solution, or in suspension. They may be administered by a variety of means; those of principal interest include: orally, topically or parenterally by injection (intravenously or intramuscularly).

Such tablets and capsules, designed for oral administration, may be in unit dosage form, and may contain conventional excipients, such as binding agents, for example, syrup, acacia, gelatin, sorbitol, tragacanth, or polyvinylpyrrolidone; fillers, for example, lactose, sugar, cornstarch, calcium phosphate, sorbitol, or glycerine; lubricants, for example, magnesium stearate, talc, polyethylene glycol, silica; disintegrants, for example, potato starch, acceptable wetting agents such as sodium lauryl sulphate. The tablets may be coated according to methods well known in the art. Oral liquid preparations may be in the form of aqueous or oily suspensions, or solutions, or they may be presented as a dry product for reconstitution with water or other suitable vehicle before use. Such liquid preparations may contain conventional additives such as suspending agents, for example, sorbitol, methyl cellulose, glucose/sugar syrup, gelatin, hydroxyethylcellulose, or carboxymethyl cellulose. Suppositories will contain conventional suppository bases, such as cocoa butter or other glycerides.

Compositions for injection, the preferred route of delivery, may be prepared in unit dosage form in ampules, or in multidose containers. The compositions may take such forms as suspensions, solutions, or emulsions in oily or aqueous vehicles, and may contain formulatory agents. Alternatively, the active ingredient may be in powder form for reconstitution, at the time of delivery, with a suitable vehicle, such as sterile water.

The compositions may also be prepared in suitable forms for absorption through the mucous membranes of the nose and throat or bronchial tissues and may conveniently take the form of liquid sprays or inhalants, lozenges, or throat paints. For medication of the eyes or ears, the preparation may be presented in liquid or semi-solid form. Topical applications may be formulated in hydrophobic or hydrophilic bases as ointments, creams, lotions, paints, or powders.

The dosage to be administered depends to a large extent upon the condition and size of the subject being treated as well as the route and frequency of administration -- the parenteral route by injection being preferred for generalized infections. Such matters, however, are left to the routine discretion of the therapist according to principles of treatment well known in the antibiotic art. In general, a daily dosage consists of from about 5 to about 600 mg of active ingredient per kg. of body weight of the subject in one or more treatments per day. A preferred daily dosage for adult humans lies in the range of from about 10 to 240 mg. of active ingredient per kg. of body weight. Another factor influencing the precise dosage regimen, apart from the nature of the infection and peculiar identity of the individual being treated, is the molecular weight of the chosen species of this invention (I).

The compositions for human delivery per unit dosage, whether liquid or solid, may contain from 0.1% to 99% of active material, the preferred range being from about 10-60%. The composition will gen -ally contain from about 15 mg. to about 1500 mg. of the active ingredient; however, in general, it is preferable to employ a dosage amount in the range of from about 250 mg to 1000 mg. In parenteral administration, the unit dosage is usually the pure compound I in sterile water solution or in the form of a soluble powder intended for solution.

The preferred method of administration of the formula I antibiotic is parenteral by i.v. infusion, i.v. bolus, or i.m. injection.

For adults, 5-50 mg. of Formula I antibiotic per kg. of body weight given 2, 3, or 4 times per day is preferred. Preferred dosage is 250 mg. to 1000 mg. of the Formula I antibiotic given two (b.i.d.) three (t.i.d.) or four (q.i.d.) times per day. More specifically, for mild infections, and particularly urinary tract infections, a dose of 250 mg. t.i.d. or q.i.d. is recommended. For moderate infections against highly susceptible gram positive and gram negative organisms, a dose of 500 mg. t.i.d. or q.i.d. is recommended. For severe, life-threatening infections against organisms at the upper limits of sensitivity to the antibiotic, a dose of 1000 t.i.d. or q.i.d. is recommended.

For children, a dose of 5-25 mg/kg of body weight given 2. 3, or 4 times per day is preferred; a dose of 10 mg/kg t.i.d. or q.i.d. is usually recommended.

Antibiotic compounds of Formula I are of the broad class known as carbapenems or 1- carbadethiapenems. Certain of these carbapenems are susceptible to attack by a renal enzyme known as dehydropeptidase (DHP). This attack or degradation may reduce the efficacy of the carbapenem antibiotic. Inhibitors of DHP and their use with carbapenem antibiotics are disclosed in the prior art [see published European Patent Applications No. 79102615.6, filed July 24, 1979 (application no. 15573) and No. 82107174.3, filed August 9, 1980 (application no. 72014)].

The present I compounds may, where DHP inhibition is desired or necessary, be combined or used with the appropriate DHP inhibitor as described in the aforesaid published applications. Thus, to the extent that the cited European patent applications 1.) define the procedure for determining DHP susceptibility of the present carbapenems and 2.) disclose suitable inhibitors, combination compositions and methods of treatment, they are incorporated herein by reference. A preferred weight ratio of I compound: DHP inhibitor in the combination compositions is about 1:1. A preferred DHP inhibitor is 7-(L-2-amino-2-carboxyethylthio)-2-(2,2-dimethylcyclopropanecarboxamido)-2-heptenoic acid or a useful salt thereof.

These combination compositions and their use is another embodiment of the present invention.

In the foregoing word description, it is to be understood that there is considerable latitude in selection of precise reaction parameters. Suggestion of this latitude and its breadth is generally indicated by the enumeration of equivalent solvent systems. temperature ranges, protecting groups, and range of identities of involved reagents.

The following examples recite a precise scheme of synthesis. It is to be underst jd that the purpose of this recitation is to further illustrate the invention and not to impose any limitation. Temperature is in °C.

### Synthesis of 1-OH Compounds

### EXAMPLE 1

### Step A:

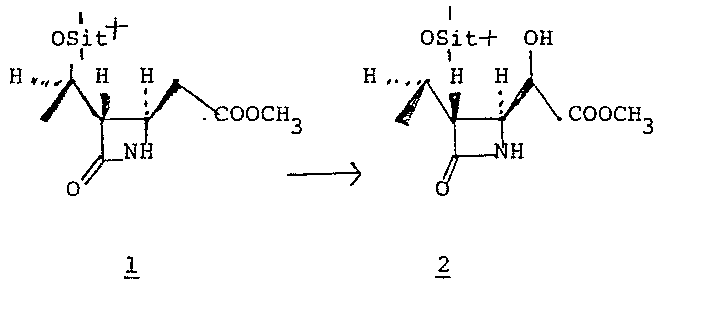

Tetrahydrofuran (THF, 20 ml) was cooled to -78° under N₂, diisopropylamine (1.2 ml) was added followed by nBuLi (3.6 ml of a 2.3 M solution). The mixture was stirred at -78° for 10 min. HMPA (1.6 ml) is added and the mixture was stirred at -78° for 15 minutes. The methyl ester 1 (1.2 g) dissolved in 10 ml THF was added over 2 minutes and the reaction mixture stirred 0.5 hours at -78°.

Trimethylphosphite (0.5 ml) was then added and the nitrogen atmosphere was replaced by oxygen and the -78° bath was replaced with one at -35°. The mixture was stirred rapidly for 1 hour. Saturated ammonium chloride was added, the organic phase was separated and the aqueous phase extracted with ethyl acetate. The combined organic phase was washed with saturated NaCl, dried over MgSO₄ and evaporated to give a solid residue. Recrystallization from hot ether gave 0.5 g of the desired alcohol 2 (1 isomer). Chromatographic separation of the mother liquor gave unreacted starting material 0.237 g and 0.280 g of alcohol as a mixture of isomers. The major isomer has the following spectral data: n.m.r. (200 MHz ) 0.08 S(CH₃Si); 0.9 S(CH₃-C-Si) 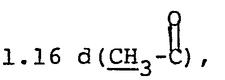 3.2 d of d(C-3H); 3.86 (CH30); 3.9 d of d (C-4H); 4.25 (m, CH₃-C-O, HO-CH-COOCH₃); H 5.85 broad (s, NH).

### Step B:

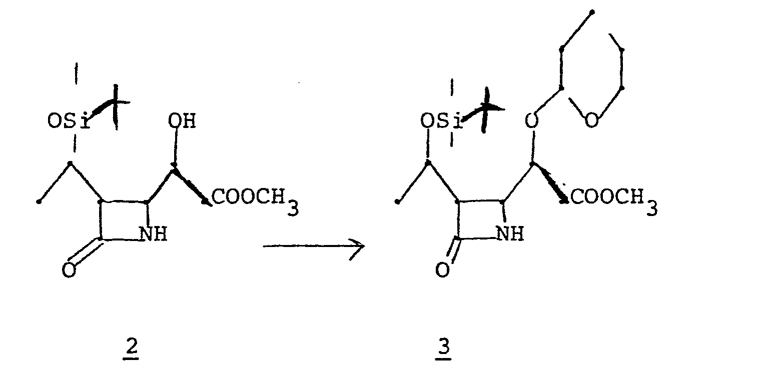

Hydroxy ester 2 (0.317 g) was suspended in 5 ml methylene chloride and treated with 0.168 ml of dihydropyran. p-Toluenesulfonic acid 2 mg was added and the mixture stirred under N₂ for 1 hour. The homogenous mixture was diluted with CH₂Cl₂, washed with 10% NaHC0₃ solution, dried and evaporated to give the tetrahydropyranyl ether 3 as an oil.

### STEP C:

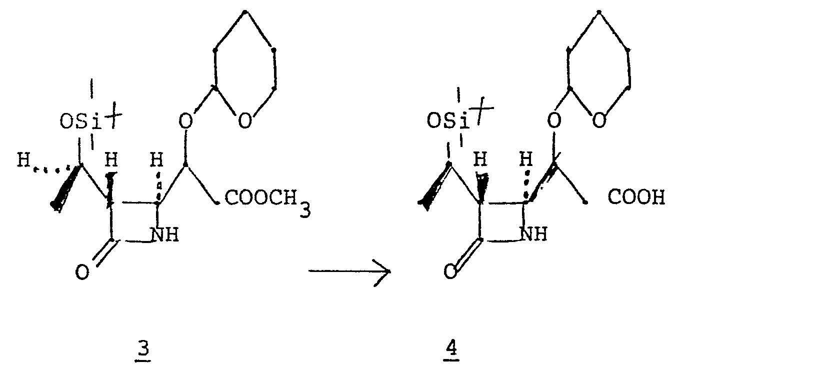

Tetrahydropyranyl ether 3 from the previous step was dissolved in MeOH (4 ml) and treated with 0.44 ml of a 2.5 N NaOH solution. The mixture was stirred at room temperature for 2 hours, then diluted with water and extracted with ether twice. The aqueous phase was acidified and extracted 3 times with EtOAc. The EtOAc extract was dried over MgS0₄ and evaporated to give 0.384 g of the tetrahydropyranyl acid 4 as a foam. n.m.r. (60 MHz, ); 0.09 (CH₃-Si); 0.9 (s, CH3-C-Si); 1.24 (d, CH₃-C) ; 4.8 (m, 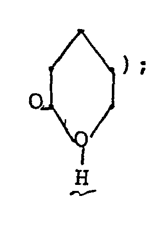 6.73 broad (s, NH).

### Step D:

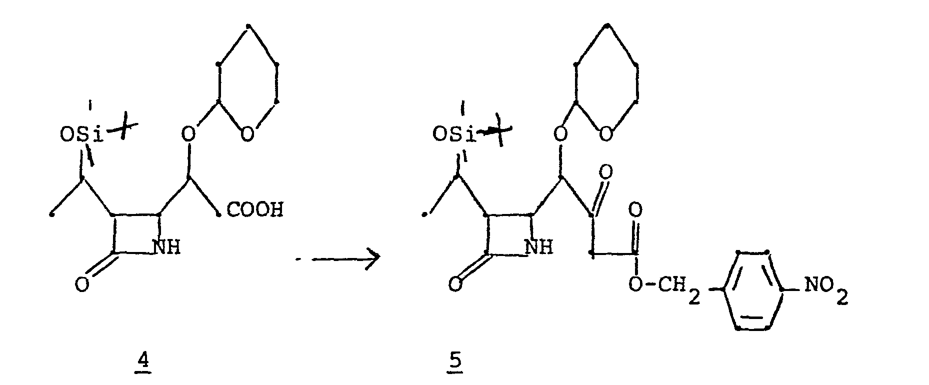

The acid 4 (200 mg) was dissolved in CH₃CN (2 ml) under N₂ and cooled to 0°. Carbonyldiimidazole 92 mg was added and the mixture was stirred at 0° for 1 1/2 hours.

Malonic acid mono-p-nitrobenzyester (247 mg) was dissolved in 4 ml anhydrous THF and cooled to 0° under N₂. Dibutylmagnesium (0.86 ml of a 0.6 M solution in Hexane) was added and the mixture was stirred 1 1/2 hours at 0°. The solvent was removed under reduced pressure and the residue was placed under N₂. To this was added the solution of the imidazolide prepared above. Dimethylformamide (DMF) 0.4 ml was added and the mixture was stirred at 50° overnight. The solvents were removed under reduced pressure, the residue was taken up in EtOAc and 10% HC1 solution. The organic phase was separated and washed with 10% NaHCO₃ solution then dried and evaporated. Chromatography of the residue on silica gel gave 182 mg of product 5 as an oil.

nmr (60 MHz, ): 0.1 (s, CH₃Si); 1.0 (s, CH₃-C-Si); 1.25 (d, 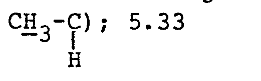 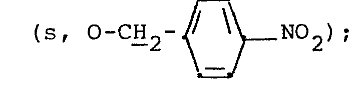 6.02 broad (s, NH); 7.6 and 8.3 (2 d, aromatic protons).

### Step E:

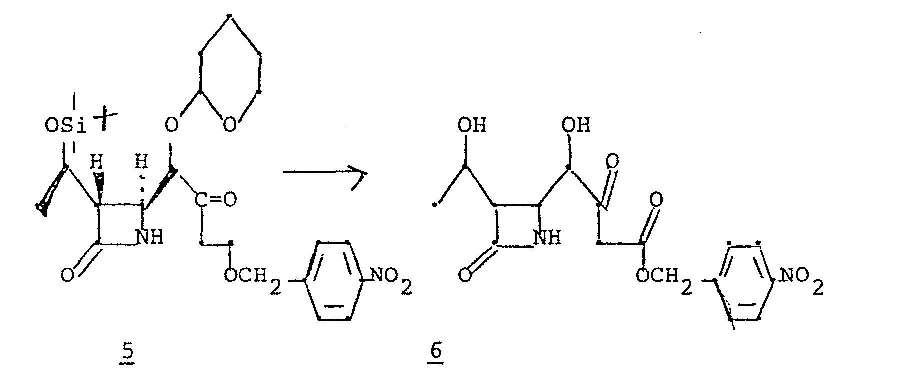

The β-keto ester 5 (182 mg) was dissolved in 4 ml of a solution of 0.1 ml conc. H₂SO₄ in 10 ml MeOH. The mixture was allowed to stand at room temperature for 1 hour. Triethylamine (0.2 ml) was added and the mixture was evaporated to dryness. The residue was the crude dihydroxy product 6 which was used for the next step without purification.

### Step F:

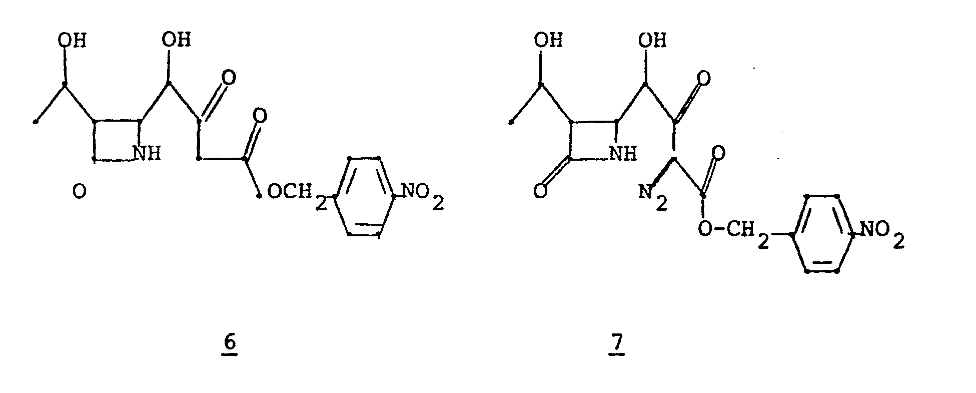

The product from the previous reaction was dissolved in 4 ml CH₃CN and treated with Et₃N (0.046 ml) and p-toluenesulfonylazide (0.064 ml). The mixture was stirred 1 hour at room temperature under N₂. The solvent was removed under reduced pressure and the residue was chromatographed on silica gel using EtOAc as eluant to give 0.040 g of product 7. i.r. (cm⁻¹) 3400 (NH + OH); 2165 (N₂); 1760 (ß-lactam C=0); 1720 (ester and carbonate C=0); 1660 (ketone C=0) ; 1520 (NO₂). n.m.r. (200 MHz, ) 1.26 (d, 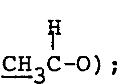 3.09 (d of d C-3H); 2.94 (d of d C-4H); 4.14 (m, 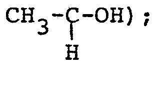 4.86 (d, HO-CH-C=O); 5.4 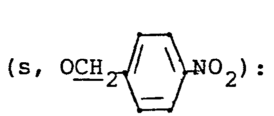 H 5.92 (s, NH); 7.55 and 8.26, (2 d, aromatic H).

### Step G:

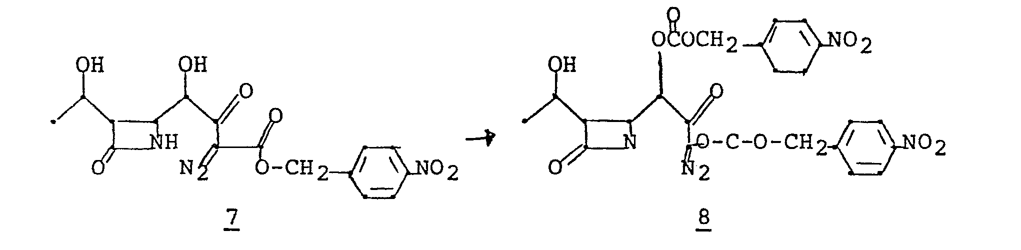

6 7 The dihydroxydiazo compound 7 (40 mg) was dissolved in 1 ml CH₃CN cooled to 0° under N₂ - treated with p-nitrobenzyloxycarbonyl chloride (43 mg) and dimethylaminopyridine (25 mg). The mixture was stirred at 0° for 45 minutes for 15 minutes at room temperature. The reaction mixture was diluted with water and EtOAc, the organic phase was separated and washed with water, dried, and evaporated. The residue was purified by preparative t.l.c. on silica gel using EtOAc as eluant to give the monohydroxy compound 8 (28 mg) as a foam. i.r. (cm⁻¹) 3400 (OH + NH); 2165 (N₂); 1760 (ß-lactam carbonyl); 1720 (ester and carbonate carbonyl) ; 1662 (ketone carbonyl); 1520 (NO₂). n.m.r. (60 MHz, ) 1.13 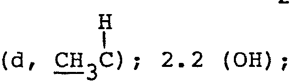 3.3 d of d (C-3H); 4.1 (m, C-4H and 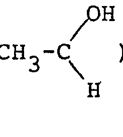 ; 5.3 and 5.4 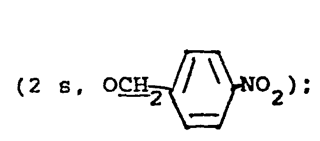 (6.1 (d, H-C-C=O); 6.26 (s, NH); ₇.55 and 8.3 (2 d, aromatic H).

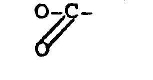

### Step H:

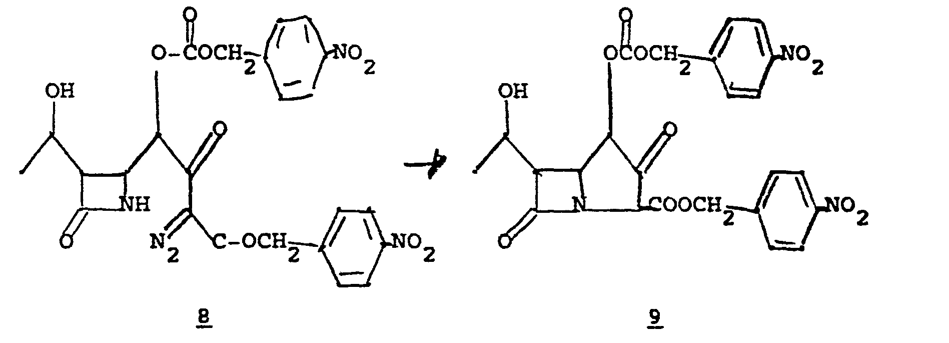

The diazo-B-ketoester 8 (28 mg) was dissolved in toluene (2 ml) and CH₂C1₂ (2 ml) under N₂ and treated with 2 mg Rh(OAc)₂. The mixture was heated at 70° for 1 hour. Infra-red spectrum showed the disappearance of the diazo group. The reaction mixture was diluted with CH₂Cl₂, filtered and evaporated to give the crude cyclic ß-ketoester 9 as a solid which was used without purification in the following step. i.r. (cm 1): 1785 (ß-lactam carbonyl): 1760 (ester and carbonate carbonyl):

### Step I:

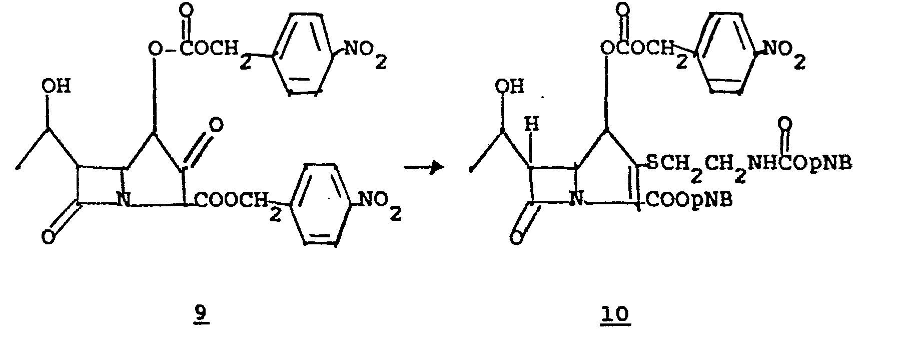

The product from the previous reaction was dissolved in CH₃CN (1 ml) cooled to 0° and treated with diisopropylethylamine (10 pl) and 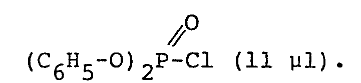 The reaction mixture was stirred at 0° for 1 hour and 15 minutes at room temperature.

The reaction mixture was cooled to 0° and treated wtih diisopropylethylamine (10 ul) and N-p-nitrobenzyloxycarbonylcysteamine (12 mg). The reaction mixture was stirred at 0° for 1 hour. The reaction mixture was diluted with EtOAc and washed with water, 5% NaHC0₃ solution then with water again; dried and evaporated to give 45 mg of the crude product. Purification by preparative t.l.c. on silica gel using 20% CH₂Cl₂ in EtOAc gave the desired product 10 (22 mg). i.r. (cm⁻¹): 3550 and 3350 (OH and NH); 1795 (ß-lactam C=0); 1760 (ester C=0); 1710 (carbonate _{C}=0); 1520 (NO₂). U.V. λ max 266, sh 325.

### Step J:

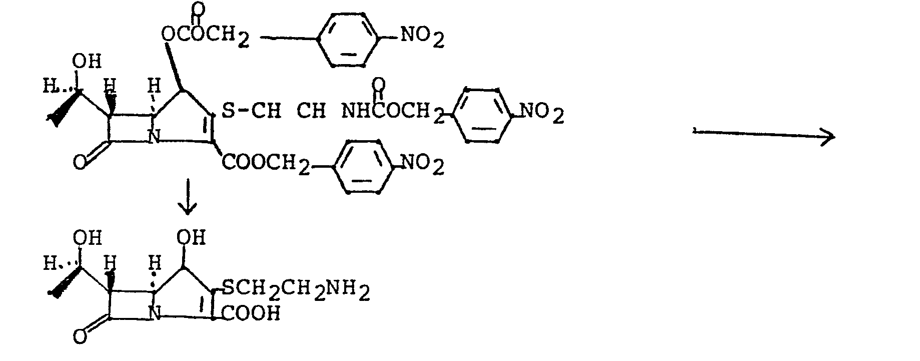

The bicyclic product 10 (5 mg) was dissolved in THF (2 ml), H₂0 1 ml, 0.5 M pH 7 MOPS buffer (0.15 ml), and EtOH (0.2 ml), PtO₂ (5 mg) was added and the mixure was hydrogenated under 40 lbs H₂ pressure for 1 1/2 hours. The catalyst was filtered and washed with water. The filtrate and washings were extracted with EtOAc 1 X and ether 2 X. The aqueous phase was concentrated and lyophilized to give 1-hydroxythienamycin. U.V. max 302.

### EXAMPLE 2

### Step A: Preparation of 1-methylthio-2-(N,N-dimethyl- carbamimidoylmethylthio)-6-(l-hydroxyethyl)-1-carbapen-2-em-3-carboxylic acid

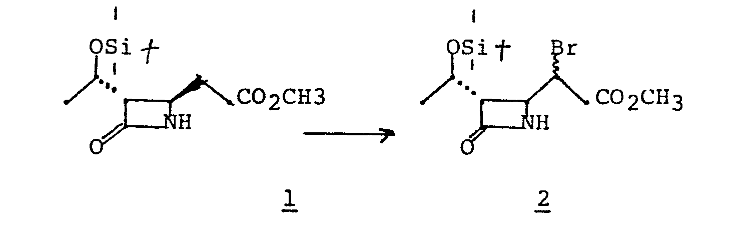

Add by syringe 17.6 ml (27.9 mm) of 1.58 M n-butyl lithium to a solution of 3.9 ml (27.9 mm) diisopropylamine in 28 ml of anhydrous tetrahydrofuran at -78°C. Stir the mixture for 15 minutes and then add 4.8 ml (27.9 mm) of hexamethylphosphoramide at -78°. Allow this mixture to stir for 20 minutes before adding by syringe a 1 M solution of azetidinone ester, 1, 4 gm (13.3 mm), in tetrahydrofuran. Continue stirring at -78° for 40 minutes where upon a 1M solution of tosylbromide in tetrahydrofuran is added. Quench the reaction mixture after an additional 10 minutes at -78° with saturated aqueous ammonium chloride solution (25 ml) and allow the mixture to warm to room temperature. Dilute the reaction mixture with 100 ml of diethyl ether. Wash with water and 1 N HC1 solution, dry over sodium sulfate and filter through a magnesium sulfate cake. Remove solvents under reduced pressure. Chromatograph on silica gel to obtain a 65% yield of the desired bromo ester 2 as a separable mixture of a and ß bromo isomers.
ß-Bromo ester: NMR: 0.02 (6H, s) Si(CH3)2; 1.02 (9H, s) (CH₃)C-Si ; 1.13 (3H, d) CH3CH(OSi); 3.02 (lH, m) azetidenone H₄; 3.8 (3H, s) -OCH₃; 4.1 (lH, dd) azetidenone H₃; 4.26 (lH, m) CH₃-CH-(OSi); 4.4 (lH, d) HCBrC0₂CH₃; 6.2 (lH, broad s) -N-H.
a-Bromo ester: NMR: 0.02 (6H, d) Si(CH₃)₂; 1.02 (9H, s) (CH₃ -Si ; 1.3 (3H, d) CH₃CH(OSi); 2.98 (1H, m) azetidenone H₄; 3.82 (3H, s) -OCH₃; 3.82 (1H, m) azetidenone H₃; 4.22 (1H, s) HCBrCO₂CH₃, 4.3 (1H, m) CH₃-CH(OSi); 6.2 (1H, broad s) -N-H.

### Step B:

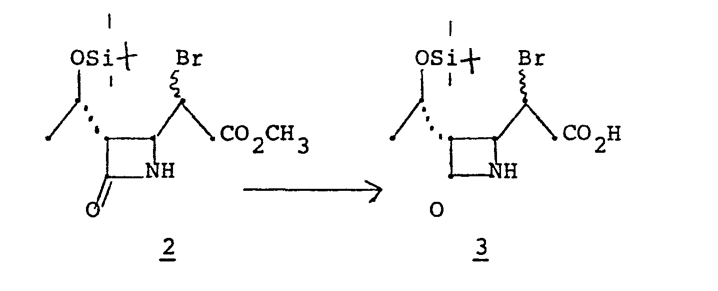

Dissolve 2.6 gm (6.9 mm) of bromoester 2 in 10 ml of methanol. Cool solution to 0° and add 2.9 ml of 2.4 M aqueous sodium hydroxide. Stir the reaction mixture for one hour at 0° then warm to room temperature and stir for 2 additional hours. Remove the methanol under vacuum, take the residue up in diethyl ether and wash with water. The aqueous phase acidified (pH 2) with 3N hydrochloric acid and extracted three times with methylene chloride. Dry the solution over sodium sulfate and filter through a magnesium sulfate cake. Remove solvents under reduced pressure to obtain a 75% yield of the desired bromoacid 3.

### Step C:

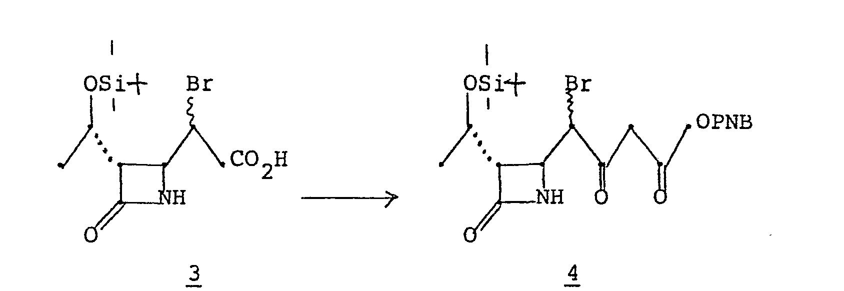

Add .194 gm (1.2 mm) 1.2 eq of carbonyl diimidazole to a suspension of .365 gm (.99 mm) of bromoacid 3 in acetonitrile at 0°. Stir the resulting solution for 40 minutes allowing it to warm to room temperature. Cool the reaction mixture to 0° again and add 600 mg 1.2 eq of paranitrobenzyl magnesium malonate. Allow the suspension to stir for 14 hours at room temperature. Remove the acetonitrile under vacuum. Dissolve the residue in ethylacetate and wash two times with 1 N hydrochloric acid, and one time with saturated aqueous sodium bicarbonate solution. Dry the organic phase over sodium sulfate and filter through a magnesium sulfate cake. Chromatograph the reaction mixture on silica gel to obtain a 45-50% yield of the desired bromoacetoacetate adduct 4. NMR: 3.7 (2H, s) -COCH ₂CO- 5.29 (2H, m)

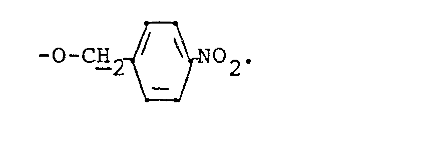

### Step D:

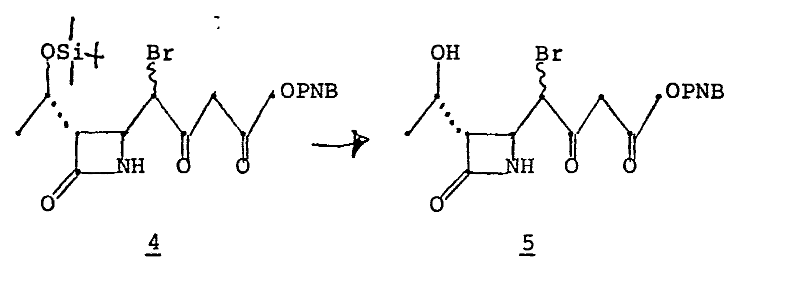

Dissolve the bromoacetoacetate derivative 4 (200 mg) in 4 ml of methanol at room temperature. Add to this a solution made up of .195 ml 12 N HC1 and .5 ml H₂0. The reaction mixture is then stirred for 3 hours. Remove the methanol under reduced pressure. Take the residue up in methylene chloride and wash with a dilute sodium bicarbonate solution. Dry the methylene chloride solution over sodium sulfate and filter through magnesium sulfate. Remove solvents under reduced pressure to obain a 70% yield of the desired free hyroxy ethyl adduct 7.

The product is characterized by a loss of the t-butyldimethylsilyl group in the NMR and the appearance in the IR of a distinct O-H stretch.

### Step E_{:}

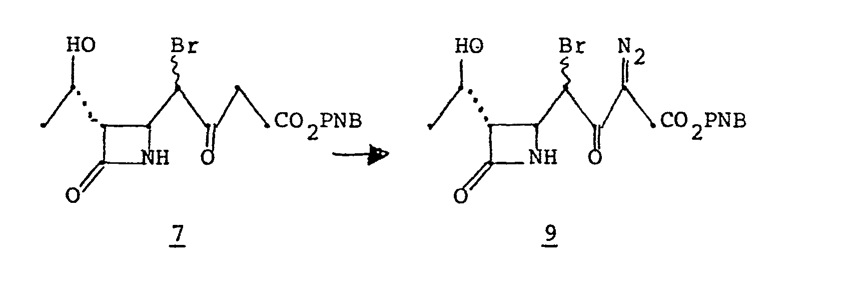

Dissolve .428 gm (1.1 mm) of the free hydroxy bromo acet acetate adduct, 7, in 4 ml of acetonitri..e. Add to this solution at 0° .168 ml of triethylamine and .178 ml of tosyl azide. Stir the reaction for 1 hour then warm to room temperature and stir for an additional 1.5 hours. Concentrate the reaction under reduced pressure and chromatograph on silica to obtain a 30% yield of the desired bromo, diazo adduct 9, as a mixture of α and β bromo compounds. IR: 2127 cm 1 (-_{N=N}) NMR: 3.14 (lH, dd) azetidinone H-4. 5.26 and 5.48 (1H, d) αand β Br-CH-CO.

### Step F:

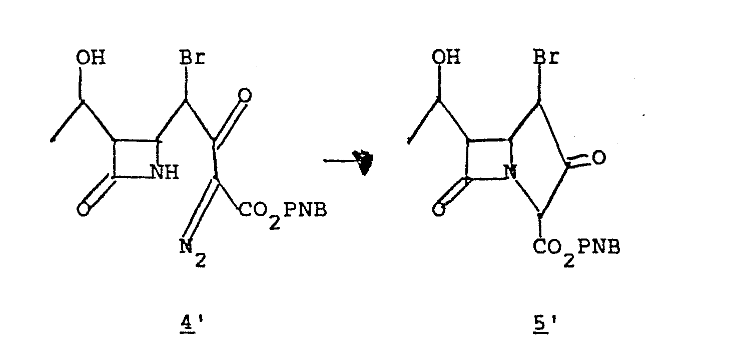

The diazo β-keto ester 4' (38.6 mg) in toluene (1 ml) is heated at 80°C in the presence of rhodium acetate (1.7 mg) for 10 min. The mixture is diluted with ethylacetate (10 ml) and washed with water and brine. The organic layer is separated, dried over MgSO₄ and evaporated in vacuo to give bicyclic keto ester 5'.

### Step G:

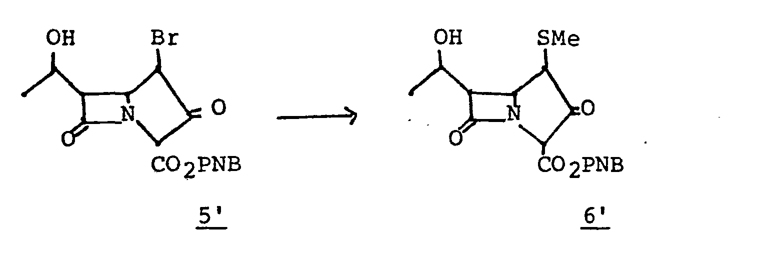

The bromo bicyclic ß-keto ester 5' (100 mg) in DMF (1.0 ml) is treated with methyl mercaptan (300 pl) and diisopropylethyl amine (500 µl) at 0°C overnight. The mixture is evaporated in vacuo to give product 6'.

### Step H:

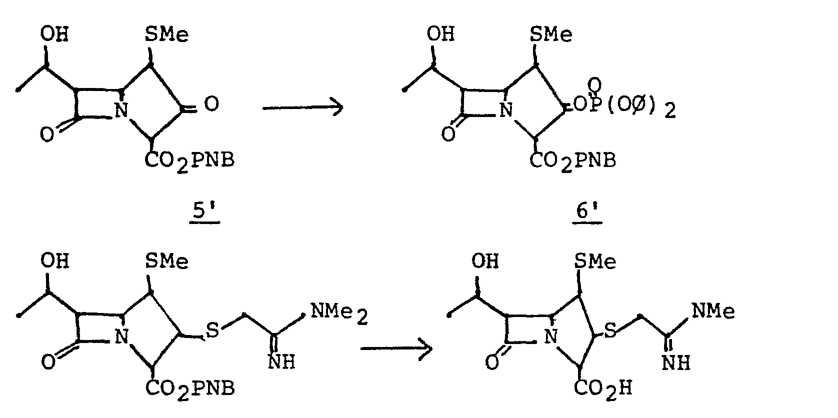

At 0°C, under N₂, the bicyclic ß-keto ester 1 (100 mg) in 1 ml acetonitrile is treated with 1.05 equivalents of diphenylchlorophosphate and 1.10 eq. of diisopropylethylamine for 20 minutes. The mixture is cooled to -25°C and 1.10 equivalents of N,N-dimethylcarbamimidoylmethylthio hydrochloride and 1.10 eq of diisopropylethylamine are added. The mixture is stirred for 30 minutes then diluted with ethyl ether to give a precipitate which is collected by filtration. The crude 3 is re-dissolved in 5 ml DI water, 5 ml THF and hydrogenated under 50 psi H₂ in the presence of 100 mg of 10% Pd/C and 1 eq of N_{A}HCO₃ for 2 hours. After filtering off the catalyst, the filtrate is extracted with ether, concentrated, and chromatographed on a Dowex-50X8 (Na cycle) column which is eluted with DI water to give product 4.

### EXAMPLE 3

### Step A: Preparation of 1-aminothienamycin

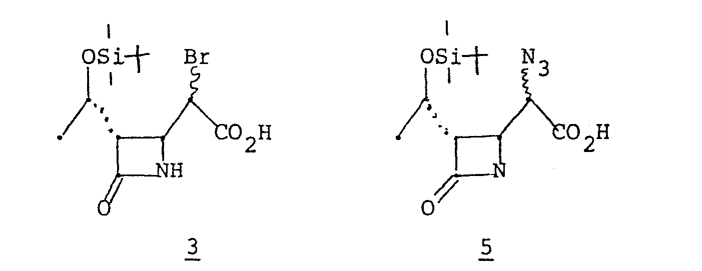

Dissolve 2.2 mg (6 mmol) bromo acid, 3, in 10 ml anhydrous dimethylformamide. Cool solution to 0° and add 1.15 ml (1.1 eq) of anhydrous diisopropyl ethyl amine. Follow immediately with addition of .8 ml (1.05 eq) of trimethylsilylchloride, freshly distilled from calcium hydride. Stir the reaction mxiture for 20 minutes before adding 1.17 gm (4 eq.) of lithium azide. Stir the reaction at room temperature for 14 hours. Remove the dimethylformamide under reduced pressure. Dissolve the residue in water and extract with diethyl ether. Acidify the aqueous phase with 3N hydrochloric acid and extract three times with methylene chloride. Dry the methylene chloride extracts over anhydrous sodium sulfate and filter through a magnesium sulfate cake. Remove the solvents under reduced pressure to obtain a 85% crude yield of the desired azido acid 5. IR: 2080 cm⁻¹ (-N=N-N-), 1754 cm⁻¹ (CO), 1700 cm⁻¹ (_{CO}).

### Step B:

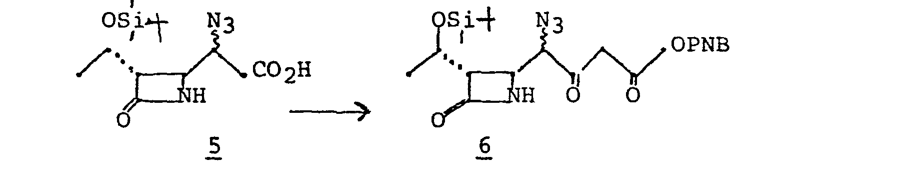

Preparation of 6 from 5 proceeds in the same fashion as transformation 3 to 4 in Step C, Example 2. IR: 1754 cm⁻¹ (C=0), 2080 cm⁻¹ (-N=_{N}-_{N}-) NMR: 3.7 (2H, s), -COCH₂CO-, 5.29 (2H, m)

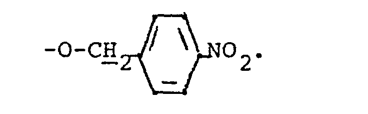

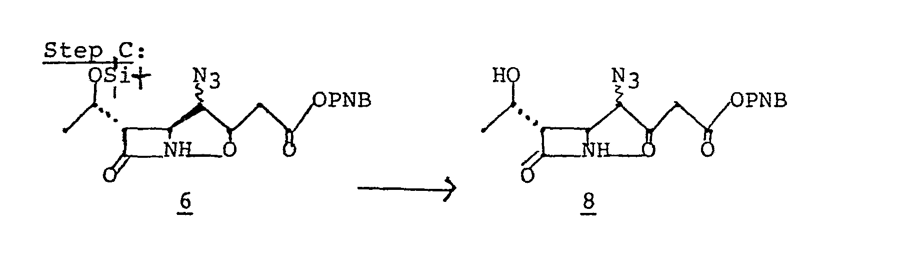

The transformation of silylhydroxyethyl azido adduct 6 to the free hydroxyethyl azido intermediate 8 proceeds in the same fashion as transformation 4 to 7 in Step D, Example 2. Yields of the desired adduct are also in the 70% range. The product was characterized by loss of the t-butyldimethylsilyl group in the NMR and the appearance in the IR of a distinct O-H stretch.

### Step D:

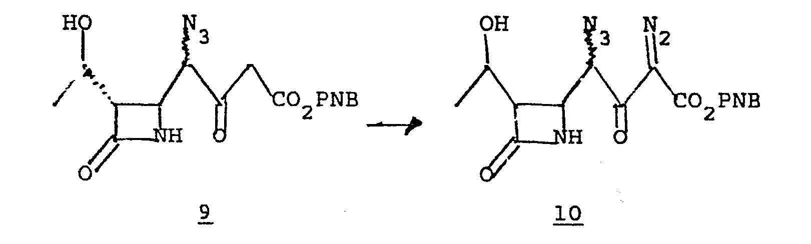

The transformation of the azido acetoacetate adduct 8 to give azido diazo adduct 10 proceeds in the same fashion as transformation 7 to 9 in Step E, Example 2. Chromatographed yield of the desired diazotransfer product was about 30%. NMR: 3.2 (lH, dd) azetidinone H-4, 4.98 and 5.1 (1H, d) α and ß-N₃-CH-CO.

### Step E:

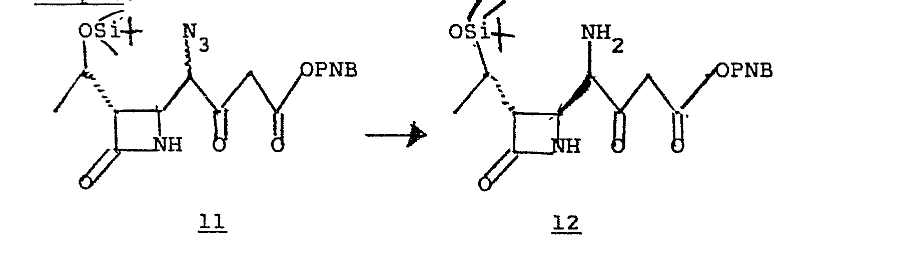

Saturate a solution of triethylamine (2 mmol, 2 eq) in 6 ml of dry methylene chloride at 0° with hydrogen sulfide gas. Add to this solution 1 mmol of the azido acetoacetate derivative 11. Stir the reaction mixture for 3 hours. Remove the solvent under reduced pressure. Dissolve the residue in 10 ml of methylene chloride and wash two times witr water. Dry over sodium sulfate and filter through magnesium sulfate cake. The product is characterized by the disappearance of the 2080 cm⁻¹ IR absorption and the appearance of a distinct N-H stretch.

### Step F_{:}

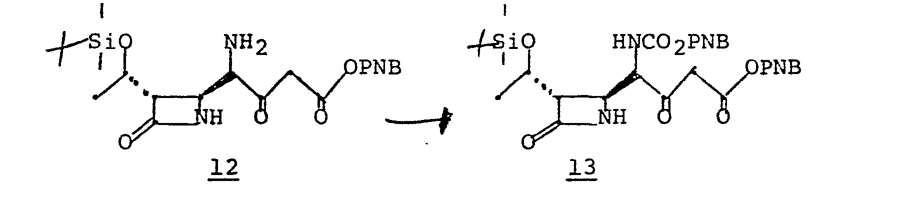

Dissolve 1 mmol amino acetoacetate derivative 12, in 5 ml of acetonitrile. Add to this solution 1.1 eq. of dimethylaminopyridine followed by addition of 1.1 eq. of para-nitro benzylchloroformate. Stir the reaction mixture for 14 hours. Remove the acetonitrile under vacuum. Dissolve the residue in methylene chloride and wash with dilute aqueous hydrochloric acid (1 N) one time and one time with saturated aqueous sodium chloride. Dry over sodium sulfate and filter through a magnesium sulfate cake. Remove solvent under vacuum to obtain the desired carbamate, 13.

### Step G:

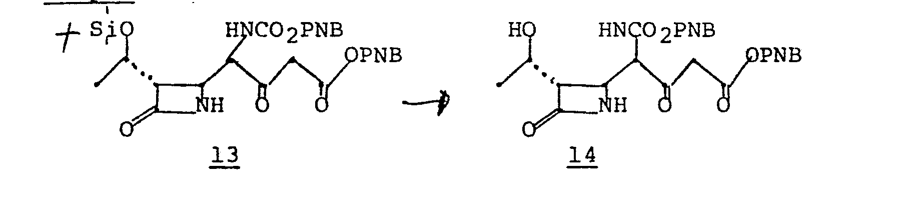

The transformation of silyl hydroxyethyl carbamate adduct, 13, to the free hdyroxy ethyl carbamate intermediate 14 proceeds in the same fashion as transformation 4 to 7 in Step D, Example 2.

### Step H_{:}

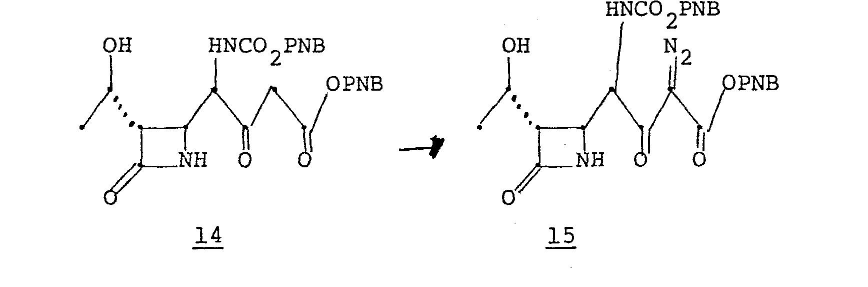

The transformation of carbamate acetonacetate adduct 13 to give carbamate diazoacetoacetate intermediate 14 proceeds in the same fashion as transformation 7 to 9 in this Example. Chromatography gives the desired diazotransfer product.

### Step I_{:}

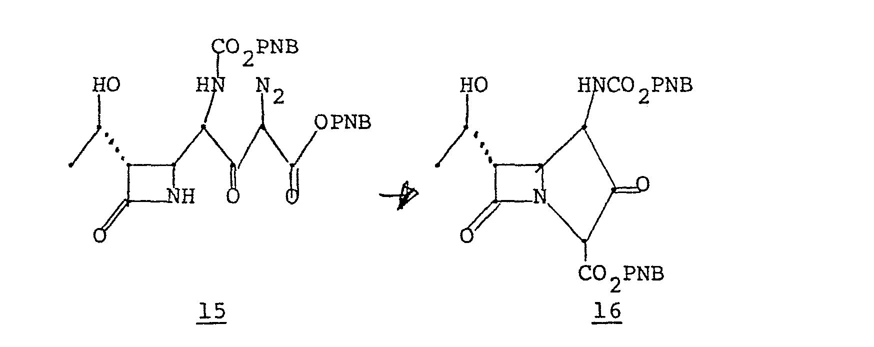

Dissolve the carbamate diazo acetoacetate derivative 15 in toluene. Add a catalytic amount of Rh₂(OAc)₄ and heat the reaction mixture for 1 hour at 80°C. Isolate the crude reaction product by removing the solvent under reduced pressure.

### Step J:

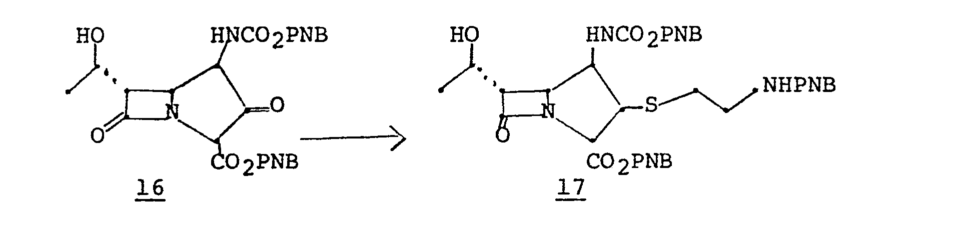

Dissolve bicyclic B-keto ester 16 in acetonitrile. Cool solution to 0° and add 1 eq. of diisopropyl ethylamine. Stir for 5 minutes and add 1 eq. of diphenylphosphonylchloride. Remove the ice bath and stir for 35 minutes. Cool the reaction to 0° again and add a second equivalent of diisopropyl ethylamine and 1 eq. of para-nitrobenzylcysteamine. Stir the reaction for an additional hour at 0°C. Dilute with methylene chloride and wash one time with pH 7 phosphate buffer. Dry over sodium sulfate and filter through magnesium sulfate.

Chromatograph the resulting residue to obtain the desired cysteamine substituted adduct 17.

### Step K:

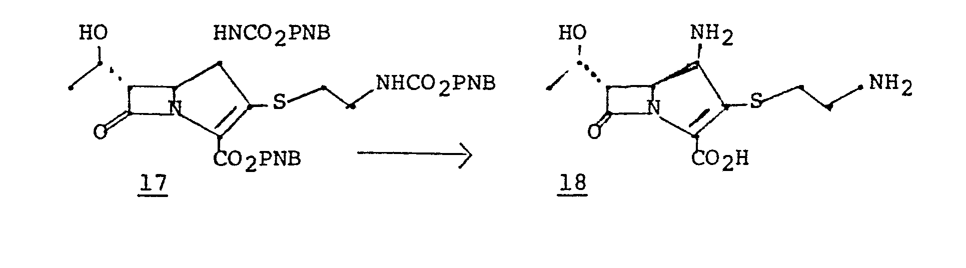

Dissolve intermediate 17 in tetrahydrofuran, ethanol, water and MOPS buffer. Add a catalytic amount of PtO₂ and place reaction under 40-45 psi H₂ on a Parr shaker. Filter the reaction after 30 minutes on a celite bed and wash with water. Extract the aqueous filtrate with ethyl acetate followed by diethyl ether. Concentrate the product under reduced pressure.

### EXAMPLE 4

### Preparation of l-chloro-2-ethylthio-6-(1-hydroxyethyl) -1-carbapen-2-em-3-carboxylic acid

### Step A:

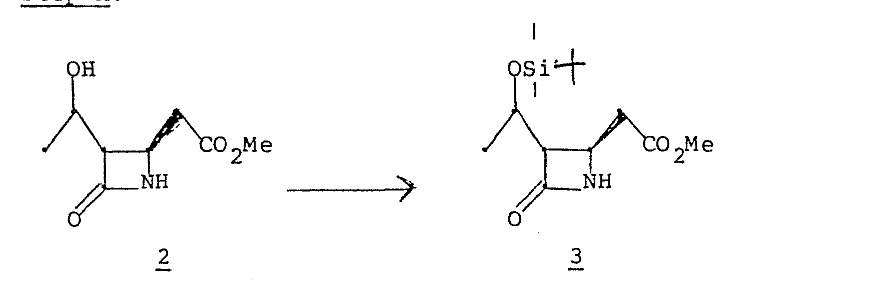

The free hydroxyl azetidinone 2 (374 mg) in DMF is treated with imidazole (688 mg) and t-butyldimethylchlorosilane (603 mg) at room temperature for 7 hours. The mixture is evaporated in vacuo and the residue is re-dissolved in ethyl acetate and washed with 1N HC1, water and brine. The organic layer is separated, dried over MgSO₄ and evaporated in vacuo to give 3.

### Step B:

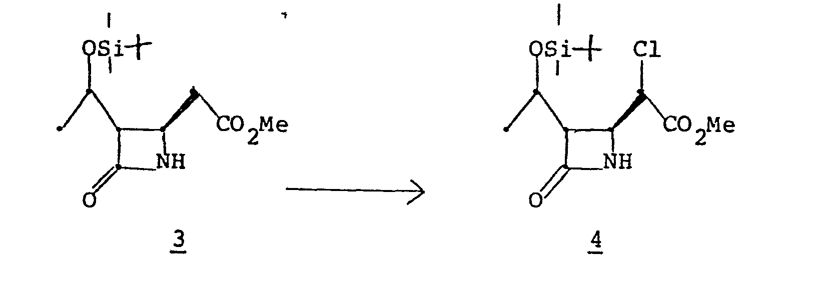

Under N₂, at -78°C, diisopropylamine (1.68 ml) in 12.5 ml THF is treated with n-butyllithium (12.5 ml, 1.6 M hexane) for 10 min. To the solution is added starting material 3 (1.51 g in 5 ml THF) and the mxture is stirred for 20 min., then is treated with 6 eq. of N-chlorosuccinimide. After stirring 2 hr. at -78°C, the mixture is hydrolyzed with 0.1 N HC1 and extracted with ethyl acetate. The organic layer is washed with water, brine then dried over MgSO₄ and evaporated in vacuo. The residue so obtained is dissolved in 50 ml methanol and treated with 10% sodium thiosulfate aqueous solution (10 ml) at 0°C for 5 min. then extracted with ethyl acetate. The organic layer is washed with water, brine, dried over Na₂SO₄, and evaporated in vacuo to give product 4.

### Step C:

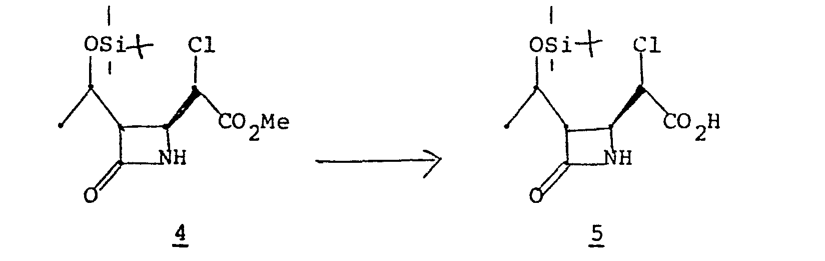

The chloro ester 4 (1.0 g) is treated with NaOH sc.ution (2.5 N, 1.4 ml) in methanol (5 ml) and water (1 ml) at room temperature for 5 hours. The mixture is acidified with 1 N HC1 to pH 1.0 then extracted with ethyl acetate. The organic layer is separated and dried over M_{G}SO₄ and evaporated to give 0.70 g of 5.

### Step D_{:}

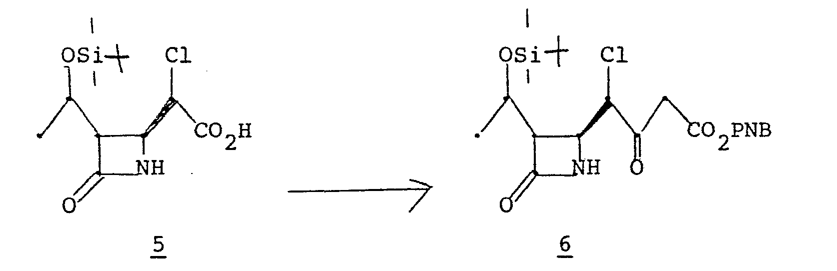

The carboxylic acid 5 (300 mg) in acetonitrile is treated with 1,1'-carbonyldiimidazole (194.6 mg) at room temperature for 30 min. To the solution is added magnesium p-nitrobenzylmalonate (1.00 g), then the mixture is heated at 65°C for 3 hours. The final reaction mixture is evaporated in vacuo to give an oily residue which is re-dissolved in 10 ml ethyl acetate and washed with water and brine. The organic layer is separated, dried over MgSO₄ then evaporated in vacuo to give crude product which is purified by silica gel TLC plates eluting with 50% EtoAc/cyclohexane to give product 6.

### Step E:

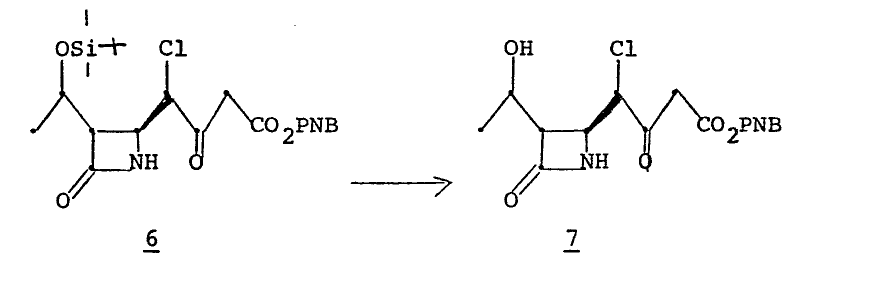

The starting material 6 (400 mg) is dissolved in 4 ml methanol and treated with 6 N HC1 (0.41 ml) at room temperature for 80 minutes. The mixture is diluted with ethyl acetate and washed with 0.1 N pH 7.0 phosphate buffer, water and brine. The organic layer is separated, dried over MgSO₄ and evaporated in vacuo to give crude product 7.

### Step F:

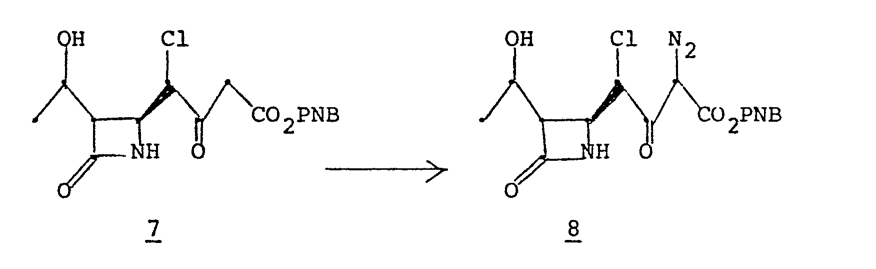

The 8-keto ester 7 (269 mg) dissolved in 3.2 ml acetonitrile is gently stirred with Amberlite XE-301-S0₂N₃ (1.5 g, 3.33 meq of N₃/g) and triethylamine(0.46 ml) at room temperature for 1.5 hours. The mixture is filtered from polymer beads and the filtrate is evaporated in vacuo to give oily residue which is purified by TLC (silica agel) eluting with ethyl acetate to give diazo keto ester 8.

### Step G:

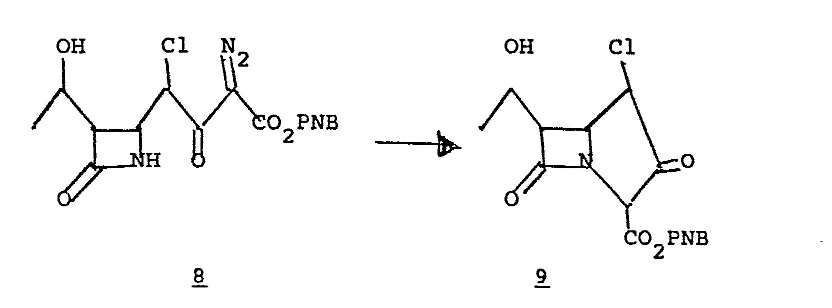

The diazo compound 8 (145 mg) is dissolved in 4.1 ml toluene and 2.5 ml ethyl acetate. The mixture is heated at 80-85°C in the presence of rhodium octanoate (2.9 mg) for 5 minutes. The solution is allowed to cool to room temperature then diluted with 10 ml ethyl acetate and washed with water and brine. The organic layer is separated, dried over MgSO and evaporated in vacuo to give bicyclic keto ester 9.

### Step G':

The diazo compound 8 (50 mg) is dissolved in toluene (10 ml) in a 50-ml quartz tube. The mixture is photolyzed under the influence of UV at room temperature for 1 hour, then evaporated in vacuo to give bicyclic keto ester 9.

### Step H:

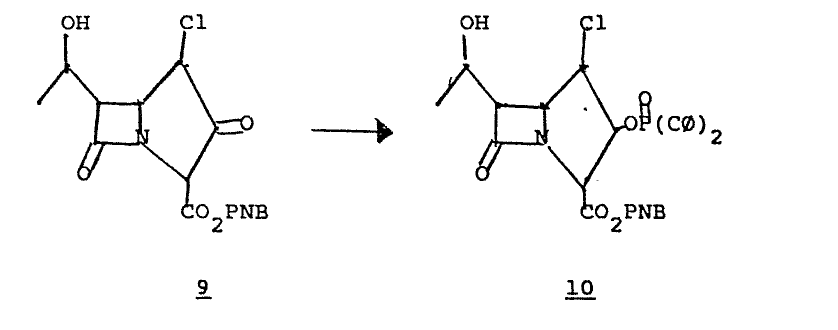

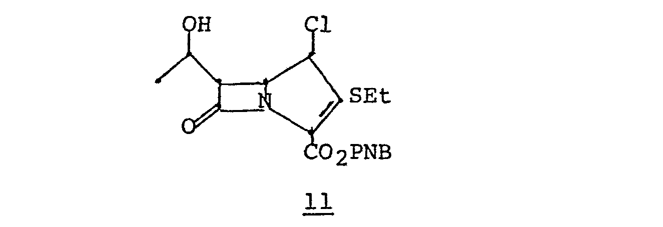

At 0°C, under N₂ atmosphere, the 1-chloro bicyclic ß-keto ester 9 (100 mg) in 1 ml acetonitrile is treated with diphenylchlorophosphate (56.7 µl) and diisopropylethylamine (50 ul) for 10 min. To the mixture is added ethanethiol (48 µl) and diisopropylamine (50 µl) and the mxiture is stirred at room temperature for 6 hours. TLC purification of the reaction mxiture (eluting with ethyl acetate) gives Product 11.

### Step I:

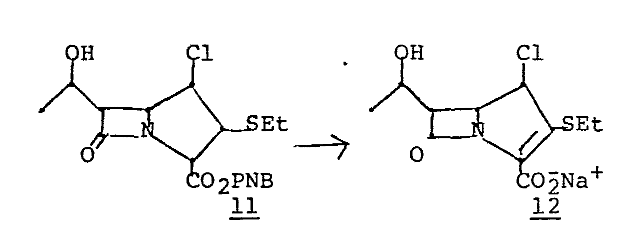

The l-chloro-2-ethylthiocarbapenem ester 11 (7.2 mg) is dissolved in THF (0.62 ml) and ethanol (0.62 ml). The solution is tranferred to a hydrogenation flask which contains 0.48 ml D.I. water, 1.53 mg of NaHC0₃ and 3.6 mg of 10% Pd/C. The mixture is hydrogenated at 50 psi for 2.5 hours, diluted with 7.2 ml water, then filtered through super cell which is washed with additional 7.2 ml water. The filtrate is extracted with 4 x 20 ml ether. The aqueous layer is separated, conc. to 2 ml then chromatographed by an XAD-2 column (2 x 6 cm). The desirable fractions from column are combined, concentrated and lyophilized to give Product 12.

### EXAMPLE 5

### Preparation of 1-OR-1-carbapen-2-em-3-carboxylic acids

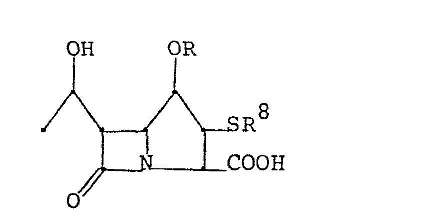

wherein R is representative shown below, and defined generally under R⁹ above. The definition of R⁸ is similarly stated.

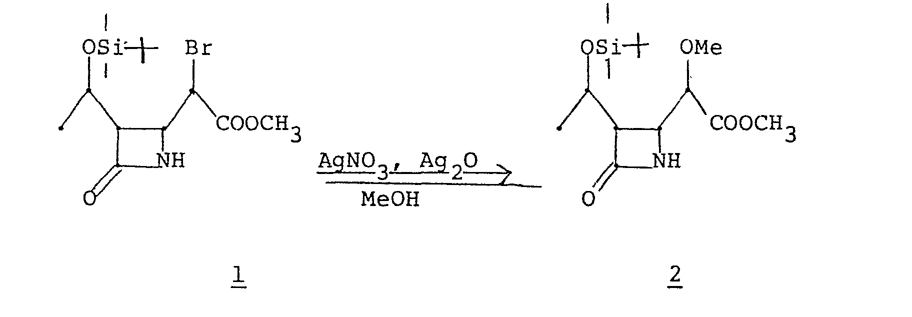

The bromoester (379 mg) is dissolved in 5 ml anhydrous MeOH and treated with A₉₂0 (248 mg) and AgNO₃ (172 mg). The mxture is stirred at room temperature for 3 hours. The silver salts are filtered off and the filtrate is evaporated and the residue chromatographed on silica gel to give the desired methoxy compound.

Substitution of other alcohols (ROH such as ethyl, isopropyl, benzyl, phenol) in the above reactions gives 2 where OMe is substituted by OR.

### EXAMPLE 6

### Preparation of 1-SR-2-SR⁸⁻1-carbapen-2-em-3-carboxylic acids

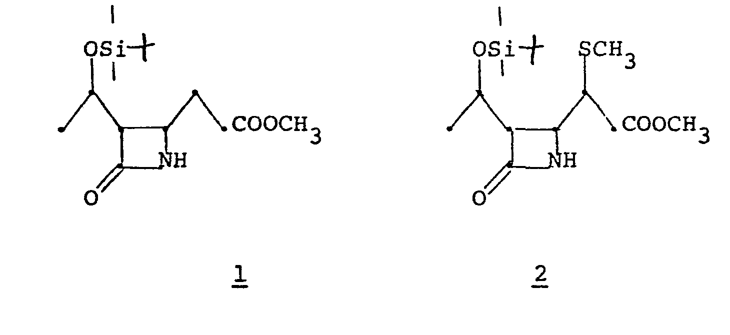

The methyl ester (60 mg) was converted to the dianion as described in Step A of Example 1.

To the solution of the dianion at -78° was added CH₃S-S-CH₃ (40 pl) and the mixture was stirred at -78° for 1 hour at -35° for another 45 minutes. The reaction mixture was worked up as described in Step A of Example 1 to give crude product which was pruified by preparative t.l.c. on silica gel using 50% Et₂0 in petroleum ether to give 25 mg of the desired product as a mixture of isomers. n.m.r. (60 MHz, ): 0.1 s (CH₃Si); 0.9 s (CH₃-C-Si); 1.3 d (CH₃-C-); 2.2 s (CH₃S); 2.95 d of d (C-3 H) ; 3.8 s 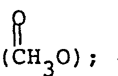 4.3 m (C-4H and CH₃-C-O); 5.9 broad s (NH). H

Following the procedure of Example 6, except substituting the reagent Ø-SS-Ø, the SØ species is obtained rather than the -SCH₃ species 2.

### EXAMPLE 8

### Step A: Preparation of l-amino-2-ethylthio-6-(l-hydroxyethyl)-1-carbapen-2-em-3-carboxylic acid

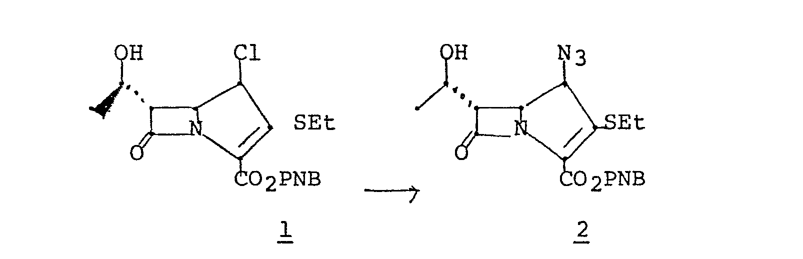

The 1-chlorocarbapenem 1 (0.2 g) in 1 ml DMF is stirred with lithium azide (0.2 g) at room temperature overnight. The mixture is diluted with 10 ml ethylacetate and wasned with water. The organic layer is separated, dried over MgSO₄ and concentrated to give product 2 which is purified by preparative t.l.c.

### Step B:

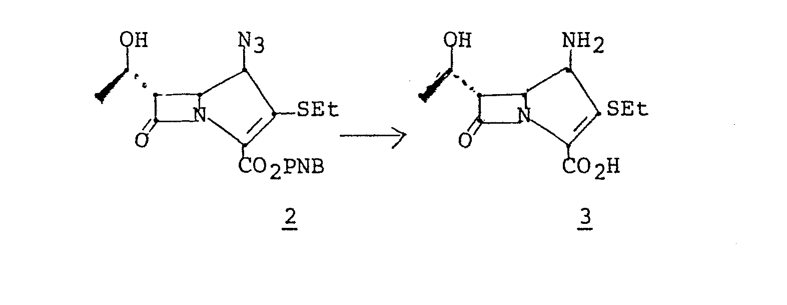

The azido carbapenem 2 obtained in Step A is dissolved in 10 ml THF and 10 ml ethanol and mixed with 8 ml of water contaiing 20 mg of sodium bicarbonte and 50 mg of 10% Pd/C. The mixture is hydrogenated at 50 psi for 2.5 hr. then filtered from catalysts. The filtrate is extracted with ether, concentrated to 3 ml then chromatographed on a XAD-2 column (2.2 x 10 cm) eluting with water to give Product 3 as solids after lyophilization.

### EXAMPLE 9

### Step A: Preparation of l-cysteminyl-2-ethylthio-6-(l-hydroxyethyl)-l-carbapen-2-em-3-carboxylic acid

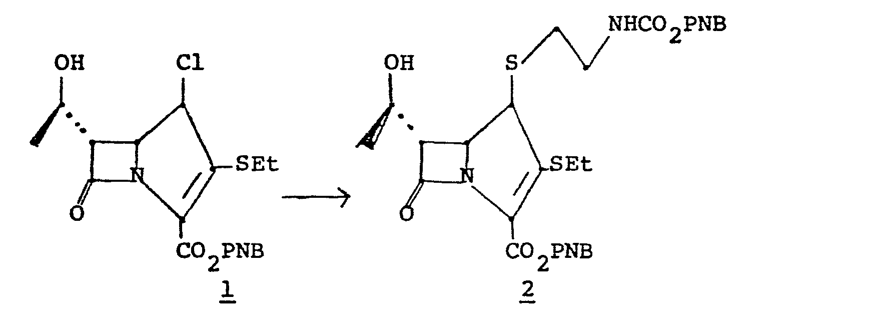

The 1-chlorocarbapenem 1 (100 mg) dissolved in DMF (1 ml) is treated with p-nitrobenzoxylcarboxyl- aminoethanethiol (80 mg) and diisopropylethylamine (50 mg) at room temperature overnight. The mxiture is diluted with 10 ml ethylacetate and washed with water and brine. The organic layer is separated, dried over MgSO₄, concentrated chromatographed on silica gel plates eluting with ethylacetate to give Product 2.

### Step B:

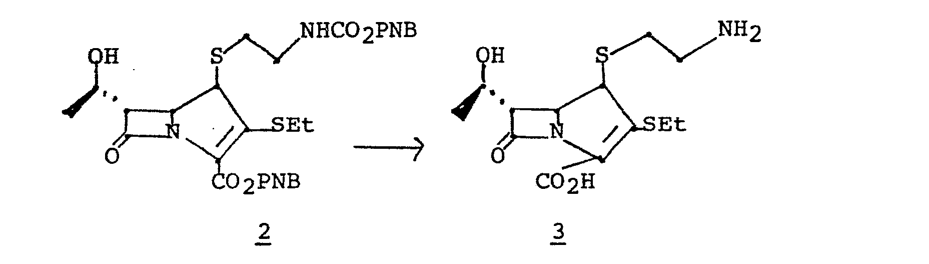

The bis-protected carbapenem 1 in Step A dissolved in 10 ml THF/10 ml ethanol/8 ml water and hydrogenated at 50 psi for 3 hours in the presence of 50 mg of 10% Pd/C and 25 mg of sodium bicarbonate. The mixture is then filtered from catalysts and the filtrate is extracted with ether, concentrated to 3 ml and chromatographed by a Dowex-50X4 (Na ) column (2.2 x 10 cm) eluting with water to give 3.

### EXAMPLE 10

### Step A: Preparation of 1-(2-cyanoethylthio) thienamycin

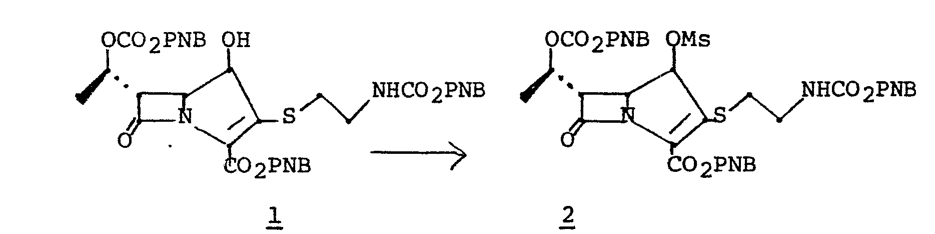

The 1-hydroxythienamycin 1 (100 mg) is treated with methanesufonylchloride (200 mg) and triethylamie (200 µl) in DMF (2 ml) at room temperature overnight. The mixture is diluted with 50 ml ethyl acetate and washed with water and brine. The organic layer is separated, dried over MgS0₄, and evaporated in vacuo to give 2.

### Step B_{:}

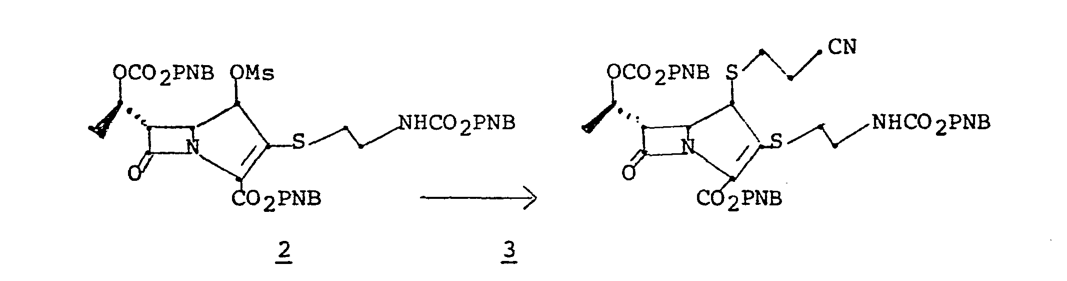

The 1-mesyl thienamycin 2 obtained in Step A is treated with cyanoethylthiol (60 µl) and diisopropylethylamine (48 µl) in 1 ml DMF and room temperature for 3 hours. The mixture is diluted with 50 ml ethyl acetate and washed with water and brine. The organic layer is separated, dried over MgSO₄, concentrated and chromatographed by silica gel plates eluting wth ethyl acetate to give 3.

### Step C:

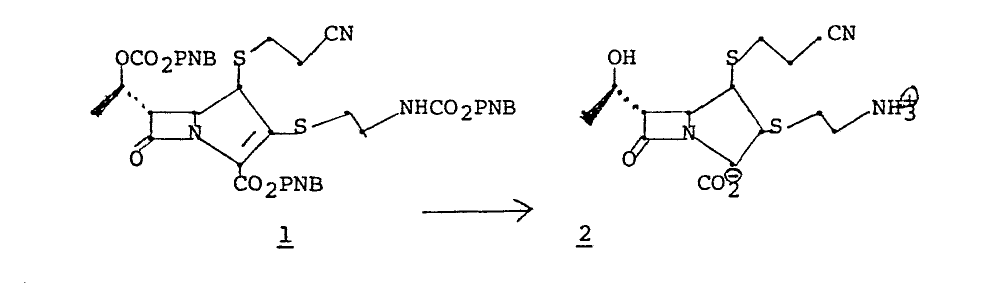

The bis-protected carbapenem 1 obtained in Step B dissolved in 10 ml THF/10 ml ethanol/8 ml H₂0 is hydrogenated at 50 psi for 2.5 hours in the presence of 50 mg of 10% Pd/C and 25 mg potassium bicarboate. The mixture is filtered from catalysts and the filtrate is extracted with ether. The aqueous layer is separated, concentrated then chromatographed by a XAD-2 column (2.2 x 10 cm) eLuting with water to give 2 as solids after lyophilization.

### EXAMPLE 11

### Preparation of 1-Acetylamino-2-ethylthio-6-(1-hydroxyethyl)-1-carbapen-2-em-3-carboxylic acid sodium salt

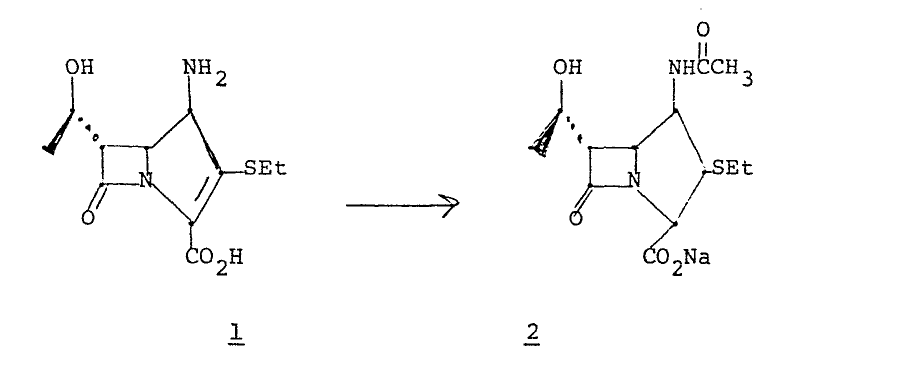

At 0°C, the 1-amino carbapenem 1 is dissolved in 10 ml of water and 10 ml of dioxane. The solution is adjusted to and maintained at pH 8.5-9.0 with 10% NaOH. To the solution is added acetic anhydride (150 µl). After stirring at 0°C for 15 min, the mixture is adjusted to pH 7.0 with phosphoric acid and extracted thoroughly with ether. the aqueous layer is concentrated and chromatographed by a XAD-2 column eluting with D.I. water to give desired aqueous solution o 2, which is lyophilized to give solid 2.

### EXAMPLE 12

### Preparation of l-Formimidoylamino-2-ethylthio-6-(1-hydroxyethyl)-1-carbapen-2-em-3-carboxylic acid

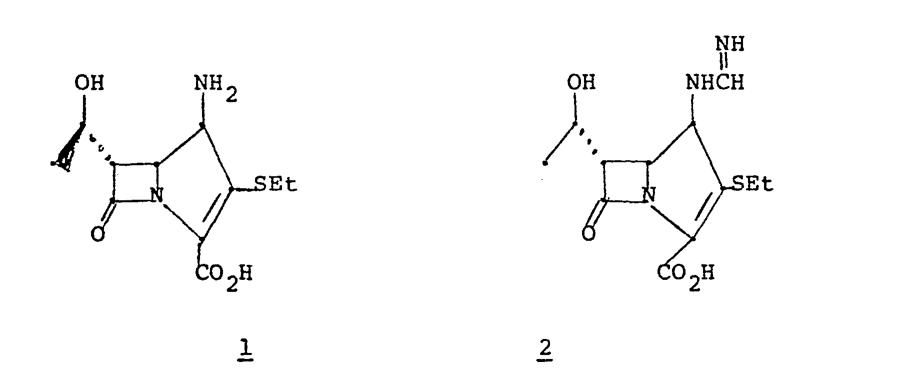

At 0°C, 1-amino carbapenem 1 (100 mg) is dissolved in 10 ml 0.1 M pH 7.0 phosphate buffer. The solution is maintained at pH 8.5-9.0 with 10% NaOH while it is treated with ethylformimidate hydrochloride (400 mg). The mixture is stirred for 20 min. then adjusted to pH 7.0 with phosphoric acid, concentrated and chromatographed by a Dowex-50X8 (Na⁺) column (2.2 x 10 cm) which is eluted with D.I. water to give aqueous solution of 2. After the aqueous solution is concentrated and lyophilized, a solid product 2 is obtained.

### EXAMPLE 13

### Preparation of 1-Acetylaminothienamycin Step A

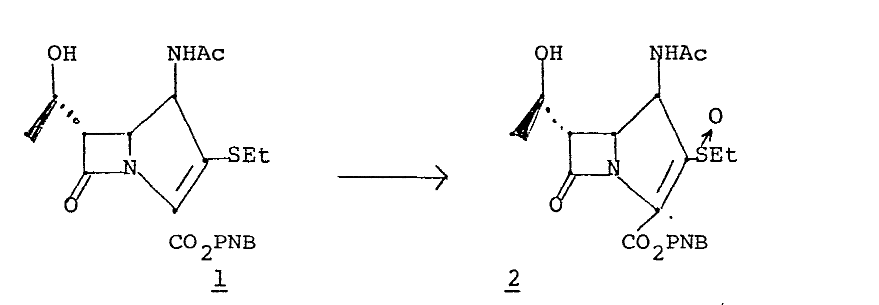

At 0°C, under N₂ atmosphere, the N-acetylamino carbapenem 1 (100 mg) is dissolved in 1.0 ml methylene chloride and treated with m-chloroperoxybenzoic acid (38 mg). The mixture is stirred for 15 min, then chromatographed by a silica gel plate eluting with ethyl acetate to give sulfoxide 2.

### Step B:

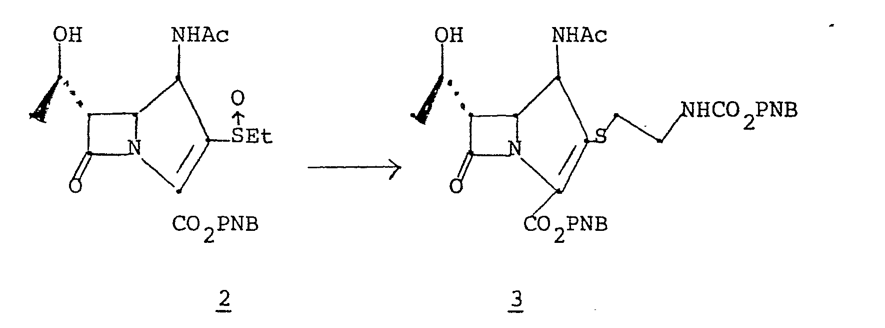

The sulfoxide 2 obtained from Step A dissolved in 0.5 ml DMF is treated with p-nitrobenz- oxycarbonylaminoethylthiol (85 mg) and diisopropylethylamine (57.6 mg) at 0°C for 3 hours. the mxiture is evaporated in vacuo to give crude product 3 which is re-dissolved in ethyl acetate and washed with water and chromatographed by silica gel plates eluting wih ethyl acetate to give desirable product 3.

### Step C:

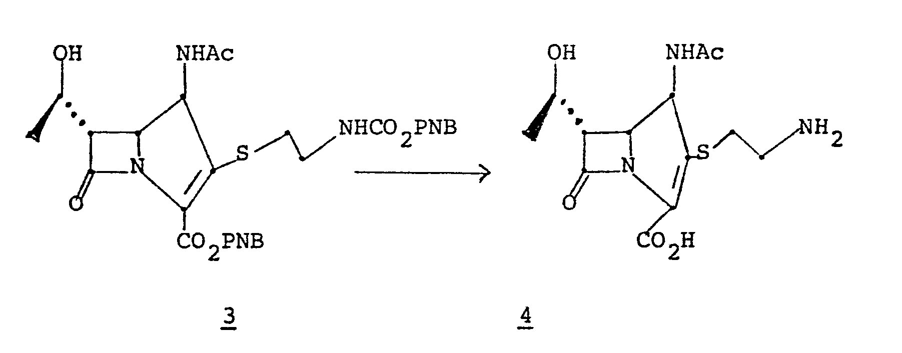

The starting material 3 (115 mg) dissolved in 10 ml THF and 10 ml ethanol is mixed with 8 ml of water containing 25 mg of sodium bicarbonate, and 58 mg of 10% Pd/C. The mixture is hydrogenated at 50 psi for 2.5 hours, then filtered from catalysts. The filtrate is extracted thoroughly with ether and the aqueous layer is separated, concentrated and chromatographed by a Dowex-50X4 (Na⁺) column (2.2 x 10 cm) which is eluted with water to give an aqueous solution of 4. After lyophilization, solid product 4 is obtained.

### EXAMPLE 14

### Step A: Preparation of 1-amino-2-(2-cyanoethylthio)-6-(1-hydroxyethyl)-1-carbapen-2-em-3-carboxylic acid

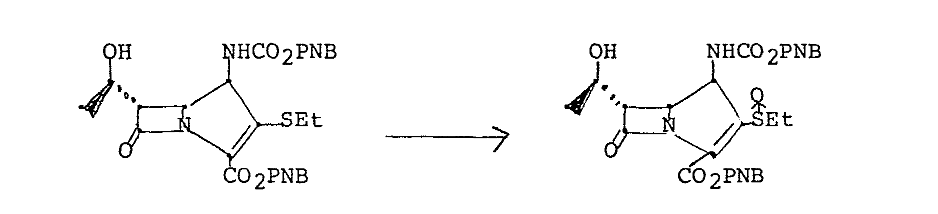

The bis-protected 1-amino carbapenem 1 (20 mg) is dissolved in methylene chloride (0.2 ml) and treated with m-chloroperoxybenzoic acid (7.5 mg) at 0°C for 20 min. The mixture is isolated by silica gel chromatography eluting wtih ethyl acetate to give sulfoxide 2.

### Step B:

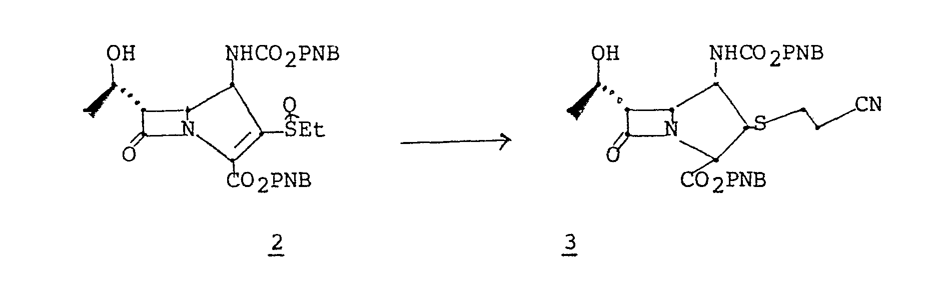

The sulfoxide 2 obtained from Step A is dissolved in DMF 0.1 ml and treated with cyanoethylthio (15 ul) and diisopropylethylamine (12 µl) at 0°C for 2 hours. The mixture is chromatographed on a silica gel plate, eluting with ethyl acetate to give product 3.

### Step C:

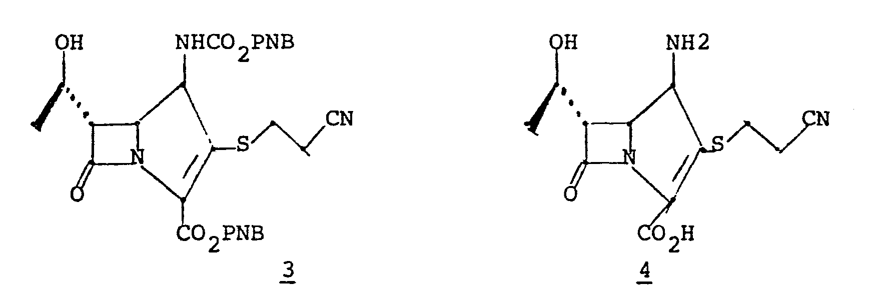

The starting material 3 (10 mg) in 1 ml THF, 1 ml ethanol, 0.8 ml water is hydrogenated at 50 psi in the presence of 10% Pd/C (6 mg) and sodium bicarboate (2.5 mg) for 2.5 hours. The mixture is filtered from catalysts and the filtrate is extracted with ether. The aqueous layer is separated, concentrated and chromatographed by a Dowex-50X4 (Na⁺) (2.2 x 5 cm) eluting with D.I. water to give product 4 in aqueous solution which is concentrated and lyophilized to give solid product 4.

### EXAMPLE 14a

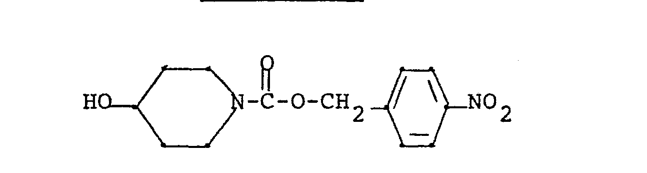

### Step A: 1-(p-nitrobenzyloxycarbonyl)-4-hydroxypiperidine

To a stirred solution of 4-hydroxypiperidine (1.01 g, 10 mM) and 4-dimethylaminopyridine (1.22 g, 70 mm) in EtOAc (30 ml) and water (20 ml) cooled to 0° is added a solution of p-nitrobenzyloxycarbonyl chloride (2.15 g, 10 mM) in EtOAc 50 ml. After stirring for 50 minutes, the organic phase is separated and washed with water, dried and evaporated. The residue is chromatographed on silica gel using CHC1_{3/}MeOH to give the desired product.

### Step B: l-(p-nitrobenzyloxycarbonyl)-4-methane- sulfonyloxy-piperidine

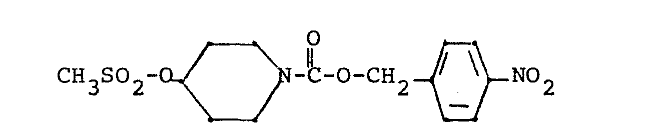

A mixture of 1 (2.8 g, 10 mM) of Et₂N (2.0 g, 20 mM) in CH₂C1₂ 20 ml cooled to 0° is treated with methane sulfonyl chloride (2.28 g, 20 mm) and the mixture is stirred at 0° for 1 hour at room temperature for 3 hours. The reaction mixture is diluted with methylene chloride washed with 5% NaHCO₃ solution, water and brine, then dried and evaporated to give a residue which is chromatographed on silica gel to give the desired 2.

### Step C: 1-p-nitrobenzyloxycarbonyl-4-benzoxylthio- piperidine

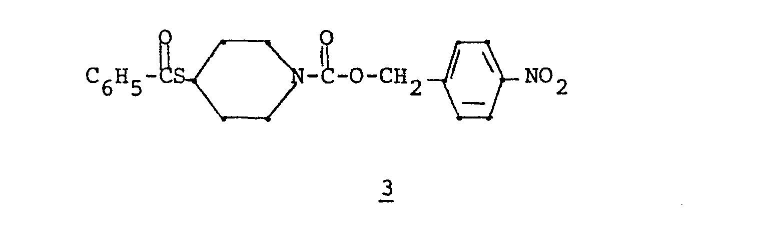

To a stirred solution of 2 (3.58 g, 10 mm) and thiolbenzoic acid (1.38 g, 10 mm) in toluene (20 ml) is added 1,5-diazabicyclo[5.4.0]undec-5-ene (DBV) (1.7 g, 11 mm). After stirring for 3 hours at 80° the reaction mixture is diluted with EtOAc, washed with solid NaHCO₃, dried and evaporated to give crude 3 which is purified by chromatography.

### Step D: 1-p-nitrobenzyloxycarbonyl-4-mercapto- piperidine

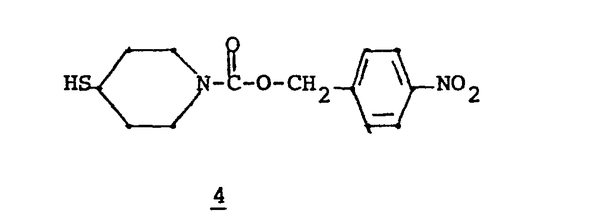

To a stirred solution of 3 (2.0 g, 5 mMol) in THF 50 ml is added LiBH₄ (1.0 g excess) at 0° under N₂ after stirring 1 hour at room temperature then reaction mixture is acidified with 5% HC1, diluted with EtOAc (200 ml) washed with water, brine and dried. The solvent is removed under reduced pressure and the residue is chromatographed on silica gel to give 4.

### EXAMPLE 14b

### Step A: 1-p-Nitrobenzyloxycarbonylthiomorpholine

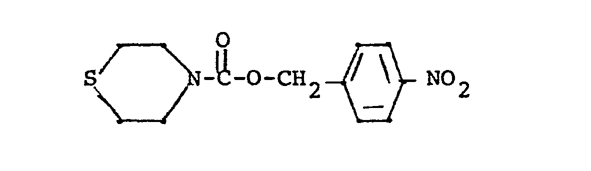

Starting with thiomorpholine and treating it with 1.15 g of p-nitrobenzyloxycarbonylchloride as in Step H of Example 1, 1-p-nitrobenzyloxycarbonylthiomorpholine is obtained.

### Step B: 1-p-Nitrobenzyloxycarbonyl-3-chlorothio- morpholine

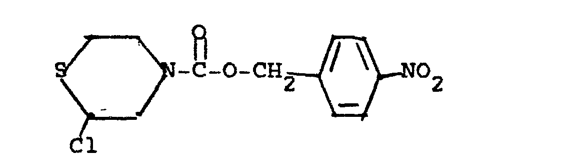

To a stirred solution of 1 (2.82 g, 10 mmol) in CHC1₃ 15 ml is added N-chlorosuccinimide (1.₅ g, 11 mMol) at room temperature under N . The reaction mixture is stirred for 10 minutes and the solvent is removed under reduced pressure at 0°. The residue which is 9 is used without purification in the next step.

### Step C: 1-p-Nitrobenzyl(1R,5S,6S)-2-[(RS)-N-p-nitrobenzyloxycarbonyl-3-thiomorpholinylthio]-6-[(R)-1-hydroxyethyl]-1-p-nitrobenzyloxy- carbonyloxy-l-carbapen-2-em-3-carboxylate

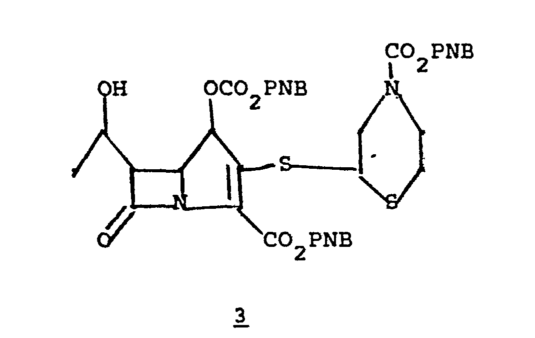

To a solution of the bicyclic ketone (2 of Table I) (36 mg) in CH₃CN (2 ml) cooled to 0° under N₂ is added iPr₂NEt (19 ml) followed by diphenyl chlorophosphate (22 ml). The mixture is stirred for 1 hour, the solvent is removed under reduced pressure and the residue is dissolved in DMF (1 ml) and cooled to 0°. Sodium hydrosulfide (5.6 mg) in DMF (0.6 ml) is added followed by iPr₂NEt (19µl). The mixture is stirred at 0° for 25 minutes and treated with a solution of 1-p-nitrobenzyloxycarbonyl-3-chlorothio- morpholine (30 mg) in DMF (1 ml) followed by iPr₂NEt (19µl). The reaction mixture is stirred for 1 hour at 0°, diluted with EtOAc. washed with water, 5% NaHCO₃ solution, solid NaCl then dried and evaporated to give a residue which is chromatographed on silica gel to give 3.

### Step D: (lR.5S,6S)-2-[(RS)-3-thiomorpholinethio]-6-[(R)-1-hydroxyethyl]-1-hydroxy-1-carbapen-2- em-3-carboxylic acid

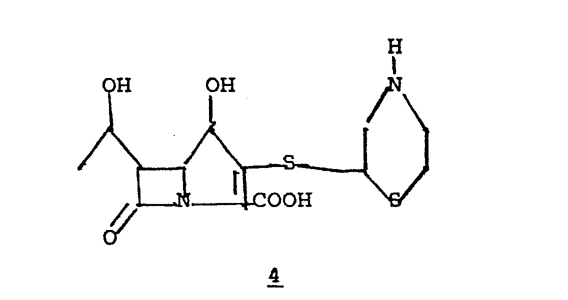

The product of Step C is treated under the reaction conditions of Step J, Example 1, to give the product 4.

### EXAMPLE 14c

Starting with thiazolidine and following the Steps A to D of Example 7, one obtains: (1R,5S,6S)-2-[(RS)-2-thazolidinethio]-6-[(R)-1-hydroxy-ethyl)-1-hydroxy-l-carbapen-2-em-3-carboxylic acid.

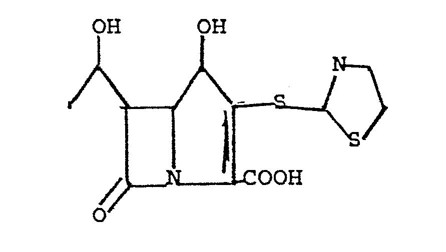

### EXAMPLE 14d

Starting with 3-hydroxypiperidine and following the Steps A to D of Example 15a, one obtains: l-p-nitrobenzyloxycarbonyl-3-mercapto- piperidine.

### EXAMPLE 14e

Starting with 3-hydroxypyrrolidine and following Steps A to D of Example 1, one obtains: l-p-nitrobenzyloxycarbonyl-3-mercaptopyrrolidine.

### EXAMPLE 14f

### Preparation of 1-chloro-2-(3'-pyrrolidylthio)-6-(1'-hydroxyethyl)-1-carbapen-2-em-3-carboxylic acid

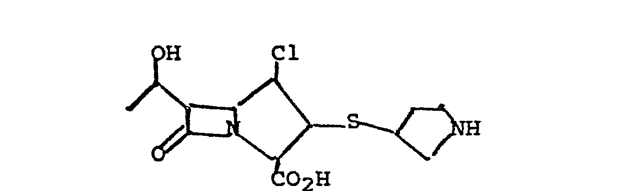

### Step A: Preparation of p-nitrobenzyl-l-chloro-2-[N-(p-nitrobenzyloxycarbonyl)-3'-pyrrolidylthio]-6-(1'-hydroxyethyl)-1-carbapen-2-em-3-carboxylate 3

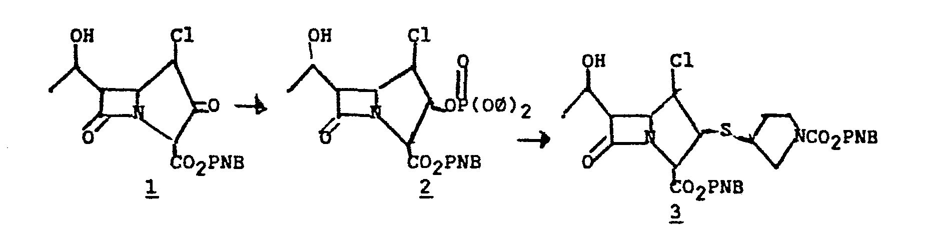

Diisopropylethylamine (50µl) and diphenylchlorophosphate (56.7µl) were added to an ice-cold solution of bicyclic keto ester 1 (100 mg) in acetonitrile (1.0 ml). The resulting solution was stirred in the cold and under a N ₂atmosphere for 20 minutes to effect conversion to the vinylphosphate

2. To the solution was added N-p-nitrobenzyl y-carbonyl-3-mercapto-pyrrolidine (93 mg) and diisopropylethylamine (50 pl). The resulting solution was kept in the cold for 20 hours, then diluted with ethylacetate, washed with brine, dried over Na₂S0₄, filtered and evaporated in vacuo to give crude product 3 which was purified by chromatography on a column of silica gel (1.5 x 15 cm) eluting with 30% EtOAc/cyclohexane.

### Step B:

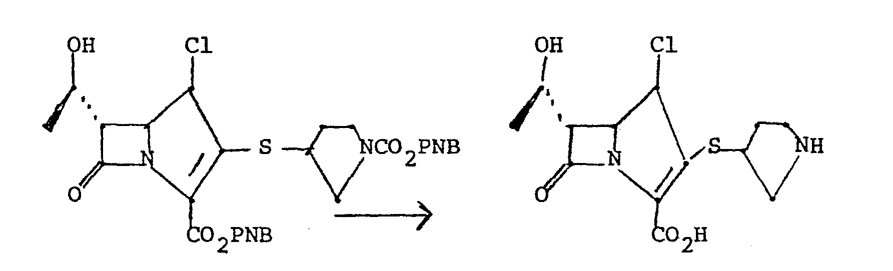

A mixture of diprotected carbapenem 1 (115 mg), THF (10 ml), ethanol (10 ml), 0.1 M pH 7 phosphate buffer (10 ml) and 10% Pd/C (58 mg) were hydrogenated at 50 psi for 2.0 hours. The mixture was filtered through a cellite pad to remove the catalyst which was washed with 10 ml water. The filtrate was extracted wtih 2 x 50 ml ethyl acetate, concentrated under vacuum to 5 ml and loaded onto a Dowex-50X4 (Na⁺ cycle) column (2.2 x 10 cm). The column was eluted with water to give 2 as solid after lyophilization.

### EXAMPLE 14g

### Preparation of l-chloro-2-(N-acetimidoyl-3'-pyrrolidyl- thio)-6-(1'-hydroxyethyl)-1-carbapen-2-em-3-carboxylic acid

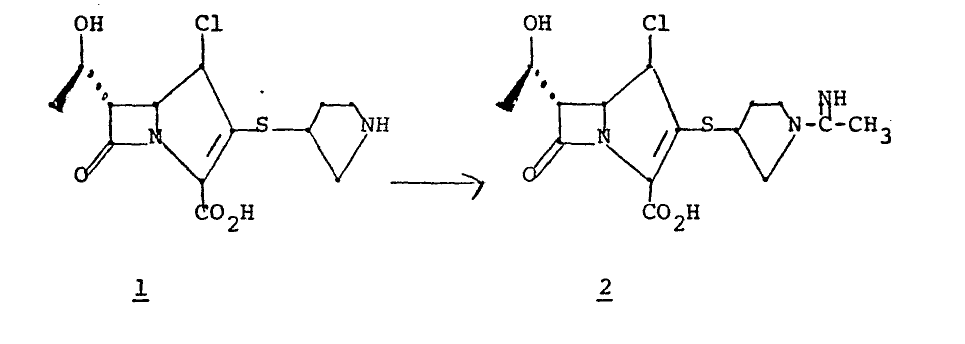

At 0°C, the free amine 1 (100 mg) was dissolved in 10 ml 0.1 M pH 7.0 phosphate buffer. The solution was brought to pH 8.5 with 2.5 N NaOH prior to addition of ethyl acetimidate hydrochloride (400 mg). The pH was re-adjusted to 8.5 wit 2.5 N NaOH after imidate was added. The mixture was stirred in the cold for 10 minutes, then acidified with phosphoric acid to pH 7.0, extracted with ethyl acetate. The aqueous layer was separated, concentrated to 5 ml volumn and chromatographed by a Dowex-50X4 (Na⁺ cycle) column (2.2 x 10 cm). The column was eluted with D.I. water to give 2 as solid after lyophilization.

### EXAMPLE 14h

Following the procedure of Example 14 except replacing acetimidate with another imidate, products (1) to (3) were obtained:

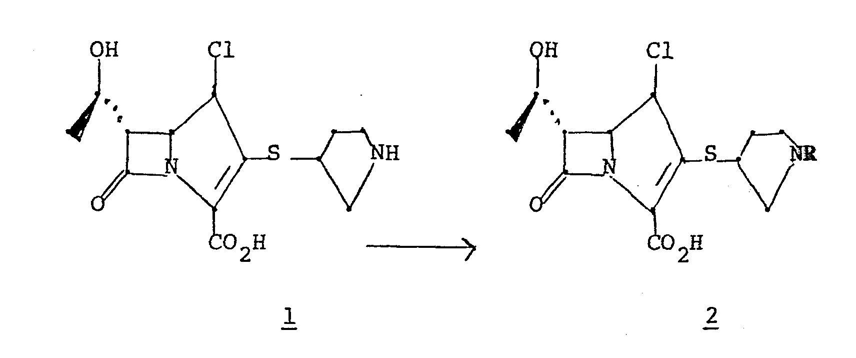

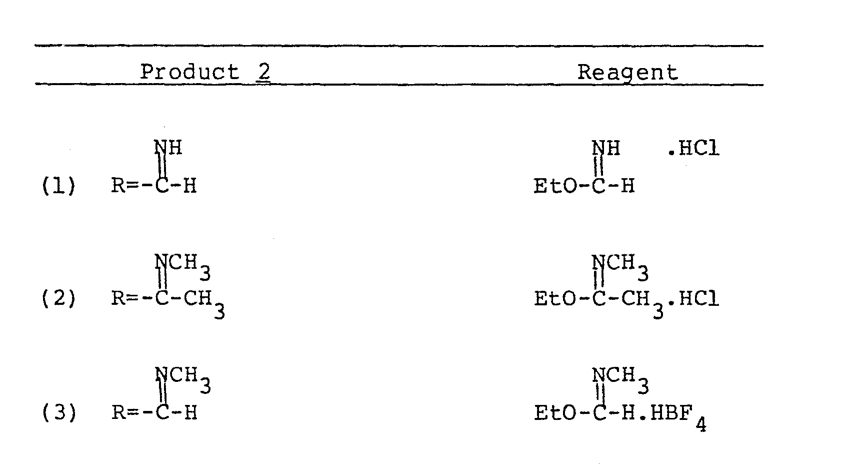

### EXAMPLE 14i

### Preparation of N-quanyl-3-mercaptopyrrolidine

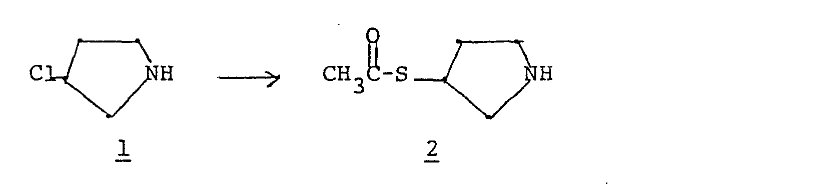

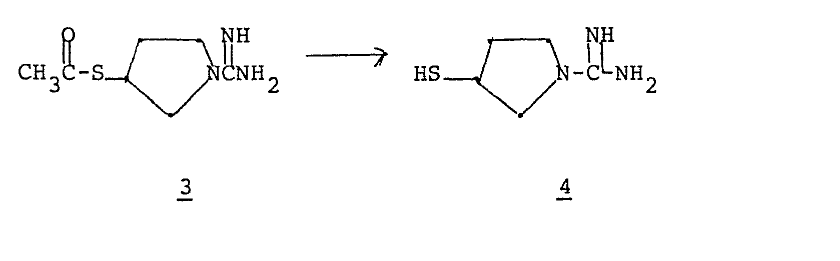

### Step A: Preparation of 3-(S-acetylthio)pyrrolidine 2 3-Chloro-pyrrolidine 1 was prepared according to a known procedure (British Patent 1,042,894).

The starting material 1 (1.05 g) was dissolved in 5 ml DMF and treated with sodium thiolacetate (0.5 g) at 60°C for 6 hours. The mixture was diluted with 20 ml ethyl acetate and washed with 10% NaHCO₃, water and brine. The organic layer was separated, dried over Na₂S0₄, then evaporated in vacuo to give product 2.

### Step B: Preparation of N-quanyl-3-mercaptopyrrolidine tetrafluoroborate

The 3-(S-acetylthio)pyrrolidine 2 (1.45 g) was dissolved in 5 ml DMF and treated with 2-methyl-2-thiopseudourea sulfate (0.56 g) and triethylamine (0.4 ml) at room temperature for 16 hours. The mixture hydrolyzed in water (5 ml) which was adjusted to pH 10 with IN NaCH at 0°C. After 30 minutes reaction, the mixture was acidified with HBF₄ to pH 2.0 and extracted wth ethyl acetate. The aqueous layer was separated and mixed with methanol to precipitate the product. The crude product was purified by crystallization from hot ethanol.

### EXAMPLE 14j

### Preparation of N-imidoyl-3-mercaptopyrrolidine hydrochloride

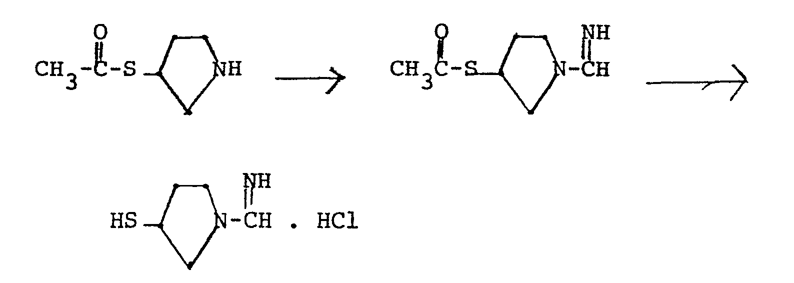

The 3-(S-acetylthio)pyrrolidine 1 (1.45 g) in 5 ml DMF was treated with O-ethyl formimidate hydrochloride (0.5 g) and triethylamine (0.4 ml) at room temperature for 6 hours. The mixture was hydrolyzed with 0.1 N NaOH (5 ml) at 0°C for 30 minutes, then acidified with HC1 to pH 2.0 and extracted with ethyl acetate. The aqueous layer was separated and mixed with methanol to precipitate product 3 which was purified by crystallization from methanol-H₂0.

### EXAMPLE 14k

### Preparation of l-acetylamino-2-(N-quanyl-3'-pyrrolidyl)-6-(1'-hydroxyethyl)-1-carbapen-2-em-3-carboxylic acid

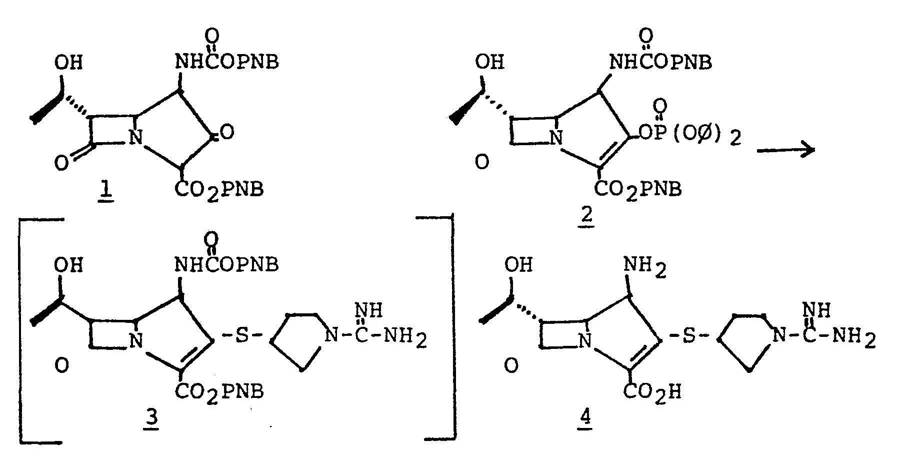

The bicyclic keto ester (33 mg) dissolved in acetonitrile (0.47 ml) at 0°C under N₂ atmosphere was treated with diphenyl chlorophosphate (21 ul) and diisopropylethylamine (20 µl) for 30 minutes. The mixture was cooled to -35°C in an ethylene glycol-water-dry ice bath then treated with DMSO solution of N-quanyl-3-mercaptopyrrolidine hydrochloride (20 mg) and diisopropylethylamine (20 µl) for 30 minutes. The mixture was diluted with 5 ml ether and centrifuged to precipitate oil product 3. The crude product 3 was re-dissolved in THF (3.72 ml) and 0.1 M pH 7.0 phosphate buffer (2.80 ml) and hydrogenated at 50 psi hydrogen in the presence of 50 mg of 10% Pd/C at room temperature for 1 hour, then filtered from catalyst. The filtrate was extracted with ether, concentrated to 4 ml and charged to a Dowex-50x4 (Na⁺ cycle) column (2.2 x 6 cm) which was eluted with D.I. water to give product 4 as solid after lyophilization.

### EXAMPLE 15

Following the procedures described in the foregoing examples and text, the following bicyclic ß-keto esters useful as intermediates in the preparation of the compounds of the present invention are obtained. Remarks relative to procedure are presented in the footnote to Table 1.

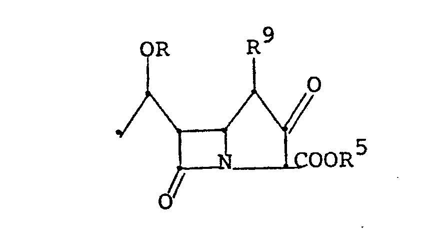

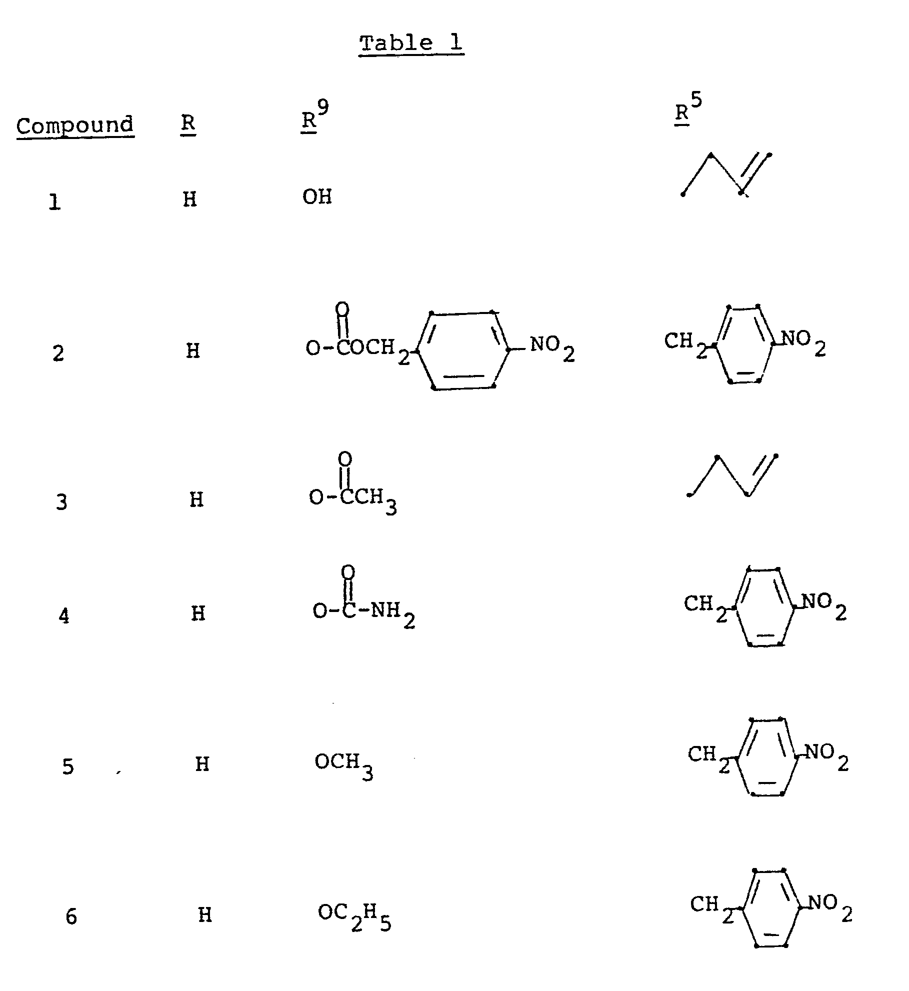

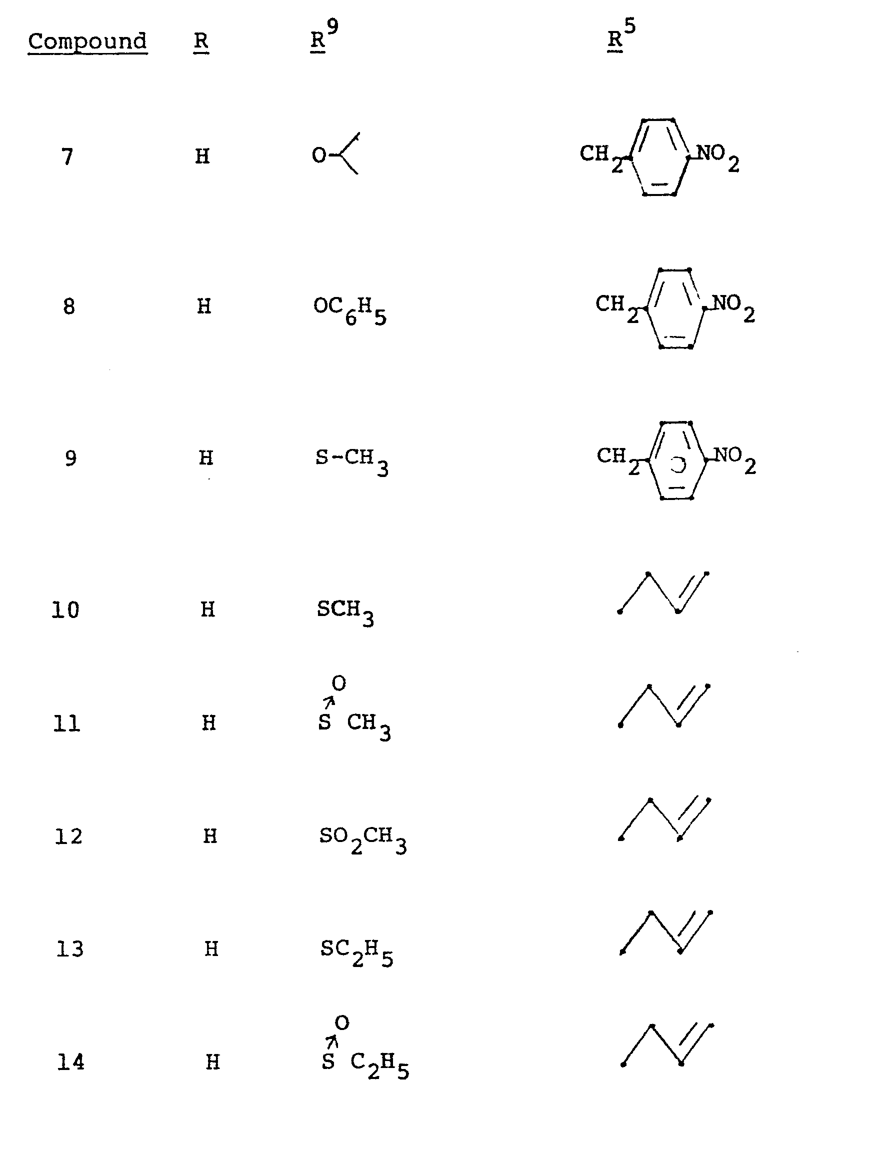

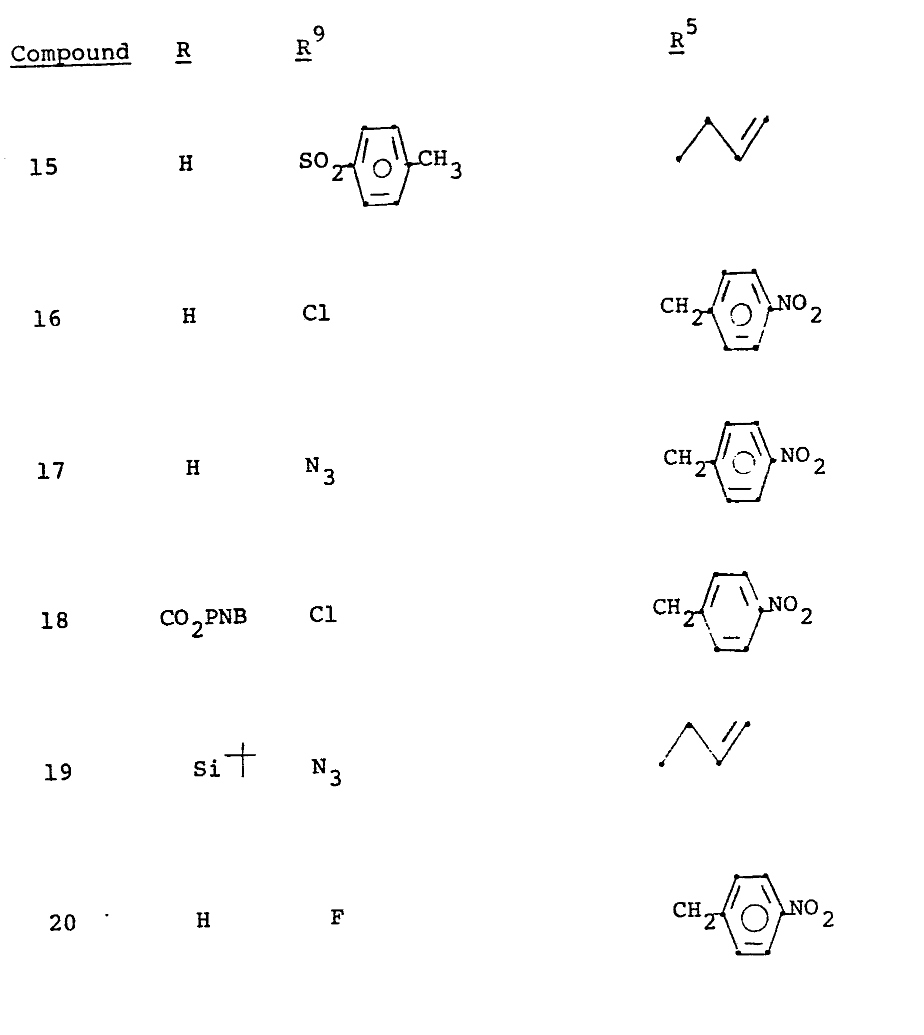

### Footnote to Table 1

1) Starting with the product of Step C, Example 1, using malonic acid mono-allyl ester instead of the corresponding p-nitrobenzyl ester in Step D; followed by Steps E, F and H of Example 1.
2) As described in Example 1.
3) As described in Example 1, except that the product of Step F is treated with 1 equivalent of acetic anhydride instead of p-nitrobenzyloxycarbonylchloride in Step G.
4) As described in Example 1, except that the product of Step E is treated with 1 equivalent of chlorosulfonyl isocyanate, followed by acid hydrolysis in Step F.
5) Starting with the product of Example 5 and carrying it through Steps C, D, E, F and H of Example 1.
6) As for compound 5 except that C₂H₅-OH is substituted for CH₃OH in the procedure of Example 5.
7) As for compound 5 except that isopropyl alcohol is substituted for methanol in the procedure of Example 5.
8) As for compound 5 except that phenol is substituted for methanol in the procedure of Example 5.
9) Starting with the product of Example 6, and carrying it through steps C, D, E, F and H of Example 1, except that malonic acid monoallyl ester instead of malonic acid mono p-nitrobenzyl ester is used in Step D of Example 1.
10) As for compound 9 except that the procedure of Example 6 is repeated on the product of Example 6.
ll) As for compound 9 except that the product of Example 6 is oxidized with 1 equivalent of m-chloroperbenzoic acid in methylene chloride in solvent at room temperature before carrying it through further procedures.
12) As for compound 11 except that the product of Example 6 is oxidized with 2 equivalents of m-chloroperbenzoic acid.
13) As described for compound 9 except that diethyl disulfide replaces dimethyldisulfide in the procedure of Example 6.
14) As for compound 13 except that the product from the procedure of Example 6 is oxidized with 1 equivalent of m-chloroperbenzoic acid in methylene chloride.
15) As for compound 11 except that the product of the procedl e of Example 6 is oxidized with 1 equivalent of m-chloroperbenzoic acid in methylene chloride.
16) Starting with compound 5 of Example 4 and carrying it through Steps D to G of Example 4 except using allyl as a protecting group.
17) Treating compound 9 of Example 4 with lithium azide in DMF.
18) Treating compound 9 of Example 4 with p-nitro- benzoxylchloroformate and p-dimethylaminopyridine in DMF.
19) Treating compound 16 of Table 1 with lithium azide then silylating the product with t-butyl- dimethylchlorosilane/imidazole in DMF.
20) Treating compound 16 of Table I with lithium fluoride in DMF.

### EXAMPLE 16

Following the procedures of the foregoing examples and text, the following compounds of the present invention are obtained. Remarks relative to the procedures are presented in the footnote to Table 2.

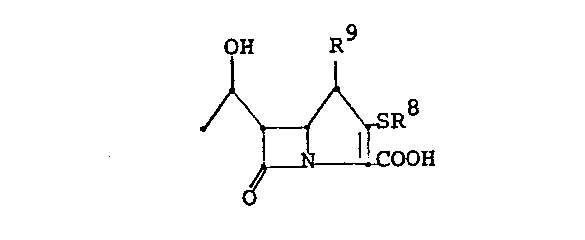

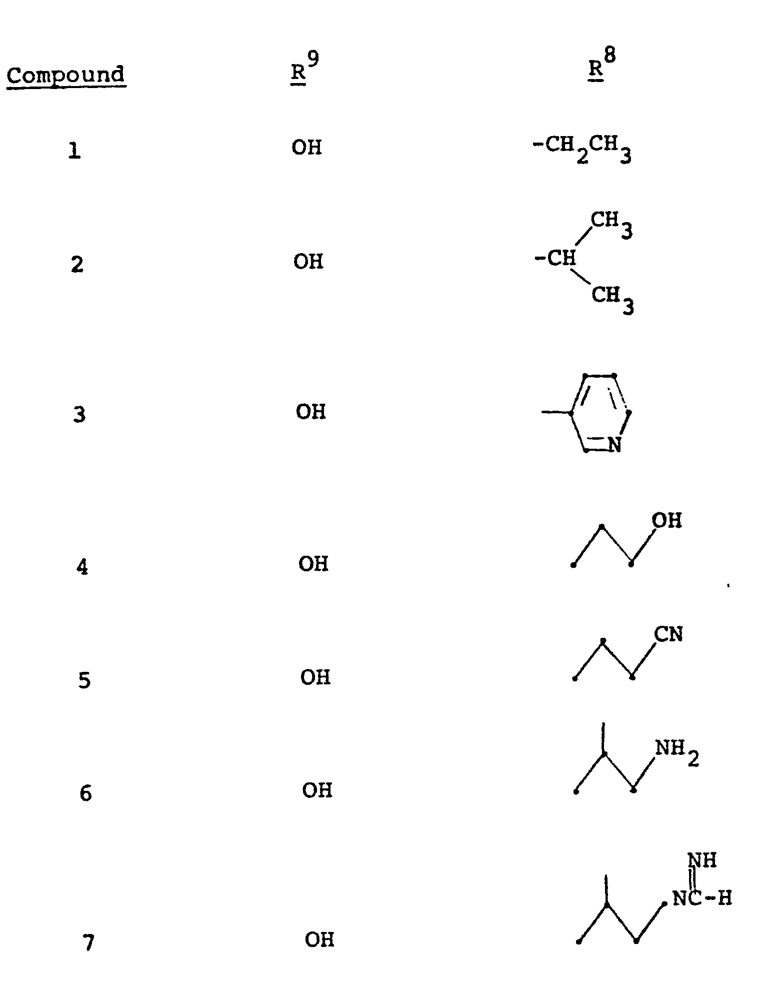

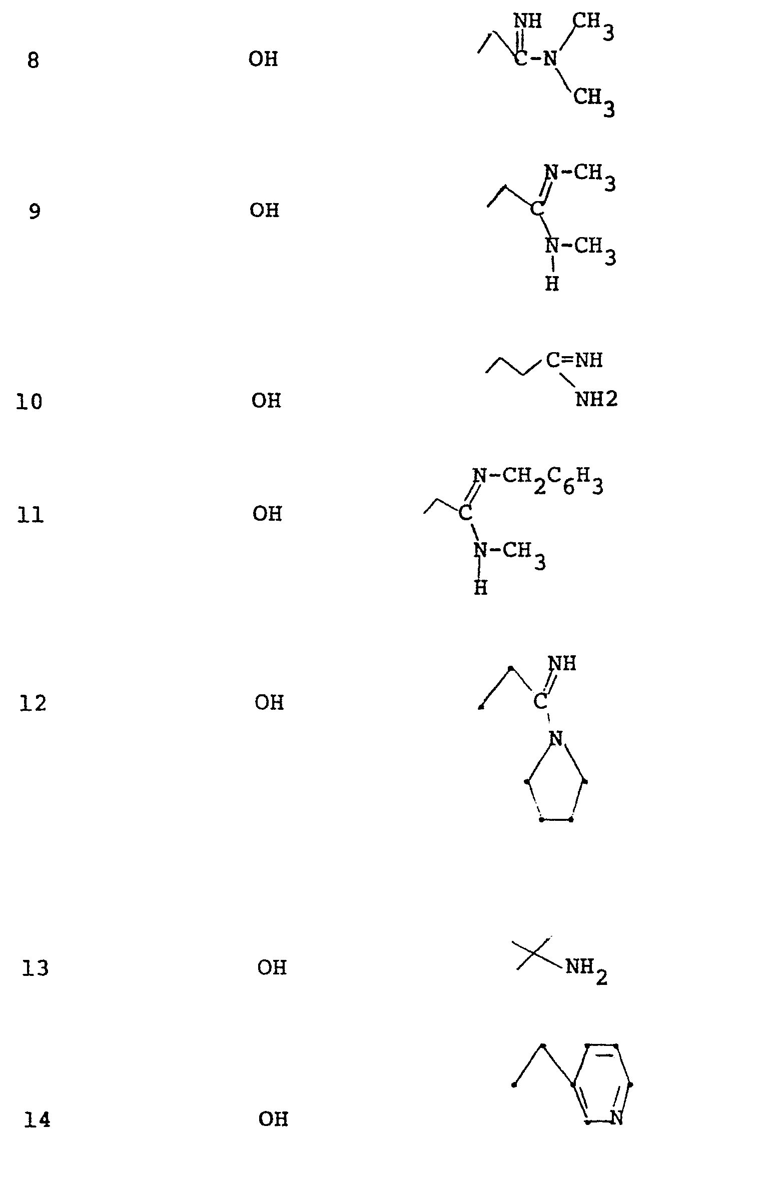

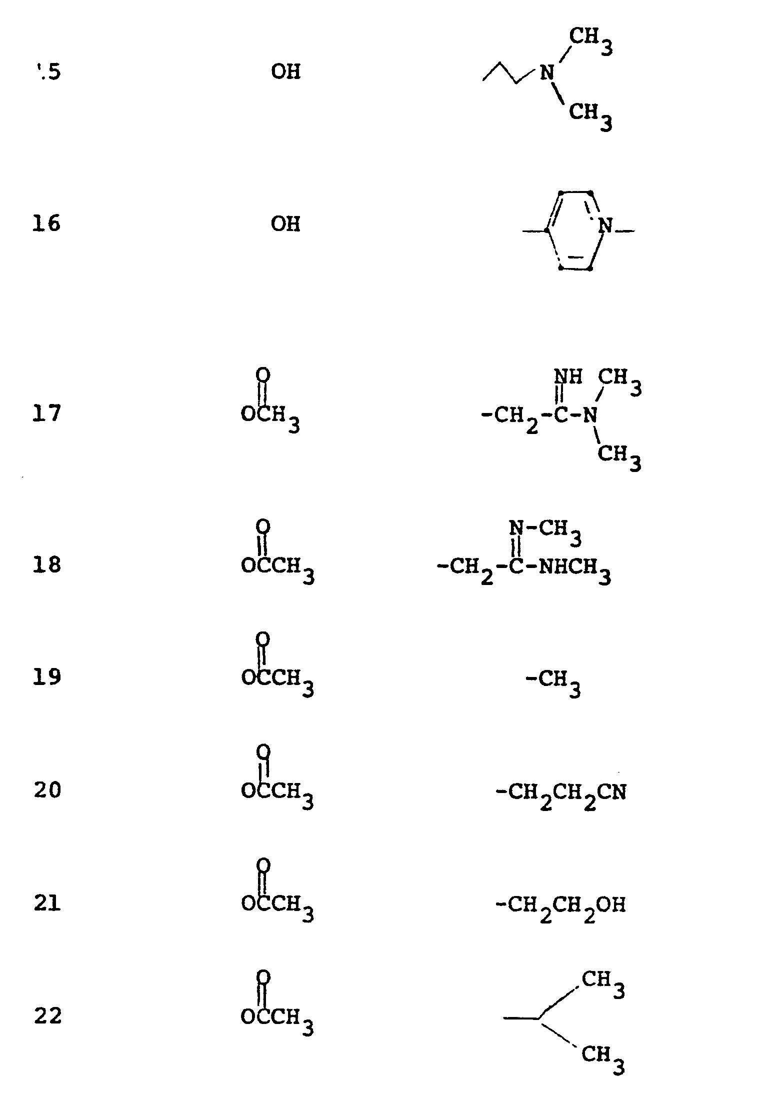

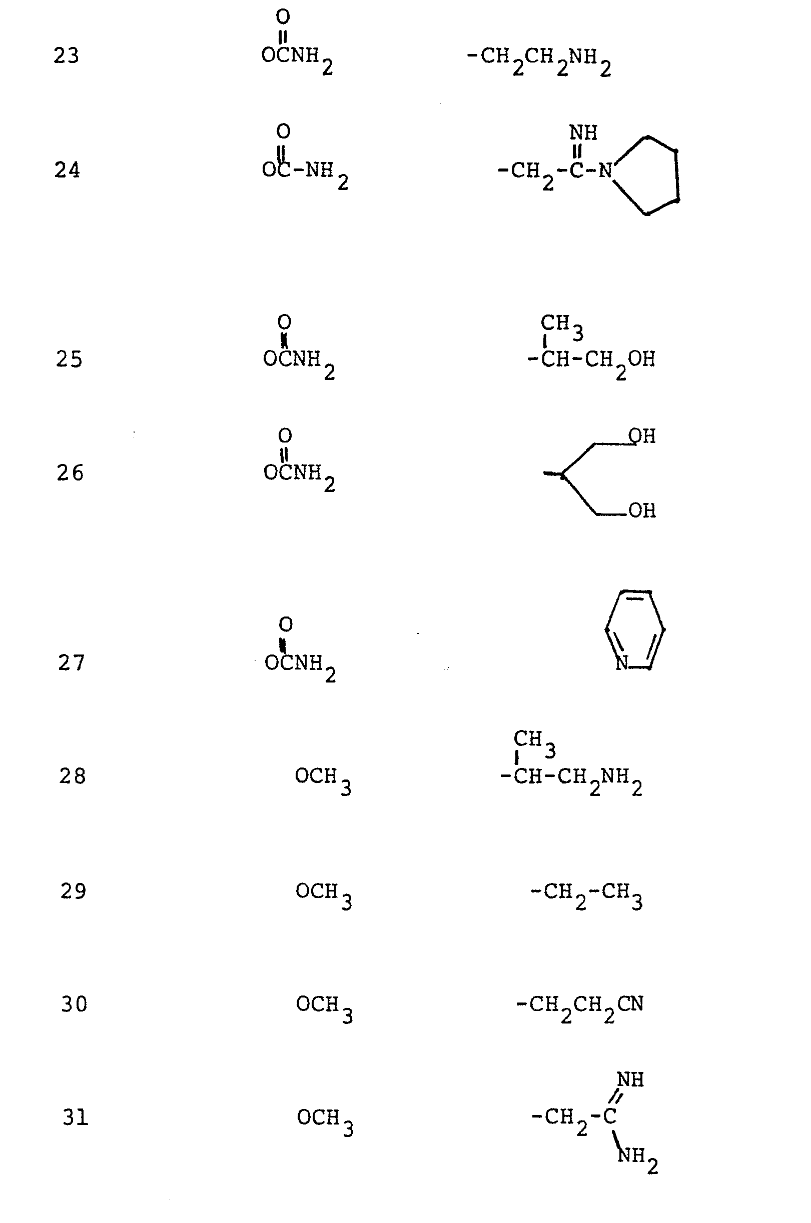

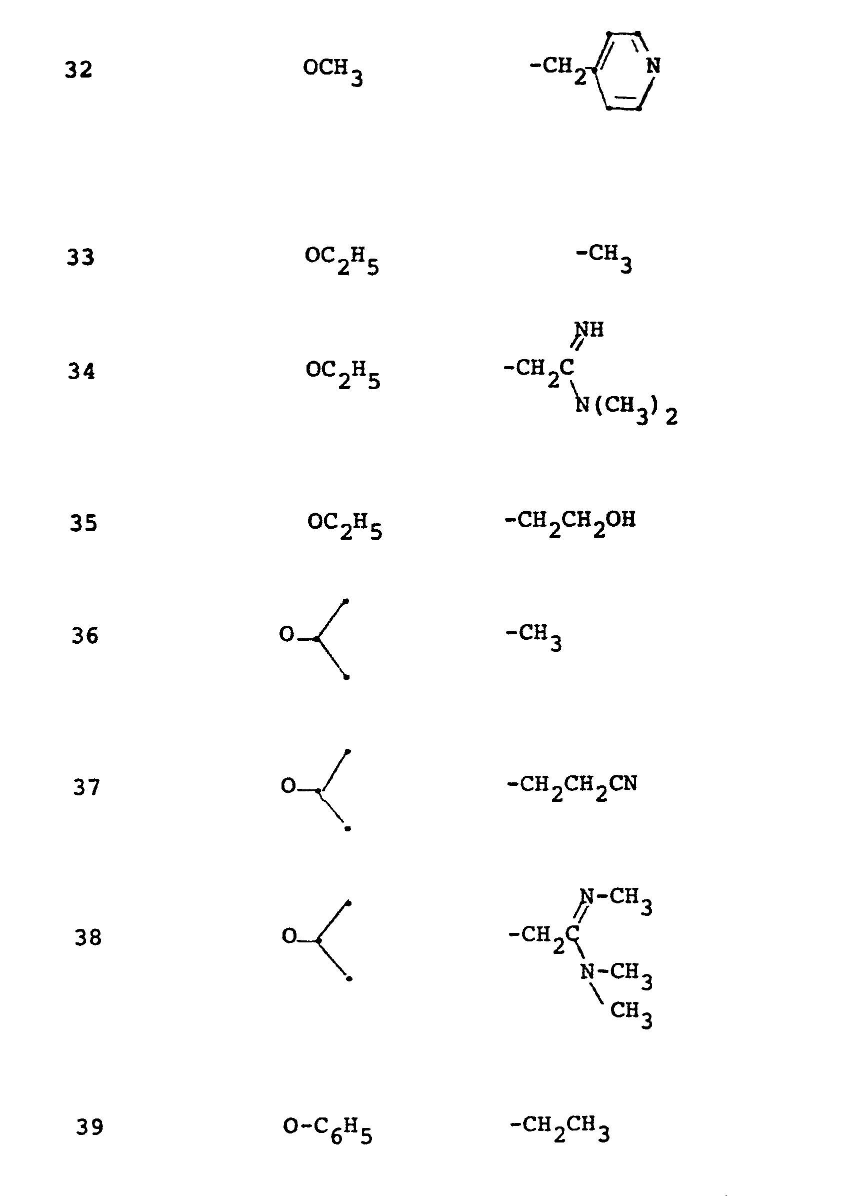

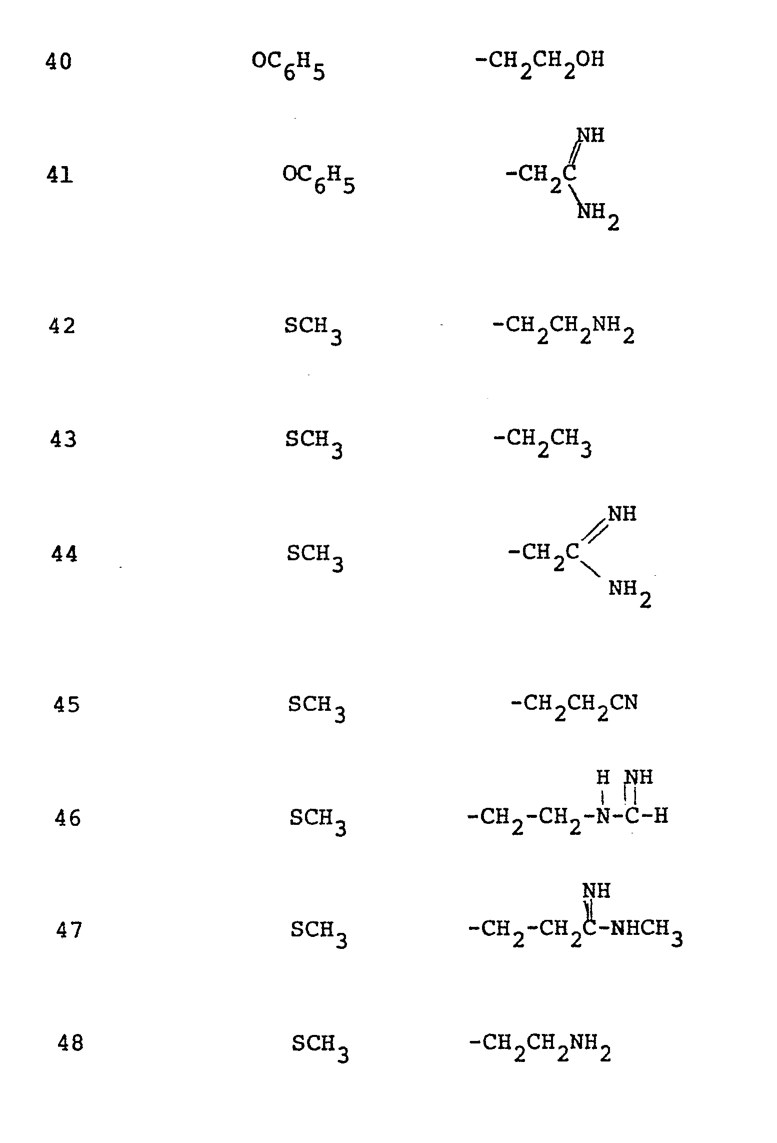

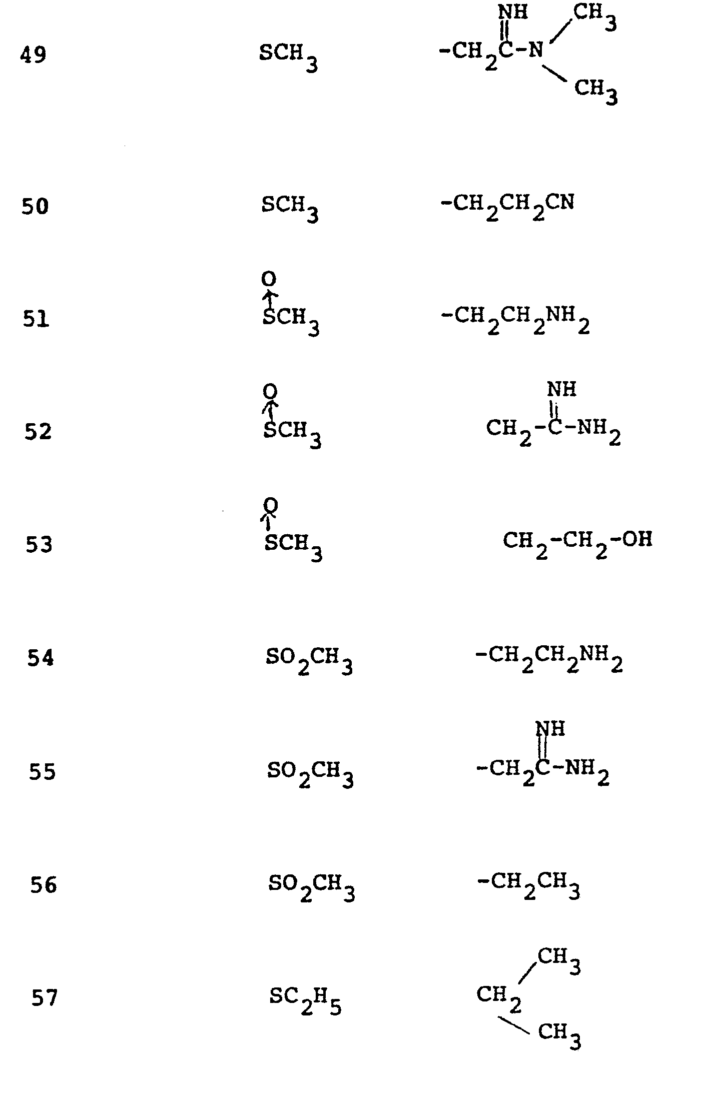

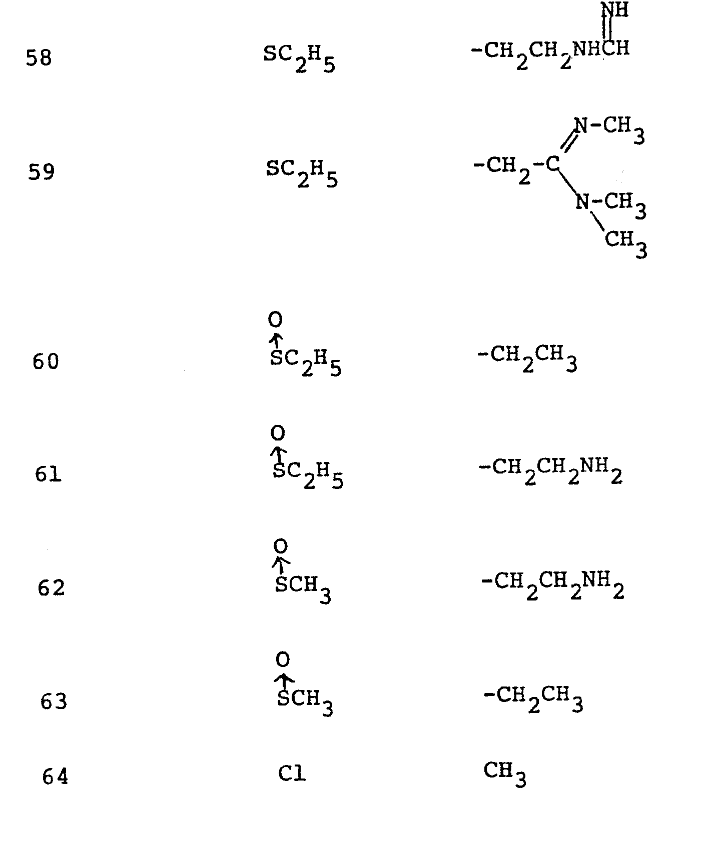

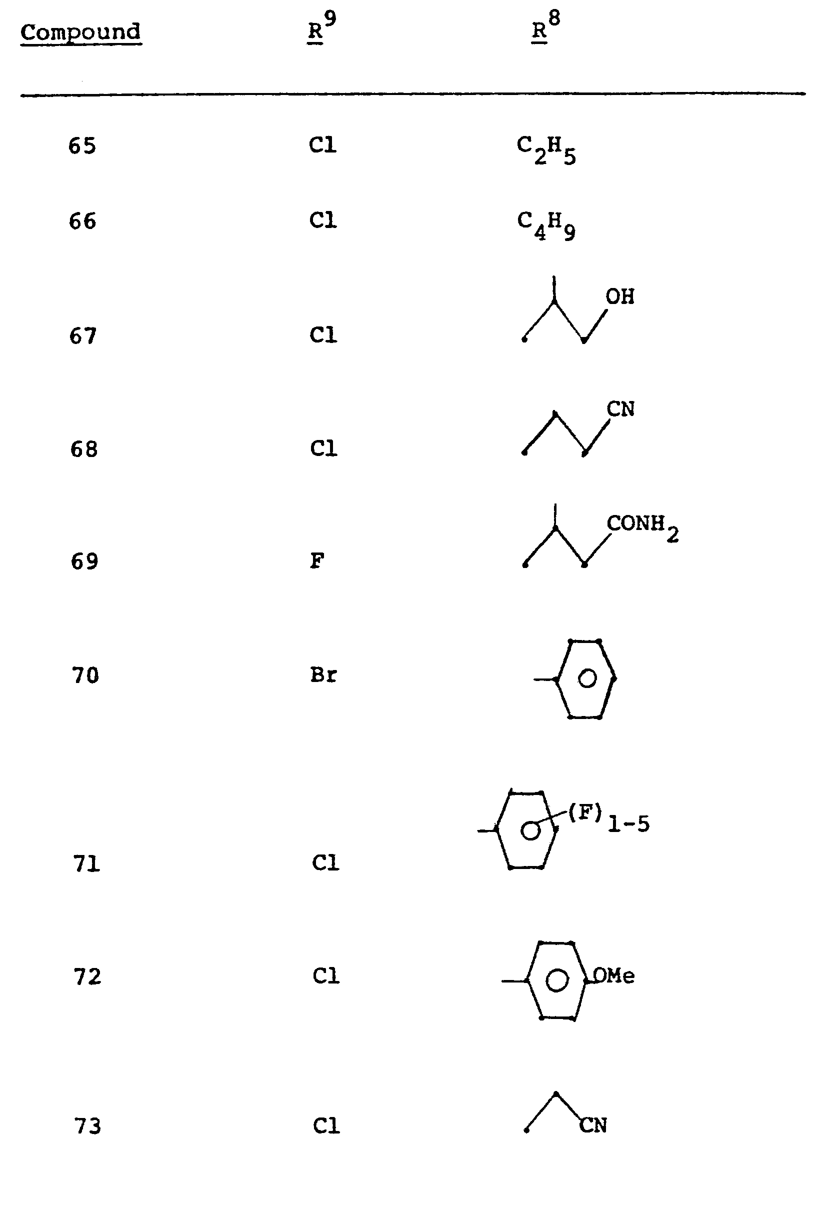

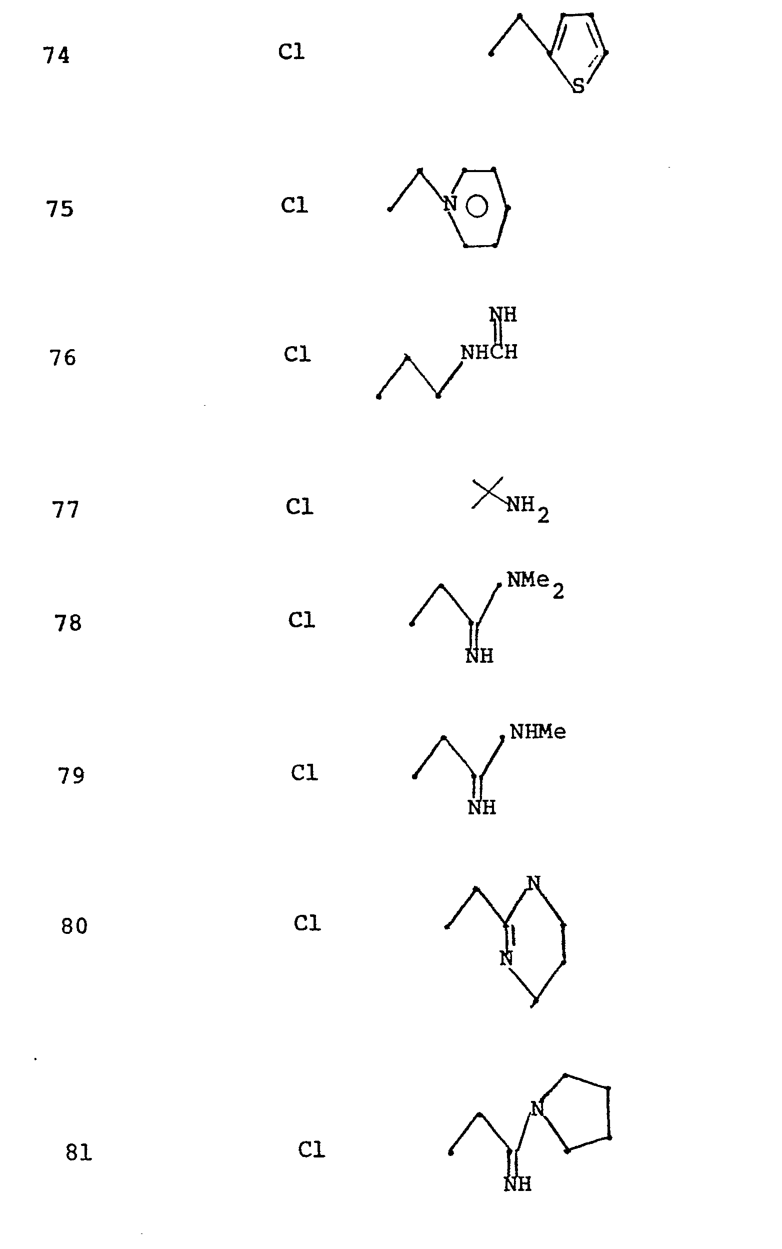

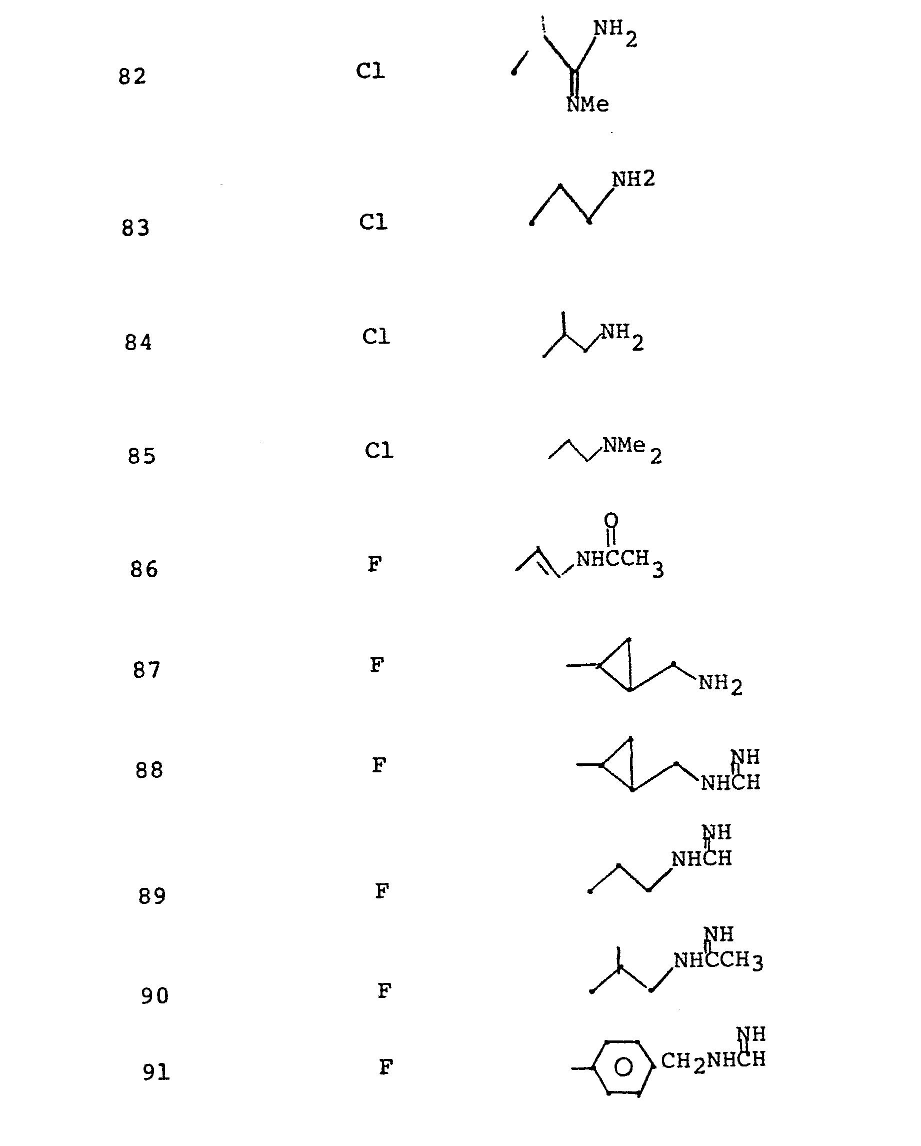

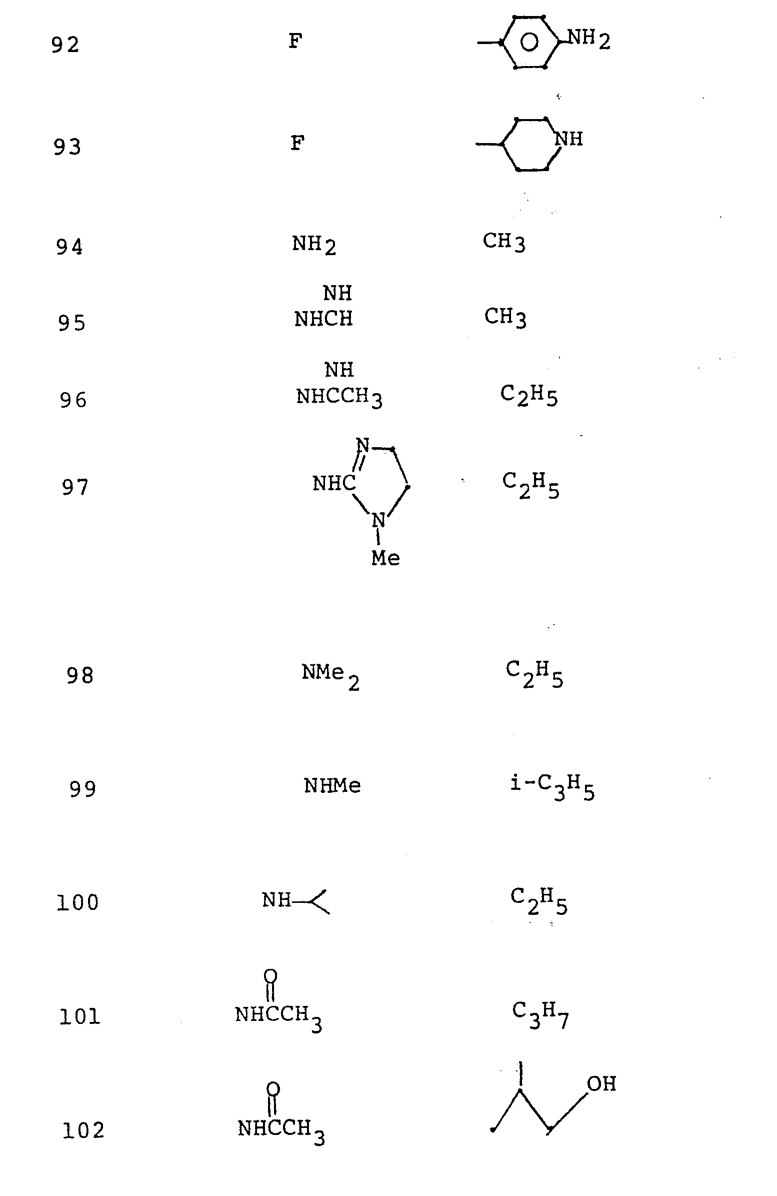

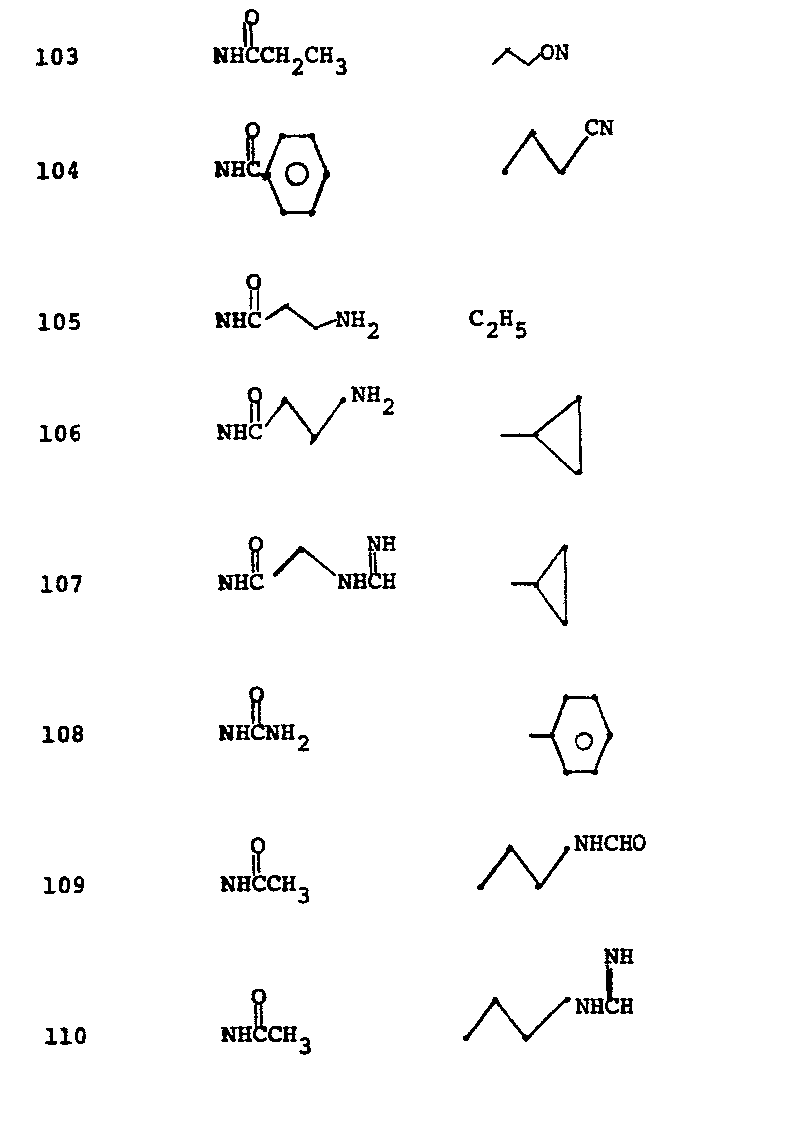

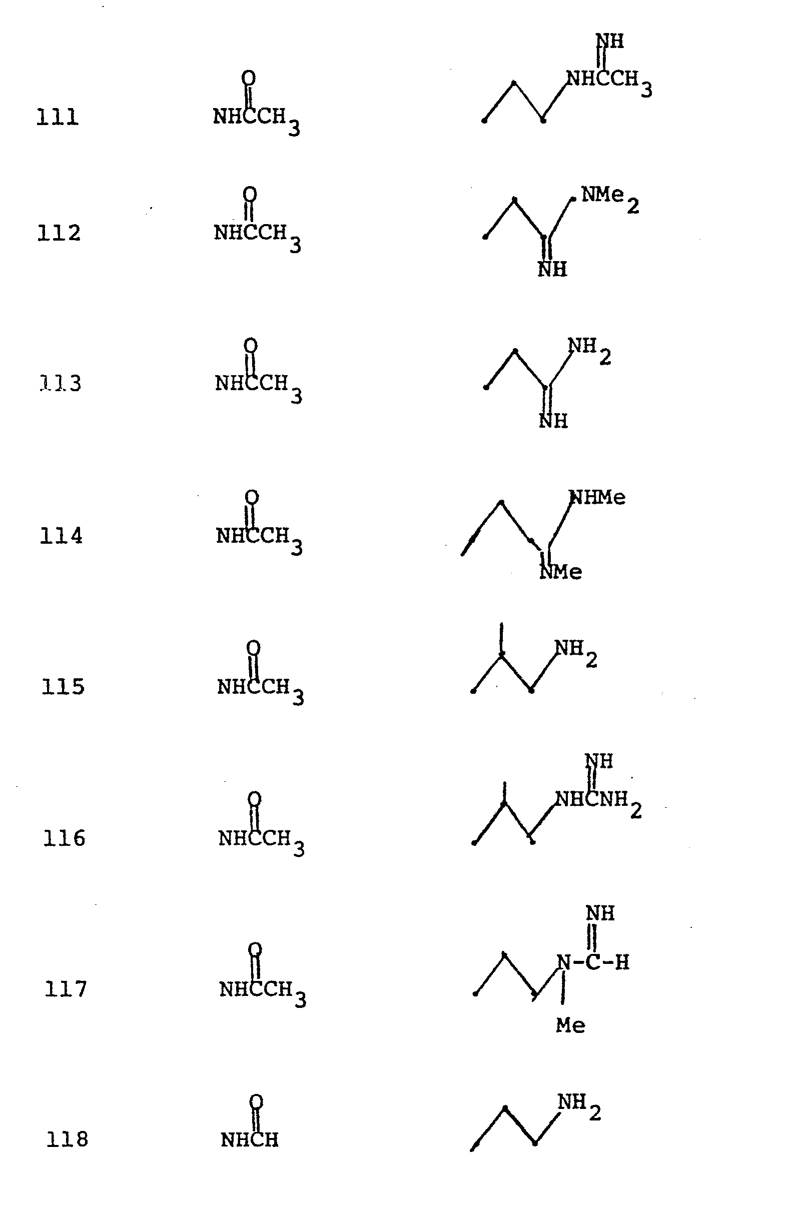

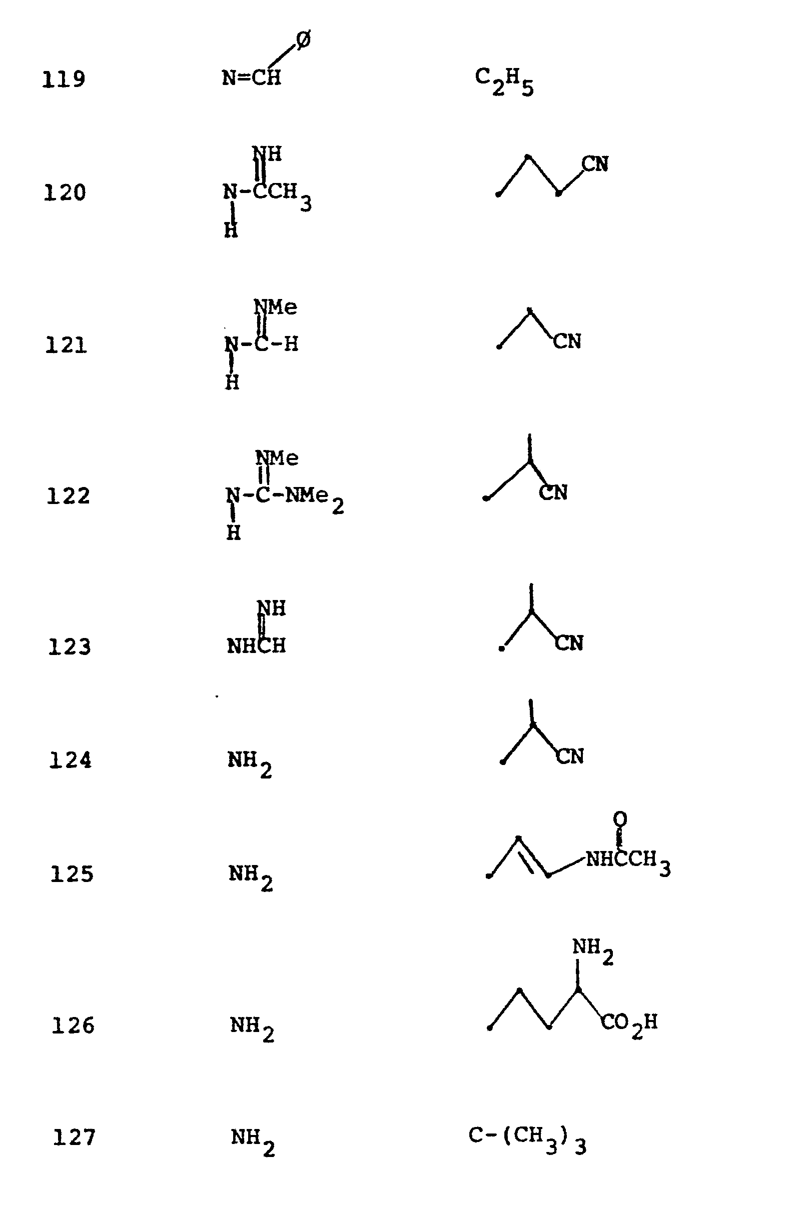

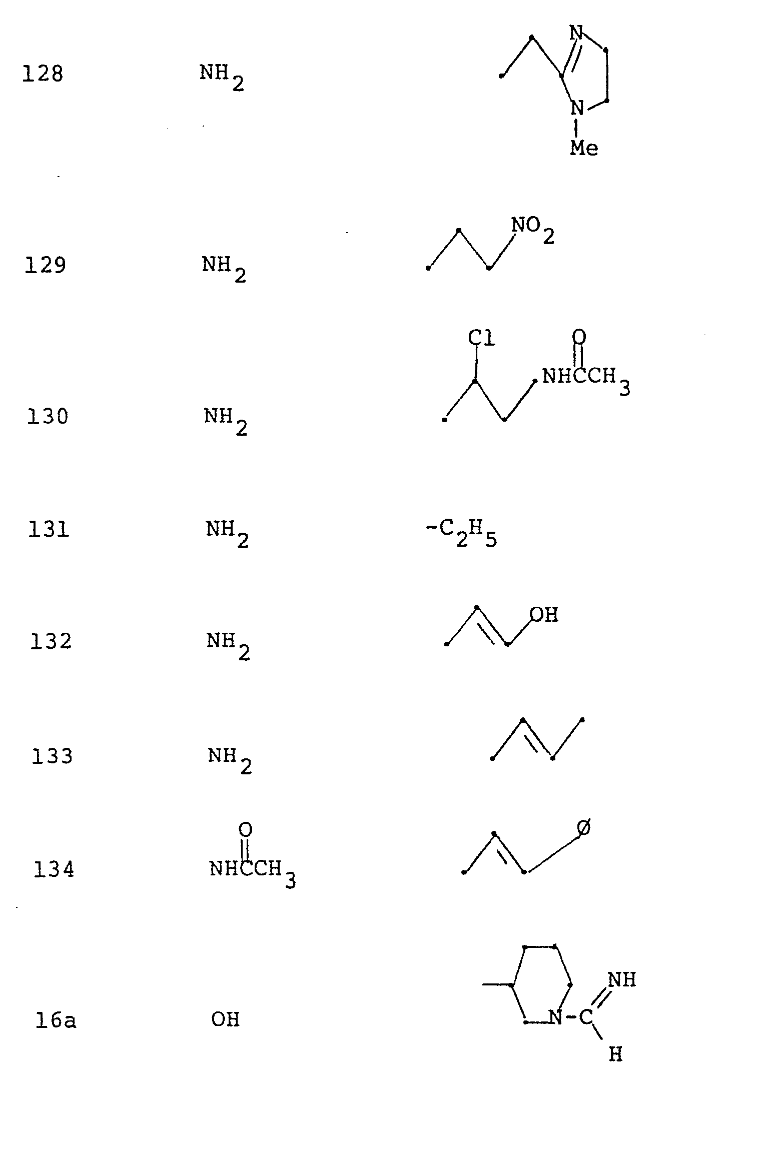

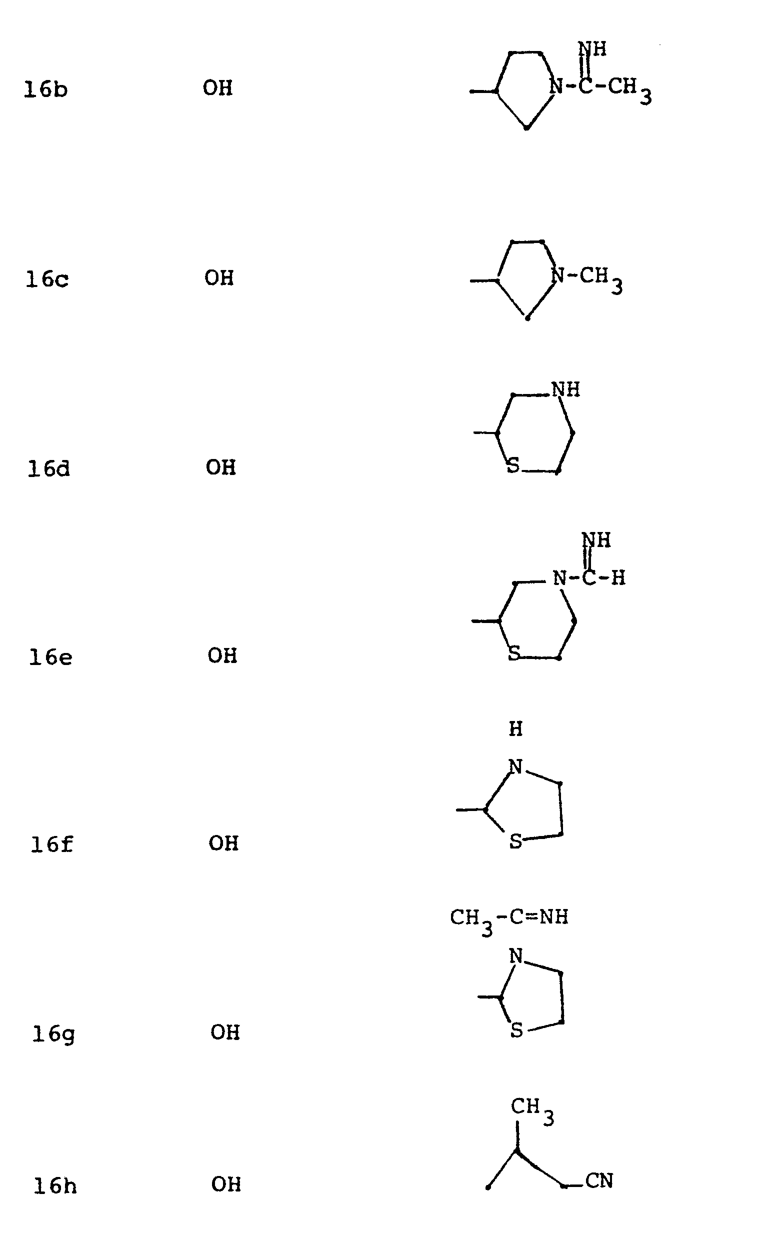

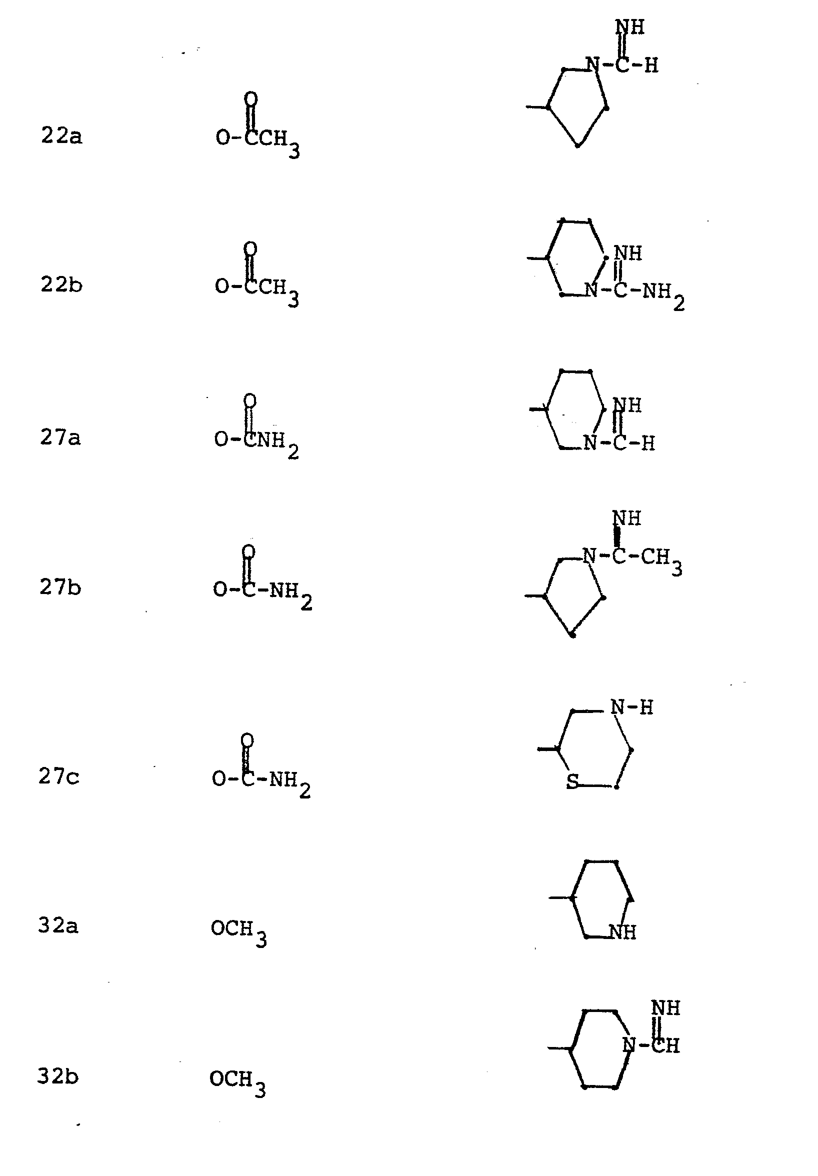

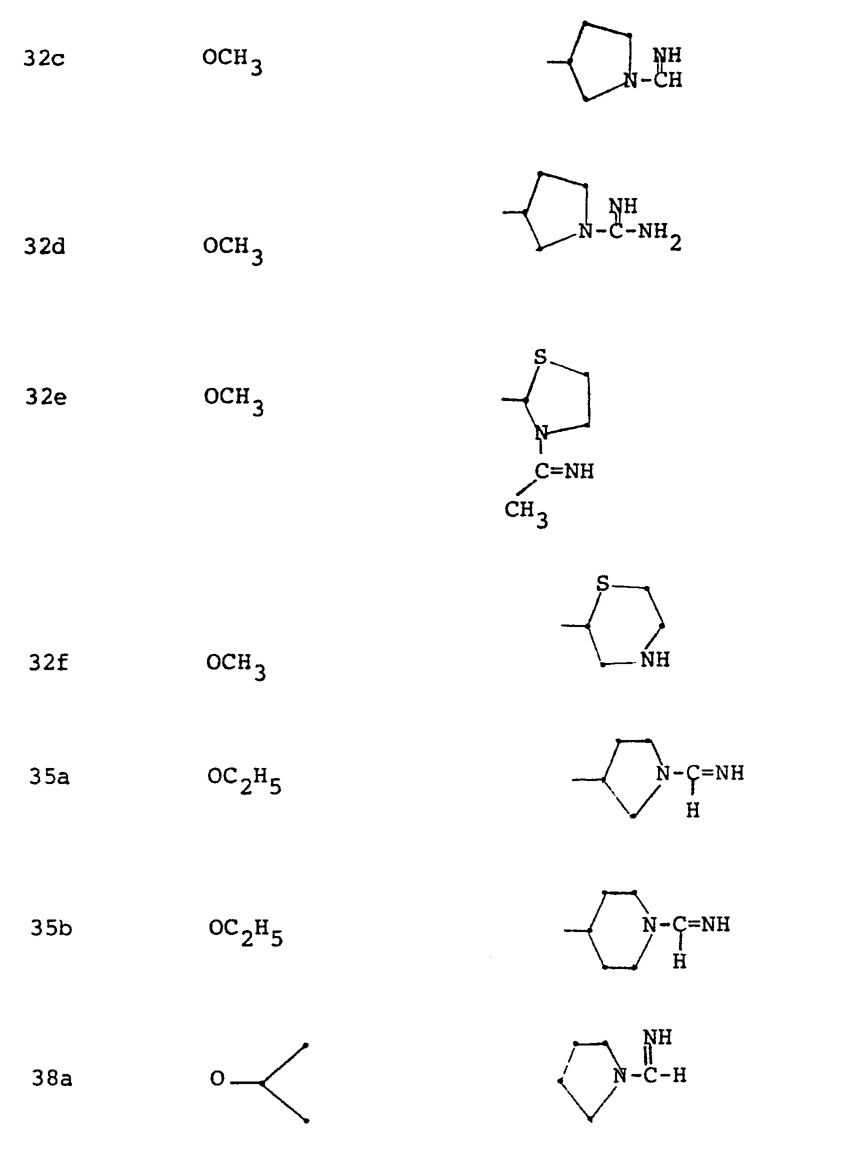

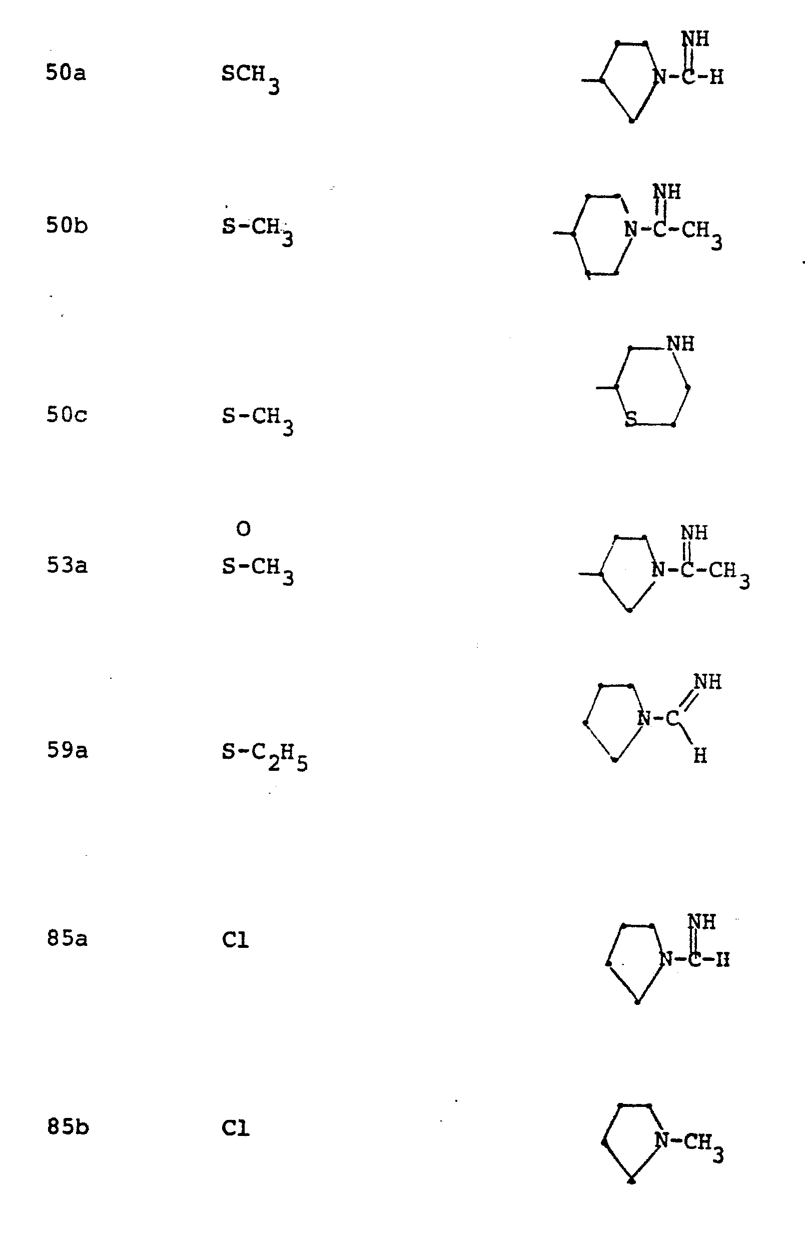

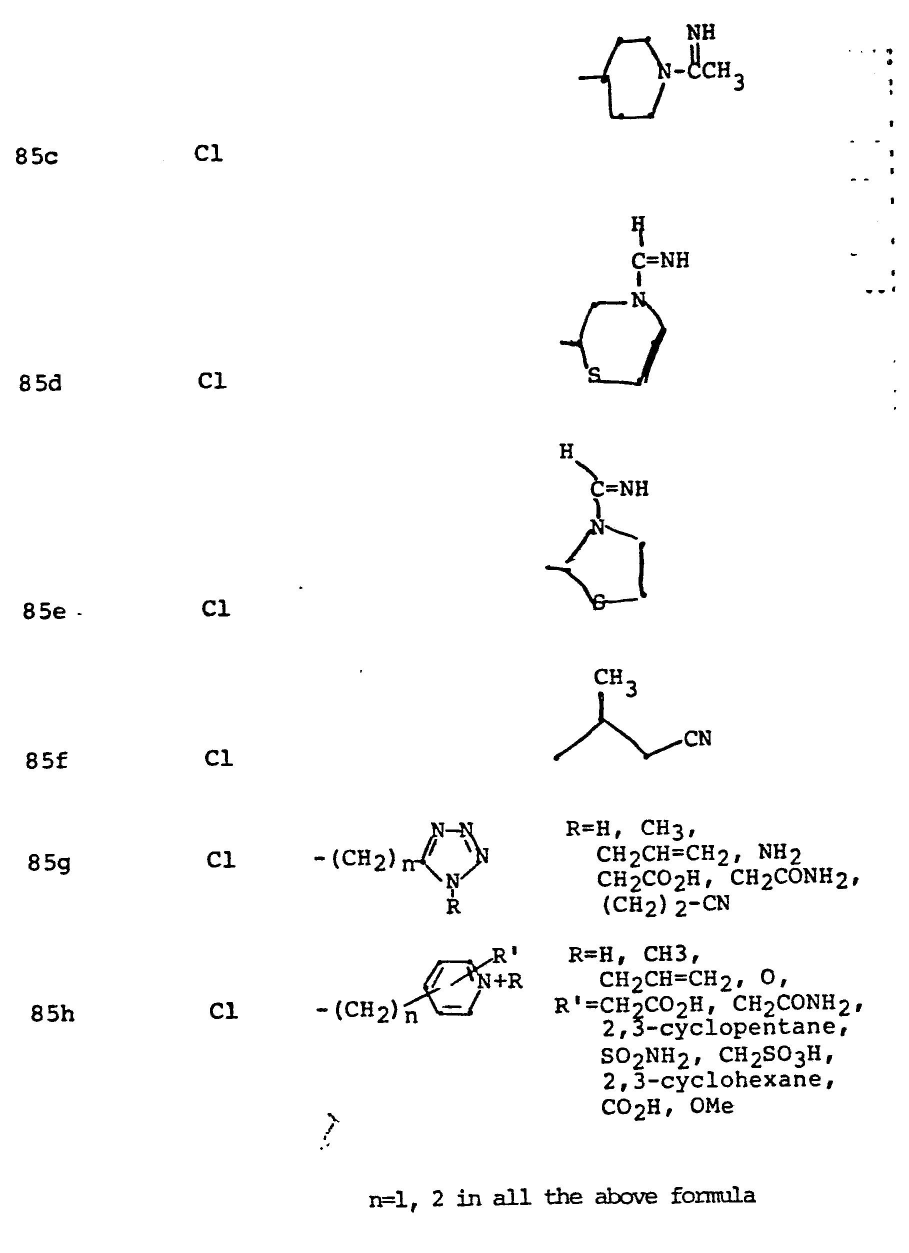

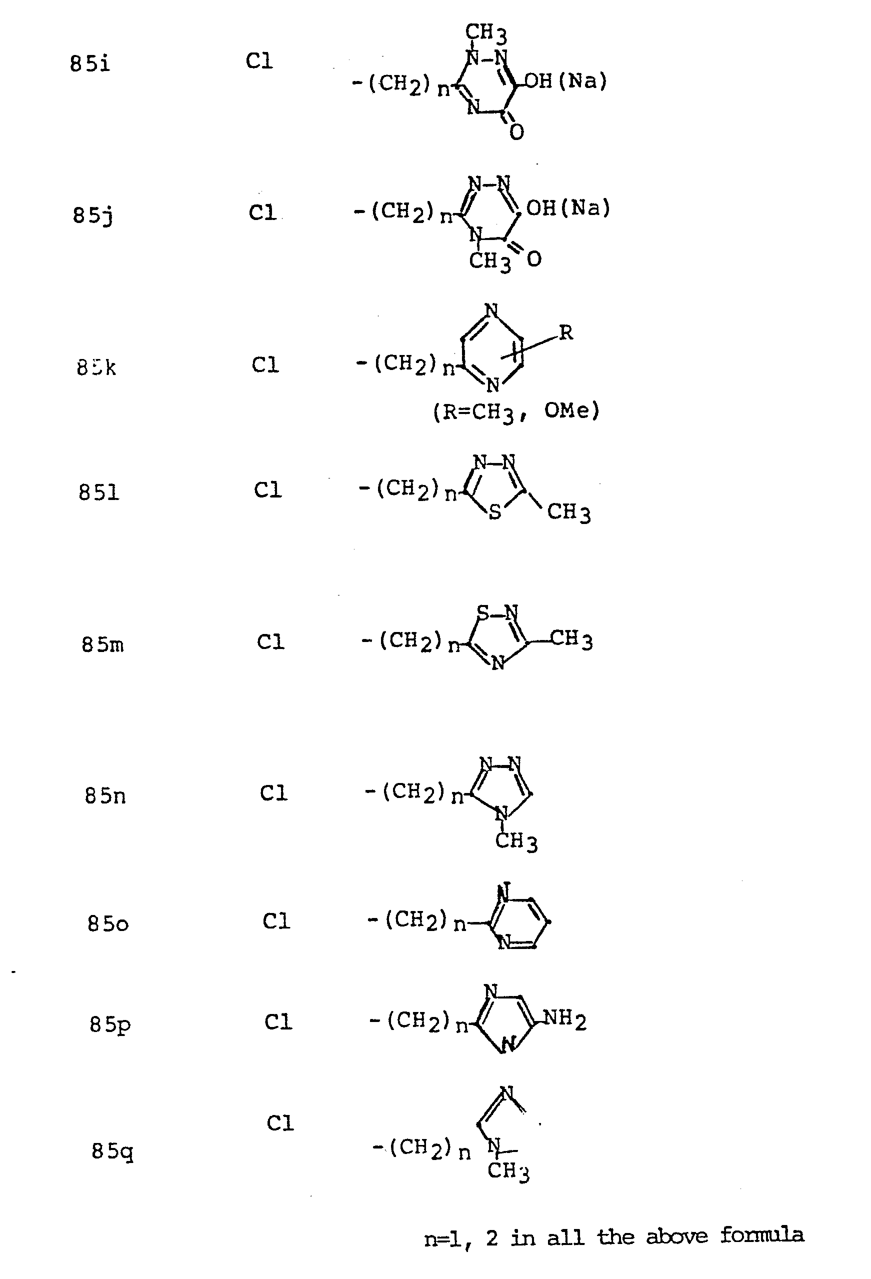

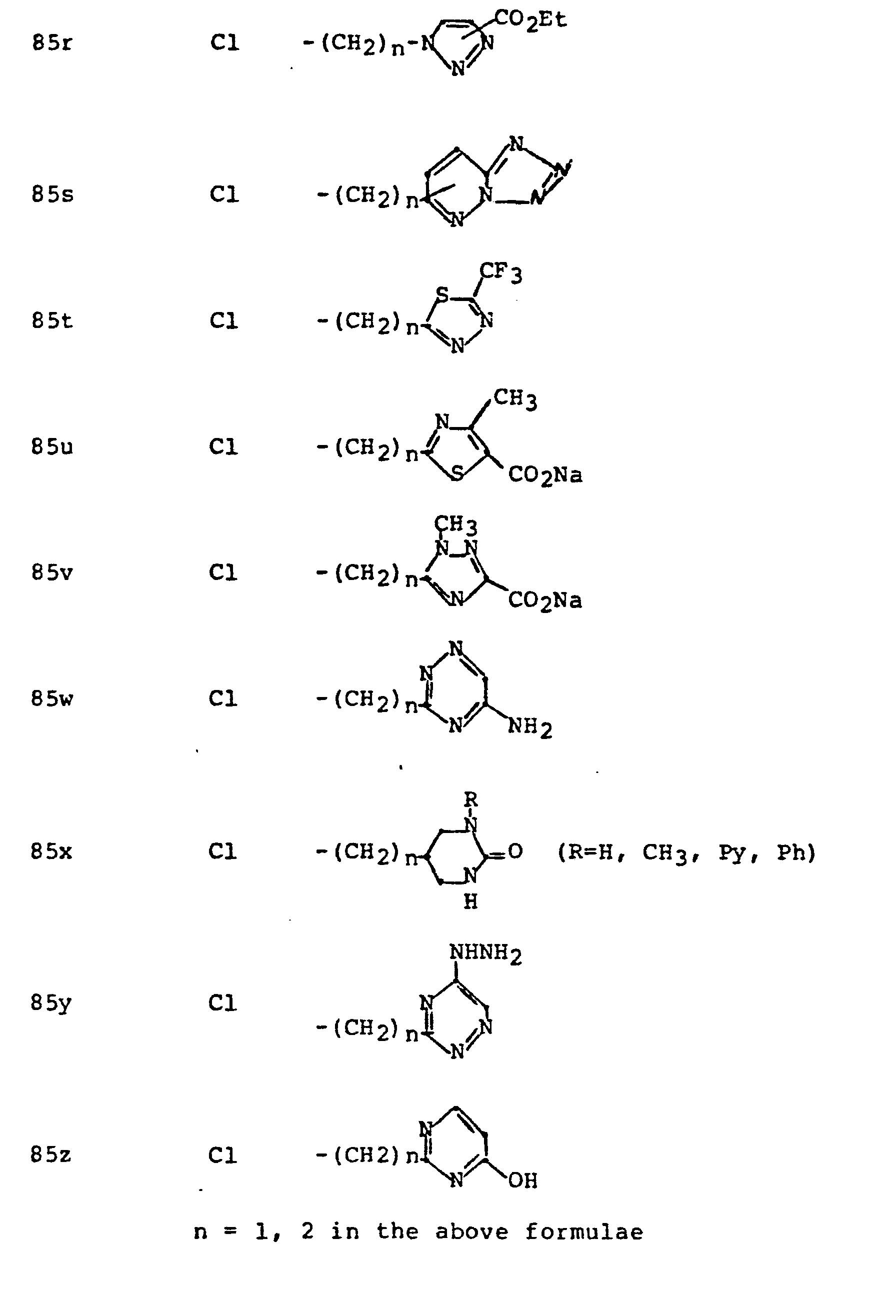

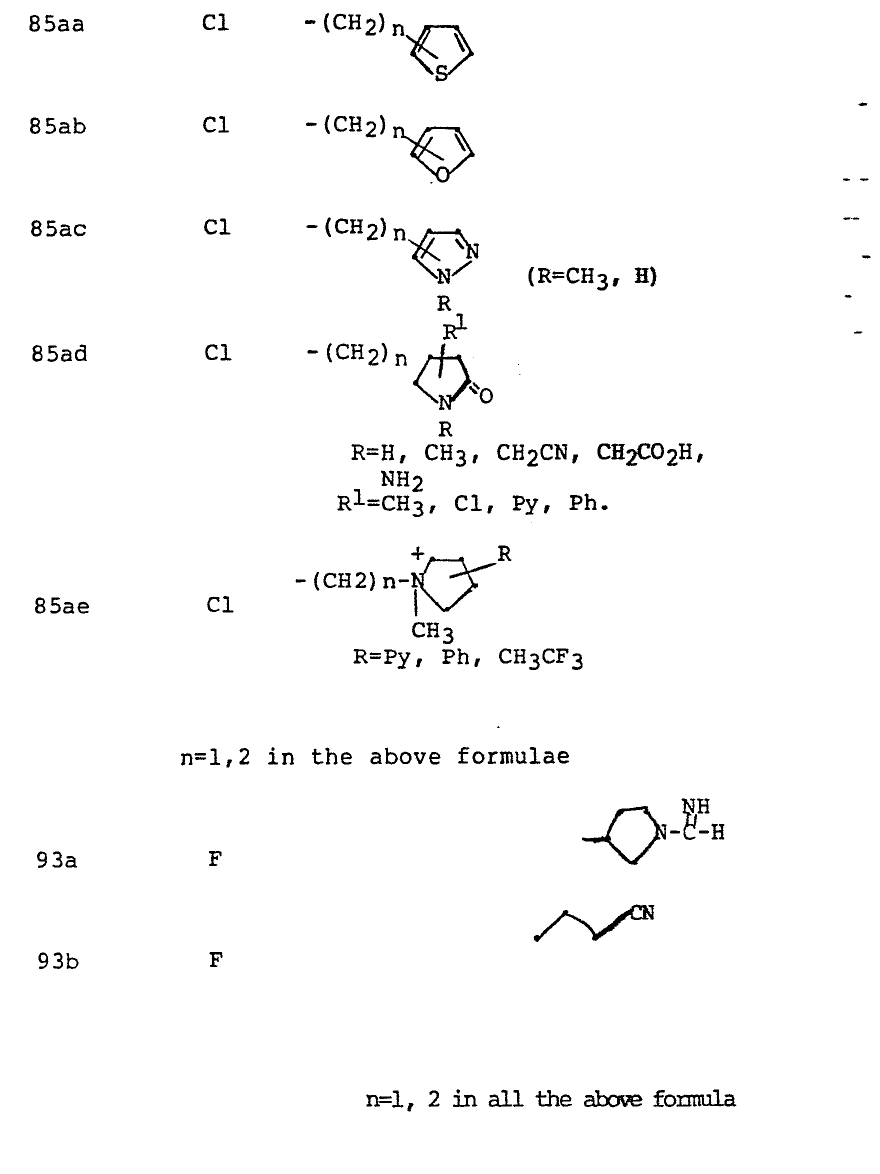

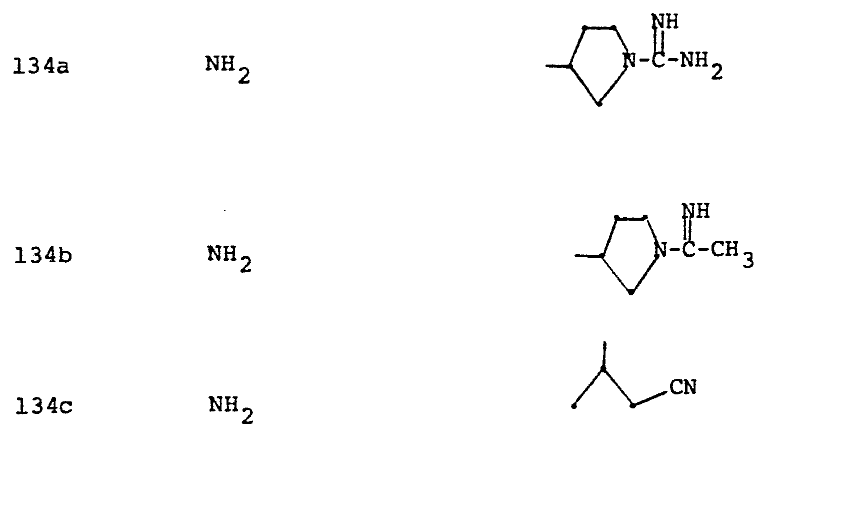

### Footnote to Table 2

1) Starting with the compound of Table 1 and carrying it through Steps I and J of Example 1, except that CH₃CH₂SH is used in place of 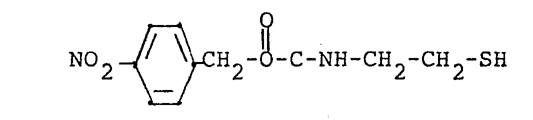 (N-CBZ-CA)
2) As for compound 1 except that 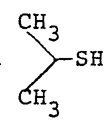 is used instead of CH₃CH₂SH.
3) As for compound 1 except that 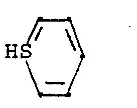 is used instead of CH₃CH₂SH.
4) As for compound 1 except that 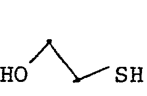 is used instead of CH₃CH₂S^{H}.
5) As for compound 1 except tht 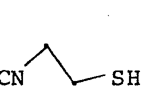 is used instead of CH₃CH₂^{SH.}
6) As for compound 1 except that 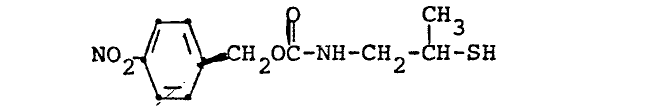
7) By treatment of compound 6 with 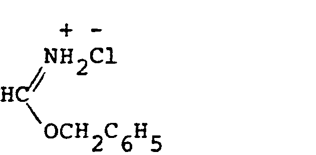 and NaOH at pH 8.5 in H₂0 at (
8) As for compound 1 except that 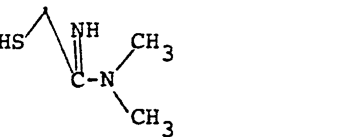 is used instead of CH₃CH₂SH and the product of Step I is not isolated.
9) As for compound 8 except that NH 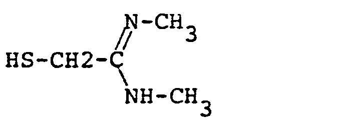 is used instead of 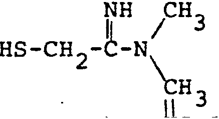
10) As for compound 8 except that 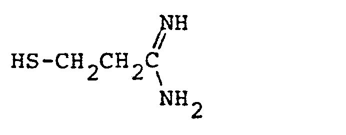 is used instead of 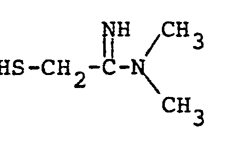
11) As for compound 8 except that NH 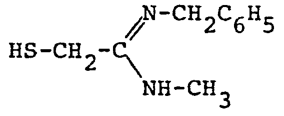 is used instead of 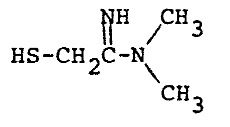
12) As for compound 8 except that 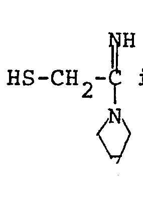 is used instead of HS- 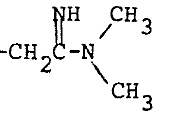
14) As for compound 1 except that 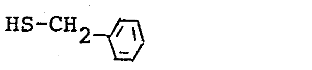 is used instead of CH₃CH₂SH
15) As for compound 8 except that 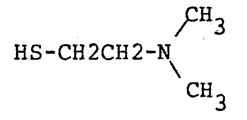 is used instead of 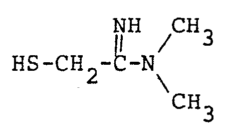
16) As for compound 1 except that HS 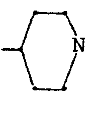 is used instead of HS-CH₂CH₃
17) Starting with compound 3 of Table 1 and carrying it through Step I of Example 1 usinc 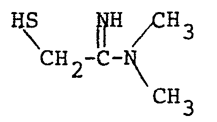 instead of N-CBZ-CA; then deblocking the allyl ester using catalytic amounts of Pd (Ø₃P)₄, (Ø)₃P and 1.1 equivalent of potassium ethyl hexanoate at 0° in ethylacetate. The compound is isolated as the Zwitter-ion.
1 18) As for compound 17 except that 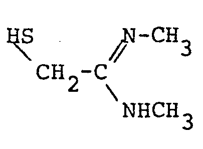 is used instead of 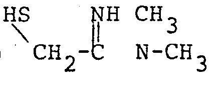
19) Starting with compound 3 of Table 1 and carrying it through Step I of example 1 using CH₃SH instead of N-CBZ-CA and deblocking the allyl ester of the product using catalytic amounts of Pd(Ø3P)4 and (Øl₃P and 1.1 equivalent of potassium ethyl hexanoate at 0° in ethyl acetate. The compound is isolated as the sodium salt.
20) As for compound 19 except that HSCH₂CH₂CN is used instead of CH₃SH.
21) As for compound 19 exept that HSCH₂CH₂OH is used instead of CH₃SH.
22) As for compound 19 except that 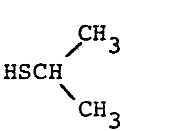 is used instead of CH₃SH.
23) Starting with compound 4 of Table 1 and carrying it through Steps I and J of Example 1.
24) As for compound 23 except that 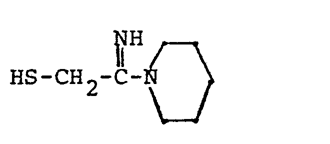 is used instead of N-CBZ-CA and the product of Step I is not isolated.
25) As for compound 22 except that 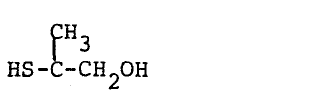 is used instead of N-CBZ-CA
26) As for compound 23 except that 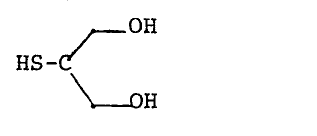 is used instead of N-CBZ-CA
27) As for compound 23 except that 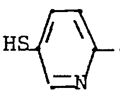 is used instead of N-CBZ-CA
28) Starting with compound 5 of Table 1 and taking it through Steps I and J of Example 1 except that 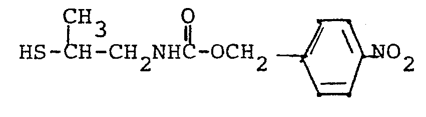 is used in Step I instead of N-CBZ-CA
29) As for compound 28 except that HS-CH₂CH₃ is used instead of N-CBZ-CA in Step I
30) As for compound 28 except that HS-CH₂CH₂CN is used instead of N-CBZ-CA in Step I
31) As for compound 28 except that 1 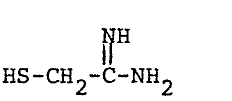 is used instead of N-CBZ-CA in Step I and the product of Step I is not isolated
32) As for compound 28 except that 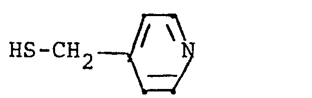 is used instead of N-CBZ-CA in Step I
33) Starting with compound 6 of Table 1 and taking it through Steps I and J of example 1 except that CH₃SH is used instead of N-CBZ-CA in Step I
34) As for compound 33 except that 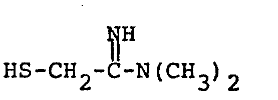 is used instead of CH₃SH and the product of Step I is not isolated
35) As for compound 33 except that HS-CH₂CH₂0H is used instead of CH₃SH
36) Starting with compound 7 of Table 1 and carrying it through Steps I and J of Example 1 but using CH₃SH instead of N-CBZ-CA in Step I
37) As for compound 36 except that HSCH₂CH₂CN is used instead of CH₃SH in Step I
38) As for compound 36 except that is 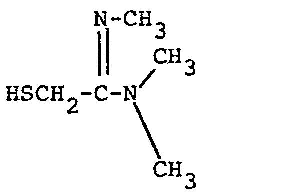 used instead of CH₃SH and the product of Step I is not isolated.
39) Starting with compound 8 of Table 1 and carrying it through Steps I and J of Example 1 but using -S-CH₂CH₃ instead of N-CBZ-CA in Step I.
40) As for compound 39 except that HSCH₂CH₂OH is used instead of HSCH₂CH₃.
41) As for compound 39 exept that 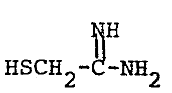 is used instead of HSCH₂CH₃ in Step I and the product of Step I is not isolated.
42) Starting with compound 9 of Table 1 and carrying it through Step I of Example 1 and deprotecting the allyl ester as described for compound 17 followed by Step J.
43) As for compound 42 except that CH₃CH₂SH is used instead of H-CBZ-CA in Step I; the product is isolated as the sodium salt. Step J is not used.
44) As for compound 43 except that 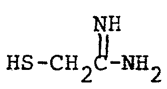 is used instead of N-CBZ-CA in Step I and the product of Step I is not isolated.
45) As for compound 43 except that CNCH₂CH₂SH is used instead of CH₃CH₂SH.
46) By treatment of compound 42 with 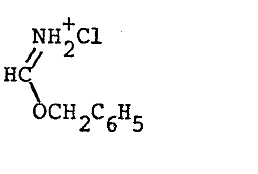 and NaOH at pH 8 in H₂0.
47) As for compound 44 except that 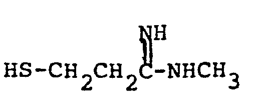 is used instead of 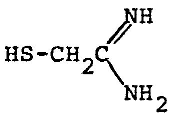 in Step I.
48) Starting with compound 10 of Table 1 and carrying out Step I of Example 1. The allyl ester is removed as described for compound 19 followed by Step J of Example 1.
49) Starting with compound 10 of Table 1 and carrying out Step I of Example 1, using 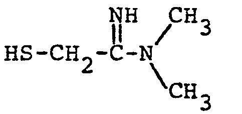 instead of N-CBZ-CA, followed by Step J, the intermediate product of Step I not being isolated.
50) As for compound 49 using HS-CH₂CH₂CN instead of 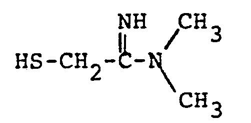 in Step I.
51) Starting with compound 11 of Table I and following the reactions described for compound 48.
52) Starting with compound 11 of Table 1 and following the reaction described for compound 49 but using 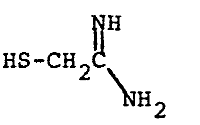 instead of 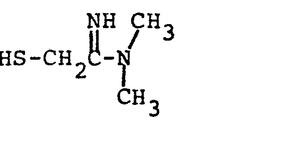
53) As for compound 52 using HS-CH₂CH₂OH instead of ] 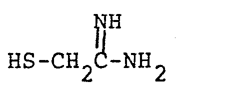
54) Startingwith compound 12 of Table 1 and carrying out the procedure described for compound 48.
55) Starting with compound 12 of Table 1 and carrying out the procedure described for compound 52.
56) Starting wth compound 12 of Table 1 and carrying out the procedure described for compound 52 except that HS-CH₂CH₃ is used instead of 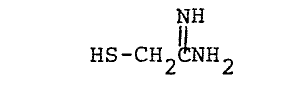
57) Starting with compound 13 of Table 1 and carrying it through Step I of Example 1 using HSCH₂CH₃ instead of N-CBZ-CA, then deblocking the allyl ester as described for compound 19.
58) Starting with compound 13 of Table 1 and carrying it through Step I of Example 1, deblocking the allyl ester as described for compound 19 and carrying it through Step J of Example 1 followed by formamidation as described for compound 7.
59) As described for compound 57 except that 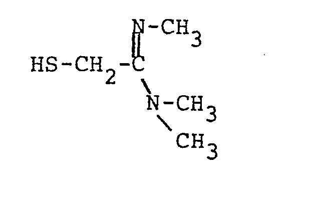
60) Starting with compound 14 of Table 1 and carrying out Step I of Example 1 but using HSCH₂CH₃ in place of N-CBZ-CA. The allyl ester is then deblocked as described for compound 19.
61) Starting with compound 14 of Table 1 and carrying out Step I of Example 1, deblocking the allyl ester as described for compound 19 and then carrying out STep J of Example 1.
62) Starting with compound 15 of Table 1 and proceding as described for compound 61.
63) Starting with compound 15 of Table 1 and proceding as described for compound 60.
64) Starting with compound 9 of Example 4 and carrying it through Steps H and I except that methylmercaptane is used instead of ethanethiol in Step H, Example 4.
65) Starting with compound 11 of Example 4 and carrying it through Step I using potassium bicarbonate in place of sodium bicarbonate.
66) As for compound 64 except tht butylmercaptane is used instead of methylmercaptane.
67) As for compound 64 except that 2-hydroxy 1-methylethylmercaptane is used instead of methylmercaptane.
68) As for compound 64, 2-cyanoethylthio is used.
69) Starting with compound 9 of Example 4 and treating it with lithium fluoride in DMF then carrying it through Step H and I except that 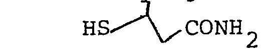 is used instead of ethanthiol in Step H and I, respectively.
70) Starting with compound 1 of Example 7 and carrying it through Step H and I of example 4 except that thiophenol is used instead of ethanthiol in Step H.
71) As for Example 64, except that fluorophenylthio is used instead of ethanethiol.
72) As for compound 64, except that p-methoxyphenyl- thio used instead of ethanethiol.
73) As for compounds 64 and 65, except that benzylthio is used instead of ethanethiol.
74) As for compound 64, except that 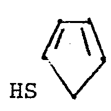 is used instead of ethanethiol.
75) As for compound 64, except that 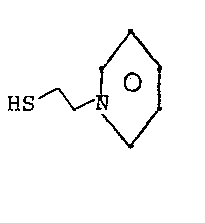 is used instead of ethanethiol in Step H and 0.1 M MOPS buffer is used instead of sodium bicarbonate in Step I, Example 4.
76-84) As for compound 75, except that the corresponding thiol is used instead of 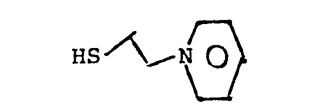
85-93) Starting with Compound 9 of Example 4 and treating it with lithium fluoride in DMF then carrying it through Steps H and I except that the corresponding mercaptane reagents are used instead of ethanethiol.
94) Starting wth compound 1 of Example 14 and carrying it through Steps A, B and C, except that methylmercaptane is used instead of cyanoethylthio in Step B, Example 14.
95) Starting with compound 94 and carried it through the procedure of Example 12.
96) As for compound 2 of Example 12, except that ethylacetimidate is used instead of ethyl formimidate.
97) As for compound 2 of Example 12, except that 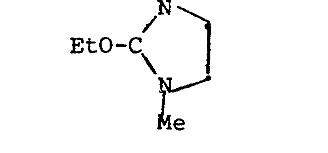 is used instead of ethyl formimidate.
98) Starting with compound 1 of Example 12 and trreating it with excessive methyliodide in DMF.
99) As for compound 98.
100) As for compound 98 except that isoprozliodide is used instead of methyliodide.
101) As for compound 2, Example 11, except that the 2-S analog is used as the starting material.
102) As for compound 2, Example 11, except that the 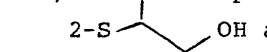 analog is used as the starting material.
103) As for compound 2, Example 11, except that the 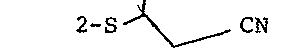 analog is used as the starting material which is acylated with 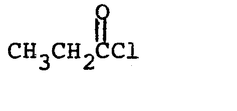
104) As for compound 103, except that 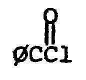 is used instead of 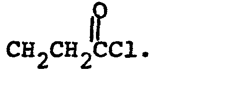
105) As for compound 2, Example 11, except that 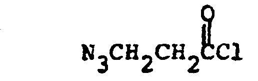 is used instead of acetic anhydride and followed by hydrogenation of azido compound.
106) As for compound 105 except that 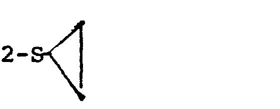 analog is acylated with 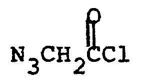 instead of 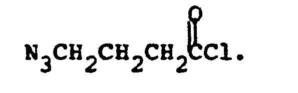
107) Compound 106 is treated iwth etyl formamidate as in Example 12.
108) As for compound 105 except that 1-amino-2-sø analog is treated with 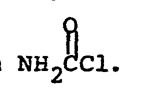
109) Starting with compound 4, Example 13 and treating it with acetic anhydride/formic acid.
110) Starting with compound 4, Example 13 and carrying it through the procedure of Example 12.
111) As for compound 110 except that ethyl acetimidate is used instead of ethyl formimidate.
112) Starting with compound 2 of Example 13 and carrying it through Steps B and C except that 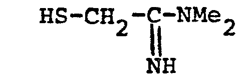 is used instead of HS-(CH₂)₂-NHCO₂PNB.
113-118 As for compound 112 except that the corresponding mercapto reagents are used instead of I 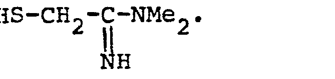
119 Starting with compound 1, Example 12, and treating it with phenylaldehyde.
120) Starting with compound 4, Example 14, and treating it with ethyl acetimidate.
121) As for compound 120, except that thₗ 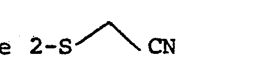 analog is used as starting material and ethylformimidate is used instead of ethylacetimidate.
122) As for compound 120, except that the 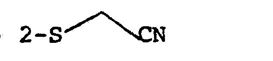 analog is used as starting material and is used instead of ethylacetimide. 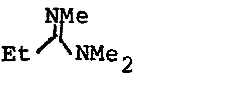
123) As for compound 120 except that the 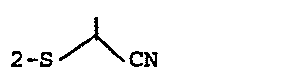 analog is used as starting material.
124) As for compound 4, Example 14, except 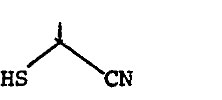 is used instead of 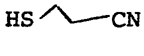 in STep B.
125-133) As for compound 124 except that the corresopnding thiol rea^{q}ents are used instead of 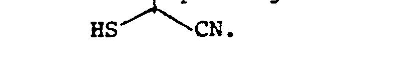
134) As for compound 4, Example 13, except that 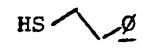 is used instead of 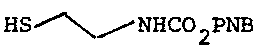 in Step B, Example 13.
16a) As for compound 1, except that 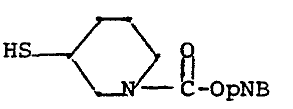 is used instead of N-CBZ-CA and the final product is formimidated as for compound 7.
16b) As for compound 1, except that 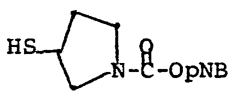 is used instead of N-CBZ-CA and the final product is treated with 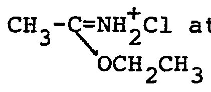 at pH 8.5 as for compound 7.
16c) As for compound 8, except that 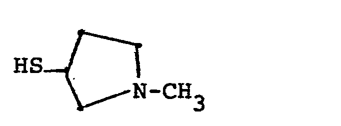 is used instead of CH₃CH₂SH and the product of Step 1 is not isolated.
16d) As described in Example 15b.
50c) Starting with compound 7 of Table 1 and taking it through Step C of Example 15b and deprotecting the allyl ester as described for compound 17 followed by Step D of Example 15b followed by acetamidination using 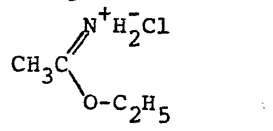
53a) As for compound 51 using 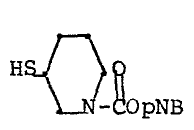 instead of N-CBZ-CA. The product is acetamidinated using 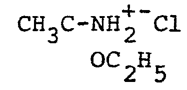
59a) As for compound 58 usinc 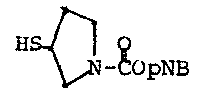 instead of N-CBZ-CA.
85a) as in Example 14h.
85b) As in Example 14f but using 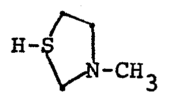 as the mercaptan.
85c) As in Example 14f, using 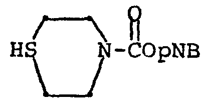 as the mercaptan and treating the final product as in Example 14g.
27b) As for compound 23 but substituting 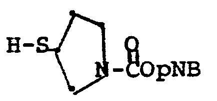 for NCNZ-CA and acetamidating the final product as described for compound 16b.
27c) Starting with compound 4 of Table 1 and carrying it through Steps C and D of Example 15b.
32a) As for compound 28 except that 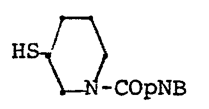 is used instead of 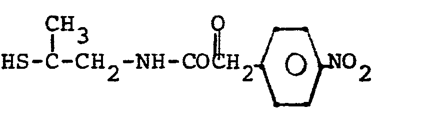
32b) As for compound 28 except that 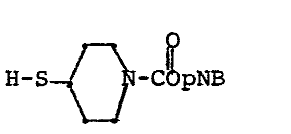 is used instead of 1 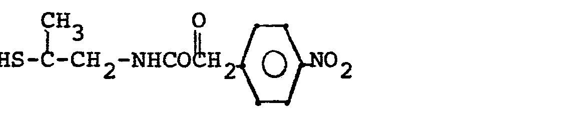 and the final product is formimidated as in Example 7.
32c) As for compound 28 except that 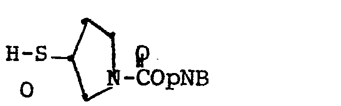 is used instead of 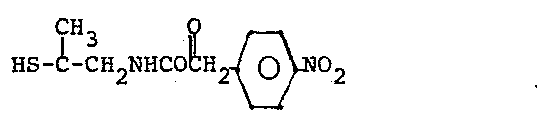 and the final product is formimidated as in Example 7.
32d) As for compound 28 except that 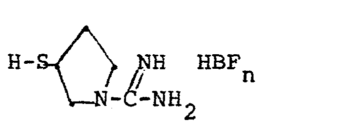 is used instead of 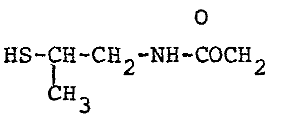 NO₂ and the product of Step I is not isolated.
32e) Starting with compound 5 of Table 1 and carrying it through Steps C & D of Example 15b, but using 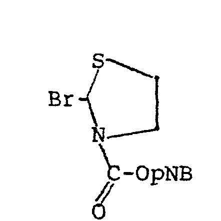 instead of ] 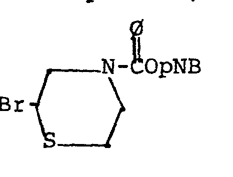 in Step C followed by acetamidination using 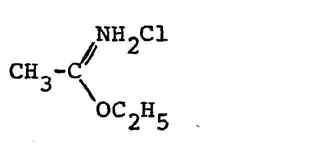
32f) Starting with compound 5 of Table 1 and carrying it through Steps C & D of Example 15b.
35a) As for compound 33 except that 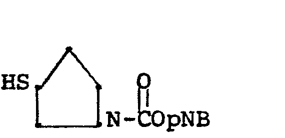 is used instead of N-CBZ-CA in Step I of Example 1 and the final product is formamidinated as for compound 7.
35b) As for 35a using 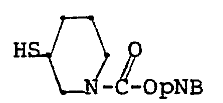 instead of 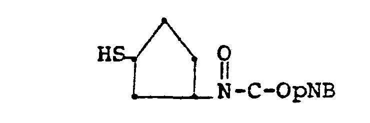
38a) As for compound 36, but using 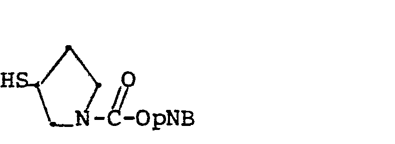 instead of N-CBZ-CA and formamidinating the final product as for compound 7.
50a) As for compound 42, using 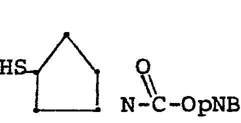 instead of N-CBZ-CA. The product is formamidinated as described for compound 7.
50b) As for compound 42, using 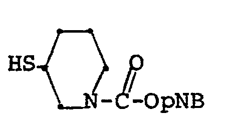 instead of N-CBZ-CA and the product is acetamidinated as for compound 7.
50c) Starting with compound 7 of Table 1 and taking it through Step C of Example 15b and deprotecting the allyl ester as described for compound 17 followed by Step D of Example 15b followed by acetamidination using 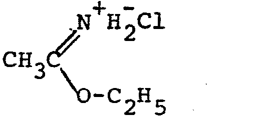
53a) As for compound 51 using 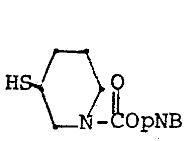 instead of N-CBZ-CA. The product is acetamidinated using 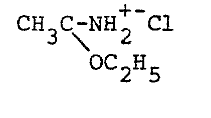
59a) As for compound 58 using 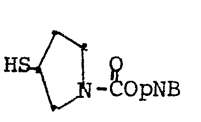 instead of N-CBZ-CA.
85a) as in Example 14h.
85b) As in Example 14f but using 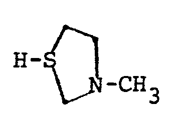 as the mercaptan.
85c) As in Example 14f, using 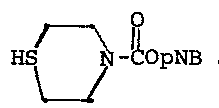 as the mercaptan and treating the final product as in Example 14g.
85d) Starting with compound 16 of Example 15 and going through the procedure of Example 14b, Steps C and D and formamidinating the final product.
85e) According to Example 14e, but starting with compound 16 of Example 15.
85f) According to Example 14f, but using 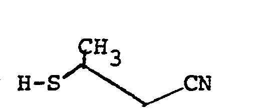 as the mercaptan.
93a) According to Example 14f starting with compound 20 of Table 1, and formimidating the final product.
93b) According to Example 14f, starting with compound 20 of Table 1 and using 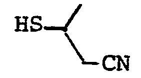 as the mercaptan.
134a) As in Example 14h.
134b) As in Example 14h but using 1 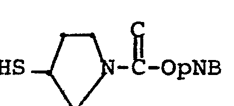 and acetamidinating the final product.
134c) As in Example 14h but using 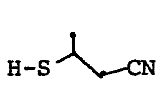 as the mercaptan.

### EXAMPLE 17

### Preparation of Pharmaceutical Compositions

One such unit dosage form is prepared by mixing 120 mg of the compound of Example 16, compound 78 with 20 mg of lactose and 5 mg of magnesium stearate and placing the 145 mg mixture into a No. 3 gelatin capsule. Similarly, by employing more of the active ingredient and less lactose, other dosage forms can be put up in No. 3 gelatin capsules, and, should it be necessary to make more than 145 mg of ingredients together, larger capsules such as compressed tablets and pills can be prepared. The following examples are illustrative of the preparation of pharmaceutical formulations:

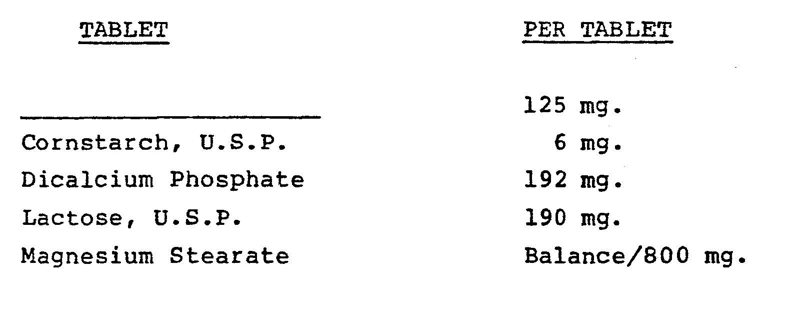

The active ingredient is blended with the dicalcium phosphate, lactose and about half of the cornstarch. The mixture is then granulated with 15% cornstarch paste (16 mg) and rough-screened. It is dried at 45°C and screened again through No. 16 screens. The balance of the cornstarch and magnesium stearate is added and the mixture is compressed into tablets, approximately 0.5 inch in diameter each weighing 800 mg.

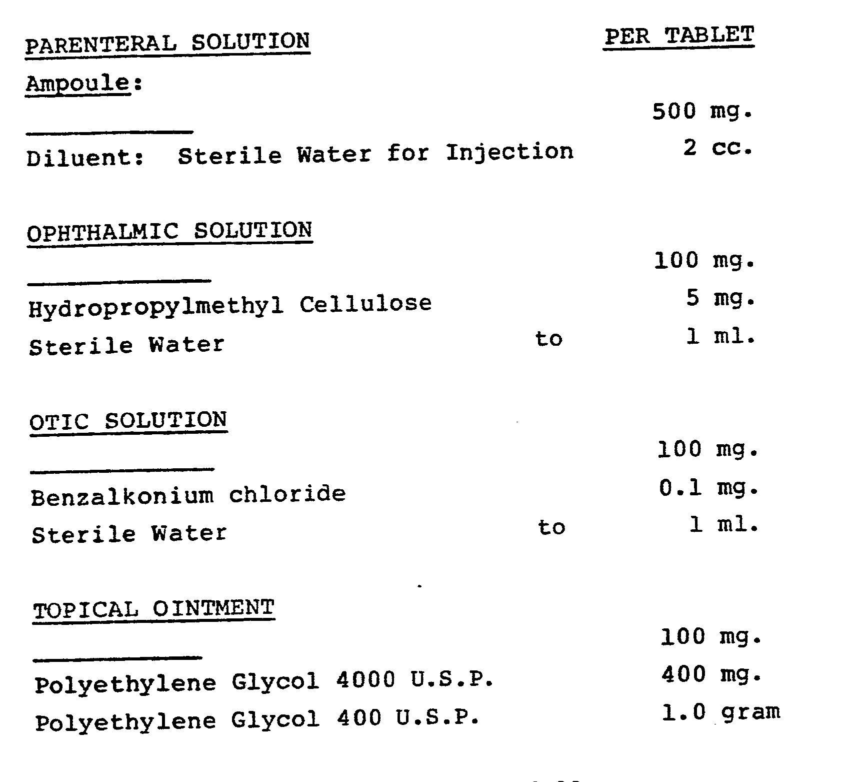

## Claims

1. A compound having the structure: 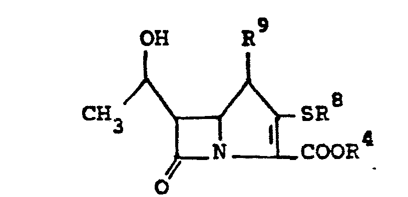 wherein:
R⁴ is H; a pharmaceutically acceptable cation or a pharmaceutically acceptable ester moiety,
R⁹ is halogen. OR, OSO₂R, 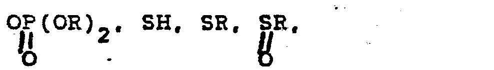 SO₂R, NH₂, NHR, N(R)₂, N⊕(R)₃, 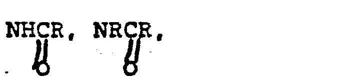 wherein R is hydrogen, C₁₋₆ alkyl, C₂₋₆ alkenyl, C2-6 alkynyl: phenyl: phenylalkyl having 7-12 carbon atoms: cycloalkyl having 3-6 carbon atoms; cycloalkylalkyl having 3-6 carbon atoms in the ring and 1-6 carbon atoms in the alkyl moiety; heterocyclic groups including heterocyclyl, heterocyclyalkyl, heteroaryl, and heteroarylalkyl:
wherein, relative to said heterocyclic group rihgs, the rings comprise 3-6 atoms, 1-4 of which are selected from O, N, or S, and the alkyl moiety comprises 1-6 carbon atoms; representative examples of such heterocyclic values for R include: 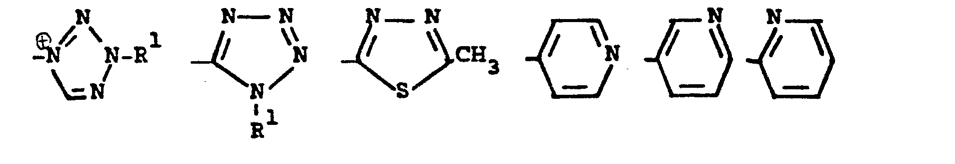 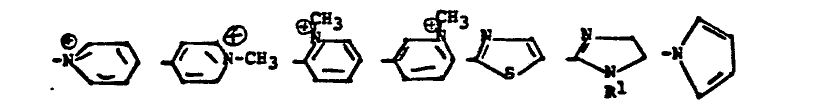 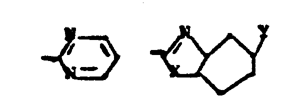 (X-N, O, S; Y-H, Cl, OCH₂CH₃, CO₂R) 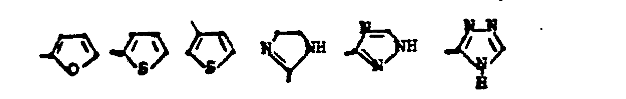 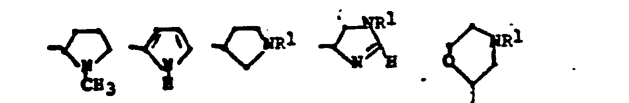 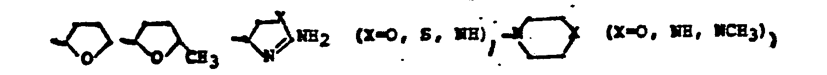 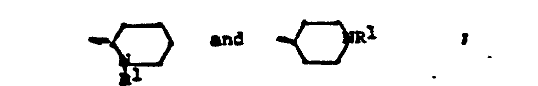 the foregoing acyclic and ring structure values for R may be subatituted by one or more members selected from: Cl, Br, F, OH, OR¹, 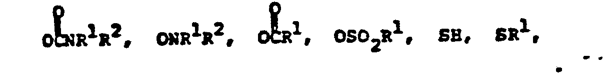 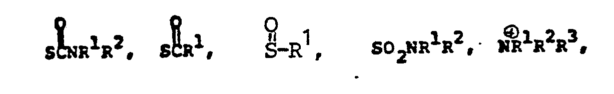 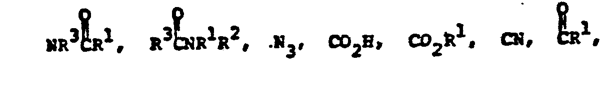 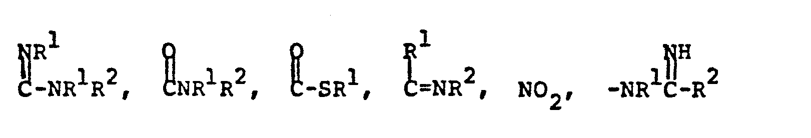 and 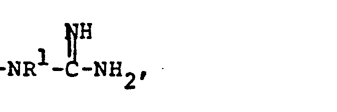
wherein the R¹, R² and R³ are independently selected from: hydrogen, C₁₋₁₀ alkyl, C₂₋₁₀ alkenyl, and C₂₋₁₀ alkynyl, having cycloalky, cycloalkylalkyl, and alkylcycloalkyl, having 3-6 carbon atoms in the cycloalkyl ring and 1-6 carbon atoms in the alkyl moieties; aryl, such as phenyl; aralkyl, aralkenyl, and aralkynyl wherein the aryl moiety is phenyl and the aliphatic portion has 1-6 carbon atoms; heterocyclic groups including heteroalkyl, heteroaralkyl, heterocyclyl and heterocyclylalkyl and wherein the hetero atom or atoms in the above-named heterocyclic moieties are selected from the group consisting of 1-4 oxygen, nitrogen or sulphur atoms and wherein the alkyl moieties associated with said heterocyclic moieties have 1-6 carbon atoms; and
R⁸ is independently selected-from the group consisting of hydrogen, substituted and unsubstituted: alkyl, alkenyl, and alkynyl, having ::rom 1-10 carbon atoms; cycloalkyl, cycloalkylalkyl, and alkylcycloalkyl, having 3-6 carbon atoms in the cycloalkyl ring and 1-6 carbon atoms in the alkyl noieties; aryl, such as phenyl; aralkyl, aralkenyl, end aralkynyl wherein the aryl moiety is phenyl and the aliphatic portion has 1-6 carbon atoms; a heterocyclic group as defined in R⁹ above and the group 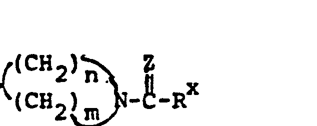
wherein n is 1-4 and m is 1-4, Z is NH, NR¹, 0 or S and R^{x} is H, or substituted or unsubstituted
Cₗ₋₆ alkyl, C₃₋₆ cycloalkyl, C₂₋₆ alkenyl,
C₂₋₆ alkynyl or C₆₋₁₀ non-heterocyclic aryl; or carbamimidoyl; wherein the substitutent or
substituents relative to the above-named radical values for R⁸ are selected from the group consisting of: 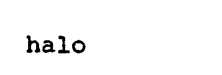 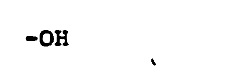 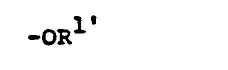 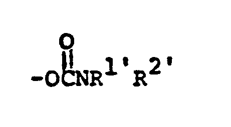 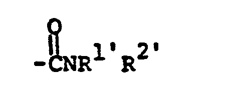 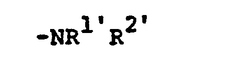 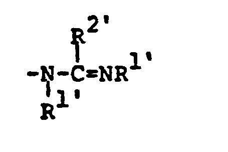 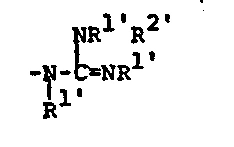 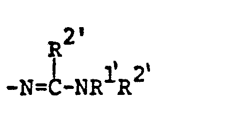 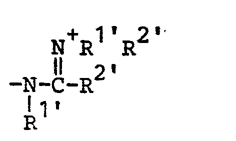 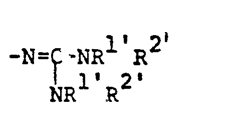 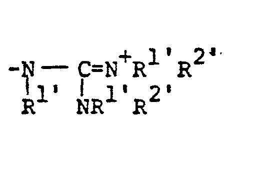 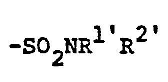
sulfamoyl 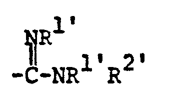 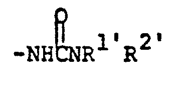 ureido 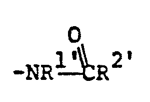 amido 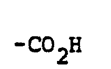 carboxy 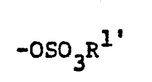 sulphate 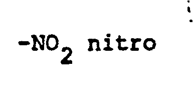 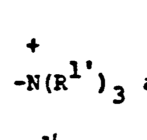 ammonium (R ^{1'} groups independently chosen 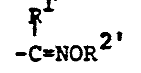 oximino 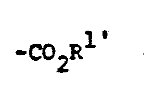 carboxylate 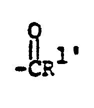 acyl 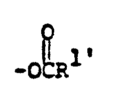 acyloxy 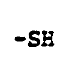 mercapto 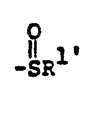 alkyl and aryl sulfinyl 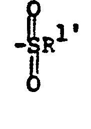 alkyl and aryl sulfonyl 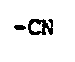 cyano 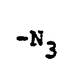 azido 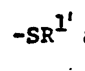 alkyl- and arylthio 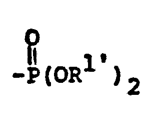 phosphono 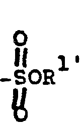 sulfo 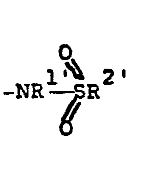 sulfonamido wherein, the groups R^{1'} and R^{2'} are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; aryl; phenyl; aralkyl, aralkenyl, and aralkynyl wherein the aryl moiety is phenyl and the aliphatic portion has 1-6 carbon atoms; heterocyclic groups as defined above, including heteroalkyl, heteroaralkyl, heterocyclyl and heterocyclylalkyl and wherein the hetero atom or atoms in the above-named heterocyclic moieties are selected from the group consisting of 1-4 oxygen. nitrogen or sulphur atoms and wherein the alkyl moieties associated with said heterocyclic moieties have 1-6 carbon atoms..

2. A compound of Claim 1 wherein:
R⁹ is halogen; and
R is substituted or unsubstituted C₁₋₆ alkyl. heteroaryl, heteroalkyl, carbamimidoyl or 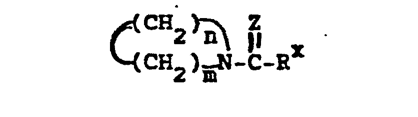 3. A compound of Claim 2 wherein R⁸ is selected from 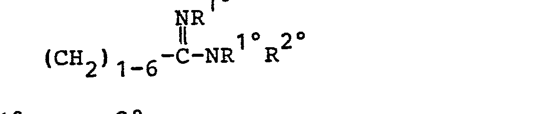 wherein R^{1°} and R^{2°} are independently selected from hydrogen, alkyl, alkenyl, alkynyl, aryl, cycloalkyl, cycloalkenyl, heteroaryl, heterocyclyl, heteroarylalkyl, alkylaryl, alkylarylalkyl.
4. A compound of Claim 2 wherein R is 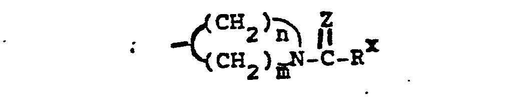
5. A compound of Claim 2 wherein R⁹ is chloro or fluoro.
6. A compound of Claim ₅ wherein R⁸ is substituted or unsubstituted C₁₋₆ alkyl. carbamimidoyl or, 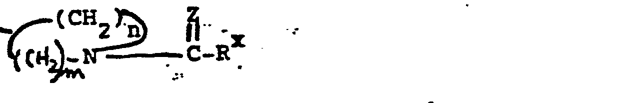
7. A compound of Claim 6 wherein R⁴ is H and R⁸ is: 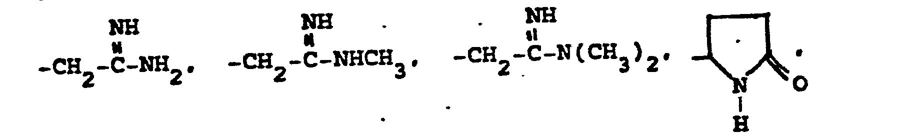 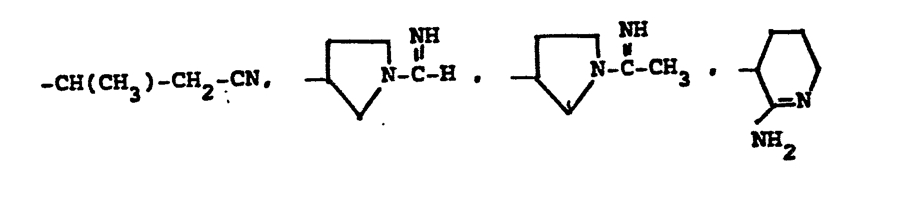 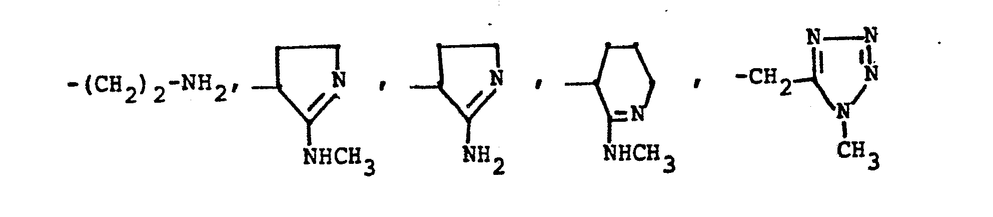 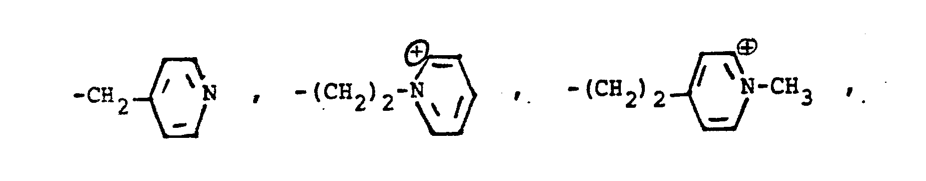 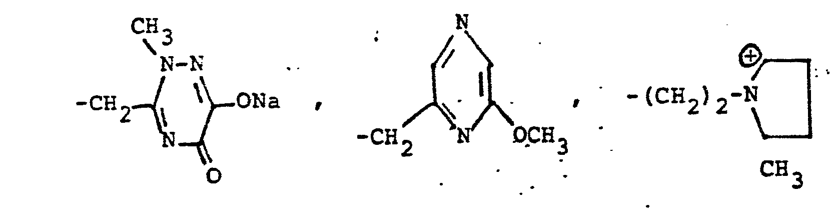 or 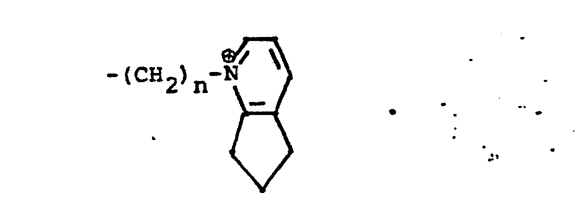
8. An antibiotic composition containing a compound of Claim 1.
9. An antibiotic composition containing a compound of Claim 1 and a dehydropeptidase inhibitor.
